# EUROPEAN PATENT SPECIFICATION

(11) **EP 0 625 164 B1**
(45) Date of publication and mention of the grant of the patent: **07.04.1999**
(21) Application number: 92921273.6
(22) Date of filing: 30.09.1992
(51) Int. Cl.: C07K 7/56, A61K 38/12

(54) **CYCLIC COMPOUNDS USEFUL AS INHIBITORS OF PLATELET GLYCOPROTEIN IIb/IIIa**
ZYKLISCHE VERBINDUNGEN ZUR VERWENDUNG ALS INHIBITOREN DES GLYCOPROTEIN IIB/IIIA
COMPOSES CYCLIQUES UTILES COMME INHIBITEURS DU COMPLEXE GLYCOPROTEIQUE PLAQUETTAIRE IIb/IIIa

(30) Priority: 30.09.1991 US 767848; 09.09.1992 US 949285
(43) Date of publication of application: 23.11.1994
(73) Proprietor: THE DU PONT MERCK PHARMACEUTICAL COMPANY, Wilmington, Delaware 19898 (US)
(72) Inventor: DEGRADO, William, Frank, Moylan, PA 19065 (US); JACKSON, Sharon, Anne, Wilmington, DE 19809 (US); MOUSA, Shaker, Ahmed, Lincoln University, PA 19352 (US); PARTHASARATHY, Anju, New Castle, DE 19720 (US); SWORIN, Michael, Newark, DE 19711 (US)
(74) Representative: von Kreisler, Alek, Dipl.-Chem.
(86) International application number: US9208144
(87) International publication number: WO9307170

(56) References cited:
- EP-A- 0 425 212
- EDITORS: SMITH ET AL 'PEPTIDES - Proceedings of the 12th American Peptide Symposium, June 1991, Cambridge, USA' 1992 , ESCOM , LEIDEN, HOLLAND
- EDITORS: SMITH ET AL 'PEPTIDES - Proceedings of the 12th American Peptide Symposium, June 1991, Cambridge, USA' 1992 , ESCOM , LEIDEN, HOLLAND
- EDITORS: GIRALT ET AL 'PEPTIDES 1990 - Proceedings of the 21th European Peptide Symposium, September 1990, Platja d'Aro, Spain' 1991 , ESCOM , LEIDEN, HOLLAND
- BIOCHEMICAL AND BIOPHYSICAL RESEARCH COMMUNICATIONS vol. 177, no. 1, May 1991, DULUTH, MINNESOTA, USA pages 74 - 82 KUMAGAI ET AL 'Effect of cyclic RGD peptide on cell adhesion and tumor metastasis'

## Description

### FIELD OF THE INVENTION

This invention relates to novel cyclic compounds containing carbocyclic ring systems useful as antagonists of the platelet glycoprotein IIb/IIIa complex, to pharmaceutical compositions containing such cyclic compounds, and to the use of these compounds for preparing a medicament for the inhibition of platelet aggregation.

### BACKGROUND OF THE INVENTION

Activation of platelets and the resulting platelet aggregation and secretion of factors by the platelets have been associated with different pathophysiological conditions including cardiovascular and cerebrovascular thromboembolic disorders, for example, the thromboembolic disorders associated with unstable angina, myocardial infarction, transient ischemic attack, stroke, atherosclerosis and diabetes. The contribution of platelets to these disease processes stems from their ability to form aggregates, or platelet thrombi, especially in the arterial wall following injury.

Platelets are known to play an essential role in the maintenance of hemostasis and in the pathogenesis of arterial thrombosis. Platelet activation has been shown to be enhanced during coronary thrombolysis which can lead to delayed reperfusion and reocclusion. Clinical studies with aspirin, ticlopidine and a monoclonal antibody for platelet glycoprotein IIb/IIIa provide biochemical evidence for platelet involvement in unstable angina, early stage of acute myocardial infarction, transient ischemic attack, cerebral ischemia, and stroke.

Platelets are activated by a wide variety of agonists resulting in platelet shape change, secretion of granular contents and aggregation. Aggregation of platelets serves to further focus clot formation by concentrating activated clotting factors in one site. Several endogenous agonists including adenosine diphosphate (ADP), serotonin, arachidonic acid, thrombin, and collagen, have been identified. Because of the involvement of several endogenous agonists in activating platelet function and aggregation, an inhibitor which acts against all agonists would represent a more efficacious antiplatelet agent than currently available antiplatelet drugs, which are agonist-specific.

Current antiplatelet drugs are effective against only one type of agonist; these include aspirin, which acts against arachidonic acid; ticlopidine, which acts against ADP; thromboxane A₂ synthetase inhibitors or receptor antagonists, which act against thromboxane A₂; and hirudin, which acts against thrombin.

Recently, a common pathway for all known agonists has been identified, namely platelet glycoprotein IIb/IIIa complex (GPIIb/IIIa), which is the membrane protein mediating platelet aggregation. A recent review of GPIIb/IIIa is provided by Phillips et all (1991) Cell 65: 359-362. The development of a GPIIb/IIIa antagonist represents a promising new approach for antiplatelet therapy. Recent studies in man with a monoclonal antibody for GPIIb/IIIa indicate the antithrombotic benefit of a GPIIb/IIIa antagonist.

There is presently a need for a GPIIb/IIIa-specific antiplatelet agent which inhibits the activation and aggregation of platelets in response to any agonist. Such an agent should represent a more efficacious antiplatelet therapy than the currently available agonist-specific platelet inhibitors.

GPIIb/IIIa does not bind soluble proteins on unstimulated platelets, but GPIIb/IIIa in activated platelets is known to bind four soluble adhesive proteins, namely fibrinogen, von Willebrand factor, fibronectin, and vitronectin. The binding of fibrinogen and von Willebrand factor to GPIIb/IIIa causes platelets to aggregate. The binding of fibrinogen is mediated in part by the Arg-Gly-Asp (RGD) recognition sequence which is common to the adhesive proteins that bind GPIIb/IIIa.

Several RGD-containing peptides and related compounds have been reported which block fibrinogen binding and prevent the formation of platelet thrombi. For example, see Cadroy et all (1989) J. Clin. Invest. 84: 939-944; Klein et all U.S. Patent No. 4,952,562; EP-A-0319506; EP-A-0422938; EP-A-0422937; EP-A-0341915;

WO 89/07609; WO 90/02751; WO 91/04247; and EP-A-0343085. Further, RGD-containing peptides which are cyclized via disulfide or carbon-carbon bonds (but not via amide bonds) are known from R.F. Nutt et al., in J.A. Smith et all (Eds.), Proceedings of the 12th American Peptide Symposium, 1991, pages 914-916, ESCOM, Leiden (NL), 1992 and EP-A-425212.

In the present invention we use conformationally-constraining carbocyclic ring systems as templates for cyclizing peptides such that they have high affinity and selectivity for GPIIb/IIIa.

### DETAILED DESCRIPTION OF THE INVENTION

This invention is directed to
(1) compounds of the formula (I): 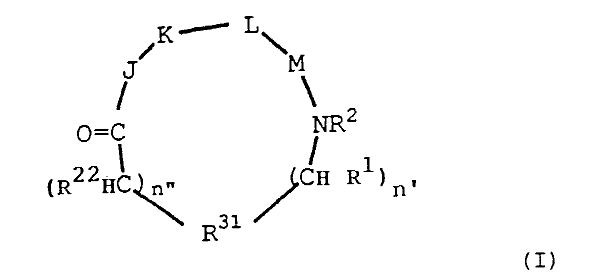 or a pharmaceutically acceptable salt or prodrug form thereof wherein:
   - R³¹: is
   (a) a 6 membered aromatic carbocyclic ring of the formula 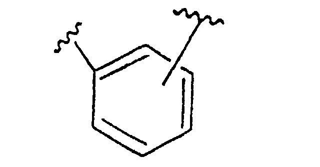 wherein said carbocyclic ring is substituted independently with 0-2 R¹⁰, or
   (b) a 10 membered bicyclic carboxylic ring of the formula 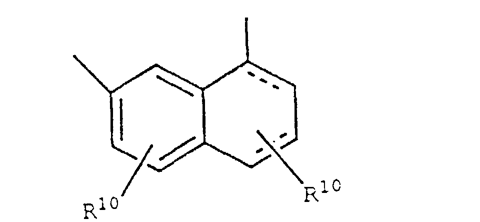 wherein the dashed bond may be a single or double bond;
      n" is 0 or 1;
      n' is 0 or 1;
   - R¹ and R²²: are independently selected from the following groups:
   hydrogen,
      C₁-C₈ alkyl substituted with 0-2 R¹¹,
      C₂-C₈ alkenyl substituted with 0-2 R¹¹,
      C₂-C₈ alkynyl substituted with 0-2 R¹¹,
      C₃-C₈ cycloalkyl substituted with 0-2 R¹¹,
      C₆-C₁₀ bicycloalkyl substituted with 0-2 R¹¹,
   aryl substituted with 0-2 R¹²,
   a heterocylic ring system substituted with 0-2 R¹²_{,} composed of 5-10 atoms including 1-3 N, S, or O heteroatoms, =O, F, Cl, Br, I, -CN, -CO₂R¹³, -C(=O)R¹³, -C(=O)NR¹³R¹⁴, -CHO, -CH₂OR¹³, -OC(=O)R¹³, -OC(=O)OR¹³, -OR¹³, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -SR¹³, -S(=O)R^{13a}, -SO₂NR¹³R¹⁴, -CH₂NR¹³R¹⁴, -NR¹³R¹⁴, -NHC(=NH)NHR¹³, -C(=NH)NHR¹³,NO₂;
   - R¹¹: is selected from one or more of the following:
   =O, F, Cl, Br, I, -CF₃, -CN, -CO₂R¹³, -C(=O)R¹³, -C(=O)NR¹³R¹⁴, -CH₂OR¹³, -OC(=O)R¹³, -OC(=O)OR¹³, -OR¹³, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -SR¹³, -S(=O)R^{13a},
   -SO₂NR¹³R¹⁴, -CH₂NR¹³R¹⁴, -NR¹³R¹⁴, -NHC(=NH)NHR¹³, -C(=NH)NHR¹³, =NOR¹⁴, NO₂, C₁-C₅ alkyl, C₂-C₄ alkenyl, C₃-C₆ cycloalkyl, C₃-C₆ cycloalkylmethyl, C₂-C₆ alkoxyalkyl, C₁-C₄ alkyl (substituted with -NR¹³R¹⁴, -CF₃, NO₂, -SO₂R¹³, or -S(=O)R^{13a})
   aryl substituted with 0-2 R¹²,
   a heterocylic ring system substituted with 0-2 R¹², composed of 5-10 atoms including 1-3 nitrogen, oxygen, or sulfur heteroatoms;
   - R¹²: is selected from one or more of the following:
   phenyl, benzyl, phenethyl, phenoxy, benzyloxy, halogen, hydroxy, nitro, cyano, C₁-C₅ alkyl, C₃-C₆ cycloalkyl, C₃-C₆ cycloalkylmethyl, C₇-C₁₀ arylalkyl, C₁-C₄ alkoxyl -CO₂R¹³, hydroxamic acid, hydrazide, oxime, boronic acid, sulfonamide, formyl, C₃-C₆ cycloalkoxy, -OC(=O)R¹³, -C(=O)R¹³,-OC(=O)OR¹³, -OR¹³, -CH₂OR¹³, -NR¹³R¹⁴, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -S(=O)R^{13a}, -SR¹³, -SO₂NR¹³R¹⁴, C₂-C₆ alkoxyalkyl, C₁-C₄ hydroxyalkyl, methylenedioxy, ethylenedioxy,
   - R¹³: is H, C₁-C₇ alkyl, aryl, -(C₁-C₆ alkyl)aryl, or C₃-C₆ alkoxyalkyl;
   - R^{13a}: is C₁-C₇ alkyl, aryl, -(C₁-C₆ alkyl)aryl, or C₃-C₆ alkoxyalkyl;
   - R¹⁴: is OH, H, C₁-C₄ alkyl, or benzyl;
   - R²: is H or C₁-C₈ alkyl;
   - R¹⁰: is selected from one or more of the following:
   phenyl, benzyl, phenethyl, phenoxy, benzyloxy, halogen, hydroxy, nitro, cyano, C₁-C₅ alkyl, C₃-C₆ cycloalkyl, C₃-C₆ cycloalkylmethyl, C₇-C₁₀ arylalkyl, C₁-C₄ alkoxyl -CO₂R¹³, -C(=O)NHOR¹³, -C(=O)NHNR¹³R¹⁴, oxime, boronic acid, C₃-C₆ cycloalkoxy, -OC(=O)R¹³, -C(=O)R¹³,-OC(=O)OR¹³, -OR¹³, -CH₂OR¹³, -NR¹³R¹⁴, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴ -NR¹⁴SO₂R^{13a}, -SO₃H, -SO₂R^{13a}, -S(=O)R^{13a}, -SR¹³, -SO₂NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -OCH₂CO₂H, 2-(1-morpholino)ethoxy, C₁-C₄ alkyl (substituted with -NR¹³R¹⁴, -CF₃, NO₂, or -S(=O)R^{13a});
   - J: is β-Ala or an _{L}-isomer or _{D}-isomer amino acid of structure -N(R³)C(R⁴) (R⁵)C(=O)-, wherein:
   R³ is H or CH₃
   R⁴ is H or C₁-C₃ alkyl;
   R⁵ is H, C₁-C₈ alkyl, C₃-C₆ cycloalkyl, C₃-C₆ cycloalkylmethyl, C₁-C₆ cycloalkylethyl, phenyl, phenylmethyl, CH₂OH, CH₂SH, CH₂OCH₃, CH₂SCH₃, CH₂CH₂SCH₃, (CH₂)ₛNH₂, (CH₂)ₛNHC(=NH) (NH₂), (CH₂)ₛNHR¹⁶, where s = 3-5;
   R³ and R⁵ can also be taken together to form -(CH₂)ₜ- (t = 2-4) or -CH₂SC(CH₃)₂-; or
   R⁴ and R⁵ can also be taken together to form (CH₂)ᵤ-, where u = 2-5;
   R¹⁶ is an amino protecting group;
   - K: is a _{D}-isomer or _{L}-isomer amino acid of structure -N(R⁶)CH(R⁷)C(=O)-, wherein:
   R⁶ is C₁-C₈ alkyl;
   R⁷ is selected from:
   -(C₁-C₇ alkyl)X; 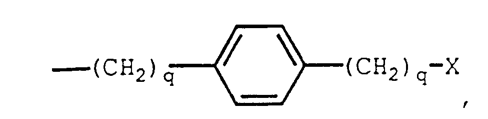 where q is independently 0,1; 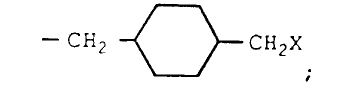 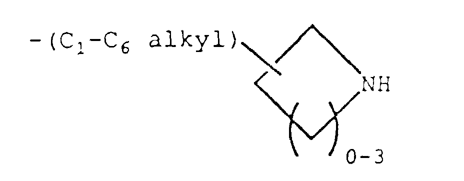 -(CH₂)ₘO-(C₁-C₄ alkyl)-X, where m = 1,2;
   -(CH₂)ₘS-(C₁-C₄ alkyl)-X, where m = 1,2; and
   X is selected from: 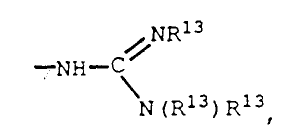 -N(R¹³)R¹³, -C(=NH)(NH₂)*,* -SC(NH)-NH₂;
   - L: is -Y(CH₂)ᵥC(=O)-, wherein:
   Y is NH, N(C₁-C₃ alkyl), O, or S; and v = 1,2;
   - M: is a _{D}-isomer or _{L}-isomer amino acid of structure -NR¹⁷-CH(R⁸)C(=O)-, wherein:
   R¹⁷ is H, C₁-C₃ alkyl;
   R⁸ is -CH₂CO₂R¹³, CH₂SO₃R^{13a}, -CH(CH₃)CO₂R¹³, -SO₂NR¹³R¹⁴, -CH₂-boronic acid, or -CH₂-tetrazole.

   This invention is further directed to
(2) compounds of the formula: 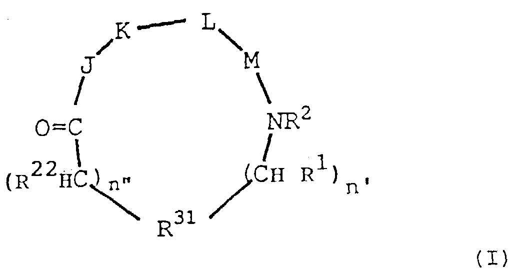 or a pharmaceutically acceptable salt or prodrug form thereof wherein:
   - R³¹: is selected from: 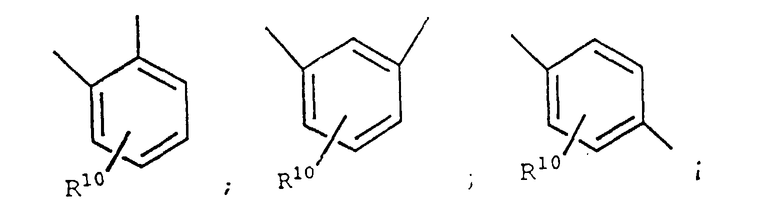 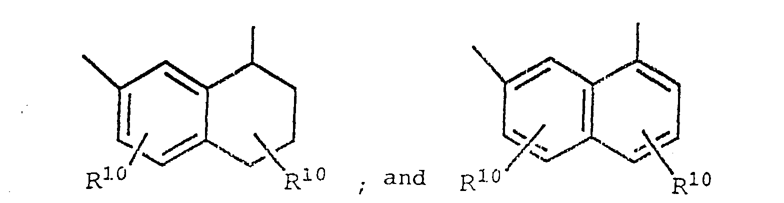 wherein R³¹ may be substituted independently with 0-2 R¹⁰;
   n" is 0 or 1;
   n' is 0 or 1;
   R¹ and R²² are independently selected from H, C₁-C₄ alkyl, phenyl, benzyl, phenyl-(C₂-C₄)alkyl, C₁-C₄ alkoxy;
   R² is H Or C₁-C₈ alkyl;
   R¹⁰ is H, C₁-C₈ alkyl, phenyl, halogen, or C₁-C₄ alkoxy;

   - J: is β-Ala or an _{L}-isomer or _{D}-isomer amino acid of structure -N(R³)C(R⁴) (R⁵)C(=O)-, wherein:
   R³ is H or CH₃
   R⁴ is H or C₁-C₃ alkyl;
   R⁵ is H, C₁-C₈ alkyl, C₃-C₆ cycloalkyl, C₃-C₆ cycloalkylmethyl, C₁-C₆ cycloalkylethyl, phenyl, phenylmethyl, CH₂OH, CH₂SH, CH₂OCH₃, CH₂SCH₃, CH₂CH₂SCH₃, (CH2)ₛNH₂, (CH₂)ₛNHC(=NH) (NH₂), (CH₂)ₛNHR¹⁶, where s = 3-5;
   R³ and R⁵ can also be taken together to form -(CH₂)ₜ- (t = 2-4) or -CH₂SC(CH₃)₂-; or
   R⁴ and R⁵ can also be taken together to form -(CH₂)ᵤ-, where u = 2-5;
   R¹⁶ is an amino protecting group;
   - K: is an _{L}-isomer amino acid of structure -N(R⁶)CH(R⁷)C(=O)-, wherein:
   R⁶ is C₁-C₈ alkyl;
   R⁷ is -(CH₂)ₚNHC(=NH) (NH₂), where p = 3-5; 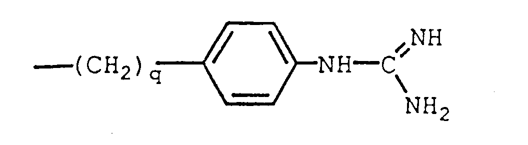 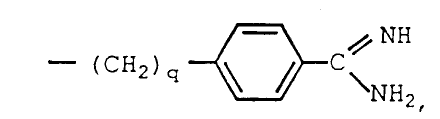 where q = 0,1;
   -(CH₂)ᵣX,where r = 4-6; 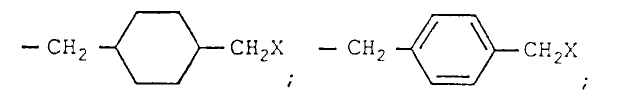 -(CH₂)ₘS(CH₂)₂X, where m = 1,2;
   -(C₃-C₇ alkyl)-N-(C₁-C₆ alkyl) 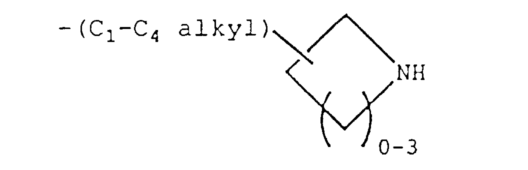 -(CH₂)ₘ-O-(C₁-C₄alkyl)-NH-(C₁-C₆ alkyl), where m = 1,2;
   -(CH₂)ₘ-S-(C₁-C₄ alkyl)-NH-(C₁-C₆ alkyl), where m =1,2; and
   X is -NH₂ or -NHC(=NH)(NH₂);
   - L: is -Y(CH₂)ᵥC(=O)-, wherein:
   Y is NH, O, or S; and v = 1,2;
   - M: is an _{L}-isomer amino acid of structure -NH-CH (CH₂SO₃H)C(=O)-.

   Preferred compounds (1) of the present invention include compounds of Formula (I), or a pharmaceutically acceptable salt or prodrug form thereof wherein:
   - R³¹: is selected from: 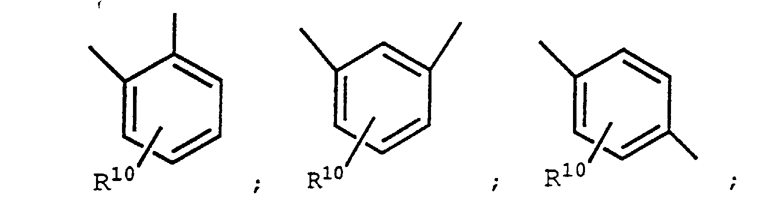 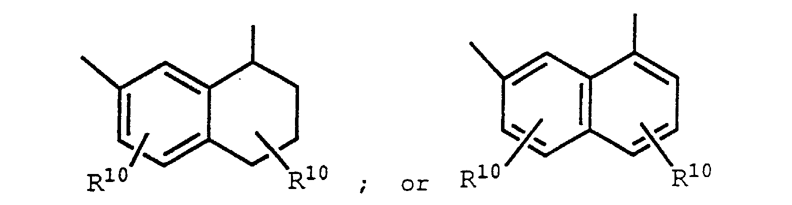 wherein R³¹ may be substituted independently with 0-2 R¹⁰;
   - R¹ and R²²: are independently selected from H, C₁-C₄ alkyl, phenyl benzyl, phenyl-(C₂-C₄)alkyl, C₁-C₄ alkoxy;
   - R¹⁰: is H, C₁-C₈ alkyl, phenyl, halogen, or C₁-C₄ alkoxy;
   - K: is an _{L}-isomer amino acid of structure -N(R⁶)CH(R⁷)C(=O)-, wherein:
   R⁶ is C₁-C₈ alkyl;
   R⁷ is -(CH₂)ₚNHC(=NH) (NH₂), where p = 3-5; 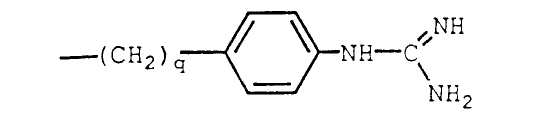 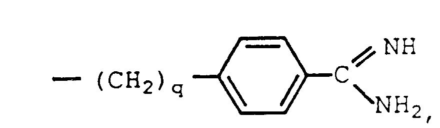 where q = 0,1;
   -(CH₂)ᵣX,where r = 4-6; 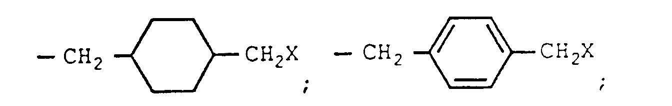 -(CH₂)ₘS(CH₂)₂X, where m = 1,2;
   -(C₃-C₇ alkyl)-N-(C₁-C₆ alkyl) 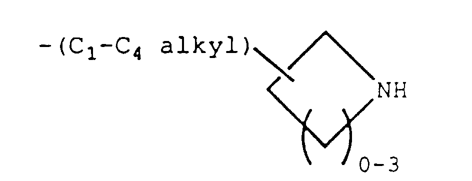 -(CH₂)ₘ-O-(C₁-C₄ alkyl)-NH-(C₁-C₆ alkyl), where m = 1,2;
   -(CH₂)ₘ-S-(C₁-C₄ alkyl)-NH-(C₁-C₆ alkyl), where m = 1,2; and
   X is -NH₂ or -NHC(=NH)(NH₂); or
   - L: is -Y(CH₂)ᵥC(=O)-, wherein:
   Y is NH, O, or S; and v = 1,2;
   - M: is an _{L}-isomer amino acid of structure -NH-CH(R⁸)C(=O)-, wherein:
   R⁸ is -CH₂CO₂H, or -CH(CH₃)CO₂H.

   Preferred compounds (1) and (2) of the invention are 1,3-disubstituted phenyl compounds of the formula (II): 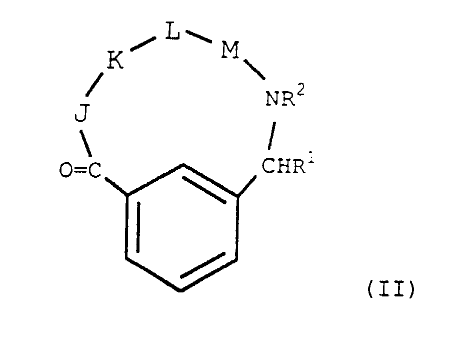 wherein:
   - R¹: is H, C₁-C₄ alkyl, phenyl, benzyl, or phenyl-(C₂-C₄)alkyl;
   - R²: is H or methyl;
   - J: is β-Ala or an _{L}-isomer or _{D}-isomer amino acid of structure -N(R³)C(R⁴)(R⁵)C(=O)-, wherein:
   R³ is H or CH₃;
   R⁴ is H or C₁-C₃ alkyl;
   R⁵ is H, C₁-C₈ alkyl, C₃-C₆ cycloalkyl, C₃-C₆ cycloalkylmethyl, C₁-C₆ cycloalkylethyl, phenyl, phenylmethyl, CH₂OH, CH₂SH, CH₂OCH₃, CH₂SCH₃, CH₂CH₂SCH₃, (CH₂)ₛNH₂ or (CH₂)ₛNHC(=NH)(NH₂), where s = 3-5; or
   R³ and R⁵ can be taken together to form -CH₂CH₂CH₂-; or
   R⁴ and R⁵ can be taken together to form -(CH₂)ᵤ-, where u = 2-5;
   - K: is an _{L}-isomer amino acid of structure -N(R⁶)CH(R⁷)C(=O)-, wherein:
   R⁶ is C₁-C₈ alkyl;
   R⁷ is -(CH₂)ₚNHC(=NH)(NH₂), where p = 3-5; 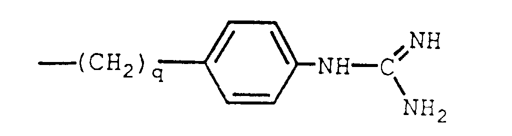 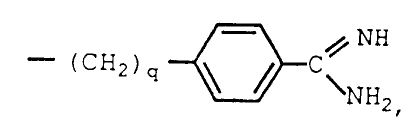 where q = 0,1;
   -(CH₂)ᵣX,where r = 4-6; 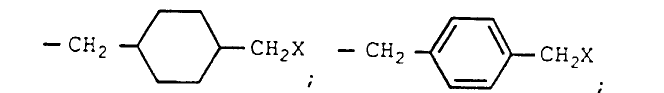 -(CH₂)ₘS(CH₂)₂X, where m = 1,2;
   -(CH₂)ₘ-O-(C₁-C₄ alkyl)-NH-(C₁-C₆ alkyl), where m = 1,2;
   -(CH₂)ₘ-S-(C₁-C₄ alkyl)-NH-(C₁-C₆ alkyl), where m = 1,2; and
   -(C₃-C₇ alkyl)-N-(C₁-C₆ alkyl) 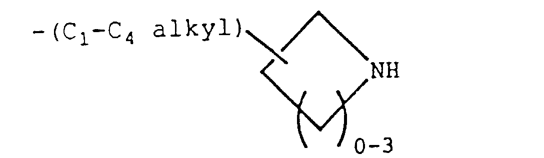 X is -NH₂ or -NHC(=NH)(NH₂),provided that X is not NH₂ when r = 4;
   - M: is an _{L}-isomer amino acid of structure -NH-CH(R⁸)C(=O)-, wherein:
   R⁸ is -CH₂CO₂H,-CH₂SO₃H, or -CH(CH₃)CO₂H.

   Further preferred compounds of the invention are 1,3-disubstituted phenyl compounds of the formula above, wherein
   - J: is β-Ala or an _{L}-isomer or _{D}-isomer amino acid of structure -N(R³)C(R⁴) (R⁵)C(=O)-, wherein:
   R³ is H or CH₃;
   R⁴ is H;
   R⁵ is H, C₁-C₈ alkyl, C₃-C₆ cycloalkyl, C₃-C₆ cycloalkylmethyl , C₁-C6₄ cycloalkylethyl, phenyl , phenylmethyl, CH₂OH, CH₂SH, CH₂OCH₃, CH₂SCH₃, CH₂CH₂SCH₃, (CH₂)ₛNH₂ or (CH₂)ₛNHC(=NH) (NH₂), where s = 3, 4, 5; or
   R³ and R⁵ can be taken together to form -CH₂CH₂CH₂-;
   - K: is an _{L}-isomer amino acid of structure
   -N(CH₃)CH (R⁷)C(=O), wherein :
   R⁷ is-(CH₂)ₚNHC(=NH)(NH₂), where p = 3-4 ; 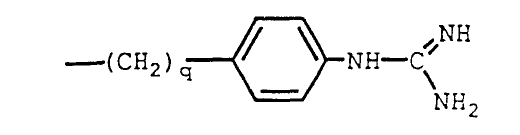 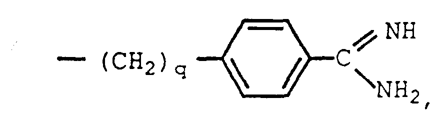 where q = 0,1;
   -(CH₂)ᵣX, where r = 4-6; 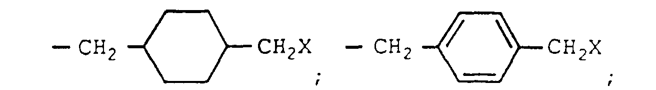 -(CH₂)ₘS(CH₂)₂X, where m = 1,2;
   -(C₄-C₇ alkyl)-N-(C₁-C₆ alkyl) 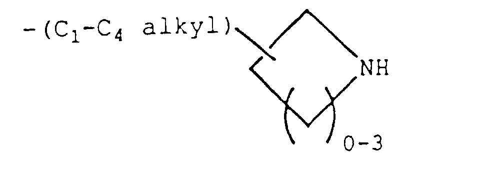 -(CH₂)ₘ-O-(C₁-C₄ alkyl)-NH-(C₁-C₆ alkyl), where m = 1,2;
   -(CH₂)ₘ-S-(C₁-C₄ alkyl)-NH-(C₁-C₆ alkyl), where m = 1,2; and
   X is -NH₂ or -NHC(=NH)(NH₂),provided that X is not -NH₂ when r = 4;
   - L: is -YCH₂C(=O)-, wherein:
   Y is NH or O;
   - M: is an _{L}-isomer amino acid of structure -NHCH(R⁸)C(=O)-, wherein:
   R⁸ is -CH₂CO₂H or -CH₂SO₃H.

   More preferred compounds of the present invention are compounds of formula (II) above, wherein:
   - R¹: is H;
   - R²: is H;
   - J: is β-Ala or an _{L}-isomer or _{D}-isomer amino acid of formula -N(R³)CH(R⁵)C(=O) -, wherein:
   R³ is H and R⁵ is H, CH₃, CH₂CH₃, CH(CH₃)₂, CH(CH₃)CH₂CH₃, CH₂CH₂CH₃, CH₂CH₂CH₂CH₃, CH₂CH₂SCH₃, CH₂CH(CH₃)₂, (CH₂)₄NH₂; or
   R³ is CH₃ and R⁵ is H; or
   R³ and R⁵ can be taken together to form -CH₂CH₂CH₂-.
   - K: is an _{L}-isomer amino acid of formula -N(CH₃)CH(R⁷)C(=O)-, wherein:
   R⁷ is-(CH₂)₃NHC(=NH) (NH₂);
   - L: is -NHCH₂C(=O)-; and
   - M: is an _{L}-isomer amino acid of formula -NHCH(CH₂COOH)C(=O)-.

   Preferred compounds (1) and (2) of the invention are 1,4-disubstituted phenyl compounds of the formula (III): 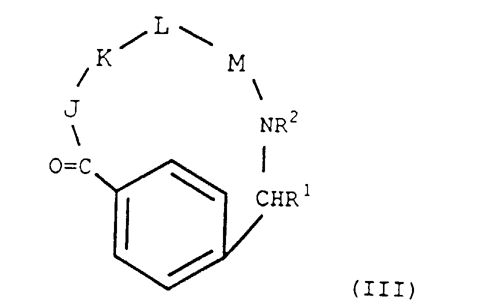 wherein:
   - R¹: is H, C₁-C₄ alkyl, phenyl, benzyl, or phenyl-(C₂-C₄)alkyl;
   - R²: is H or methyl;
   - J: is β-Ala or an _{L}-isomer or _{D}-isomer amino acid of structure -N(R³)C(R⁴) (R⁵)C(=O)-, wherein:
   R³ is H or CH₃;
   R⁴ is K or C₁-C₃ alkyl;
   R⁵ is H, C₁-C₈ alkyl, C₃-C₆ cycloalkyl, C₃-C₆ cycloalkylmethyl, C₁-C₆ cycloalkylethyl, phenyl, phenylmethyl, CH₂OH, CH₂SH, CH₂OCH₃, CH₂SCH₃, CH₂CH₂SCH₃, (CH₂)_{S}NH₂ or (CH₂)ₛNHC(=NH) (NH₂), where s = 3-5; or
   R³ and R⁵ can be taken together to form -CH₂CH₂CH₂-; or
   R⁴ and R⁵ can be taken together to form -(CH₂)ᵤ-, where u = 2-5;
   - K: is an _{L}-isomer amino acid of structure -N(R⁶)CH(R⁷)C(=O)-, wherein:
   R⁶ is C₁-C₈ alkyl;
   R⁷ is -(CH₂)ₚNHC(=NH)(NH₂), where p = 3-4; 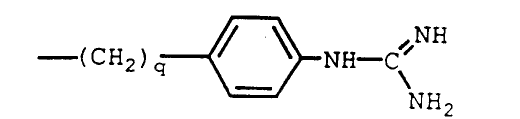 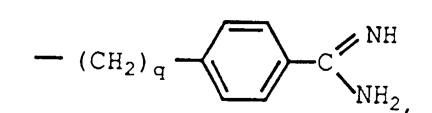 where q = 0,1;
   -(CH₂)ᵣX,where r = 4-6; 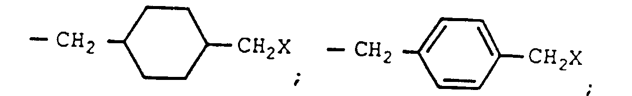 -(CH₂)ₘS(CH₂)₂X, where m = 1,2;
   -(C₃-C₇ alkyl)-N-(C₁-C₆ alkyl) 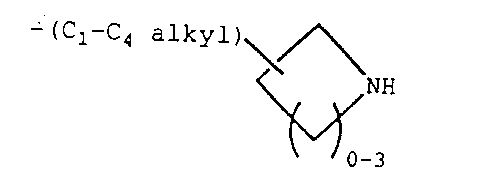 -(CH₂)ₘ-O-(C₁-C₄ alkyl)-NH-(C₁-C₆ alkyl), where m = 1,2;
   -(CH₂)ₘ-S-(C₁-C₄ alkyl)-NH-(C₁-C₆ alkyl), where m = 1,2; and
   X is -NH₂ or -NHC(=NH)(NH₂),provided that X is not NH₂ when r = 4;
   M is an _{L}-isomer amino acid of structure -NH-CH(R⁸)C(=O)-, wherein:
   R⁸ is -CH₂CO₂H,-CH₂SO₃H, or -CH(CH₃)CO₂H.

   Specifically preferred are the following compounds having IC₅₀ values of less than or equal to 100 µM for inhibiting the aggregation of platelets (as described below) :
   The compound of formula (II) wherein R¹ and R² are H; J is _{D}-Val; K is NMeArg; L is Gly; and M is Asp. The compound of formula (II) wherein R¹ and R² are H; J is _{D}-2-aminobutyric acid; K is NMeArg; L is Gly; and M is Asp.
   The compound of formula (II) wherein R¹ and R² are H; J is _{D}-Leu; K is NMeArg; L is Gly; and M is Asp.
   The compound of formula (II) wherein R¹ and R² are H; J is _{D}-Ala; K is NMeArg; L is Gly; and M is Asp.
   The compound of formula (II) wherein R¹ and R² are H; J is Gly; K is NMeArg; L is Gly; and M is Asp.
   The compound of formula (II) wherein R¹ and R² are H; J is _{D}-Pro; K is NMeArg; L is Gly; and M is Asp.
   The compound of formula (II) wherein R¹ and R² are H; J is _{D}-Lys; K is NMeArg; L is Gly; and M is Asp.
   The compound of formula (II) wherein R¹ and R² are H; J is β-Ala; K is NMeArg; L is Gly; and M is Asp.
   The compound of formula (II) wherein R1 and R² are H; J is _{D}-Val; K is NMeArg; L is β-Ala; and M is Asp.
   The compound of formula (II) wherein R¹ and R² are H; J is Pro; K is NMeArg; L is Gly; and M is Asp.
   The compound of formula (II) wherein R¹ and R² are H; J is NMeGly; K is NMeArg; L is Gly; and M is Asp.
   The compound of formula (II) wherein R¹ is methyl (isomer 1); R² are H; J is _{D}-Val; K is NMeArg; L is Gly; and M is Asp.
   The compound of formula (II) wherein R¹ is methyl (isomer 2); R² are H; J is _{D}-val; K is NMeArg; L is Gly; and M is Asp.
   The compound of formula (II) wherein R¹ is phenyl (isomer 1); R² are H; J is _{D}-val; K is NMeArg; L is Gly; and M is Asp.
   The compound of formula (II) wherein R¹ is phenyl (isomer 2); R² are H; J is _{D}-Val; K is NMeArg; L is Gly; and M is Asp.
   The compound of formula (III) wherein R¹ and R² are H; J is _{D}-Val; K is NMeArg; L is Gly; and M is Asp.
   The compound of formula (II) wherein R¹ and R² are H; J is D-Val; K is D-NMeArg; L is Gly; and M is Asp.
   The compound of formula (II) wherein R¹ and R² are H; J is D-Nle; K is NMeArg; L is Gly; and M is Asp.
   The compound of formula (II) wherein R¹ and R² are H; J is _{D}-Phg; K is NMeArg; L is Gly; and M is Asp.
   The compound of formula (II) wherein R¹ and R² are H; J is _{D}-Ile; K is NMeArg; L is Gly; and M is Asp.
   The compound of formula (II) wherein R¹ and R² are H; J is _{D}-Phe; K is NMeArg; L is Gly; and M is Asp.
   The compound of formula (II) wherein R¹ and R² are H; J is NMeGly; K is NMeArg; L is Gly; and M is Asp.
   The compound of formula (II) wherein R¹ and R² are H; J is _{D}-Nle; K is NMeArg; L is Gly; and M is Asp.
   The compound of formula (II) wherein R¹ and R² are H; J is _{D}-Tyr; K is NMeArg; L is Gly; and M is Asp.
   The compound of formula (II) wherein R¹ and R² are H; J is _{D}-Val; K is NMeAmf; L is Gly; and M is Asp.
   The compound of formula (II) wherein R¹ and R² are H; J is _{D}-Val; K is NMeArg; L is Ala; and M is Asp.
   The compound of formula (II) wherein R¹ and R² are H; J is _{D}-Val; K is NMeArg; L is Gly; and M is αMeAsp.
   The compound of formula (II) wherein R¹ and R² are H; J is _{D}-Val;; K is NMeArg; L is Gly; and M is βMeAsp.
   The compound of formula (II) wherein R¹ and R² are H; J is _{D}-Val; K is NMeArg; L is Gly; and M is NMeAsp.
   The compound of formula (II) wherein R¹ and R² are H; J is D-Val; K is NMeArg; L is Gly; and M is D-Asp.
   The compound of formula (II) wherein R¹ is H; R² is CH₃; J is _{D}-Val; K is NMeArg; L is Gly; and M is Asp.
   The compound of formula (II) wherein R¹ and R² are H; J is _{D}-Abu; K is di-NMeorn; L is Gly; and M is Asp. 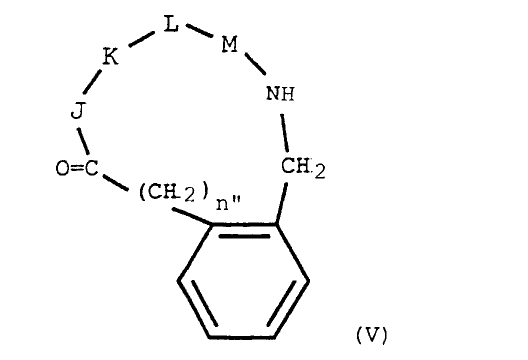
   The compound of formula (V) wherein n"=1; J is _{D}-Val; K is NMeArg; L is Gly; and M is Asp.
   The compound of formula (V) wherein n"=0; J is _{D}-Val; K is NMeArg; L is Gly; and M is Asp. 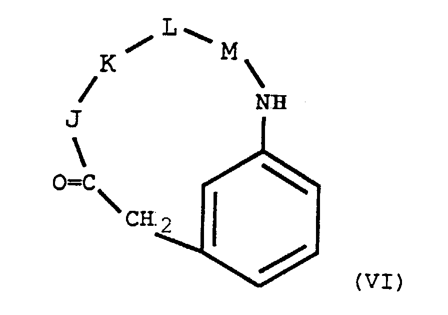
   The compound of formula (VI) wherein J is _{D}-Val; K is NMeArg; L is Gly; and M is Asp. 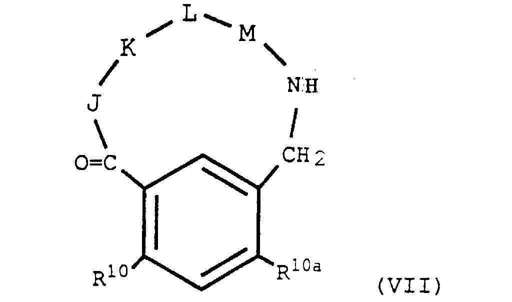
   The compound of formula (VII) wherein R¹⁰ is H; R^{10a} is Cl; J is _{D}-Val; K is NMeArg; L is Gly; and M is Asp.
   The compound of formula (VII) wherein R¹⁰ is H; R^{10a} is I; J is _{D}-Val; K is NMeArg; L is Gly; and M is Asp.
   The compound of formula (VII) wherein R¹⁰ is H; R^{10a} is I; J is _{D}-Abu; K is NMeArg; L is Gly; and M is Asp.
   The compound of formula (VII) wherein R¹⁰ is H; R^{10a} is OMe; J is _{D}-Val; K is NMeArg; L is Gly; and M is Asp.
   The compound of formula (VII) wherein R¹⁰ is H; R^{10a} is Me; J is _{D}-Val; K is NMeArg; L is Gly; and M is Asp.
   The compound of formula (VII) wherein R^{10a} is H; R¹⁰ is Cl; J is _{D}-Val; K is NMeArg; L is Gly; and M is Asp.
   The compound of formula (VII) wherein R^{10a} is H; R¹⁰ is MeO; J is _{D}-Val; K is NMeArg; L is Gly; and M is Asp.
   The compound of formula (VII) wherein R^{10a} is H; R¹⁰ is Me; J is _{D}-Val; K is NMeArg; L is Gly; and M is Asp. 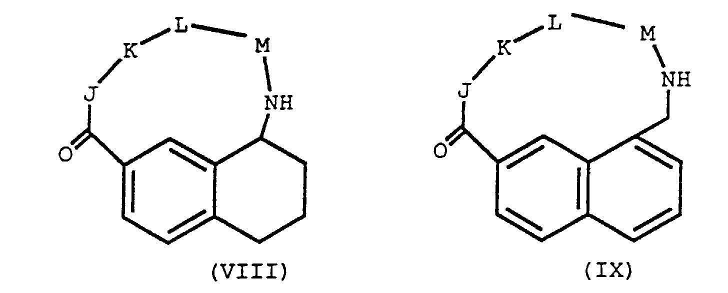
   The compound of formula (VIII) wherein J is _{D}-Val; K is NMeArg; L is Gly; and M is Asp.
   The compound of formula (IX) wherein J is _{D}-Val; K is NMeArg; L is Gly; and M is Asp.
   More specifically preferred are the following compounds of formula (I), all of which have IC₅₀ values of less than 1.0 µM for inhibiting the aggregation of platelets (as described below).
   The compound of formula (II) wherein R¹ and R² are H; J is _{D}-Val; K is NMeArg; L is Gly; and M is Asp.
   The compound of formula (II) wherein R¹ and R² are H; J is _{D}-2-aminobutyric acid; K is NMeArg; L is Gly; and M is Asp.
   The compound of formula (II) wherein R¹ and R² are H; J is _{D}-Leu; K is NMeArg; L is Gly; and M is Asp.
   The compound of formula (II) wherein R¹ and R² are H; J is _{D}-Ala; K is NMeArg; L is Gly; and M is Asp.
   The compound of formula (II) wherein R¹ and R² are H; J is Gly; K is NMeArg; L is Gly; and M is Asp.
   The compound of formula (II) wherein R¹ and R² are H; J is _{D}-Pro; K is NMeArg; L is Gly; and M is Asp.
   The compound of formula (II) wherein R¹ and R² are H; J is _{D}-Lys; K is NMeArg; L is Gly; and M is Asp.
   The compound of formula (II) wherein R¹ and R² are H; J is β-Ala; K is NMeArg; L is Gly; and M is Asp.
   The compound of formula (II) wherein R¹ and R² are H; J is NMeGly; K is NMeArg; L is Gly; and M is Asp.
   The compound of formula (II) wherein R¹ is methyl (isomer 1); R² are H; J is _{D}-Val; K is NMeArg; L is Gly; and M is Asp.
   The compound of formula (II) wherein R¹ is methyl (isomer 2); R² are H; J is _{D}-Val; K is NMeArg; L is Gly; and M is Asp.
   The compound of formula (II) wherein R¹ is phenyl (isomer 1); R² are H; J is _{D}-Val; K is NMeArg; L is Gly; and M is Asp.
   The compound of formula (III) wherein R¹ and R² are H; J is _{D}-Val; K is NMeArg; L is Gly; and M is Asp.
   The compound of formula (II) wherein R¹ and R² are H; J is D-Val; K is D-NMeArg; L is Gly; and M is Asp.
   The compound of formula (II) wherein R¹ and R² are H; J is D-Nle; K is NMeArg; L is Gly; and M is Asp.
   The compound of formula (II) wherein R¹ and R² are H; J is D-Phg; K is NMeArg; L is Gly; and M is Asp.
   The compound of formula (II) wherein R¹ and R² are H; J is D-Phe; K is NMeArg; L is Gly; and M is Asp.
   The compound of formula (II) wherein R¹ and R² are H; J is D-Ile; K is NMeArg; L is Gly; and M is Asp.
   The compound of formula (V) wherein n"=1; J is D-Val; K is NMeArg; L is Gly; and M is Asp.
   The compound of formula (V) wherein n"=0; J is D-Val; K is NMeArg; L is Gly; and M is Asp.
   The compound of formula (VI) wherein J is D-Val; K is NMeArg; L is Gly; and M is Asp.
   The compound of formula (VII) wherein R¹⁰ is H; R^{10a} is Cl; J is D-Val; K is NMeArg; L is Gly; and M is Asp.
   The compound of formula (VII) wherein R¹⁰ is H; R^{10a} is I; J is D-Val; K is NMeArg; L is Gly; and M is Asp.
   The compound of formula (VII) wherein R¹⁰ is H; R^{10a} is I; J is D-Abu; K is NMeArg; L is Gly; and M is Asp.
   The compound of formula (VII) wherein R¹⁰ is H; R^{10a} is Me; J is D-Val; K is NMeArg; L is Gly; and M is Asp.
   The compound of formula (VII) wherein R^{10a} is H; R¹⁰ is Cl; J is D-Val; K is NMeArg; L is Gly; and M is Asp.
   The compound of formula (VII) wherein R^{10a} is H; R10 is MeO; J is D-Val; K is NMeArg; L is Gly; and M is Asp.
   The compound of formula (VII) wherein R^{10a} is H; R10 is Me; J is D-Val; K is NMeArg; L is Gly; and M is Asp.
   The compound of formula (II) wherein R¹ and R² are H; J is D-Tyr; K is NMeArg; L is Gly; and M is Asp.
   The compound of formula (II) wherein R¹ and R² are H; J is D-Val; K is NMeAmf; L is Gly; and M is Asp.
   The compound of formula (II) wherein R¹ and R² are H; J is D-Val;; K is NMeArg; L is Gly; and M is βMeAsp.
   The compound of formula (II) wherein R¹ is H; R² is CH₃; J is D-Val; K is NMeArg; L is Gly; and M is Asp.
   The compound of formula (III) wherein R¹ and R² are H; J is D-Val; K is NMeArg; L is Gly; and M is Asp.
   The compound of formula (VIII) wherein J is D-Val; K is NMeArg; L is Gly; and M is Asp.
   The invention is also directed to
(3) the use of a compound according to (1) and (2) above for preparing a medicament for the treatment of thromboembolic disorders; and
(4) a pharmaceutical composition comprising a pharmaceutically acceptable carrier and a therapeutichally effective amount of a compound according to (1) and (2) above.

In the present invention it has been discovered that the compounds above are useful as inhibitors of glycoprotein IIb/IIIa (GPIIb/IIIa). As discussed above, GPIIb/IIIa mediates the process of platelet activation and aggregation. The compounds of the present invention inhibit the activation and aggregation of platelets induced by all known endogenous platelet agonists. The above medicament (3) of the present invention is suitable for the treatment of conditions involving platelet activation and aggregation, including cardiovascular and cerebrovascular thromboembolic disorders, including, for example, thromboembolic disorders associated with unstable angina, myocardial infarction, transient ischemic attack, stroke, atherosclerosis, and diabetes, by administering to a host in need of such treatment a pharmaceutically effective amount of the compounds described above. The compounds of the present invention are useful for inhibiting the binding of fibrinogen to blood platelets, inhibiting aggregation of blood platelets, treating thrombus formation or embolus formation, or preventing thrombus or embolus formation in a mammal. The compounds of the invention may be used for preparing a medicament for blocking fibrinogen from acting at its receptor site in a mammal.

The compounds of the present invention can also be co-administered with suitable anti-coagulant agents, such as heparin or warfarin, or anti-platelet agents, such as aspirin. The compounds of the present invention may also be combined with thrombolytic or fibrinolytic agents, such as plasminogen activators, anistreplase, urokinase, or streptokinase, to achieve synergistic effects in the treatment of thromboembolic disorders.

GPIIb/IIIa is known to be overexpressed in metastatic tumor cells. The compounds of the present invention may also be used for preparing medicaments for the treatment of metastatic cancer.

The compounds herein described may have asymmetric centers. Unless otherwise indicated, all chiral, diastereomeric and racemic forms are included in the present invention. Many geometric isomers of olefins, C=N double bonds can also be present in the compounds described herein, and all such stable isomers are contemplated in the present invention. Two distinct isomers (cis and trans) of the peptide bond are known to occur; both can also be present in the compounds described herein, and all such stable isomers are contemplated in the present invention. Unless otherwise specifically noted, the _{L}-isomer of the amino acid is used at positions J, K, L, and M of the compounds of the present invention. The D and L-isomers of a particular amino acid are designated herein using the conventional 3-letter abbreviation of the amino acid, as indicated by the following examples: _{D}-Leu, _{D}-Leu, _{L}-Leu, or L-Leu.

When any variable (for example, R¹ through R⁸, m, n, p, X, Y ) occurs more than one time in any constituent or in any formula, its definition on each occurrence is independent of its definition at every other occurrence. Also, combinations of substituents and/or variables are permissible only if such combinations result in stable compounds.

As used herein, "alkyl" is intended to include both branched and straight-chain saturated aliphatic hydrocarbon groups having the specified number of carbon atoms; "alkoxy" represents an alkyl group of indicated number of carbon atoms attached through an oxygen bridge; "cycloalkyl" is intended to include saturated ring groups, such as cyclopropyl, cyclobutyl, cyclopentyl, cyclohexyl, cycloheptyl and cyclooctyl; and "biycloalkyl" is intended to include saturated bicyclic ring groups such as [3.3.0]bicyclooctane, [4.3.0]bicyclononane, [4.4.0]bicyclodecane (decalin), [2.2.2]bicyclooctane. "Alkenyl" is intended to include hydrocarbon chains of either a straight or branched configuration and one or more unsaturated carbon-carbon bonds which may occur in any stable point along the chain, such as ethenyl and propenyl; and "alkynyl" is intended to include hydrocarbon chains of either a straight or branched configuration and one or more triple carbon-carbon bonds which may occur in any stable point along the chain, such as ethynyl and propynyl. "Halo" or "halogen" as used herein refers to fluoro, chloro, bromo and iodo; and "counterion" is used to represent a small, negatively charged species such as chloride, bromide, hydroxide, acetate and sulfate.

As used herein, "aryl" is intended to mean phenyl or naphthyl; "carbocyclic" is intended to mean any stable 5- to 7- membered monocyclic or bicyclic or 7- to 14-membered bicyclic or tricyclic carbon ring, any of which may be saturated, partially unsaturated, or aromatic. Examples of such carbocyles include cyclopentyl, cyclohexyl, phenyl, biphenyl, naphthyl, indanyl or tetrahydronaphthyl (tetralin).

As used herein, the term "heterocycle" or "heterocyclic ring system" is intended to mean a stable 5- to 7- membered monocyclic or bicyclic or 7- to 10-membered bicyclic heterocyclic ring which may be saturated, partially unsaturated, or aromatic, and which consists of carbon atoms and from 1 to 3 heteroatoms selected from the group consisting of N, O and S and wherein the nitrogen and sulfur heteroatoms may optionally be oxidized, and the nitrogen may optionally be quaternized, and including any bicyclic group in which any of the above-defined heterocyclic rings is fused to a benzene ring. The heterocyclic ring may be attached to its pendant group at any heteroatom or carbon atom which results in a stable structure. The heterocyclic rings described herein may be substituted on carbon or on a nitrogen atom if the resulting compound is stable. Examples of such heterocycles include pyridyl, pyrimidinyl, furanyl, thienyl, pyrrolyl, pyrazolyl, imidazolyl, tetrazolyl, benzofuranyl, benzothiophenyl, indolyl, indolenyl, quinolinyl, isoquinolinyl or benzimidazolyl, piperidinyl, 4-piperidonyl, pyrrolidinyl, 2-pyrrolidonyl, pyrrolinyl, tetrahydrofuranyl, tetrabydroquinolinyl, tetrahydroisoquinolinyl, decahydroquinolinyl or octahydroisoquinolinyl.

By "stable compound" or "stable structure" is meant herein a compound that is sufficiently robust to survive isolation to a useful degree of purity from a reaction mixture, and formulation into an efficacious therapeutic agent.

The term "substituted", as used herein, means that an one or more hydrogen on the designated atom is replaced with a selection from the indicated group, provided that the designated atom's normal valency is not exceeded, and that the substitution results in a stable compound.

As used herein, the term "amine protecting group" means any group known in the art of organic synthesis for the protection of amine groups. Such amine protecting groups include those listed in Greene, "Protective Groups in Organic Synthesis" John Wiley & Sons, New York (1981) and "The Peptides: Analysis, Sythesis, Biology, Vol. 3, Academic Press, New York (1981).

Any amine protecting group known in the art can be used. Examples of amine protecting groups include the following: 1) acyl types such as formyl, trifluoroacetyl, phthalyl, and p-toluenesulfonyl; 2) aromatic carbamate types such as benzyloxycarbonyl (Cbz) and substituted benzyloxycarbonyls, 1-(p-biphenyl)-1-methylethoxycarbonyl, and 9-fluorenylmethyloxycarbonyl (Fmoc); 3) aliphatic carbamate types such as tert-butyloxycarbonyl (Boc), ethoxycarbonyl, diisopropylmethoxycarbonyl, and allyloxycarbonyl; 4) cyclic alkyl carbamate types such as cyclopentyloxycarbonyl and adamantyloxycarbonyl; 5) alkyl types such as triphenylmethyl and benzyl; 6) trialkylsilane such as trimethylsilane; and 7) thiol containing types such as phenylthiocarbonyl and dithiasuccinoyl.

As used herein, "pharmaceutically acceptable salts and prodrugs" refer to derivatives of the disclosed compounds that are modified by making acid or base salts, or by modifying functional groups present in the compounds in such a way that the modifications are cleaved, either in routine manipulation or in vivo, to the parent compounds. Examples include:
mineral or organic acid salts of basic residues such as amines; alkali or organic salts of acidic residues such as carboxylic acids; esters of carboxylates; and acetate, formate and benzoate derivatives of alcohols and amines.

Pharmaceutically acceptable salts of the compounds of the invention can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418.

The term "amino acid" as used herein means an organic compound containing both a basic amino group and an acidic carboxyl group. Included within this term are modified and unusual amino acids, such as those disclosed in, for example, Roberts and Vellaccio (1983) The Peptides, 5: 342-429.

The term "amino acid residue" as used herein means that portion of an amino acid (as defined herein) that is present in a peptide or pseudopeptide. The term "peptide" as used herein means a linear compound that consists of two or more amino acids (as defined herein) that are linked by means of peptide or pseudopeptide bonds.

The compounds of the present invention can be prepared in a number of ways well known to one skilled in the art of organic synthesis. Preferred methods include those methods described below.

The following abbreviations are used herein:
- _{D}-Abu: _{D}-2-aminobutyric acid
- β-Ala or bAla: 3-aminopropionic acid
- Boc: t-butyloxycarbonyl
- Boc-iodo-Mamb: t-butyloxycarbonyl-3-aminomethyl-4-iodo-benzoic acid
- Boc-Mamb acid: t-butyloxycarbonyl-3-aminomethylbenzoic
- Boc-ON: [2-(tert-butyloxycarbonyloxylimino)-2-phenylacetonitrile
- Cl₂Bzl: dichlorobenzyl
- CBZ: Carbobenzyloxy
- DCC: dicyclohexylcarbodiimide
- DIEA: diisopropylethylamine
- di-NMeOrn: N-αMe-N-γMe-ornithine
- DMAP: 4-dimethylaminopyridine
- HBTU: 2-(1H-Benzotriazol-1-yl)-1,1,3,3-tetramethyluronium hexafluorophosphate
- NMeArg or MeArg: α-N-methyl arginine
- NMeAmf: N-Methylaminomethylphenylalanine
- NMeAsp: α-N-methyl aspartic acid
- NMeGly or MeGly: N-methyl glycine
- NMe-Mamb: N-methyl-3-aminomethylbenzoic acid
- NMM: N-methylmorpholine
- OcHex: O-cyclohexyl
- OBzl: O-benzyl
- TBTU: 2-(1H-Benzotriazol-1-yl)-1,1,3,3-tetramethyluronium tetrafluoroborate
- Tos: tosyl

The following conventional three-letter amino acid abbreviations are used herein; the conventional one-letter amino acid abbreviations are not used herein:
- Ala =: alanine
- Arg =: arginine
- Asn =: asparagine
- Asp =: aspartic acid
- Cys =: cysteine
- Gln =: glutamine
- Glu =: glutamic acid
- Gly =: glycine
- His =: histidine
- Ile =: isoleucine
- Leu =: leucine
- Lys =: lysine
- Met =: methionine
- Nle =: norleucine
- Phe =: phenylalanine
- Phg =: phenylglycine
- Pro =: proline
- Ser =: serine
- Thr =: threonine
- Trp =: tryptophan
- Tyr =: tyrosine
- Val =: valine

### Peptide Synthesis

The compounds of the present invention can be synthesized using standard synthetic methods known to those skilled in the art. Generally, peptides are elongated by deprotecting the α-amine of the C-terminal residue and coupling the next suitably protected amino acid through a peptide linkage using the methods described. This deprotection and coupling procedure is repeated until the desired sequence is obtained. This coupling can be performed with the constituent amino acids in a stepwise fashion, or condensation of fragments (two to several amino acids), or combination of both processes, or by solid phase peptide synthesis according to the method originally described by Merrifield, J. Am. Chem. Soc., 85, 2149-2154 (1963).

The compounds of the invention may also be synthesized using automated peptide synthesizing equipment. In addition to the foregoing, procedures for peptide synthesis are described in Stewart and Young, "Solid Phase Peptide Synthesis", 2nd ed, Pierce Chemical Co., Rockford, IL (1984); Gross, Meienhofer, Udenfriend, Eds., "The Peptides: Analysis, Synthesis, Biology, Vol. 1, 2, 3, 5, and 9, Academic Press, New York, (1980-1987); Bodanszky, "Peptide Chemistry: A Practical Textbook", Springer-Verlag, New York (1988); and Bodanszky et all "The Practice of Peptide Sythesis" Springer-Verlag, New York (1984).

The coupling between two amino acid derivatives, an amino acid and a peptide, two peptide fragments, or the cyclization of a peptide can be carried out using standard coupling procedures such as the azide method, mixed carbonic acid anhydride (isobutyl chloroformate) method, carbodiimide (dicyclohexylcarbodiimide, diisopropylcarbodiimide, or water-soluble carbodiimides) method, active ester (p-nitrophenyl ester, N-hydroxysuccinic imido ester) method, Woodward reagent K method, carbonyldiimidazole method, phosphorus reagents such as BOP-Cl, or oxidation-reduction method. Some of these methods (especially the carbodiimide) can be enhanced by the addition of 1-hydroxybenzotriazole. These coupling reactions may be performed in either solution (liquid phase) or solid phase.

The functional groups of the constituent amino acids must be protected during the coupling reactions to avoid undesired bonds being formed. The protecting groups that can be used are listed in Greene, "Protective Groups in Organic Synthesis" John Wiley & Sons, New York (1981) and "The Peptides: Analysis, Sythesis, Biology, Vol. 3, Academic Press, New York (1981).

The α-carboxyl group of the C-terminal residue is usually protected by an ester that can be cleaved to give the carboxylic acid. These protecting groups include: 1) alkyl esters such as methyl and t-butyl, 2) aryl esters such as benzyl and substituted benzyl, or 3) esters which can be cleaved by mild base treatment or mild reductive means such as trichloroethyl and phenacyl esters. In the solid phase case, the C-terminal amino acid is attached to an insoluble carrier (usually polystyrene). These insoluble carriers contain a group which will react with the carboxyl group to form a bond which is stable to the elongation conditions but readily cleaved later. Examples of which are: oxime resin (DeGrado and Kaiser (1980) *J. Org. Chem.* **45,** 1295-1300) chloro or bromomethyl resin, hydroxymethyl resin, and aminomethyl resin. Many of these resins are commercially available with the desired C-terminal amino acid already incorporated.

The α-amino group of each amino acid must be protected. Any protecting group known in the art can be used. Examples of these are: 1) acyl types such as formyl, trifluoroacetyl, phthalyl, and p-toluenesulfonyl; 2) aromatic carbamate types such as benzyloxycarbonyl (Cbz) and substituted benzyloxycarbonyls, 1-(p-biphenyl)-1-methylethoxycarbonyl, and 9-fluorenylmethyloxycarbonyl (Fmoc); 3) aliphatic carbamate types such as tert-butyloxycarbonyl (Boc), ethoxycarbonyl, diisopropylmethoxycarbonyl, and allyloxycarbonyl; 4) cyclic alkyl carbamate types such as cyclopentyloxycarbonyl and adamantyloxycarbonyl; 5) alkyl types such as triphenylmethyl and benzyl; 6) trialkylsilane such as trimethylsilane; and 7) thiol containing types such as phenylthiocarbonyl and dithiasuccinoyl. The preferred α-amino protecting group is either Boc or Fmoc. Many amino acid derivatives suitably protected for peptide synthesis are commercially available.

The α-amino protecting group is cleaved prior to the coupling of the next amino acid. When the Boc group is used, the methods of choice are trifluoroacetic acid, neat or in dichloromethane, or HCl in dioxane. The resulting ammonium salt is then neutralized either prior to the coupling or in situ with basic solutions such as aqueous buffers, or tertiary amines in dichloromethane or dimethylformamide. When the Fmoc group is used, the reagents of choice are piperidine or substituted piperidines in dimethylformamide, but any secondary amine or aqueous basic solutions can be used. The deprotection is carried out at a temperature between 0 °C and room temperature.

Any of the amino acids bearing side chain functionalities must be protected during the preparation of the peptide using any of the above-identified groups. Those skilled in the art will appreciate that the selection and use of appropriate protecting groups for these side chain functionalities will depend upon the amino acid and presence of other protecting groups in the peptide. The selection of such a protecting group is important in that it must not be removed during the deprotection and coupling of the α-amino group.

For example, when Boc is chosen for the α-amine protection the following protecting groups are acceptable: ρ-toluenesulfonyl (tosyl) moieties and nitro for arginine; benzyloxycarbonyl, substituted benzyloxycarbonyls, or tosyl for lysine; benzyl or alkyl esters such as cyclopentyl for glutamic and aspartic acids; benzyl ethers for serine and threonine; benzyl ethers, substituted benzyl ethers or 2-bromobenzyloxycarbonyl for tyrosine; ρ-methylbenzyl, ρ-methoxybenzyl, acetamidomethyl, benzyl, or t-butylsulfonyl for cysteine; and the indole of tryptophan can either be left unprotected or protected with a formyl group.

When Fmoc is chosen for the α-amine protection usually tert-butyl based protecting groups are acceptable. For instance, Boc can be used for lysine, tert-butyl ether for serine, threonine and tyrosine, and tert-butyl ester for glutamic and aspartic acids.

Once the elongation and cyclization of the peptide is completed all of the protecting groups are removed. For the liquid phase synthesis the protecting groups are removed in whatever manner as dictated by the choice of protecting groups. These procedures are well known to those skilled in the art.

When a solid phase synthesis is used, the peptide should be removed from the resin without simultaneously removing protecting groups from functional groups that might interfere with the cyclization process. Thus, if the peptide is to be cyclized in solution, the cleavage conditions need to be chosen such that a free α-carboxylate and a free α-amino group are generated without simultaneously removing other protecting groups. Alternatively, the peptide may be removed from the resin by hydrazinolysis, and then coupled by the azide method. Another very convenient method involves the synthesis of peptides on an oxime resin, followed by intramolecular nucleophilic displacement from the resin, which generates a cyclic peptide (Osapay, Profit, and Taylor (1990) *Tetrahedron Letters* **43**, 6121-6124). When the oxime resin is employed, the Boc protection scheme is generally chosen. Then, the preferred method for removing side chain protecting groups generally involves treatment with anhydrous HF containing additives such as dimethyl sulfide, anisole, thioanisole, or p-cresol at 0 °C. The cleavage of the peptide can also be accomplished by other acid reagents such as trifluoromethanesulfonic acid/trifluoroacetic acid mixtures.

Unusual amino acids used in this invention can be synthesized by standard methods familiar to those skilled in the art ("The Peptides: Analysis, Sythesis, Biology, Vol. 5, pp. 342-449, Academic Press, New York (1981)). N-Alkyl amino acids can be prepared using proceedures described in previously (Cheung et al., (1977) *Can*. *J. Chem.* **55**, 906; Freidinger et all, (1982) *J. Org. Chem.* **48**, 77 (1982)).

The compounds of the present invention may be prepared using the procedures further detailed below.

Representative materials and methods that may be used in preparing the compounds of the invention are described further below.

Manual solid phase peptide synthesis was performed in 25 mL polypropylene filtration tubes purchased from BioRad Inc. Oxime resin (substitution level = 0.96 mmol/g) was prepared according to published procedures (DeGrado and Kaiser (1980) *J. Org. Chem.* **45**, 1295). All chemicals and solvents (reagent grade) were used as supplied from the vendors cited without further purification. t-Butyloxycarbonyl (Boc) amino acids and other starting amino acids may be obtained commercially from Bachem Inc., Bachem Biosciences Inc. (Philadelphia, PA), Advanced ChemTech (Louisville, KY), Peninsula Laboratories (Belmont, CA), or Sigma (St. Louis, MO). 2-(1H-Benzotriazol-1-yl)-1,1,3,3-tetramethyluronium hexafluorophosphate (HBTU) and TBTU were purchased from Advanced ChemTech. N-methylmorpholine (NMM), *m*-cresol, D-2-aminobutyric acid (Abu), trimethylacetylchloride, diisopropylethylamine (DIEA), 3-cyanobenzoic acid and [2-(tert-butyloxycarbonyloxylimino)-phenylacetonitrile] (Boc-ON) were purchased from Aldrich Chemical Company. Dimethylformamide (DMF), ethyl acetate, chloroform (CHCl₃), methanol (MeOH), pyridine and hydrochloric acid (HCl) were obtained from Baker. Acetonitrile, dichloromethane (DCM), acetic acid (HOAc), trifluoroacetic acid (TFA), ethyl ether, triethylamine, acetone, and magnesium sulfate were purchased from EM Science. Palladium on carbon catalyst (10% Pd) was purchased from Fluka Chemical Company. Absolute ethanol was obtained from Quantum Chemical Corporation. Thin layer chromatography (TLC) was performed on Silica Gel 60 F₂₅₄ TLC plates (layer thickness 0.2 mm) which were purchased from EM Separations. TLC visualization was accomplished using UV light, iodine, and/or ninhydrin spray. Melting points were determined using a Thomas Hoover or Electrothermal 9200 melting point apparatus and are uncorrected. HPLC analyses were performed on either a Hewlett Packard 1090, Waters Delta Prep 3000, Rainin, or DuPont 8800 system. NMR spectra were recorded on a 300 MHz General Electric QE-300, Varian 300, or Varian 400 spectrometer. Fast atom bombardment mass spectrometry (FAB-MS) was performed on a VG Zab-E double-focusing mass spectrometer using a Xenon FAB gun as the ion source or a Finnigan MAT 8230.

### Synthesis of 3 and 4-substituted Boc-aminomethylbenzoic Acid Derivatives

3 and 4-substituted Boc-aminomethylbenzoic acid derivatives useful as intermediates in the synthesis of the compounds of the invention are prepared using standard procedures, for example, as described in *Tett. Lett.,* 4393 (1975); *Modern Synthetic Reactions,* H.O. House (1972); or Hartinget et al. *J. Am*. *chem. Soc.,* 50: 3370 (1928), and as shown schematically below.

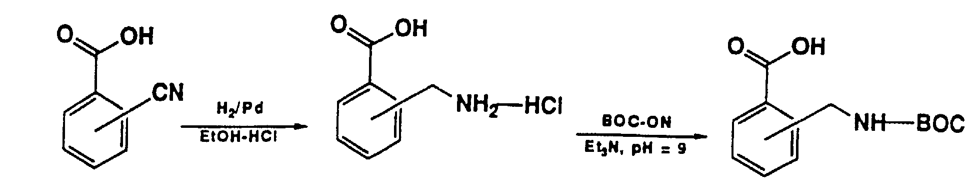

### 3-Aminomethylbenzoic acid•HCl

3-Cyanobenzoic acid (10.0 g, 68 mmol) was dissolved in 200 ml ethanol by heating in a 35-50°C water bath. Concentrated HCl (6.12 ml, 201 mmol) was added and the solution was transferred to a 500 ml nitrogen-flushed round bottom flask containing palladium on carbon catalyst (1.05 g, 10% Pd/C). The suspension was stirred under an atmosphere of hydrogen for 38 hours, filtered through a scintered glass funnel, and washed thoroughly with H₂O. The ethanol was removed under reduced pressure and the remaining aqueous layer, which contained a white solid, was diluted to 250 ml with additional H₂O. Ethyl ether (250 ml) was added and the suspension was transferred to a separatory funnel. Upon vigorous shaking, all solids dissolved and the aqueous layer was then washed two times with ether, evaporated under reduced pressure to a volume of 150 ml, and lyophilized to give the title compound (3-aminomethylbenzoic acid·HCl) (8.10 g, 64%) as a beige solid. ¹H NMR (D₂O) 4.27 (s, 2H), 7.60 (t, 1H), 7.72 (d,1H), 8.06 (d, 2H).

### t-Butyloxycarbonyl-3-aminomethylbenzoic Acid (Boc-Mamb)

The title compound was prepared according to a modification of standard procedures previously reported in the literature (Itoh, Hagiwara, and Kamiya (1975) *Tett. Lett.,* 4393). 3-Aminomethylbenzoic acid (hydrochloride salt) (3.0 g, 16.0 mmol) was dissolved in 60 ml H₂O. To this was added a solution of Boc-CN (4.33 g, 17.6 mmol) in 60 ml acetone followed by triethylamine (5.56 ml, 39.9 mmol). The solution turned yellow and the pH was adjusted to 9 (wet pH paper) by adding an additional 1.0 ml (7.2 mmol) triethylamine. The solution was stirred overnight at room temperature at which time the acetone was removed under reduced pressure and the remaining aqueous layer was washed three times with ether. The aqueous layer was then acidified to pH 2 with 2N HCl and then extracted three times with ethyl acetate. The combined organic layers were washed three times with H₂O, dried over anhydrous magnesium sulfate, and evaporated to dryness under reduced pressure. The material was recrystallized from ethyl acetate/ hexane to give two crops of the title compound (2.58 g, 64%) as an off-white solid. mp 123-125°C ;¹H NMR (CDCl₃) 1.47 (s, 9 H), 4.38 (br s, 2 H), 4.95 (br s, 1H), 7.45 (t, 1H), 7.55 (d, 1H), 8.02 (d, 2H).

### t-Butyloxycarbonyl-N-methyl-3-aminomethylbenzoic Acid (Boc-Mamb)

The title compound can be prepared according to standard procedures, for examples, as disclosed in Olsen, *J. Org. chem.* (1970) 35: 1912), and as shown schematically below.

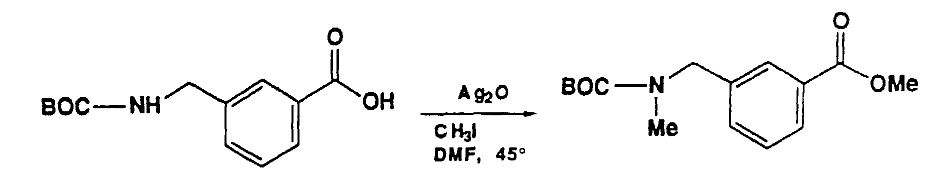

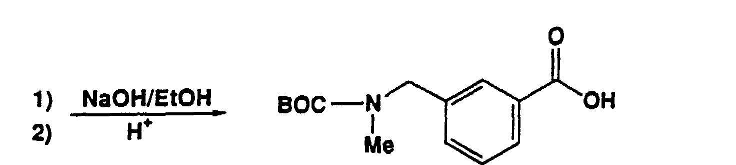

### Synthesis of Aminomethylbenzoic Acid Analogs

Intermediates of the formula below may be prepared using standard synthetic procedures, for example, as shown in the indicated reaction schemes shown below.

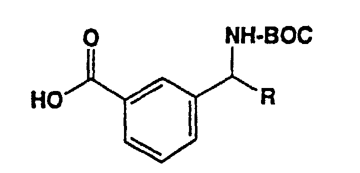

For R = CH₃, CH₂CH₃, CH₂CH₂CH₃, CH₂CH₂CH₂CH₃, CH(CH₃)₂, C(CH₃)₃, CH(CH₃)CH₂CH₃, benzyl, cyclopentyl, cyclohexyl; see Scheme 1.

For R = CH₃, CH₂CH₂CH₂CH₃, phenyl; see Scheme 2.

For R = CH₃, phenyl; see Scheme 3 and 4.

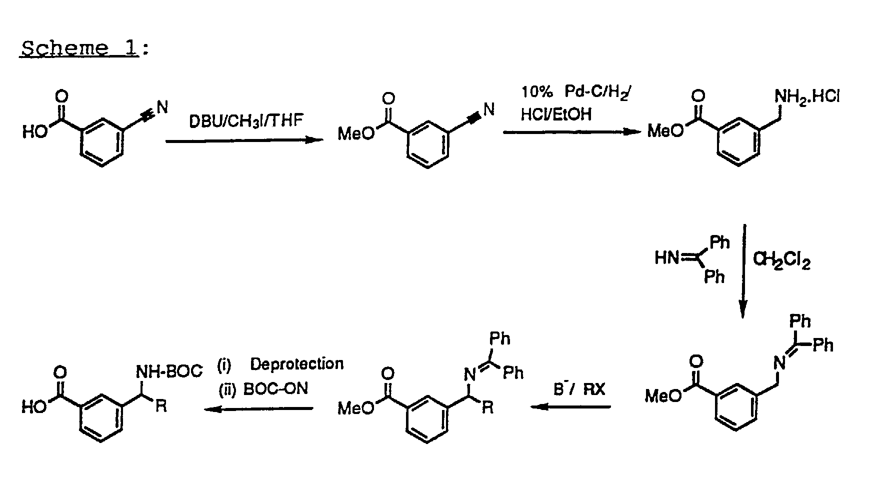

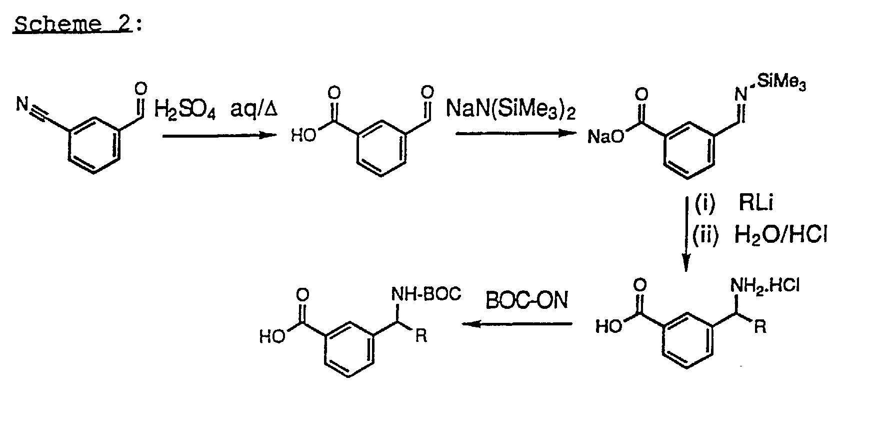

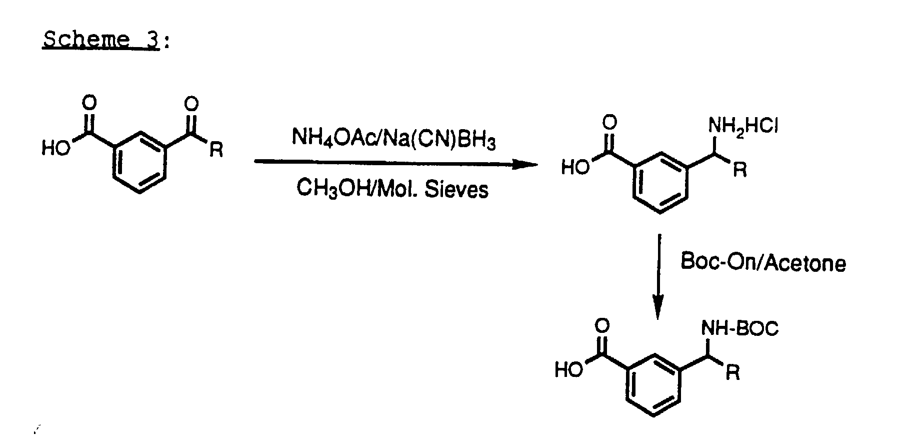

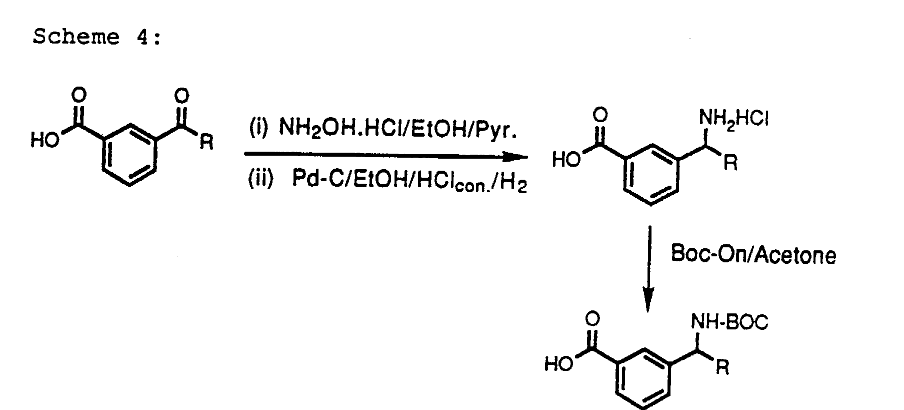

### 3-[1'-(t-butyloxycarbonyl)amino]ethylbenzoic acid (BOC-MeMAMB)

The title compound for the purpose of this invention was prepared according to the Scheme 4 (above).

3-Acetylbenzoic acid (0.50 g, 3 mmol), hydroxylamine hydrochloride (0.70 g, 10 mmol) and pyridine (0.70 ml, 9 mmol) were refluxed in 10 ml ethanol, for 2 h. Reaction mixture was concentrated, residue triturated with water, filtered and dried. Oxime was isolated as a white solid (0.51 g ; 94.4% yield).
¹HNMR (CD₃OD) 7.45-8.30(m, 4H), 2.30(s, 3H). MS (CH₄-CI) [M+H-O] = 164.

A solution of the oxime (0.51 g, 3 mmol) in ethanol, containing 10% Pd on carbon (1.5 g) and conc. 5 HCl (0.25 ml, 3 mmol) was hydrogenated at 2.07bar (30 psi) H₂ pressure in a Parr hydrogenator for 5 h. Catalyst was filtered and the filtrate concentrated. Residue was triturated with ether. Amine hydrochloride was isolated as a white solid (0.48 g ; 85.7% yield). ¹HNMR (CD₃OD) 7.6-8.15(m, 4H), 4.55(q, 1H), 1.70(s, 3H). MS [M+H] = 166.

Amine hydrochloride (0.40 g, 2 mmol) was dissolved in 15 ml water. A solution of BOC-ON (0.52 g, 2.1 mmol) in 15 ml acetone was added, followed by the addition of triethylamine (0.8 ml, 6 mmol). Reaction was allowed to proceed for 20 h. Reaction mixture was concentrated, partitioned between ethyl acetate and water. Aqueous layer was acidified to pH 2 using 10% HCl solution. Product was extracted in ethyl acetate, which after the usual work up and recrystallization from ethyl acetate/hexane, gave the title compound as a white solid (0.30 g ; 57% yield). m p 116-118°C.
¹HNMR (CDCl₃) 7.35-8.2(m, 4H), 4.6(bs, 1.5H), 1.50(d, 3H), 1.40(s, 9H). MS (NH₃-CI) [M+NH₄] = 283.

### 3-[1'-(t-butyloxycarbonyl)amino]benzy]benzoic acid(BOC-PhMAMB)

The title compound for the purpose of this invention was prepared according to the Scheme 4 (above), by the procedure similar to that for the methyl derivative.

A solution of 3-benzoylbenzoic acid (2.00 g, 9 mmol), hydroxylamine hydrochloride (2.00 g, 29 mmol) and pyridine (2.00 ml, 25 mmol) in ethanol was refluxed for 12 h. After the usual extractive work up, white solid was obtained (2.41 g). The product still contained traces of pyridine, but was used in the next step without further purification.

The crude product (2.00 g, ~8 mmol) was dissolved in 200 ml ethanol. 10% Pd-C (2.00 g) and con. HCl (1.3 ml, 16 mmol) were added. Reaction mixture was hydrogenated at 2.07 bar (30 psi) for 1 h. The catalyst was filtered and the reaction mixture concentrated. Upon trituration of the residue with ether and drying under vacuum, amine hydrochloride was obtained as a white solid (2,12 g ; 97% yield). ¹HNMR (CD₃OD) 7.4-8.15(m, 10H), 5.75 (s, 1H) . MS (CH₄-CI) [M+H-OH] = 211.

Amine hydrochloride (1.00 g, 4 mmol) was converted to its BOC-derivative by a procedure similar to the methyl case. 0.60 g (48% yield) of the recrystallized (from ethanol/hexane) title compound was obtained as a white solid. m p 190-192° C. ¹HNMR (CD₃OD) 7.2-8.0(m, 10H), 5.90 (2s, 1H, 2 isomers), 1.40(s, 9H). MS (NH₃-CI) [M+NH₄-C₄H₈] = 289

### t-Butyloxycarbonyl-D-2-aminobutyric Acid

The title compound was prepared by a modification of procedures previously reported in the literature (Itoh, Hagiwara, and Kamiya (1975) *Tett. Lett.,* 4393), as shown in the scheme below.

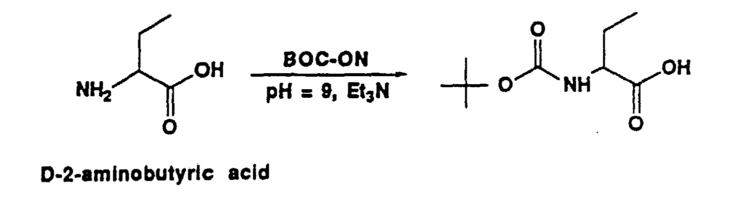

D-2-aminobutyric acid (1.0 g, 9.70 mmol) was dissolved in 20 ml H₂O and a solution of Boc-ON (2.62 g, 10.6 mmol) in 20 ml acetone was added. A white precipitate formed which dissolved upon addition of triethylamine (3.37 ml, 24.2 mmol) to give a pale yellow solution (pH = 9, wet pH paper). The solution was stirred at room temperature overnight at which time the acetone was removed under reduced pressure. The remaining aqueous layer was extracted with ether three times, acidified to pH 2 with concentrated HCl, and then extracted with ethyl acetate three times. The combined organic layers were dried over anhydrous magnesium sulfate and evaporated under reduced pressure to give t-butyloxycarbonyl-D-2-aminobutyric acid as an oil (2.05 g,greater than quantitative yield, contains solvent), which was used without further purification. ¹H NMR (CDCl₃) 0.98 (t, 3H), 1.45 (s, 9H), 1.73 (m, 1H), 1.90 (m, 1H), 4.29 (m, 1H), 5.05 (m, 1H).

### Synthesis of t-Butyloxycarbonyl-3-aminophenylacetic Acid

t-Butyloxycarbonyl-3-aminophenylacetic acids useful as intermediates in the synthesis of the compounds of the invention are prepared using standard procedures, for example, as described in Collman and Groh (1982) *J. Am. chem. Soc.,* 104: 1391, and as shown schematically below.

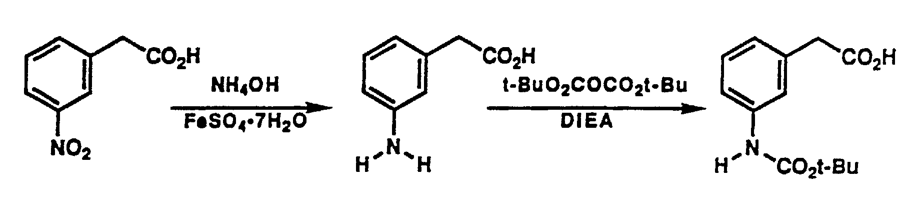

### t-Butyloxycarbonyl-3-aminophenylacetic Acid

A solution of 3-aminophenylacetic acid (Aldrich, 10 g, 66 mmol), di-*tert*-butyl dicarbonate (15.8 g, 72 mmol), and DIEA (8.6 g, 66 mmol) in 50 ml of dichloromethane was stirred overnight at room temperature. The reaction mixture was concentrated, partitioned between dichloromethane-H₂O, the water layer was separated, acidified to pH 3 with 1N HCl, and extracted with dichloromethane. The extracts were washed with H₂O, brine, dried over anhydrous sodium sulfate, and evaporated to dryness under reduced pressure. This material was purified by recrystallization from heptane to provide the title compound (3.7 g, 22%) as a white solid. mp 105°C; ¹H NMR (CDCl₃) 7.35 (s, 1H), 7.25 (m, 3H), 6.95 (m, 1H), 6.60 (br s, 1H), 3.65 (s, 2H), 1.50 (s, 9H).

### Synthesis of 4, 5, and 6-Substituted 3-Aminomethylbenzoic Acid•HCl, and 4, 5, and 6-Substituted t-Butyloxycarbonyl-3-aminomethylbenzoic Acid Derivatives

4, 5, and 6-Substituted 3-aminomethylbenzoic acid•HCl, and 4, 5, and 6-substituted t-butyloxycarbonyl-3-aminomethylbenzoic acid derivatives useful as intermediates in the synthesis of the compounds of the invention are prepared using standard procedures, for example, as described in Felder et al *Helv. Chim. Acta,* 48: 259 (1965); de Diesbach *Helv. Chim. Acta,* 23: 1232 (1949); Truitt and Creagn *J. Org. Chem.,* 27: 1066 (1962); or Sekiya et al *Chem. Pharm. Bull.,* 11: 551 (1963), and as shown schematically below.

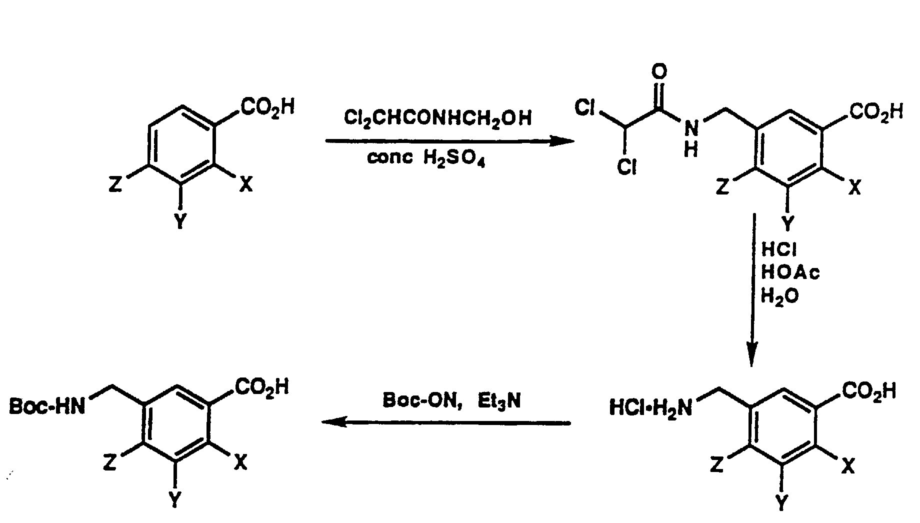

### Synthesis of 4-Chloro-3-aminomethylbenzoic Acid•HCl

The title compound was prepared by modification of procedures previously reported in the literature (Felder et al (1965) *Helv. Chim. Acta,* 48: 259). To a solution of 4-chlorobenzoic acid (15.7 g, 100 mmol) in 150 ml of concentrated sulfuric acid was added N-hydroxymethyl dichloroacetamide (23.7 g, 150 mmol) in portions. The reaction mixture was stirred at room temperature for 2 days, poured onto 375 g of ice, stirred for 1 hour, the solid was collected by filtration, and washed with H₂O. The moist solid was dissolved in 5% sodium bicarbonate solution, filtered, and acidified to pH 1 with concentrated HCl. The solid was collected by filtration, washed with H₂O, and air-dryed overnight to give 4-chloro-3-dichloroacetylaminomethylbenzoic acid (26.2 g, 89%) as a white powder.

A suspension of 4-chloro-3-dichloroacetylaminomethylbenzoic acid (26.2 g, 88 mmol) in 45 ml of acetic acid, 150 ml of concentrated HCl, and 150 ml of H₂O was heated to reflux for 3 hours, filtered while hot, and allowed to cool to room temperature. The solid was collected by filtration, washed with ether, washed with acetone-ether, and air-dryed overnight to give the title compound (7.6 g, 39%) as off-white crystals. mp 278-9°C; ¹H NMR (D₆-DMSO) 13.40 (br s, 1H), 8.75 (br s, 3H), 8.20 (s, 1H), 7.95 (dd, 1H), 7.70 (d, 1H), 4.20 (br s, 2H).

### t-Butyloxycarbonyl-4-chloro-3-aminomethylbenzoic Acid

A suspension of 4-chloro-3-aminomethylbenzoic acid•HCl (6.7 g, 30 mmol) and triethylamine (9.3 g, 92 mmol) in 50 ml of H₂O, was added to a solution of Boc-ON (9.2 g, 38 mmol) in 50 ml of tetrahydrofuran cooled to 0°C. The reaction mixture was stirred at room temperature overnight, and the volatile compounds were removed by concentration under reduced pressure. The residue was diluted with H₂O, washed with ether, acidified to pH 3 with 1N HCl, and extracted with ethyl acetate. The extracts were washed with H₂O, brine, dried over anhydrous magnesium sulfate, and evaporated to dryness under reduced pressure. This material was triturated with ether-hexane to provide the title compound (7.4 g, 87%) as a white powder. mp 159°C (dec); ¹H NMR (D₆-DMSO) 13.20 (br s, 1H), 7.90 (s, 1H), 7.80 (dd, 1H), 7.60 (br s, 1H), 7.55 (d, 1H), 4.20 (br d, 2H), 1.40 (s, 9H).

### 4 and 6-Substituted t-Butyloxycarbonyl-3-aminomethylbenzoic Acid Derivatives

The compounds listed below were prepared using the general procedure described above for t-butyloxycarbonyl-4-chloro-3-aminomethylbenzoic acid.

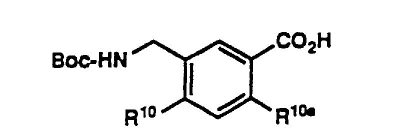

| R^{10a} | R¹⁰ | mp °C |
|---|---|---|
| H | Cl | 159 |
| H | I | 168 |
| H | Me | 155 |
| H | MeO | 171 |
| Cl | H | 150 |
| I | H | 182 |
| Me | H | 166 |
| MeO | H | 79 |

### Synthesis of 2-Aminomethylbenzoic Acid•HCl and 2-Aminomethylphenylacetic Acid•HCl

2-Aminomethylbenzoic acid·HCl and 2-aminomethylphenylacetic acid·HCl useful as intermediates in the synthesis of the compounds of the invention are prepared using standard procedures, for example, as described in Naito et al *J. Antibiotics,* 30: 698 (1977); or Young and Sweet *J. Am. Chem. Soc.,* 80: 800 (1958), and as shown schematically below.

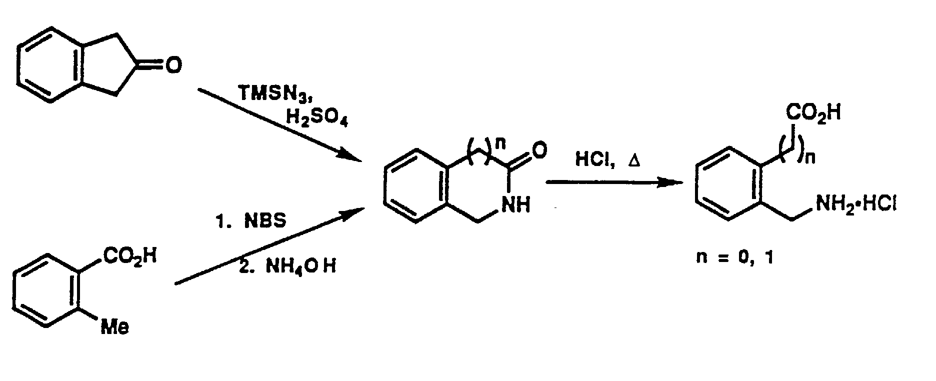

### 2-Aminomethylphenylacetic Acid δ-Lactam

The title compound was prepared by modification of procedures previously reported in the literature (Naito et all (1977) *J. Antibiotics,* 30: 698). To an ice-cooled suspension of 2-indanone (10.8 g, 82 mmol) and azidotrimethylsilane (9.4 g, 82 mmol) in 115 ml of chloroform was added 25 ml of concentrated sulfuric acid at a rate to maintain the temperature between 30-40°C. After an additional 3 hours, the reaction mixture was poured onto ice, and the water layer was made basic with concentrated ammonium hydroxide. The chloroform layer was separated, washed with H₂O, brine, dried over anhydrous magnesium sulfate, and evaporated to dryness under reduced pressure. This material was purified by sublimination (145°C, <133 Pa (<1 mm)), followed by recrystallization from benzene to give the title compound (5.4 g, 45%) as pale yellow crystals. mp 149150°C; ¹H NMR (CDCl₃) 7.20 (m, 5H), 4.50 (s, 2H), 3.60 (s, 2H).

### 2-Aminomethylphenylacetic Acid•HCl

The title compound was prepared by modification of procedures previously reported in the literature (Naito et all (1977) *J. Antibiotics,* 30: 698). A mixture of 2-aminomethylphenylacetic acid δ-lactam (6.4 g, 44 mmol) and 21 ml of 6N HCl was heated to reflux for 4 hours. The reaction mixture was treated with activated carbon (Norit A), filtered, evaporated to dryness, and the residual oil triturated with acetone. Filtration provided the title compound (5.5 g, 62%) as colorless crystals. mp 168°C (dec); ¹H NMR (D₆-DMSO) 12.65 (br s, 1H), 8.35 (br s, 3H), 7.50 (m, 1H), 7.35 (m, 3H), 4.05 (ABq, 2H), 3.80 (s, 2H).

### 2-Aminomethylbenzoic Acid γ-Lactam

The title compound was prepared by modification of procedures previously reported in the literature (Danishefsky et all (1975) *J. Org. Chem*., 40: 796). A mixture of methyl *o*-toluate (45 g, 33 mol), N-bromosuccinimide (57 g, 32 mol), and dibenzoyl peroxide (0.64 g) in 175 ml of carbon tetrachloride was heated to reflux for 4 hours. The cooled reaction mixture was filtered, evaporated to dryness under reduced pressure, dissolved in 250 ml of methanol, and concentrated ammonium hydroxide (75 ml, 1.11 mol) was added. The reaction mixture was heated to reflux for 5 hours, concentrated, filtered, and the solid washed with H₂O followed by ether. This material was purified by recrystallization from H₂O to give the title compound (11.0 g, 26%) as a white solid. mp 150°C; ¹H NMR (CDCl₃) 7.90 (d, 1H), 7.60 (t, 1H), 7.50 (t, 2H), 7.00 (br s, 1H), 4.50 (s, 2H).

### 2-Aminomethylbenzoic Acid•HCl

The title compound was prepared using the general procedure described above for 2-aminomethylphenylacetic acid•HCl. The lactam (3.5 g, 26 mmol) was converted to the title compound (2.4 g, 50%) as colorless crystals. mp 233°C (dec); ¹H NMR (D₆-DMSO) 13.40 (br s, 1H), 8.35 (br s, 3H), 8.05 (d, 1H), 7.60 (m, 3H), 4.35 (br s, 2H).

### Alternatives to Mamb: Other Cyclic Peptide Intermediates

Alternatives to Mamb useful as carbocylic residues R³¹ in the cyclic peptides of the invention include aminoalkyl-naphthoic acid and aminoalkyl-tetrahydronaphthoic acid residues. Representative aminoalkyl-naphthoic acid and aminoalkyl-tetrahydronaphthoic acid intermediates useful in the synthesis of cyclic peptides of the present invention are described below. The synthesis of these intermediates is outlined below in Scheme 4a.

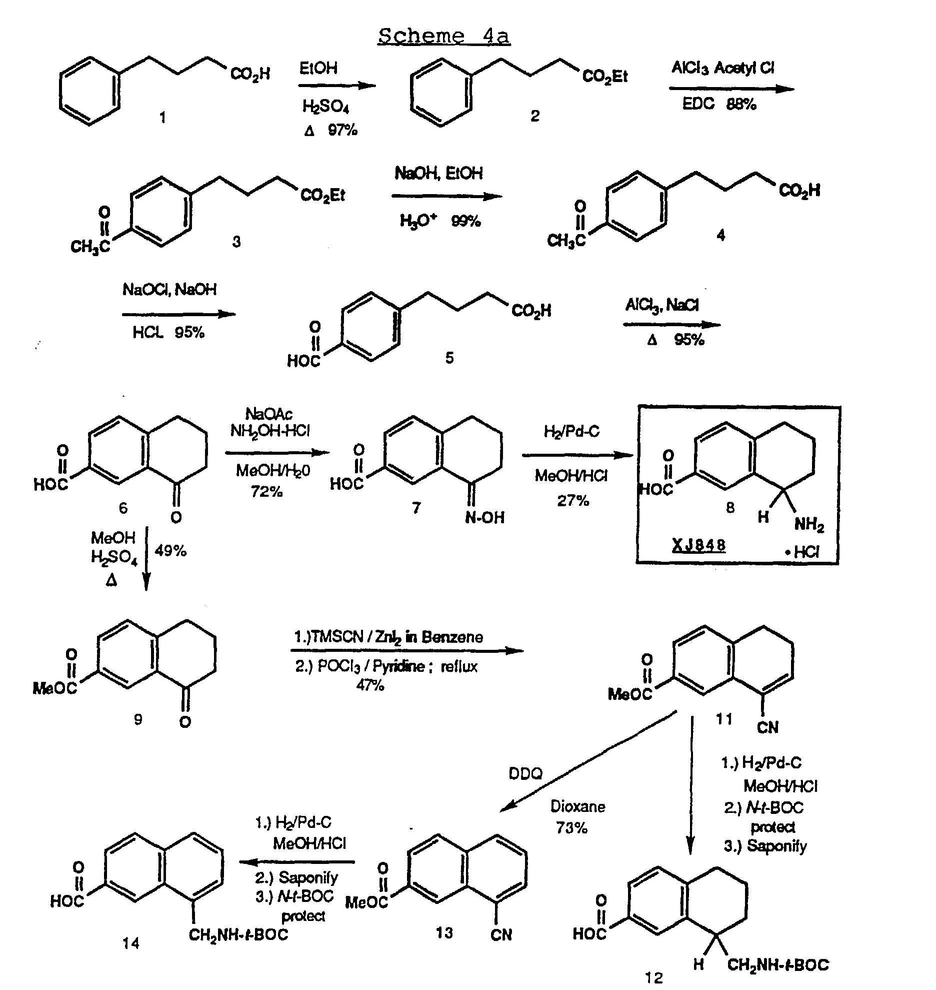

The title compound was prepared according to a modification of standard procedures previously reported in the literature (Earnest, I., Kalvoda, J., Rihs, G., and Mutter, M., Tett. Lett., Vol. 31, No. 28, pp 4011-4014, 1990).

### 8-Amino-5,6,7,8-tetrahydro-2-naphthoic Acid Hydrochloride (8)

As shown below in Scheme 4a, 4-phenylbutyric acid (1) was converted to the ethyl ester (2) which was acylated via aluminum chloride and acetylchloride to give 4-acetylphenylbutyric acid ethyl ester (3). This ester was subjected to saponification to give 4-acetylphenylbutyric acid (4). Subsequently, the acetyl group was oxidized to give 4-carboxyphenylbutyric acid (5) which was converted to the 1-tetralin-7-carboxylic acid (6) using aluminum chloride in a Friedel-Crafts cyclization with resonably high yield. At that point, the tetralone was split into two portions and some was converted to the oxime (7) using sodium acetate and hydroxylamine hydrochloride.The oxime was subjected to hydrogenolysis to give the racemic mixture of 8-amino-5,6,7,8-tetrahydro-2-naphthoic acid as the hydrochloride (8) for use as an intermediate for incorporation into the cyclic peptide.
Part A - A solution of 4-phenylbutyric acid (50.0 g, 0.3 mol) in ethanol (140 ml) with concentrated sulfuric acid (0.53 ml) was stirred at reflux over 5 hours. The cooled solution was poured into ice water and extracted with ethyl acetate. The combined organic layers were backwashed with brine, dried over anhydrous magnesium sulfate and evaporated to dryness under reduced pressure to give 4-phenylbutyric acid ethyl ester (56.07 g, 0.29 mol, 97%) as a yellow liquid. ¹H NMR (CDCl₃) d 7.3-7.1 (m, 5H), 4.1 (q, 2H, J=7.1 Hz), 2.7 (t, 2H, J=7.7 Hz), 2.3 (t, 2H, J=7.5 Hz), 1.95 (quintet, 2H, J=7.5 Hz), 1.25 (t, 3H, J=7.1 Hz).
Part B - To a solution of aluminum chloride (153 g, 1.15 mol), and acetyl chloride (38.5 ml, 42.5 g, 0.54 mol) in dichloromethane (1500 ml) was added, dropwise, a solution of 4-phenylbutyric acid ethyl ester (50.0 g, 0.26 mol) in dichloromethane (500 ml). All was stirred at ambient temperature for 15 minutes. The solution was poured into cold concentrated hydrochloric acid (2000 ml) and then extracted with dichloromethane. The combined organic layers were backwashed with brine, dried over anhydrous magnesium sulfate and evaporated to dryness under reduced pressure to give 4-acetylphenylbutyric acid ethyl ester (53.23 g, 0.23 mol, 88%) as a dark yellow liquid. ¹H NMR (CDCl₃) d 7.9 (d, 2H, J=8.1 Hz), 7.25 (d, 2H, J=8.4 Hz), 4.1 (q, 2H, J=7.1 Hz), 2.75 (t, 2H, J=7.6 Hz), 2.6 (s, 3H), 2.35 (t, 2H, J=7.6 Hz), 2.0 (quintet, 2H, J=7.5 Hz), 1.25 (t, 3H, J=7.1 Hz).
Part C - To a solution of 4-acetylphenylbutyric acid ethyl ester (50.0 g, 0.21mol) in ethanol (1250 ml) was added, dropwise, a solution of sodium hydroxide (50.0 g) in water (1250 ml). All was stirred at reflux over 4 hours. The solution was concentrated to half volume and then acidified to a pH equal to 1.0 using hydrochloric acid (1N). The resulting precipitate was collected and washed with water to give 4-acetylphenylbutyric acid (53.76 g, 0.26 mol, 99%) as a white solid. mp = 50-52°C; ¹H NMR (CDCl₃) d 7.9 (d, 2H, J=8.1 Hz), 7.25 (d, 2H, J=9.1 Hz), 2.75 (t, 2H, J=7.7 Hz), 2.6 (s, 3H), 2.4 (t, 2H, J=7.3 Hz), 2.0 (quintet, 2H, J=7.4 Hz).
Part D -To a solution of sodium hypochlorite (330 ml, 17.32 g, 0.234 mol) in a solution of sodium hydroxide (50%, 172 ml), warmed to 55°C, was added, portionwise as a solid, 4-acetylphenylbutyric acid (16.0 g, 0.078 mol) while keeping the temperature between 60-70°C. All was stirred at 55°C over 20 hours. The cooled solution was quenched by the dropwise addition of a solution of sodium bisulfite (25%, 330 ml). The mixture was then transferred to a beaker and acidified by the careful addition of concentrated hydrochloric acid. The resulting solid was collected, washed with water and dried, then triturated sequentially with chlorobutane and hexane to give 4-carboxyphenylbutyric acid (15.31 g, 0.074 mol, 95%) as a white solid. mp = 190-195°C; ¹H NMR (DMSO) d 12.55 (bs, 1H), 8.1 (s, 1H), 7.85 (d, 2H, J=8.1 Hz), 7.3 (d, 2H, J=8.1 Hz), 2.7 (t, 2H, J=7.5 Hz), 2.2 (t, 2H, J=7.4 Hz), 1.8 (quintet, 2H, J=7.5 Hz).
Part E - A mixture of 4-carboxyphenylbutyric acid (10.40 g, 0.05 mol), aluminum chloride (33.34 g, 0.25 mol) and sodium chloride (2.90 g, 0.05 mol) was heated with continual stirring to 190°C over 30 minutes. As the mixture cooled to 60°C, cold hydrochloric acid (1N, 250 ml) was carefully added. The mixture was extracted with dichloromethane. The combined organic layers were backwashed with dilute hydrochloric acid and water, dried over anhydrous magnesium sulfate and evaporated to dryness under reduced pressure. The resulting solid was triturated with chlorobutane to give 1-tetralon-7-carboxylic acid (9.59 g, 0.05 mol, 100%) as a brown solid. mp = 210-215°C; ¹H NMR (DMSO) d 8.4 (s, 1H), 8.1 (d, 2H, J=8.0 Hz), 7.5 (d, 1H, J=7.9 Hz), 3.0 (t, 2H, J=6.0 Hz), 2.65 (t, 2H, J=6.6 Hz), 2.1 (quintet, 2H, J=6.3 Hz).
Part E - A solution of 1-tetralon-7-carboxylic acid (1.0 g, 0.0053 mol) and sodium acetate (1.93 g, 0.024 mol) and hydroxylamine hydrochloride (1.11 g, 0.016 mol) in a mixture of methanol and water (1:1, 15 ml) was stirred at reflux over 4 hours. The mixture was cooled and then added was more water (50 ml). The solid was collected, washed with water and dried, then triturated with hexane to give 1-tetralonoxime-7-carboxylic acid (0.78 g, 0.0038 mol, 72%) as a white solid. mp = 205-215°C; ¹H NMR (DMSO) d 11.3 (s, 2H), 8.4 (s, 1H), 7.8 (d, 1H, J=7.7 Hz), 7.3 (d, 1H, J=7.7 Hz), 2.8 (t, 2H, J=5.9 Hz), 2.7 (d, 2H, J=6.6 Hz), 1.9-1.7 (m, 2H).
Part G - A mixture of 1-tetralonoxime-7-carboxylic acid (0.75 g, 0.0037 mol) in methanol (25 ml) with concentrated hydrochloric acid (0.54 ml, 0.20 g, 0.0056 mol) and palladium on carbon catalyst (0.10 g, 5% Pd/C) was shaken for 20 hours at ambient temperature under an atmosphere of hydrogen (4,14 bar (60 psi)).The reaction mixture was filtered over Celite® and washed with methanol. The filtrate was evaporated to dryness under reduced pressure and the residue was purified by flash chromatography using hexane:ethyl acetate::1:1 to give the racemic mixture of 8-amino-5,6,7,8-tetrahydro-2-naphthoic acid hydrochloride (0.225 g, 0.001 mol. 27%) as a white solid. mp = 289-291°C; ¹H NMR (DMSO) d 8.55 (bs, 3H), 8.2-8.1 (m, 1H), 7.85-7.8 (m, 1H), 7.35-7.25 (m, 1H), 4.5 (m, 1H), 2.9-2.8 (m, 2H), 2.1-1.9 (m, 3H), 1.85-1.7 (m, 1H).

### N-(BOC)-8-Aminomethyl-5,6,7,8-tetrahydro-2-naphthoic Acid (12)

As shown below in Scheme 4a, the remaining tetralone was then converted to the methyl ester (9). Using a procedure from Gregory, G.B. and Johnson, A.L, JOC, 1990, 55, 1479, the tetralone methyl ester (9) was converted, first, to the cyanohydrin by treatment with trimethylsilylcyanide and zinc iodide and then, via the *in situ* dehydration with phosphorous oxychloride in pyridine, to the methyl 8-cyano-5,6-dihydro-2-naphthoate (11). This naphthoate was divided into two portions and some was subjected to hydrogenolysis, N-BOC-protection and saponification to give N-(BOC)-8-aminomethyl-5,6,7,8-tetrahydro-2-naphthoic acid (12) as an intermediate for incorporation into the cyclic peptide.
Part A - A mixture of 1-tetralon-7-carboxylic acid (7.0 g, 0.037 mol) in methanol (13.6 ml, 10.8 g, 0.30 mol) with a catalytic amount of hydrochloriic acid (0.07 ml, 0.12 g, 0.0012 mol) was stirred at reflux over 5 hours. The cooled reaction mixture was poured into ice water and extracted with ethyl acetate. The combined organic layers were backwashed with water and brine, dried over anhydrous magnesium sulfate and evaporated to dryness under reduced pressure. The resulting solid was purified by flash chromatography using hexane:ethyl acetate::75:25. The resulting solid was triturated with hexane to give 1-tetralon-7-carboxylic acid methyl ester (3.61 g, 0.018 mol, 49%) as a yellow solid. mp = 170-172°C; ¹H NMR (CDCl₃) d 8.7 (s, 1H), 8.15 (d, 1H, J=8.1 Hz), 7.35 (d, 1H, J=8.1 Hz), 3.95 (s, 3H), 3.05 (d, 2H, J=6,1 Hz), 2.7 (t, 2H, J=6.4 Hz), 2.15 (quintet, 2H, J=6.2 Hz).
Part B - A solution of 1-tetralon-7-carboxylic acid methyl ester (3.50 g, 0.017 mol), trimethylsilylcyanide (1.98 g, 0.02 mol) and zinc iodide (0.10 g) in benzene (20 ml) was stirred at ambient temperature over 15 hours. Then added, sequentially and dropwise, was pyridine (20 ml) and phosphorous oxychloride (4.0 ml, 6.55 g, 0.0425 mol). The reaction mixture was stirred at reflux over 1 hour then evaporated to dryness under reduced pressure. The residue was taken up in chloroform, backwashed with water, dried over anhydrous magnesium sulfate and evaporated to dryness under reduced pressure to give methyl 8-cyano-5,6-dihydro-2-naphthoate (1.70 g, 0.008 mol, 47%) as a yellow solid. mp = 73-75°C; ¹H NMR (CDCl₃) d 8.0-7.9 (m, 1H), 7.3-7.2 (m, 1H), 6.95 (t, 1H, J=4.8 Hz), 3.95 (s, 3H), 2.9 (t, 2H, J=8.3 Hz), 2.6-2.4 (m, 3H)
Part C - A mixture of methyl 8-cyano-5,6-dihydro-2-naphthoate (0.80 g, 0.0038 mol) in methanol (25 ml) with concentrated hydrochloric acid (0.56 ml) and palladium on carbon catalyst (0.40 g, 5% Pd/C) was shaken for 20 hours at ambient temperature under an atmosphere of hydrogen (3.45 bar (50 psi)). The reaction mixture was filtered over Celite® and washed with methanol. The filtrate was evaporated to dryness under reduced pressure and the residue was triturated with hexane to give the racemic mixture of methyl 8-aminomethyl-5,6,7,8-tetrahydro-2-naphthoate (0.80 g, 0.0037 mol, 97%) as a white solid. mp = 172-179°C; ¹H NMR (DMSO) d 8.2-8.0 (m, 4H), 7.9-7.7 (m, 6H), 7.5-7.2 (m, 4H), 3.9-3.8 (m, 7H), 3.3-2.7(m, 10H), 2.0-1.6 (m, 8H).
Part D - A solution of methyl 8-aminomethyl-5,6,7,8-tetrahydro-2-naphthoate (0.78 g, 0.0036 mol) and triethylamine (0.55 ml, 0.40 g, 0.004 mol) in aqueous tetrahydrofuran (50%, 75 ml) was added, portionwise as a solid, 2-(*tert*-butoxycarbonyloxyimino)-2-phenylacetonitrile (0.99 g, 0.004 mol). All was stirred at ambient temperature over 3 hours. The solution was concentrated to half volume and extracted with diethylether. The aqueous layer was then acidified to a pH of 1.0 using hydrochloric acid (1N) and then extraced with ethyl acetate. The combined organic layers were dried over anhydrous magnesium sulfate and evaporated to dryness under reduced pressure. The residue was purified by flash chromatography using hexane:ethyl acetate::8:2 to give methyl N-(BOC)-8-aminomethyl-5,6,7,8-tetrahydro-2-naphthoate (0.54 g, 0.0017 mol, 47%) as a white solid. mp = 72-80°C; ¹H NMR (DMSO) d 13.8 (s, 1H), 7.8-7.65 (m, 3H), 7.6-7.5 (m, 3H), 7.25-7.20 (m, 1H), 7.15-7.05 (m, 1H), 3.9-3.8 (m, 1H), 3.2-2.8 (m, 4H), 1.8-1.6 (m, 3H), 1.4 (s, 6H).
Part E - To a solution of methyl N-(BOC)-8-aminomethyl-5,6,7,8-tetrahydro-2-naphthoate (0.50 g, 0.0016 mol) in ethanol (12.5 ml) was added, dropwise, a solution of sodium hydroxide (0.50 g) in water (12.5 ml). All was stirred a reflux over 4 hours. The reaction mixture was concentrated to half volume and then acidified to a pH equal to 1.0 using hydrochloric acid (1N). The residue was puified by flash chromatography using a gradient of hexane:ethyl acetate::1:1 to ethyl acetate to ethyl acetate: methanol::9:1 to give the racemic mixture of the title compound, N-(BOC)-2-aminomethyl-5,6,7,8-tetrahydro-2-naphthoic acid (0.19 g, 0.00062 mol, 39%) as a white solid. mp = 172-176°C; ¹H NMR (DMSO) d 7.8 (s, 1H), 7.65 (d, 1H, J=8.1 Hz), 7.15 (d, 1H, J=8.1 Hz), 7.1-7.0 (m, 1H), 3.2-3.1 (m, 2H), 3.0-2.7 (m, 4H), 1.8-1.6 (m, 4H), 1.4 (s, 9H).

### N-(BOC)-8-aminomethyl-2-naphthoic acid (14)

The remaining naphthoate (11) was treated with 2,3-dichloro-5,6-dicyano-1,4-benzoquinone (DDQ) in dioxane to aromatize the adjacent ring to give the methyl 8-cyano-2-naphthoate (13). Then, the nitrile was reduced via hydrogentation and the methyl ester saponified to the carboxylic acid. This acid was then N-BOC-protected to give N-(BOC)-8-aminomethyl-2-naphthoic acid (14) as an intermediate for incorporation into the cyclic peptide.
Part A - A solution of methyl 8-cyano-5,6-dihydro-2-naphthoate (1.0 g, 0.0047 mol) and 2,3-dichloro-5,6-dicyano-1,4-benzoquinone (1.07 g, 0.0047 mol) in dioxane (50 ml) was stirred at 120°C over 16 hours. The reaction mixture was poured into ice water and extracted with ethyl acetate. The combined organic layers were dried over anhydrous magnesium sulfate and evaporated to dryness under reduced pressure. The residue was purified by flash chromatography using ethyl acetate to give methyl 8-cyano-2-naphthoate (0.72 g, 0.0034 mol, 73%) as a tan solid. mp = 178-182°C; ¹H NMR (CDCl₃) d 8.95 (s, 1H), 8.3-8.2 (m, 1H), 8.15-8.10 (m, 1H), 8.0-7.95 (m, 2H), 7.7-7.6 (m, 1H), 4.05 (s, 1H).
Part B - A mixture of methyl 8-cyano-2-naphthoate (1.0 g, 0.0047 mol) in methanol (35 ml) with concentrated hydrochloric acid (0.69 ml) and palladium on carbon catalyst (0.20 g, 5% Pd/C) was shaken for 6 hours at ambient temperature under anatmosphere of hydrogen (3.45bar (50 psi)): The reaction mixture was filtered over Celite® and washed with methanol. The filtrate was evaporated to dryness under reduced pressure and the residue was triturated with hexane to give methyl 8-aminomethyl-2-naphthoate (0.76 g, 0.0035 mol, 75%) as an oil. ¹H NMR (DMSO) d 8.75 (s, 1H), 8.5 (bs, 2H), 8.2-8.05 (m, 3H), 7.75-7.70 (m, 2H), 4.6 (s, 2H), 3.95 (m, 3H).
Part C - To a solution of methyl 8-aminomethyl-2-naphthoate (0.75 g, 0.0035 mol) in dry tetrahydrofuran (50 ml), cooled to 0°C, was added a solution of lithium hydroxide (0.5 M, 5.83 ml). All was stirred at ambient temperature over 20 hours. Another aliquot of lithium hydroxide was added and all was stirred for an additional 20 hours. The solid was collected and the filtrate was evaporated to dryness under reduced pressure. The solids were triturated with diethyl ether to give 8-aminomethyl-2-naphthoic acid (0.67 g, 0.0033 mol, 95%) as a white solid. mp = 223-225°C; ¹H NMR (DMSO) d 8.6 (s, 1H), 8.1-7.9 (m, 1H), 7.8-7.7 (m, 4H), 7.55-7.5 (m, 1H), 7,45-7.35 (m, 2H), 4.2 (s, 2H).
Part D - A solution of 8-aminomethyl-2-naphthoic acid (0.50 g, 0.00025 mol) and triethylamine (0.038 ml, 0.028 g, 0.000275 mol) in aqueous tetrahydrofuran (50%, 5 ml) was added, portionwise as a solid, 2-(*tert*-butoxycarbonyloxyimino)-2-phenylacetonitrile (0.068 g, 0.000275 mol). All was stirred at ambient temperature over 5 hours. The solution was concentrated to half volume and extracted with diethylether. The aqueous layer was then acidified to a pH of 1.0 using hydrochloric acid (1N) and then extraced with ethyl acetate. The combined organic layers were dried over anhydrous magnesium sulfate and evaporated to dryness under reduced pressure to give the title compound, N-(BOC)-8-aminomethyl-2-naphthoic acid (0.050 g, 0.00017 mol) as a white solid. mp = 190-191°C; ¹H NMR (DMSO) d 13.1 (bs, 1H), 8.8 (s, 1H), 8.0 (q, 2H, J=7.9 Hz), 7.9 (d, 1H, J=8.1 Hz), 7.6 (t, 1H, J=7.5 Hz), 7.65-7.55 (m, 2H), 4.6 (d, 2H, J=5.5 Hz), 1.4 (s, 9H).

### Synthesis of Cyclic Peptides

t-Butyloxycarbonyl-3-aminomethylbenzoic acid (Boc-Mamb) is coupled to oxime resin by a modification of the method described by DeGrado and Kaiser (1980) *J. Org. Chem.* **45**, 1295 using 1 equivalent of the 3-aminomethylbenzoic acid (with respect to the substitution level of the resin), 1 equivalent of HBTU, and 3 equivalent of NMM. Alternatively, Boc-Mamb (1 equivalent) may be coupled to the oxime resin using 1 equivalent each of DCC and DMAP in methylene chloride. Coupling times range from 15 to 96 hours. The substitution level is then determined using either the picric acid test (Sarin, Kent, Tam, and Merrifield, (1981) *Anal. Biochem.* **117**, 145-157) or the quantitative ninhydrin assay (Gisin (1972) *Anal. Chim. Acta* **58,** 248-249). Unreacted oxime groups are blocked using 0.5 M trimethylacetylchloride / 0.5 M diisopropylethylamine in DMF for 2 hours. Deprotection of the Boc protecting group is accomplished using 25% TFA in DCM for 30 minutes. The remaining amino acids or amino acid derivatives are coupled using between a two and ten fold excess (based on the loading of the first amino acid or amino acid derivative) of the appropriate amino acid or amino acid derivatives and HBTU in approximately 8 ml of DMF. The resin is then neutralized in situ using 3 eq. of NMM (based on the amount of amino acid used) and the coupling times range from 1 hour to several days. The completeness of coupling is monitored by qualitative ninhydrin assay, or picric acid assay in cases where the amino acid was coupled to a secondary amine. Amino acids are recoupled if necessary based on these results.

After the linear peptide had been assembled, the N-terminal Boc group is removed by treatment with 25% TFA in DCM for 30 minutes. The resin is then neutralized by treatment with 10% DIEA in DCM. Cyclization with concomitant cleavage of the peptide is accomplished using the method of Osapay and Taylor ((1990) *J. Am. Chem. Soc*., **112,** 6046) by suspending the resin in approximately 10 ml/g of DMF, adding one equivalent of HOAc (based on the loading of the first amino acid), and stirring at 50-60°C for 60 to 72 hours. Following filtration through a scintered glass funnel, the DMF filtrate is evaporated, redissolved in HOAc or 1:1 acetonitrile: H₂O, and lyophilized to obtain protected, cyclized material. Alternatively, the material may be dissolved in methanol and precipitated with ether-to obtain the protected, cyclized material. This is then treated using standard procedures with anhydrous hydrogen fluoride (Stewart and Young (1984) "Solid Phase Peptide Synthesis", 2nd. edition, Pierce Chemical Co., 85) containing 1 ml/g *m*-cresol or anisole as scavenger at 0°C for 20 to 60 minutes to remove side chain protecting groups. The crude product may be purified by reversed-phase HPLC using a 2.5 cm preparative Vydac® C18 column with a linear acetonitrile gradient containing 0.1% TFA to produce pure cyclized material. The following N-α-Boc-protected amino acids may be used for the syntheses : Boc-Arg(Tos), Boc-N-α-MeArg Tos), Boc-Gly, Boc-Asp(OcHex), Boc-3-aminomethyl-4-iodo-benzoic acid, Boc-D-Ile, Boc-NMeAsp(OcHex), Boc-NMe-Mamb, Boc-D-Phg, Boc-D-Asp(OBzl), Boc-L-Asp(OcHex), Boc-αMe-Asp(OcHex), Boc-βMe-Asp(OcHex), Boc-L-Ala, Boc-L-Pro, Boc-D-Nle, Boc-D-Leu, Boc-D-Val, Boc-D-2-aminobutyric acid (Boc-D-Abu), Boc-Phe, Boc-D-Ser(Bzl), Boc-D-Ala, Boc-3-aminomethylbenzoic acid (Boc-Mamb), Boc-D-Lys(2-ClZ), Boc-β-Ala, Boc-D-Pro, Boc-D-Phe, Boc-D-Tyr(Cl₂Bzl), Boc-NMe-Amf(CBZ), Boc-aminotetra[in-carboxylic acid, Boc-aminomethylnaphthoic acid, Boc-4-aminomethylbenzoic acid, or Boc-NMeGly.

The synthesis of the compounds of the invention is further exemplified below. The Tables below set forth representative compounds of the present invention.

### Example 1

### cyclo-(Gly-NMeArg-Gly-Asp-Mamb); J = Gly, K = NMeArg, L = Gly, M = Asp, R¹ = R² = H

The title compound was prepared using the general procedure described below for cyclo-(D-Val-NMeArg-Gly-Asp-Mamb). The peptide was prepared on a 0.336 mmol scale to give the protected cyclic peptide (218 mg, 84%). The peptide (200 mg) and 200 µl of *m*-cresol were treated with anhydrous hydrogen fluoride at 0°C for 1 hour. The crude material was precipitated with ether, redissolved in aqueous HOAc, and lyophilized to generate the title compound as a pale yellow solid (158 mg, greater than quantitative yield; calculated as the acetate salt). Purification was accomplished by reversed-phase HPLC on a preparative Vydac® C18 column (2.5 cm) using a 0.23%/ min gradient of 2 to 11% acetonitrile containing 0.1% TFA and then lyophilized to give the TFA salt of the title compound as a fluffy white solid (21% recovery, overall yield 16.3%).
Mass spectrum: M+H = 533.26.

### Example 2

### cyclo-(D-Ala-NMeArg-Gly-Asp-Mamb); J = D-Ala, K = NMeArg, L = Gly, M = Asp, R¹ = R² = H

The title compound was prepared using the general procedure described below for cyclo-(D-Val-NMeArg-Gly-Asp-Mamb). Recoupling of the Boc-N-MeArg(Tos) residue was found to be necessary. The peptide was prepared on a 0.244 mmol scale to give the protected cyclic peptide (117 mg, 61%). The peptide (110 mg) and 110 µl of m-cresol were treated with anhydrous hydrogen fluoride at 0°C for 1 hour. The crude material was precipitated with ether, redissolved in aqueous HOAc, and lyophilized to generate the title compound as a pale yellow solid. Purification was accomplished by reversed-phase HPLC on a preparative Vydac® C18 column (2.5 cm) using a 0.25%/ min gradient of 2 to 11% acetonitrile containing 0.1% TFA and then lyophilized to give the TFA salt of the title compound as a fluffy white solid.
Mass spectrum: M+H = 547.23.

### Example 3

### cyclo-(D-Abu-NMeArg-Gly-Asp-Mamb); J = D-Abu, K = NMeArg, L = Gly, M = Asp, R¹ = R² = H

The title compound was prepared using the general procedure described below for Example 4. The peptide was prepared on a 0.101 mmol scale to give the protected cyclic peptide (51 mg, 63%). The peptide (43 mg) and 50 µl of *m*-cresol were treated with anhydrous hydrogen fluoride at 0°C for 30 minutes The crude material was precipitated with ether, redissolved in aqueous HOAc, and lyophilized to generate the title compound as a pale yellow solid (23 mg, 68.7%; calculated as the acetate salt). Purification was accomplished by reversed-phase HPLC on a preparative Vydac® C18 column (2.5 cm) using a 0.23%/min gradient of 7 to 14% acetonitrile containing 0.1% trifluoroacetic acid and then lyophilized to give the TFA salt of the title compound as a fluffy white solid (31% recovery; overall yield 12.4%).
Mass spectrum: M+H = 561.46.

### Example 4

### cyclo-(D-Val-NMeArg-Gly-Asp-Mamb); J = D-Val, K = NMeArg, L = Gly, M = Asp, R¹ = R² = H

To a 25 ml polypropylene tube fitted with a frit was added Boc-Mamb (0.126 g, 0.5 mmol) and 6 ml of DMF. To this was added HBTU (0.194 g, 0.5 mmol), oxime resin (0.52 g, substitution level = 0.96 mmol/g), and N-methylmorpholine (0.165 ml, 1.50 mmol). The suspension was mixed at room temperature for 24 hours. The resin was then washed thoroughly (10-12 ml volumes) with DMF (3x), MeOH (1x), DCM (3x), MeOH (2x) and DCM (3x). The substitution level was determined to be 0.389 mmol/g by quantitative ninhydrin assay. Unreacted oxime groups were blocked by treatment with 0.5 M trimethylacetylchloride/ 0.5M DIEA in DMF for 2 hours.

The following steps were then performed: (Step 1) The resin was washed with DMF(3x), MeOH (-1x), DCM (3x), MeOH (2x), and DCM (3x). (Step 2) The t-Boc group was deprotected using 25% TFA in DCM for 30 minutes. (Step 3) The resin was washed with DCM (3x), MeOH (1x), DCM (2x), MeOH (3x) and DMF(3x) (Step 4) Boc-Asp(OcHex) (0.613 g, 1.94 mmol), HBTU (0.753 g, 1.99 mmol), 8 ml of DMF, and N-methylmorpholine (0.642 ml, 5.84 mmol) were added to the resin and the reaction allowed to proceed for 2.5 hours. (Step 5) The coupling reaction was found to be complete as assessed by the qualitative ninhydrin assay. Steps 1-5 were repeated until the desired sequence had been attained. The coupling of Boc-D-Val to NMeArg was monitored by the picric acid test.

After the linear peptide was assembled, the N-terminal t-Boc group was removed by treatment with 25% TFA in DCM (30 min ) The resin was washed thoroughly with DCM (3x), MeOH (2x) and DCM (3x), and then neutralized with 10% DIEA in DCM (2 x 1 min ) The resin was washed thoroughly with DCM (3x) and MeOH (3x) and then dried. Half of the resin (0.101 mmol) was cyclized by treating with 6 ml of DMF containing HOAc (5.8 µl, 0.101 mmol) and heating at 50°C for 72 hours. The resin was then filtered through a scintered glass funnel and washed thoroughly with DMF. The DMF filtrate was evaporated to an oil, redissolved in 1:1 acetonitrile: H₂O, and lyophilized to give the protected cyclic peptide (49 mg, 60%). The peptide (42 mg) was treated with anhydrous hydrogen fluoride at 0°C, in the presence of 50 µl of *m*-cresol as scavenger, for 30 minutes to remove side chain protecting groups . The crude material was precipitated with ether, redissolved in aqueous HOAc, and lyophilized to generate the title compound as a pale yellow solid (23 mg, 70%; calculated as the acetate salt). Purification was accomplished using reversed-phase HPLC with a preparative Vydac® C18 column (2.5 cm) and a 0.23%/ minute gradient of 7 to 18% acetonitrile containing 0.1% trifluoroacetic acid to give the TFA salt of the title compound as a fluffy white solid (24% recovery; overall yield 9.4%); FAB-MS: [M+H] = 575.45.

### Solution Phase Synthesis of Example 4

The following abbreviations are used below for TLC solvent systems: chloroform/methanol 95:5 = CM; chloroform/acetic acid 95:5 = CA;
chloroform/methanol/acetic acid 95:5 = CMA
*BocNMeArg*(*Tos*)-*Gly*-*OBzl* -- 25 mmol BocNMeArg(Tos) (11.07 g, Bachem), 30 mmol Gly-OBzl tosylate (10.10 g, Bachem), 25 mmol HBTU (O-Benzotriazole-N,N,N',N',-tetramethyl-uronium-hexafluorophosphate; 9.48 g; Advanced Chemtech), and 75 mmol DIEA (diisopropylethylamine; Aldrich) were dissolved in 25 ml CH₂Cl₂. The reaction was allowed to proceed 1 h , the solvent was evaporated under reduced pressure at 50° to a syrup, wich was dissolved in 400 ml ethyl acetate. This solution was extracted with (150 ml each) 2 x 5% citric acid, 1 x water, 2 x sat. NaHCO₃, 1 x sat. NaCl. The organic layer was dried over MgSO₄, and the solvent evaporated under reduced pressure. The resulting oil was triturated with petroleum ether and dried under high vacuum for a minimum of 1 h: yield 14.7 g (99.5%) ; TLC R_{f(CM)} = 0.18 R_{f(CA)} = 0.10; NMR is consistent with structure; FABMS M+H⁺ = 590.43 (expected 590.26).
N*meArg(Tos)*-*Gly*-*OBzl* -- 14.5 g (BocNmeArg(Tos) -Gly-OBzl (24.5 mmol) was dissolved in 30 ml TFA, allowed to react for 5 min , and the solvent evaporated at 133 Pa (1 mm mecu-pressure) at r.t. The resulting syrup was dissolved in 400 ml ice cold ethyl acetate, and extracted with 100 ml ice cold sat. NaHCO3, the aqueous phase was extracted-twice with 200 ml ethyl acetate, and the combined organic phases were extracted once with 25 ml sat. NaCl. The solvent was evaporated under reduced pressure giving a viscous oil that was triturated with 300 ml ether. The resulting solid was filtered and washed with ether, giving a hydroscopic compound that was dried in a vacuum desiccator: yield 10.33 g (86.2%); TLC R_{f(CM)} = 0.03; R_{f(CMA)} = 0.20; NMR is consistent with structure; FABMS M+H⁺ = 490.21 (expected 490.20).
*Boc-D-Val-NMeArg(Tos)-Gly-OBzl* -- 9.80 mmol NmeArg(Tos)-Gly-OBzl (4.80 g), 9.82 mmol Boc-D-Val (2.13 g, Bachem), and 10.0 mmol HBTU (3.79 g) were dissolved in 10 ml methylene chloride. The flask was placed on an ice bath, and 20 mmol DIEA (3.48 ml) was added. The reaction was allowed to proceed at 0° for 15 min and 2 days at r.t. The reaction mixture was diluted with 400 ml ethyl acetate, extracted (200 ml each) 2 x 5% citric acid, 1 x sat. NaCl, dried over MgSO₄ and evaporated under reduced pressure. The resulting oil was triturated with 50, then 30 ml ether for 30 min with efficient mixing: yield 4.58 g (69%); TLC R_{f(CM)} = 0.27 (also contains a spot near the origin, which is an aromatic impurity that is removed during trituration of the product in the next step); NMR is consistent with structure; FABMS M+H⁺ = 689.59 (expected 689.43).
*Boc-D-Val-NMeArg(Tos)-Gly* -- 4.50 g Boc-D-Val-NMeArg(Tos)-Gly-OBzl (4.44 mmol) dissolved in 80 ml methanol was purged with N₂ for 10 min. 1.30 g Pd/C catalyst (10% Fluka lot #273890) was then added, and then H₂ was passed directly over the surface of the reaction. TLC showed the reaction to be complete within approximately 0.5 h . After 1 h the catalyst was removed by filtering through a bed of Celite, and the solvent removed at 40° under reduced pressure. The resulting solid was triturated well with 50 ml refluxing ether, filtered, and washed with petroleum ether: yield 3.05 g (78%); TLC R_{f(CM)} = 0.03; R_{f(CMA)} = 0.37; NMR is consistent with structure; FABMS M+H⁺ = 599.45 (expected 599.29).
*4-Nitrobenzophenone Oxime* (Ox) -- 50 g 4-nitrobenzophenone (220 mmol, Aldrich) and 30.6 g hydroxylamine hydrochloride (Aldrich, 440 mmol) were heated at reflux in 0.5 l methanol/pyridine (9:1) for 1 hr. The reaction mixture was evaporated under reduced pressure, dissolved in 500 ml ether, and extracted with 200 ml each of 5% citric acid (2 times) and sat. NaCl (1 time), dried over MgSO₄, evaporated under reduced pressure and triturated with ether giving 44.35 g (83%) of the oxime as a mixture of the cis and trans isomers: TLC R_{f(CM)} = 0.50; R_{f}(CMA) = 0.82; NMR is consistent with structure; FABMS M+H⁺ = 242.07 (expected 242.07).
*BocMamb-Ox* -- 22 mmol BocMamb (5.522 g), 20 mmol nitrobenzophenone oxime (4.84 g), and 20 mmol DMAP (4-dimethylaminopyridine; Aldrich) were dissolved in 40 ml CH₂Cl₂ . The flask was placed on an ice bath, and 21 mmol DCC (Dicyclohexylcarbodiimide; 4.33 g) was added. The reaction was allowed to proceed on ice for 30 min and at r.t. over night. The dicyclohexylurea formed was filtered, and washed with 40 ml methylene chloride. The filtrate was evaporated under reduced pressure at r.t. to a syrup, and dissolved in 400 ml ethyl acetate. This solution was extracted with (150 ml each) 2 x 5% citric acid, 1 x water, 2 x sat. NaHCO₃, 1 x sat. NaCl. The organic layer was dried over MgSO₄, and the solvent evaporated under reduced pressure. The resulting oil was triturated with petroleum ether and dried under high vacuum for a minimum of 1 h : yield 7.51 g (79%); TLC R_{f(CM)} = 0.41; R_{f(CMA)} = 0.66; NMR is consistent with structure; FABMS M+H⁺ = 476.30 (expected 476.18).
*TFA·MAMB-Ox* -- BocMamb-Ox , 7.4 g (15.5 mmol) was dissolved in 30 ml methylene chloride plus 10 ml TFA (25% TFA). The reaction was allowed to proceed at r.t. for 1 h , and the solvent evaporated under reduced pressure at r.t. for 10 min, then at 40° for 15 min. The resulting syrup was triturated with ether (200 ml) at -5°, giving. The resulting crystals were filtered after 1 hr and washed well with ether: yield 7.22 g (95%); R_{f(CMA)} = 0.25; NMR is consistent with structure; FABMS M+H⁺ = 376.22 (expected 376.12).
*Boc-Asp (OcHex) -Mamb-Ox* -- 20 mmol Boc-Asp(OcHex) (6.308 g, Bachem) and 44 mmol DIEA (7.66 ml) were dissolved in 20 ml DMF. 20 mmol HBTU (7.58 g, Advanced Chemtech) was added, and the reaction allowed to proceed for 2 minutes with vigorous stirring. TFA·Mamb-Ox (7.13 g, 15 mmol) was added, and the reaction allowed to proceed o.n. at r.t. The solvent was removed under reduced pressure giving an oil, which was dissolved in 500 ml ethyl acetate, and this solution was extracted with (150 ml each) 2 x 5% citric acid, 1 x water, 2 x sat. NaHCO₃, 1 x sat. NaCl. The organic layer was dried over MgSO₄, and the solvent evaporated under reduced pressure. The resulting oil was triturated with petroleum ether and dried under high vacuum: yield 9.76 g (97%) ; TLC R_{f(CM)} = 0.55; NMR is consistent with structure; FABMS M+H⁺ = 673.45 (expected 673.23).
*TFA Asp (OcHex) -MAMB-Ox* -- 15 mmol Boc-Asp(OcHex)-MAMB-Ox was dissolved in 50 ml 35% TFA in CH₂Cl₂, and allowed to react 90 min. The solvent was evaporated under reduced pressure at r.t. for 10 min, then at 40° for 15 min. To remove traces of TFA, 25 ml DMF was added and the solvent evaporated at 50°. The resulting syrup was triturated with ether (200 ml), then dried under high vacuum: yield 9.61 g (93%); R_{f(CMA)} = 0.45; NMR is consistent with structure; FABMS M+H⁺ = 573.56 (expected 573.23).
*Boc-D-Val-NMeArg (Tos)-Gly-Asp (OcHex)*-*MAMB-Ox* 10.0 mmol each TFA Asp(OcHex)-MAMB-Ox, Boc-D-val-NMeArg (Tos)-Gly, and HBTU, plus 30 mmol DIEA were dissolved in 20 ml DMF. After 4 h , the solvent was removed under reduced pressure, and the residue taken up in 600 ml ethyl acetate, which was extracted with 300 ml each of 5% citric acid, water and sat. NaCl. The organic layer was dried over MgSO₄, evaporated under reduced pressure, triturated with ether and dried in vacuo: yield 9.90 g (86%); R_{f(CM)} = 0.10; NMR is consistent with structure; FABMS M+H⁺ = 1153.22 (expected 1153.47).
*TFA·D-Val-NmeArg(Tos)-Gly-Asp (OcHex) -MAMB-Ox* This compound was prepared from Boc-D-Val-NMeArg(Tos)-Gly-Asp(OcHex)-MAMB-Ox (9.8 g, 8.5 mmol) by treatment with TFA/CH₂Cl₂ (1:1) for 45 min. The solvent was evaporated and the product triturated with ether: yield 9.73 g (98%); R_{f(CM)} = 0.10; NMR is consistent with structure; FABMS M+H⁺ = 1053.22 (expected 1053.4).
*cyclo(·D-Val-NmeArg(Tos) -Gly-Asp (OcHex)-MAMB)* TFA·D-Val-NMeArg(Tos)-Gly-Asp(OcHex)-MAMB-Ox (1.80 g, 1.54 mmol), and 2 mmol each of DIEA and acetic acid were dissolved in 200 ml DMF. The mixture was heated to 50° for 2 days, then evaporated under reduced pressure. The syrup was dissolved in 400 ml ethyl acetate/n-butanol (1:1), and extracted with 200 ml each of 5% citric acid (3x) and sat. NaCl (1x). The organic layer was dried over MgSO₄ and triturated twice with 200 ml ether: yield 1.07 g (86%); R_{f(CM)} = 0.10; NMR is consistent with structure; FABMS M+H⁺ = 811.2 (expected 811.38).
*cyclo(·D-Val-NMeArg-Gly-Asp-MAMB)* 0.50 g cyclo (D-Val-NMeArg(Tos)-Gly-Asp(OcHex)-MAMB) was treated with 5 ml HF at 0°C, in the presence of 0.5 ml of anisole for 30 min. The HF was removed under reduced pressure and the crude peptide triturated with ether, ethyl acetate and ether. The resulting solid was dissolved in 10% acetic acid and lyophilized: yield 0.321 g (82% calculated as the acetate salt). The product was purified with a recovery of approximately 40% using the same method as described for the material synthesized by the solid phase procedure.

### Example 4b

### cyclo-(D-Val-D-NMeArg-Gly-Asp-Mamb); J = D-Val, K = D-NMeArq, L = Gly, M = Asp, R¹ = H, R² = H

The title compound was prepared using the general procedure described for cyclo-(D-Val-NMeArg-Gly-Asp-Mamb) (Example 4). The DCC/DMAP method was used for attachment of Boc-Mamb to the oxime resin. The peptide was prepared on a 0.596 mmol scale to give the protected cyclic peptide (186 mg, 38.6%). The peptide (183 mg) and 0.183 ml of anisole were treated with anhydrous hydrogen fluoride at 0°C for 30 minutes. The crude material was precipitated with ether, redissolved in aqueous acetonitrile, and lyophilized to generate the title compound (145 mg, greater than quantitative yield; calculated as the fluoride salt). Purification was accomplished by reversed-phase HPLC on a preparative Vydac® C18 column (2.5 cm) using a 0.23%/ min gradient of 9 to 22.5% acetonitrile containing 0.1% TFA and then lyophilized to give the TFA salt of the title compound as a fluffy white solid (14.8% recovery, overall yield 5.3%); FAB-MS: [M+H] = 575.31.

### Example 5

### cyclo-(D-Leu-NMeArg-Gly-Asp-Mamb); J = D-Leu, K = NMeArg, L = Gly, M = Asp, R¹ = R² = H

The title compound was prepared using the-general procedure described above for cyclo-(D-Val-NMeArg-Gly-Asp-Mamb). The peptide was prepared on a 0.115 mmol scale to give the protected cyclic peptide (92.4 mg, 98%). The peptide (92.4 mg) and 93 µl of *m*-cresol were treated with anhydrous hydrogen fluoride at 0°C for 20 minutes. The crude material was precipitated with ether, redissolved in aqueous HOAc, and lyophilized to generate the title compound as a pale yellow solid (45.7 mg, 63%; calculated as the acetate salt). Purification was accomplished by reversed-phase HPLC on a preparative Vydac® C18 column (2.5 cm) using a 0.23%/ min gradient of 7 to 21% acetonitrile containing 0.1%
TFA and then lyophilized to give the TFA salt of the title compound as a fluffy white solid (29% recovery, overall yield 16.5%);FAB-MS: [M+H] = = 589.48.

### Example 7

### cyclo-(D-Nle-NMeArg-Gly-Asp-Mamb); J = D-Nle, K = NMeArg, L = Gly, M = Asp, R¹ = H, R² = H

The title compound was prepared using the general procedure described for cyclo-(D-Val- NMeArg-Gly-Asp-Mamb) (Example 4). The DCC/DMAP method was used for attachment of Boc-Mamb to the oxime resin. The peptide was prepared on a 0.586 mmol scale to give the protected cyclic peptide (305 mg, 63.3%). The peptide (295 mg) and 0.295 ml of anisole were treated with anhydrous hydrogen fluoride at 0°C for 30 minutes. The crude material was precipitated with ether, redissolved in aqueous acetonitrile, and lyophilized to generate the title compound (207 mg, 95.4%; calculated as the fluoride salt). Purification was accomplished by reversed-phase HPLC on a preparative Vydac® C18 column (2.5 cm) using a 0.23%/ min gradient of 5.4 to 18% acetonitrile containing 0.1% TFA and then lyophilized to give the TFA salt of the title compound as a fluffy white solid (44% recovery, overall yield
22.9%); FAB-MS: [M+H] = 589.26.

### Example 11

### cyclo-(D-Phg-NMeArg-Gly-Asp-Mamb); J = D-Phg, K = NMeArg, L = Gly, M = Asp, R¹ = H, R² = H

The title compound was prepared using the general procedure described for cyclo-(D-Val-NMeArg-Gly-Asp-Mamb) (Example 4). The DCC/DMAP method was used for attachment of Boc-Mamb to the oxime resin. The peptide was prepared on a 0.611 mmol scale to give the protected cyclic peptide (296 mg, 57.4%). The peptide (286 mg) and 0,286 ml of anisole were treated with anhydrous hydrogen fluoride at 0°C for 30 minutes. The crude material was precipitated with ether, redissolved in aqueous acetonitrile, and lyophilized to generate the title compound (210 mg, 98.9%; calculated as the fluoride salt). Purification was accomplished by reversed-phase HPLC on a preparative Vydac® C18 column (2.5 cm) using a 0.23%/ min gradient of 5.4 to 18% acetonitrile containing 0.1% TFA and then lyophilized to give the TFA salt of the title compound as a fluffy white solid (24.2% recovery, overall yield 11.9%); FAB-MS: [M+H] = 609.27.

### Example 12

### cyclo-(D-Phe-NMeArg-Gly-Asp-Mamb); J = D-Phe, K = NMeArg, L = Gly, M = Asp, R¹ = H, R² = H

The title compound was prepared using the general procedure described for cyclo-(D-Val-NMeArg-Gly-Asp-Mamb) (Example 4). The DCC/DMAP method was used for attachment of Boc-Mamb to the oxime resin. The peptide was prepared on a 0.611 mmol scale to give the protected cyclic peptide (140 mg, 26.7%). The peptide (135 mg) and 0.135 ml of anisole were treated with anhydrous hydrogen fluoride at 0°C for 30 minutes. The crude material was precipitated with ether, redissolved in aqueous acetonitrile, and lyophilized to generate the title compound (108 mg, greater than quantitative yield; calculated as the fluoride salt). Purification was accomplished by reversed-phase HPLC on a preparative Vydac® C18 column (2.5 cm) using a 0.23%/ min gradient of 7.2 to 22.5% acetonitrile containing 0.1% TFA and then lyophilized to give the TFA salt of the title compound as a fluffy white solid (35% recovery, overall yield 8.7%); FAB-MS: [M+H] = 623.28.

### Example 13f

### cyclo-(D-Lys-NMeArg-Gly-Asp-Mamb); J = D-Lys, K = NMeArg, L = Gly, M = Asp, R¹ = R² = H

The title compound was prepared using the general procedure described above for cyclo-(D-Val-NMeArg-Gly-Asp-Mamb). The DCC/DMAP method was used for attachment of Boc-Mamb to the oxime resin. The peptide was prepared on a 0.586 mmol scale to give the protected cyclic peptide (349 mg, 58.9%). The peptide (334 mg) and 334 µl of anisole were treated with anhydrous hydrogen fluoride at 0°C for 30 minutes. The crude material was precipitated with ether, redissolved in aqueous acetonitrile, and lyophilized to generate the title compound as a pale yellow solid (168 mg, 79.1%; calculated as the difluoride salt). Purification was accomplished by reversed-phase HPLC on a preparative Vydac® C18 column (2.5 cm) using a 0.23%/ min gradient of 5.4 to 14.4% acetonitrile containing 0.1% TFA and then lyophilized to give the TFA salt of the title compound as a fluffy white solid (33.6% recovery, overall yield 12.1%); FAB-MS: [M+H] = 604.32

### Example 13r

### cyclo-(D-Ile-NMeArg-Gly-Asp-Mamb); J = D-Ile, K = NMeArg, L = Gly, M = Asp, R¹ = H, R² = H

The title compound was prepared using the general procedure described for cyclo-(D-Val-NMeArg-Gly-Asp-Mamb) (example 4). The DCC/DMAP method was used for attachment of Boc-Mamb to the oxime resin. The peptide was prepared on a 0.611 mmol scale to give the protected cyclic peptide (349 mg, 69.2%). The peptide (342 mg) and 0.342 ml of anisole were treated with anhydrous hydrogen fluoride at 0°C for 30 minutes. The crude material was precipitated with ether, redissolved in aqueous acetonitrile, and lyophilized to generate the title compound (227 mg, 90%; calculated as the fluoride salt). Purification was accomplished by reversed-phase HPLC on a preparative Vydac® C18 column (2.5 cm) using a 0.23%/ min gradient of 10.8 to 19.8% acetonitrile containing 0.1% TFA and then lyophilized to give the TFA salt of the title compound as a fluffy white solid (22.5% recovery, overall yield 12.1%); FAB-MS : [M+H] = 589.34.

### Example 18

### cyclo-(NMeGly-NMeArg-Gly-Asp-Mamb); J = NmeGly, K = NMeArg, L = Gly, M = Asp, R¹ = R² = H

The title compound was prepared using the general procedure described above for cyclo-(D-Val-NMeArg-Gly-Asp-Mamb). The DCC/DMAP method was used for attachment of Boc-Mamb to the oxime resin. The peptide was prepared on a 0.43 mmol scale to give the protected cyclic peptide (205 mg, 60%). The peptide (200 mg) and 200 µl of *m*-cresol were treated with anhydrous hydrogen fluoride at 0°C for 30 minutes. The crude material was precipitated with ether, redissolved in aqueous HOAc, and lyophilized to generate (18) as a pale yellow solid (148 mg, 97%; calculated as the acetate salt). Purification was accomplished by reversed-phase HPLC on a preparative Vydac® C18 column (2.5 cm) using a 0.23%/ min gradient of 7 to 22% acetonitrile containing 0.1% TFA and then lyophilized to give the TFA salt of (18) as a fluffy white solid (14.7% recovery, overall yield 7.9%); FAB-MS: [M+H] = 547.34.

### Example 24

### cyclo-(Pro-NmeArg-Gly-Asp-Mamb); J = Pro, K = NMeArg, L = Gly, M = Asp, R¹ = R² = H

The title compound was prepared using the general procedure described above for cyclo-(D-Val-NMeArg-Gly-Asp-Mamb). The DCC/DMAP method was used for attachment of Boc-Mamb to the oxime resin. The peptide was prepared on a 0.43 mmol scale to give the protected cyclic peptide (170 mg, 48.8%). The peptide (164 mg) and 164 µl of *m*-cresol were treated with anhydrous hydrogen fluoride at 0°C for 30 minutes. The crude material was precipitated with ether, redissolved in aqueous HOAc, and lyophilized to generate (24) as a pale yellow solid (101 mg, 79% ; calculated as the acetate salt). Purification was accomplished by reversed-phase HPLC on a preparative Vydac® C18 column (2.5 cm) using a 0.23%/ min gradient of 7 to 22% acetonitrile-containing 0.1% TFA and then lyophilized to give the TFA salt of (24) as a fluffy white solid (5.8% recovery, overall yield 2.1%);FAB-MS : [M+H] = 573.46.

### Example 25

### cyclo-(D-Pro-NMeArg-Gly-Asp-Mamb); J = D-Pro, K = NMeArg, L = Gly, M = Asp, R¹ = R² = H

The title compound was prepared using the general procedure described above for cyclo-(D-Val-NMeArg-Gly-Asp-Mamb). The DCC/DMAP method was used for attachment of Boc-Mamb to the oxime resin. The peptide was prepared on a 0.43 mmol scale to give the protected cyclic peptide (211mg, 60.8%). The peptide (200 mg) and 200 µl of *m*-cresol were treated with anhydrous hydrogen fluoride at 0°C for 30 minutes. The crude material was precipitated with ether, redissolved in aqueous HOAc, and lyophilized to generate (25) as a pale yellow solid (145 mg, 93.3%; calculated as the acetate salt).
Purification was accomplished by reversed-phase HPLC on a preparative Vydac® C18 column (2.5 cm) using a 0.23%/ min gradient of 7 to 22% acetonitrile containing 0.1% TFA and then lyophilized to give the TFA salt of (25) as a fluffy white solid (6.4% recovery, overall yield 3.3%); FAB-MS: [M+H] = = 573.35.

### Example 28c

### cyclo-(β-Ala-NMeArg-Gly-Asp-Mamb); J = β-Ala, K = NMeArg, L = Gly, M = Asp, R¹ = R² = H

The title compound was prepared using the general procedure described above for cyclo-(D-Val-NMeArg-Gly-Asp-Mamb). The DCC/DMAP method was used for attachment of Boc-Mamb to the oxime resin. The peptide was prepared on a 0.586 mmol scale to give the protected cyclic peptide (264 mg, 5*i*.5%). The peptide (258 mg) and 258 µl of anisole were treated with anhydrous hydrogen fluoride at 0°C for 30 minutes. The crude material was precipitated with ether, redissolved in aqueous acetonitrile, and lyophilized to generate the title compound as a pale yellow solid (231 mg, greater than quantitative yield; calculated as the fluoride salt). Purification was accomplished by reversed-phase HPLC on a preparative Vydac® C18 column (2.5 cm) using a 0.23%/ min gradient of 5.4 to 14.4% acetonitrile containing 0.1% TFA and then lyophilized to give the TFA salt of the title compound as a fluffy white solid (53.2% recovery, overall yield 32.5%); FAB-MS: [M+H] = 547.28.

### Example 28f

### cyclo-(D-Tyr-NMeArg-Gly-Asp-Mamb); J = D-Tyr, K = NMeArg, L = Gly, M = Asp, R¹ = H, R² = H

The title compound was prepared using the general procedure described for cyclo-(D-Val-NMeArg-Gly-Asp-Mamb) (Example 4). The DCC/DMAP method was used for attachment of Boc-Mamb to the oxime resin. The peptide was prepared on a 0.313 mmol scale to give the protected cyclic peptide (342 mg, greater than quantitative yield). The peptide (331 mg) and 0.330 ml of anisole were treated with anhydrous hydrogen fluoride at 0°C for 30 minutes. The crude material was precipitated with ether, redissolved in aqueous acetonitrile, and lyophilized to generate the title compound (218 mg, greater than quantitative yield; calculated as the fluoride salt). Purification was accomplished by reversed-phase HPLC on a preparative Vydac® C18 column (2.5 cm) using a 0.23%/ min gradient of 9 to 18% acetonitrile containing 0.1% TFA and then lyophilized to give the TFA salt of the title compound as a fluffy white solid (11.3% recovery, overall yield 10.8%) ; FAB-MS: [M+H] = 639.54.

### Example 30

### cyclo-(D-Val-NMeAmf-Gly-Asp-Mamb); J = D-Val, K = NMeAmf, L = Gly, M = Asp, R¹ = R² = H

The title compound was prepared using the general procedure described for cyclo-(D-Val-NMeArg-Gly-Asp-Mamb) (Example 4). The DCC/DMAP method was used for attachment of Boc-Mamb to the oxime resin. The peptide was prepared on a 0.586 mmol scale to give the protected cyclic peptide (189 mg, 39.9%). The peptide (189 mg) and 0.189 ml of anisole were treated with anhydrous hydrogen fluoride at 0°C for 30 minutes. The crude material was precipitated with ether, redissolved in aqueous acetonitrile, and lyophilized to generate the title compound (212 mg, greater than quantitative yield; calculated as the fluoride salt). Purification was accomplished by reversed-phase HPLC on a preparative Vydac® C18 column (2.5 cm) using a 0.23%/ min gradient of 10.8 to 22.5% acetonitrile containing 0.1% TFA and then lyophilized to give the TFA salt of the title compound as a fluffy white solid (8.1% recovery, overall yield 4.1%); FAB-MS: [M+H] = 595.23.

### Example 38a

The title compound may be synthesized using procedures described in Mosher et all Tett. Lett. 29: 3183-3186, and as shown schematically below. This same procedure is a generally useful method for converting a primary amine into a guanidine functionality.

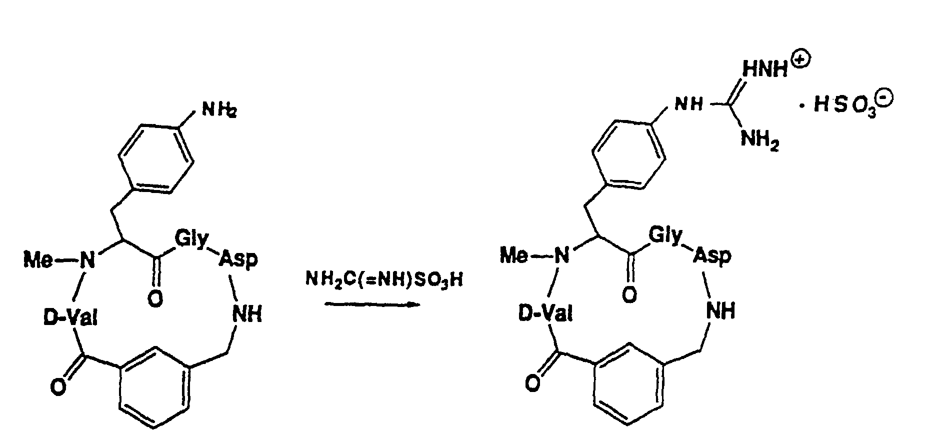

### Examples 32-35

The synthesis of Examples 32-35 is shown schematically below.

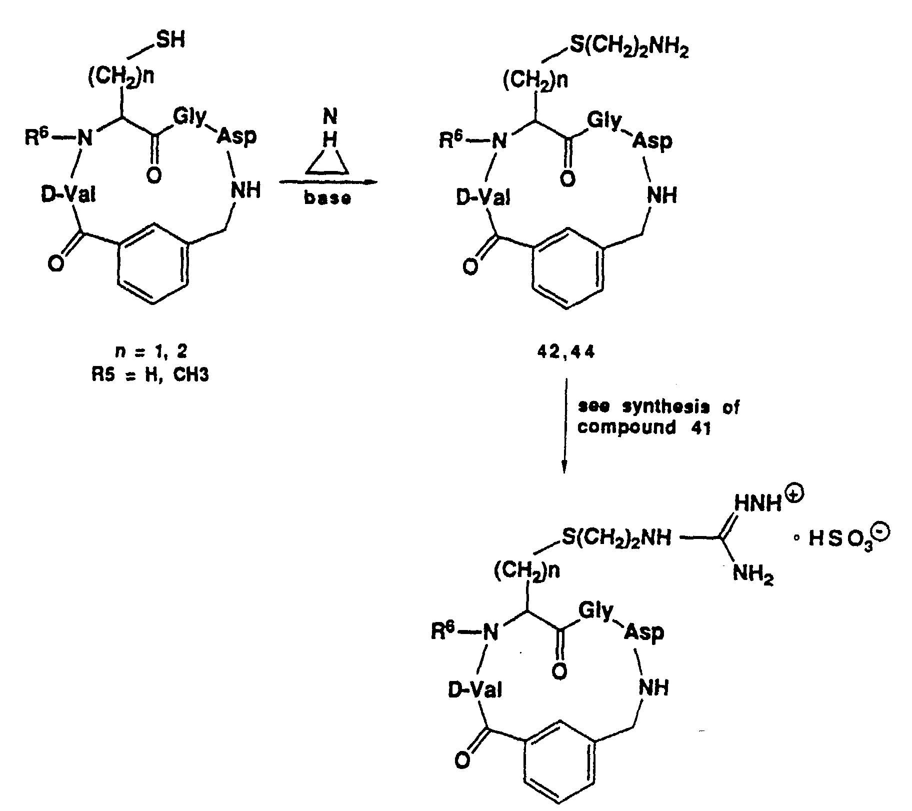

### Examples 36 and 37

Examples 36 and 37 are prepared according to standard procedures, for example, as described in Garigipati, *Tett. Lett.* (1990) 31: 1969-1972 and in Canadian Patent 2008311, as is shown schematically below. The aspartic acid group may be protected (e.g., with a phenacyl protection group) to avoid side reactions.

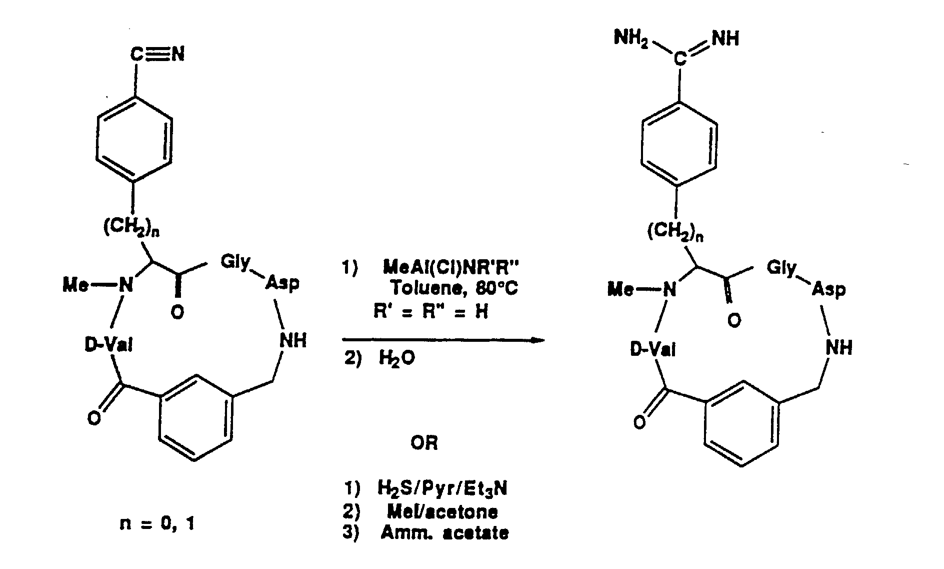

### Example 40

### cyclo-(D-Val-NMeArg-β-Ala-Asp-Mamb); J = D-Val, K = NMeArg, L = β-Ala, M = Asp, R¹ = R² = H

The title compound was prepared using the general procedure described above for cyclo-(D-Val-NMeArg-Gly-Asp-Mamb). The DCC/DMAP method was used for attachment of Boc-Mamb to the oxime resin. The peptide was prepared on a 0.586 mmol scale to give the protected cyclic peptide (227 mg, 46.9%). The peptide (219 mg) and 219 µl of anisole were treated with anhydrous hydrogen fluoride at 0°C for 30 minutes. The crude material was precipitated with ether, redissolved in aqueous acetonitrile, and lyophilized to generate (54) as a pale yellow solid (150 mg, 93.2%; calculated as the fluoride salt). Purification was accomplished by reversed-phase HPLC on a preparative Vydac® C18 column (2.5 cm) using a 0.23%/ min gradient of 7.2 to 16.2% acetonitrile containing 0.1% TFA and then lyophilized to give the TFA salt of (54) as a fluffy white solid (43.6% recovery, overall yield 16.5%); FAB-MS: [M+H] = 589.32.

### Examples 41-45

The synthesis of Examples 41-45 is shown schematically below.

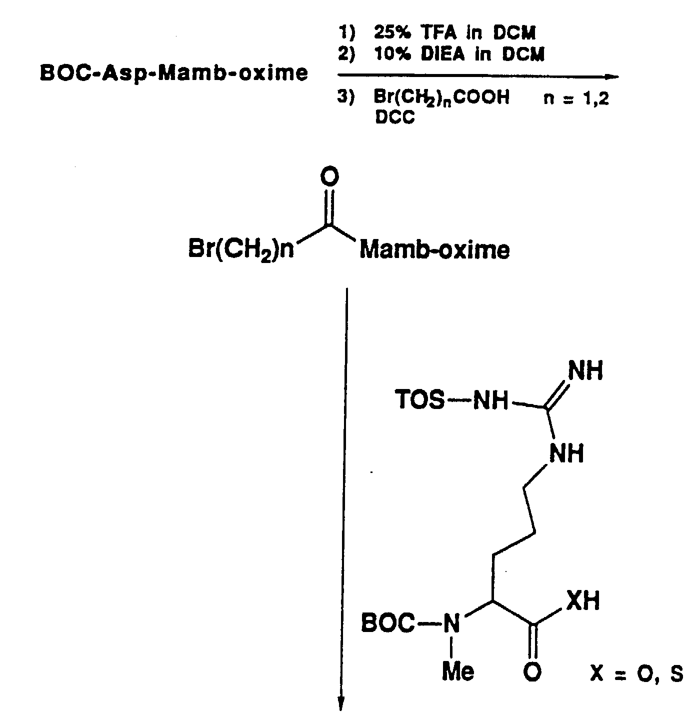

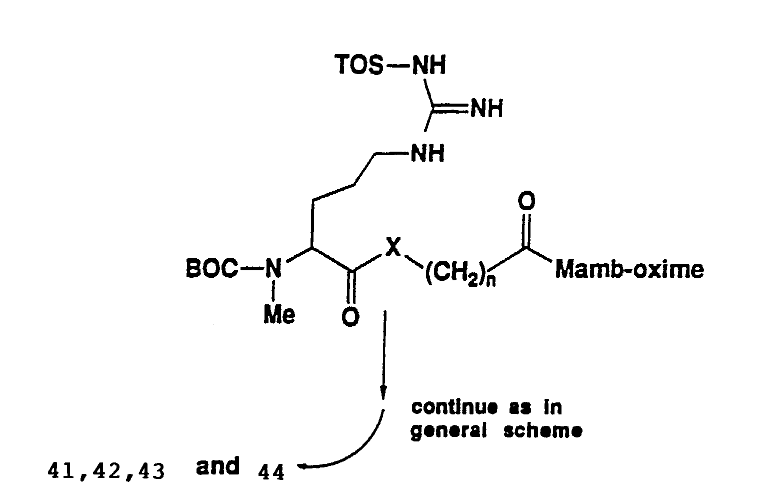

### Example 44c

### cyclo-(D-Val-NMeArg-L-Ala-Asp-Mamb); J = D-Val, K = NMeArg, L = L-Ala, M = Asp, R¹ = H, R² = H

The title compound was prepared using the general procedure described for cyclo-(D-Val-NMeArg-Gly-Asp-Mamb) (Example 4). The DCC/DMAP method was used for attachment of Boc-Mamb to the oxime resin. The peptide was prepared on a 0.611 mmol scale to give the protected cyclic peptide (375 mg, 74.6%). The peptide (360 mg) and 0.360 ml of anisole were treated with anhydrous hydrogen fluoride at 0°C for 30 minutes. The crude material was precipitated with ether, redissolved in aqueous acetonitrile, and lyophilized to generate the title compound (220 mg, 83%; calculated as the fluoride salt). Purification was accomplished by reversedphase HPLC on a preparative Vydac® C18 column (2.5 cm) using a 0.23%/ min gradient of 9 to 18% acetonitrile containing 0.1% TFA and then lyophilized to give the TFA salt of the title compound as a fluffy white solid (19.9% recovery, overall yield 10.6%); FAB-MS: [M+H] = 589.31.

### Example 45 and 45a

### cyclo-(D-Val-NMeArg-Gly-α-MeAsp-Mamb); J is D-Val; K is NMeArg; L is Gly; M is α-MeAsp; R¹ = R² = H

The title compound was prepared using the general procedure described for cyclo-(D-Val-NMeArg-Gly-Asp-Mamb). The DCC/DMAP method was used for attachment of Boc-Mamb to the oxime resin. The peptide was prepared on a 0.794 mmol scale to give the protected cyclic peptide (237 mg, 36.1%). The peptide (237 mg) and 0.237 ml of anisole were treated with anhydrous hydrogen fluoride at 0°C for 30 minutes. The crude material was precipitated with ether, redissolved in aqueous acetonitrile, and lyophilized to generate the title compound (165 mg, 94.3%; calculated as the fluoride salt). Purification was accomplished by reversed-phase HPLC on a preparative Vydac® C18 column (2.5 cm) using a 0.23%/ min gradient of 9 to 18% acetonitrile containing 0.1% TFA and then lyophilized to give the TFA salt of the title compound as a fluffy white solid; isomer #1 (8.36% recovery, overall yield 2.5%); FAB-MS: [M+H] = 589.29; isomer #2 (9.16% recovery, overall yield 2.7%); FAB-MS: [M+H] = 589.27.

### Example 46 and 46a

### cyclo-(D-Val-NMeArg-Gly-β-MeAsp-Mamb); J = D-Val, K = NMeArg, L = Gly, M = β-MeAsp, R¹ = H, R² = H

The title compound was prepared using the general procedure described for cyclo-(D-Val-NMeArg-Gly-Asp-Mamb) (Example 4). The DCC/DMAP method was used for attachment of Boc-Mamb to the oxime resin. The peptide was prepared on a 0.611 mmol scale to give the protected cyclic peptide (201 mg, 40.0%). The peptide (200 mg) and 0.200 ml of anisole were treated with anhydrous hydrogen fluoride at 0°C for 30 minutes. The crude material was precipitated with ether, redissolved in aqueous acetonitrile, and lyophilized to generate the title compound (162 mg, greater than quantitative yield; calculated as the fluoride salt). Purification was accomplished by reversed-phase HPLC on a preparative Vydac® C18 column (2.5 cm) using a 0.23%/ min gradient of 9 to 18% acetonitrile containing 0.1% TFA and then lyophilized to give the TFA salt of the title compound as a fluffy white solid; isomer #1 (12.7% recovery, overall yield 4.8%); FAB-MS : [M+H] = 589.43; isomer #2 (13.9% recovery, overall yield 5.3%); FAB-MS: [M+H] = 589.45.

### Example 46b

### cyclo-(D-Val-NMeArg-Gly-NMeAsp-Mamb); J = D-Val, K = NMeArg, L = Gly, M = NMeAsp, R¹ = H, R² = H

The title compound was prepared using the general procedure described for cyclo-(D-Val-NMeArg-Gly-Asp-Mamb) (Example 4). The DCC/DMAP method was used for attachment of Boc-Mamb to the oxime resin. The peptide was prepared on a 0.611 mmol scale to give the protected cyclic peptide (232 mg, 46.1%). The peptide (225 mg) and 0.225 ml of anisole were treated with anhydrous hydrogen fluoride at 0°C for 30 minutes. The crude material was precipitated with ether, redissolved in aqueous acetonitrile, and lyophilized to generate the title compound (160 mg, 96.4%; calculated as the fluoride salt). Purification was accomplished by reversed-phase HPLC on a preparative Vydac® C18 column (2.5 cm) using a 0.23%/ min gradient of 9 to 18% acetonitrile containing 0.1% TFA and then lyophilized to give the TFA salt of the title compound as a fluffy white solid (28.2% recovery, overall yield 10.9%); FAB-MS : [M+H] = 589.42.

### Example 46c

### cyclo-(D-Val-NMeArg-Gly-D-Asp-Mamb); J = D-Val, K = NMeArg, L = Gly, M = D-Asp, R¹ = H, R² = H

The title compound was prepared using the general procedure described above for cyclo-(D-Val-NMeArg-Gly-Asp-Mamb). The DCC/DMAP method was used for attachment of Boc-Mamb to the oxime resin. The peptide was prepared on a 0.611 mmol scale to give the protected cyclic peptide (257 mg, 51.9%). The peptide (250 mg) and 0.250 ml of anisole were treated with anhydrous hydrogen fluoride at 0°C for 30 minutes. The crude material was precipitated with ether, redissolved in aqueous acetonitrile, and lyophilized to generate the title compound (192 mg, greater than quantitative yield; calculated as the fluoride salt). Purification was accomplished by reversed-phase HPLC on a preparative Vydac® C18 column (2.5 cm) using a 0.23%/ min gradient of 9 to 18% acetonitrile containing 0.1% TFA and then lyophilized to give the TFA salt of the title compound as a fluffy white solid (44.4% recovery, overall yield 20.7%); FAB-MS: [M+H] = 575.42.

### Examples 48 and 48a

### cyclo-(D-Val-NMeArg-Gly-D-Asp-MeMAMB); J = D-Val, K = NMeArg, L = Gly, M = D-Asp, R¹ = CH₃, R² = H

MeMAMB linker was prepared via Scheme 4 (described earlier). The title compound was made by following the solution phase synthetic route to attach MeMAMB to the tripeptide. Cyclization gave the protected cyclic peptide. Deprotection was achieved by treatment of the peptide (390 mg) and anisol (0.390 ml) with anhydrous HF at 0°C for 30 minutes. The crude material was precipitated with ether, redissolved in 10% aqueous acetic acid, and lyophilized to give a mixture of the two isomers (330 mg; greater than quantitative yield; calculated as the acetate salt). Purification and the separation of the isomers was accomplished by Reverse-Phase HPLC on a preparative Vydac® C18 column (2.5 cm) using a 0.48%/min gradient of 7 to 23% acetonitrile containing 0.1% TFA. Fractions collected at Rf 24.1 min and 26.8 min were lyophilized to give the TFA salts of the isomers I and 2 respectively. FAB-MS (Isomer 1): [M+H] = 589.31; FAB-MS (isomer 2): [M+H] = 589.31.

### Examples 56 and 56a

### cyclo-(D-Val-NMeArg-Gly-D-Asp-PhMAMB); J = D-Val, K = NMeArg, L = Gly, M = D-Asp, R¹ = Ph, R² = H

PhMAMB linker was prepared via Scheme 4 (described earlier). The title compound was made by following the solution phase synthetic route to attach PhMAMB to the tripeptide. Cyclization gave the protected cyclic peptide. Deprotection was achieved by treatment of the peptide (470 mg) and anisol (0.470 ml) with anhydrous HF at 0°C for 30 minutes. The crude material was precipitated with ether, redissolved in 10% aqueous acetic acid, and lyophilized to give a mixture of the two isomers (310 mg; 82.4% overall recovery). Purification and the separation of the isomers was accomplished by Reverse-Phase HPLC on a preparative Vydac® C18 column (2.5 cm) using a 0.55%/min gradient of 18 to 36% acetonitrile containing 0.1% TFA. Fractions collected at Rf 22 min and 24.6 min were lyophilized to give the TFA salts of the isomers 1 and 2 respectively.
FAB-MS (Isomer 1): [M+H] = 651.33; FAB-MS (isomer 2): [M+H] = 651.33.

### Example 59

### cyclo-(D-Val-NMeArg-Gly-Asp-NMeMamb); J = D-Val, K = NMeArg, L = Gly, M = Asp, R¹ = H, R² = CH₃

The title compound was prepared using the general procedure described for cyclo-(D-Val-NMeArg-Gly-Asp-Mamb) (Example 4). The DCC/DMAP method was used for attachment of Boc-NMeMamb to the oxime resin. The peptide was prepared on a 0.456 mmol scale to give the protected cyclic peptide (406 mg, greater than quantitative yield). The peptide (364 mg) and 0.364 ml of anisole were treated with anhydrous hydrogen fluoride at 0°C for 30 minutes. The crude material was precipitated with ether, redissolved in aqueous acetonitrile, and lyophilized to generate the title compound (251 mg, 93.5%; calculated as the fluoride salt). Purification was accomplished by reversed-phase HPLC on a preparative Vydac® C18 column (2.5 cm) using a 0.23%/ min gradient of 9 to 18% acetonitrile containing 0.1% TFA and then lyophilized to give the TFA salt of the title compound as a fluffy white solid (34.2% recovery, overall yield 29.9%); FAB-MS: [M+H] = 589.33.

### Example 66,67

### cyclo-(D-Val-NMeArg-Gly-Asp-4-aminomethylbenzoic acid) ;

The title compound was prepared using the general procedure described above for cyclo-(D-Val-NMeArg-Gly-Asp-Mamb). The DCC/DMAP method was used for attachment of Boc-4-aminomethylbenzoic acid to the oxime resin. The peptide was prepared on a 0.43 mmol scale to give the protected cyclic peptide (212mg, 60.8%). The peptide (200 mg) and 200 µl of m-cresol were treated with anhydrous hydrogen fluoride at 0°C for 30 minutes. The crude material was precipitated with ether, redissolved in aqueous HOAc, and lyophilized to generate the crude peptide as a pale yellow solid (152 mg, 97% ; calculated as the acetate salt). Purification was accomplished by reversed-phase HPLC on a preparative Vydac® C18 column (2.5 cm) using a 0.23%/ min gradient of 7 to 22% acetonitrile containing 0.1% TFA. Two peaks were isolated to give isomer #1 (87) (17.1% recovery, overall yield 9.3%) and isomer #2 (88) (13.4% recovery, overall yield 7.3%); FAB-MS: [M+H] = 575.41 (isomer #1; 87); 575.44 (isomer #2; 88).

### Example 68a and 68b

### cyclo-(D-Val-NMeArg-Gly-Asp-aminotetralincarboxylic acid); J = D-Val, K = NMeArg, L = Gly, M = Asp, R¹ = R² = H

The title compound was prepared using the general procedure described for cyclo-(D-Val-NMeArg-Gly-Asp-Mamb) (Example 4). The DCC/DMAP method was used for attachment of Boc-aminotetralin-carboxylic acid to the oxime resin. The peptide was prepared on a 0.164 mmol scale to give the protected cyclic peptide (69 mg, 49.3%). The peptide (69 mg) and 0.069 ml of anisole were treated with anhydrous hydrogen fluoride at 0°C for 30 minutes. The crude material was precipitated with ether, redissolved in aqueous acetonitrile, and lyophilized to generate the title compound (59.7 mg, greater than quantitative yield; calculated as the fluoride salt). Purification was accomplished by reversed-phase HPLC on a preparative Vydac® C18 column (2.5 cm) using a 0.23%/ min gradient of 16.2 to 27% acetonitrile containing 0.1% TFA and then lyophilized to give the TFA salt of the title compound as a fluffy white solid. Two isomers were obtained; isomer #1 (12.5% recovery, overall yield 6.2%, FAB-MS : [M+H] = 615.34; isomer #2 (18.6% recovery, overall yield 9.3%, FAB-MS: [M+H] = 615.35.

### Example 68c

### cyclo-(D-Val-NMeArg-Gly-Asp-aminomethylnaphthoic acid); J = D-Val, K = NMeArg,L = Gly, M = Asp, R¹ = H, R² = H

The title compound was prepared using the general procedure described for cyclo-(D-Val-NMeArg-Gly-Asp-Mamb) (Example 4). The DCC/DMAP method was used for attachment of Boc-aminomethylnaphthoic acid to the oxime resin. The peptide was prepared on a 0.737 mmol scale to give the protected cyclic peptide (463 mg, 73.1%). The peptide (463 mg) and 0.463 ml of anisole were treated with anhydrous hydrogen fluoride at 0°C for 20 minutes. The crude material was precipitated with ether, redissolved in aqueous acetonitrile, and lyophilized to generate the title compound (349 mg, greater than quantitative yield; calculated as the fluoride salt). Purification was accomplished by reversed-phase HPLC on a preparative Vydac® C18 column (2.5 cm) using a 0.45%/ min gradient of 4.5 to 22.5% acetonitrile containing 0.1% TFA and then lyophilized to give the TFA salt of the title compound as a fluffy white solid (12.1% recovery, overall yield 7.8%); FAB-MS : [M+H] = 625.32.

### Example 68d

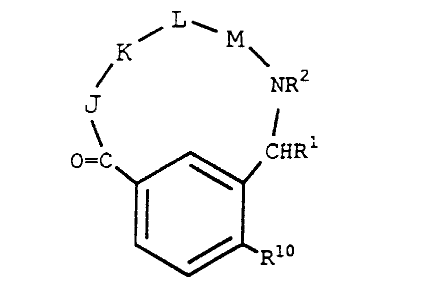

cyclo-(D-Abu-NMeArg-Gly-Asp-iodo-Mamb); J = D-Abu, K = NMeArg, L = Gly, M = Asp, R¹ = R² = H, R¹⁰ = I

The title compound was prepared using the general procedure described above for cyclo-(D-Val-NMeArg-Gly-Asp-Mamb) (Example 4). The DCC/DMAP method was used for attachment of Boc-iodo-Mamb to the oxime resin. The peptide was prepared on a 3.53 mmol scale to give the protected cyclic peptide (4.07 g, greater than quantitative yield). The peptide (4.07 g) and 4.0 ml of anisole were treated with anhydrous hydrogen fluoride at 0°C for 30 minutes. The crude material was precipitated with ether, redissolved in aqueous acetic acid, and lyophilized to generate the title compound (2.97 g, greater than quantitative yield; calculated as the acetate salt). Purification was accomplished by reversed-phase HPLC on a preparative Vydac® C18 column (2.5 cm) using a 0.16%/ min gradient of 16.2 to 22.5% acetonitrile containing 0.1% TFA and then lyophilized to give the TFA salt of the title compound as a fluffy white solid (28.7% recovery, overall yield 30.2%); FAB-MS: [M+H] = 687.33.

### Example 68e

### cyclo-(D-Abu-di-NMeOrn-Gly-Asp-Mamb); J = D-Abu, K = di-NMeOrn, L = Gly, M = Asp, R¹ = R² = H

The title compound was prepared using the general procedure described for cyclo-(D-Val-NMeArg-Gly-Asp-Mamb) (Example 4). The DCC/DMAP method was used for attachment of Boc-Mamb to the oxime resin. The peptide was prepared on a 0.498 mmol scale to give the protected cyclic peptide (150 mg, 39.3%). The peptide (150 mg) and 0.150 ml of anisole were treated with anhydrous hydrogen fluoride at 0°C for 30 minutes. The crude material was precipitated with ether, redissolved in aqueous acetonitrile, and lyophilized to generate the title compound (93 mg, 86%; calculated as the fluoride salt). Purification was accomplished by reversed-phase HPLC on a preparative Vydac® C18 column (2.5 cm) using a 0.45%/ min gradient of 3.6 to 18% acetonitrile containing 0.1% TFA and then lyophilized to give the TFA salt of the title compound as a fluffy white solid (49.3% recovery, overall yield 14.2%); FAB-MS: [M+H] = 533.34.

### Example 69

### cyclo-(D-Val-NMeArg-Gly-Asp-2-aminomethylphenylacetic acid)

The title compound was prepare by a modification of the general solution-phase chemistry route. This approach employed an amino acid succinimide ester coupling to the aromatic linker, and the dinitrobenzophenone oxime as shown schematically below in the Scheme below (n = 1).

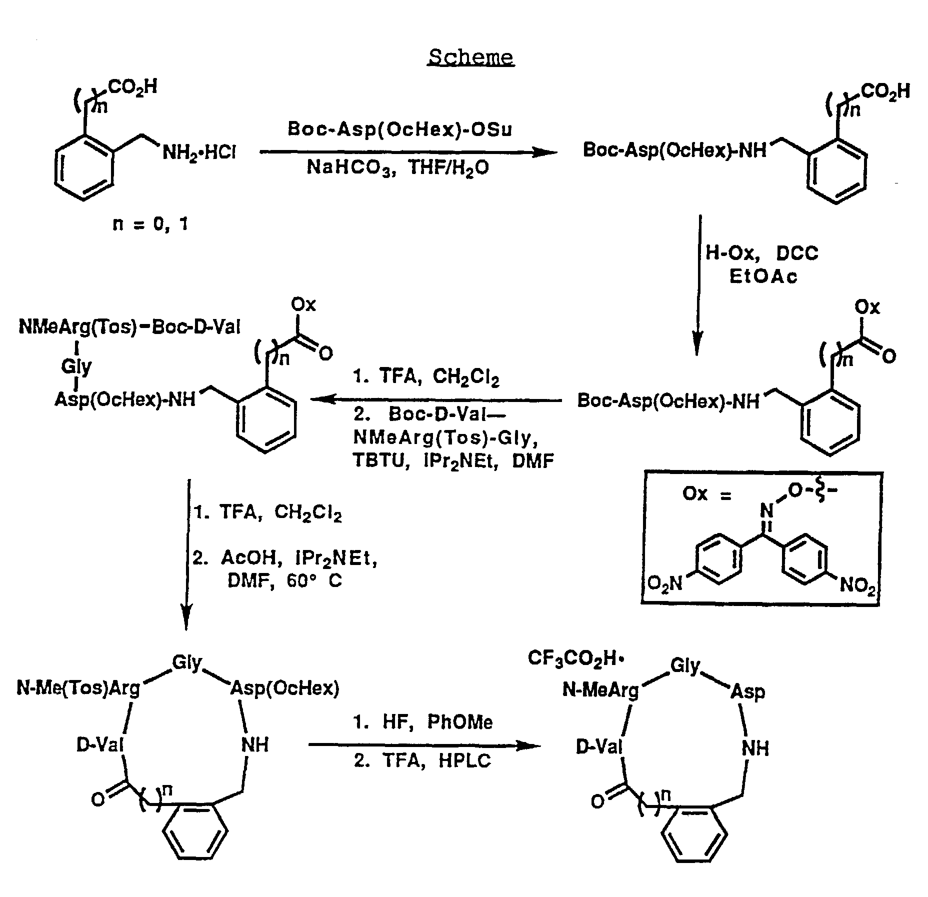

### Boc-Asp (OcHex)-2-aminomethylphenylacetic Acid

To a suspension of 2-aminomethylphenylacetic acid•HCl (4.0 g, 20 mmol) in H₂O (20 ml) was added NaHCO₃ (5.0 g, 60 mmol), followed by a solution of Boc-Asp(OcHex)-OSu (7.5 g, 18 mmol) in THF (20 ml). The reaction mixture was stirred at room temperature for 3 hours, filtered, diluted with H₂O, acidified with 1N HCl, and extracted with ethyl acetate. The extracts were washed with H₂O, brine, dried over anhydrous magnesium sulfate, and evaporated to dryness under reduced pressure. This material was triturated with ether to provide the title compound (7.0 g, 83%) as a white powder. ¹H NMR (D₆-DMSO) 12.40 (br s, 1H), 8.30 (br t, 1H), 7.20 (m, 5H), 4.65 (m, 1H), 4.35 (q, 1H), 4.25 (m, 2H), 3.65 (s, 2H), 2.70 (dd, 1H), 2.55 (dd, 1H), 1.70 (m, 4H), 1.40 (s, 9H), 1.35 (m, 6H).

### 4,4'-Dinitrobenzophenone Oxime

The title compound was prepared by modification of procedures previously reported in the literature (Chapman and Fidler (1936) *J. chem. Soc*, 448; Kulin and Leffek (1973) *Can. J. chem.,* 51: 687). A solution of chromic anhydride (20 g, 200 mmol) in 125 ml of H₂O was added dropwise over 4 hours, to a suspension of bis(4-nitrophenyl)methane (25 g, 97 mmol) in 300 ml of acetic acid heated to reflux. The reaction mixture was heated at reflux for 1 hour, cooled to room temperature, and poured into water. The solid was collected by filtration, washed with H20, 5% sodium bicarbonate, H₂O, and air-dryed to provide a 1:1 mixture of bis(4-nitrophenyl)methane/4,4'-dinitrobenzophenone via ¹H NMR. This material was oxidized with a second portion of chromic anhydride (20 g, 200 mmol), followed by an identical work-up procedure to provide the crude product. Trituration with 200 ml of benzene heated to reflux for 16 hours provided 4,4'-dinitrobenzophenone (20.8 g, 79%) as a yellow powder.

A solution of hydroxylamine hydrochloride (10.2 g, 147 mmol) was added to a suspension of 4,4'-dinitrobenzophenone (19 g, 70 mmol) in 100 ml of ethanol. The reaction mixture was heated to reflux for 2 hours, cooled to room temperature, and the solid collected by filtration. Recrystallization from ethanol provided the title compound (14.0 g, 70%) as pale yellow crystals. mp 194°C; ¹H NMR (D₆-DMSO) 12.25 (s, 1H), 8.35 (d, 2H), 8.20 (d, 2H), 7.60 (d, 4H).

### 4,4'-Dinitrobenzophenone Oxime Boc-Asp(OcHex)-2-aminomethylphenylacetate

To an ice-cooled solution of Boc-Asp(OcHex)-2-aminomethylphenylacetic acid (3.5 g, 7.6 mmol) and 4,4'-dinitrobenzophenone oxime (2.2 g, 7.5 mmol) in 50 ml of ethyl acetate and 5 ml of DMF was added DCC (1.6 g, 7.8 mmol). The reaction mixture was stirred at room temperature for 8 hours, filtered, diluted with ethyl acetate, washed with saturated sodium bicarbonate solution, H₂O, brine, dried over anhydrous magnesium sulfate, and evaporated to dryness under reduced pressure. This material was purified by column chromatography on silica gel (EM Science, 230-400 mesh) using 10:1 dichloromethane/ethyl acetate to give the title compound (4.3 g, 78%) as pale yellow crystals. ¹H NMR (D₆-DMSO) 8.30 (dd, 5H), 7.80 (d, 2H), 7.65 (d, 2H), 7.15 (m, 5H), 4.65 (m, 1H), 4.35 (q, 1H), 4.15 (m, 2H), 3.90 (s, 2H), 2.70 (dd, 1H), 2.50 (dd, 1H), 1.70 (m, 4H), 1.40 (s, 9H), 1.35 (m, 6H).

### 4,4'-Dinitrobenzophenone Oxime Boc-D-Val-NMeArg(Tos)-Gly-Asp(OcHex)-2-aminomethylphenylacetate

To a solution of 4,4'-dinitrobenzophenone oxime Boc-Asp (OcHex)-2-aminomethylphenylacetate (1.5 g, 2 mmol) in 4 ml of dichloromethane was added 2 ml of trifluoroacetic acid. The reaction mixture was stirred at room temperature for 1 hour, diluted with dichloromethane, and evaporated to dryness under reduced pressure. The oily residue was concentrated under high vacuum to remove traces of excess trifluoroacetic acid.

To a solution of the crude TFA salt and Boc-D-Val-NMeArg(Tos)-Gly (1.2 g, 2 mmol) in 5 ml of DMF was added TBTU (640 mg, 2 mmol) and DIEA (780 mg, 6 mmol). The reaction mixture was stirred at room temperature for 16 hours, concentrated under high vacuum, diluted with ethyl acetate, washed with 5% citric acid, H₂O, brine, dried over anhydrous magnesium sulfate, and evaporated to dryness under reduced pressure. This material was triturated with ether to provide the title compound (2.3 g, 95%) as a yellow powder. This material was used without further purification.

### cycle-(D-Val-NMeArg(Tos)-Gly-Asp (OcHex)-2-aminomethylphenylacetic acid)

To a solution of 4,4'-dinitrobenzophenone oxime Boc-D-Val-NMeArg (Tos)-Gly-Asp (OcHex)-2-aminomethylphenylacetate (1.2 g, 1 mmol) in 4 ml of dichloromethane was added 2 ml of trifluoroacetic acid. The reaction mixture was stirred at room temperature for 3 hours, diluted with dichloromethane, and evaporated to dryness under reduced pressure. The oily residue was concentrated under high vacuum to remove traces of excess trifluoroacetic acid

To a solution of the crude TFA salt in 100 ml of DMF was added acetic acid (0.50 ml, 8.7 mmol) and DIEA (1.52 ml, 8.7 mmol). The reaction mixture was stirred at 60°C for 3 days, concentrated under high vacuum diluted with ethyl acetate, and the solution allowed to crystallize overnight. Filtration provided the title compound (563 mg, 68%) as a yellow powder. ¹H NMR (D₆-DMSO) 8.70 (d, 1H), 8.40 (br s, 1H), 8.30 (br s, 1H), 8.05 (t, 1H), 7.65 (d, 2H), 7.25 (d, 2H), 7.20 (m, 4H), 7.10 (br d, 1H), 6.80 (br s, 1H), 6.60 (br s, 1H), 5.10 (dd, 1H), 4.65 (m, 1H), 4.55 (m, 1H), 4.40 (m, 2H), 3.85 (m, 2H), 3.65 (d, 1H), 3.45 (m, 2H), 3.05 (m, 2H), 2.80 (s, 3H), 2.80 (m, 1H), 2.60 (dd, 1H), 2.30 (s, 3H), 1.70 (m, 6H), 1.30 (m, 9H), 0.95 (d, 3H), 0.80 (d, 3H); DCI(NH₃)-MS: [M+H] = 825.

### cyclo-(D-Val-NMeArg-Gly-Asp-2-aminomethylphenylacetic acid)

A mixture of 352 mg (0.43 mmol) of cyclo-(D-Val-NMeArg(Tos)-Gly-Asp (OcHex)-2-aminomethylphenylacetic acid) and 352 µl of anisole was treated at 0°C with 5 ml of HF for 20 minutes. The excess HF was removed under reduced pressure, the residue triturated with ether, dissolved in 50% acetonitrile/H₂O, and lyophilized to provide the crude cyclic peptide•HF salt as an off-white powder. Purification was accomplished by reversed-phase HPLC on a preparative Vydac® C18 column (2.5 cm) using a 0.8% / minute gradient of 10 to 38% acetonitrile containing 0.1% trifluoroacetic acid to give the TFA salt of the title compound (225 mg, 75%) as a fluffy white solid; ¹H NMR (D₆-DMSO) 8.70 (d, 1H), 8.35 (d, 1H), 8.20 (t, 1H), 8.00 (t, 1H), 7.45 (t, 1H), 7.20 (m, 3H), 7.10 (m, 1H), 7.00 (br s, 4H), 5.10 (dd, 1H), 4.50 (dt, 1H), 4.40 (m, 2H), 3.85 (dt, 2H), 3.65 (d, 1H), 3.50 (dd, 1H), 3.45 (d, 1H), 3.10 (m, 2H), 2.90 (s, 3H), 2.75 (dd, 1H), 2.55 (dd, 1H), 2.00 (m, 1H), 1.85 (m, 1H), 1.65 (m, 1H), 1.30 (m, 2H), 0.95 (d, 3H), 0.85 (d, 3H); FAB-MS : [M+H] = 589.

### Example 70

### cyclo-(D-Val-NMeArg-Gly-Asp-2-aminomethylbenzoic acid)

The title compound was prepare by the general solution-phase procedure described above for cyclo-(D-Val-NMeArg-Gly-Asp-2-aminomethylphenylacetic acid), and as shown schematically above in the Example 69 Scheme (n = 0). The cyclic peptide (192 mg, 0.24 mmol) was deprotected with excess HF in the presence of anisole as scavenger. Purification was accomplished by reversed-phase HPLC on a preparative Vydac® C18 column (2.5 cm) using a 0.8% / minute gradient of 10 to 38% acetonitrile containing 0.1% trifluoroacetic acid to give the TFA salt of the title compound (20 mg, 12%) as a fluffy white solid; ¹H NMR (D₆-DMSO) 8.75 (d, 1H), 8.50 (d, 1H), 7.65 (t, 1H), 7.60 (t, 1H), 7.50 (m, 2H), 7.40 (m, 3H), 7.00 (br s, 4H), 5.05 (dd, 1H), 4.50 (t, 1H), 4.30 (m, 2H), 4.10 (dd, 1H), 3.70 (m, 2H), 3.15 (q, 2H), 3.05 (s, 3H), 2.80 (dd, 1H), 2.55 (dd, 1H), 2.10 (m, 1H), 1.95 (m, 1H), 1.60 (m, 1H), 1.40 (m, 2H), 1.05 (d, 3H), 0.95 (d, 3H); FAB-MS : [M+H] = 575.

### Example 71

### cyclo-(D-Val-NMeArg-Gly-Asp-3-aminophenylacetic acid)

The title compound was prepare by the general solution-phase procedure described above for cyclo-(D-Val-NMeArg-Gly-Asp-Mamb), and as shown schematically in the Scheme below. The cyclic peptide (360 mg, 0.44 mmol) was deprotected with excess HF in the presence of anisole as scavenger. Purification was accomplished by reversed-phase HPLC on a preparative LiChrospher® RP-18 column (5 cm) using a 2.3% / minute gradient of 22 to 90% acetonitrile containing 0.1% trifluoroacetic acid to give the TFA salt of the title compound (150 mg, 50%) as a fluffy white solid; ¹H NMR (D₆-DMSO) 12.40 (br s, 1H), 8.95 (s, 1H), 8.55 (m, 2H), 8.45 (t, 1H), 7.90 (d, 1H), 7.50 (m, 1H), 7.20 (t, 1H), 7.00 (br s, 4H), 6.90 (m, 2H), 5.15 (dd, 1H), 4.65 (q, 1H), 4.55 (t, 1H), 3.65 (m, 2H), 3.60 (dd, 1H), 3.10 (m, 2H), 2.85 (s, 3H), 2.85 (d, 1H), 2.70 (dd, 2H), 2.00 (m, 2H), 1.75 (m, 1H), 1.35 (m, 2H), 0.90 (d, 3H), 0.85 (d, 3H); FAB-MS: [M+H] = 575.

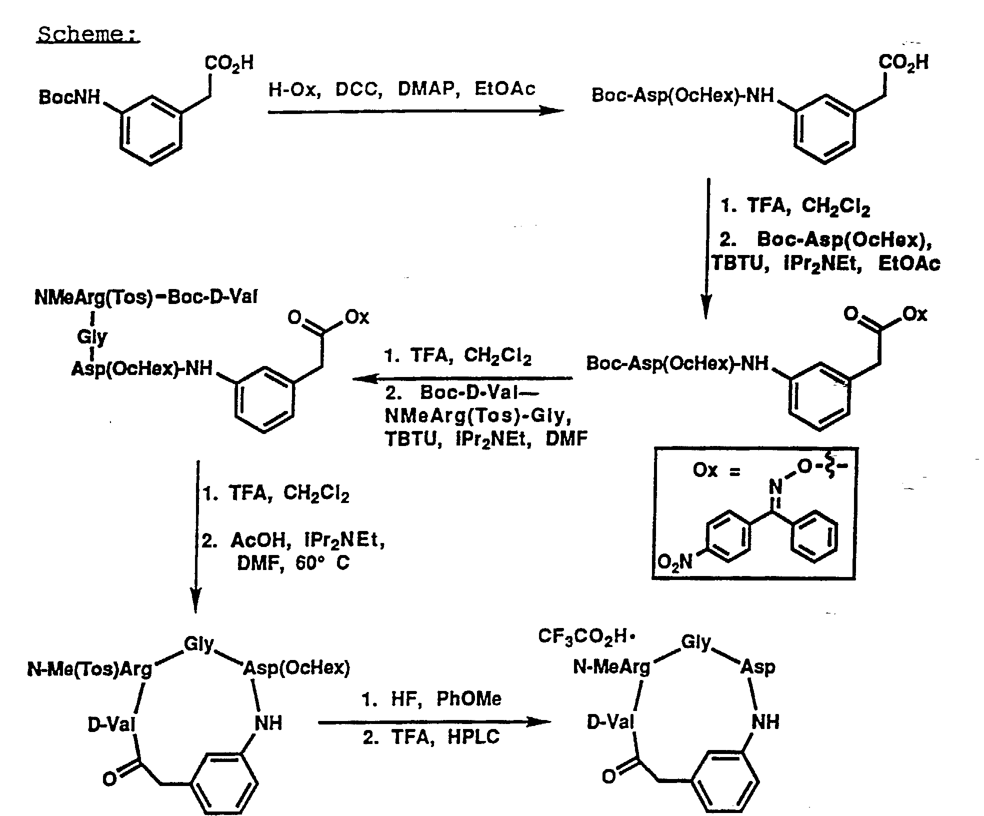

### Example 72

### cycle-(D-Val-NMeArg-Gly-Asp-3-aminomethyl-4-chlorobenzoic acid)

The title compound was prepared by the general solution-phase procedure described above for cyclo-(D-Val-NMeArg-Gly-Asp-Mamb). The cyclic peptide (240 mg, 0.28 mmol) was deprotected with excess HF in the presence of anisole as scavenger. Purification was accomplished by reversed-phase HPLC on a preparative LiChrospher® RP-18 column (5 cm) using a 1.4% / minute gradient of 22 to 90% acetonitrile containing 0.1% trifluoroacetic acid to give the TFA salt of the title compound (80 mg, 39%) as a fluffy white solid; ¹H NMR (D₆-DMSO) 9.00 (d, 1H), 8.50 (d, 1H), 8.45 (t, 1H), 7.60 (d, 2H), 7.45 (s, 1H), 7.45 (d, 2H), 7.00 (br s, 4H), 5.15 (dd, 1H), 4.45 (m, 2H), 4.20 (m, 2H), 4.10 (d, 1H), 3.55 (d, 1H), 3.10 (m, 2H), 2.90 (s, 3H), 2.65 (dd, 1H), 2.50 (m, 1H), 2.05 (m, 2H), 1.50 (m, 1H), 1.30 (m, 2H), 1.05 (d, 3H), 0.85 (d, 3H); FAB-MS : [M+H] = 609.

### Example 73

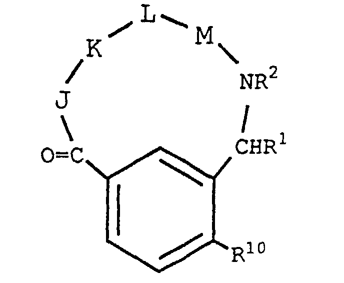

cyclo-(D-Val-NMeArg-Gly-Asp-iodo-Mamb); J = D-Val, K = NMeArg, L = Gly, M = Asp, R¹ = R² = H, R¹⁰ = I

The title compound was prepared using the general procedure described for cyclo-(D-Val-NMeArg-Gly-Asp-Mamb) (Example 4). The DCC/DMAP method was used for attachment of Boc-iodo-Mamb to the oxime resin. The peptide was prepared on a 1.05 mmol scale to give the protected cyclic peptide (460 mg, 46.8%). The peptide (438 mg) and 0.5 ml of anisole were treated with anhydrous hydrogen fluoride at 0°C for 30 minutes. The crude material was precipitated with ether, redissolved in aqueous acetic acid, and lyophilized to generate the title compound (340 mg, 95.6%; calculated as the acetate salt). Purification was accomplished by reversed-phase HPLC on a preparative Vydac® C18 column (2.5 cm) using a 0.23%/ min gradient of 12.6 to 22.5% acetonitrile containing 0.1% TFA and then lyophilized to give the TFA salt of the title compound as a fluffy white solid (39.7% recovery, overall yield 16.6%); FAB-MS : [M+H] = 701.37.

### Example 74

### cyclo-(D-Val-NMeArg-Gly-Asp-3-aminomethyl-4-methoxybenzoic acid)

The title compound was prepare by the general solution-phase procedure described above for cyclo-(D-Val-NMeArg-Gly-Asp-Mamb). The cyclic peptide (600 mg, 0.71 mmol) was deprotected with excess HF in the presence of anisole as scavenger. Purification was accomplished by reversed-phase HPLC on a preparative Vydac® C18 column (2.5 cm) using a 0.33% / minute gradient of 7 to 18% acetonitrile containing 0.1% trifluoroacetic acid to give the TFA salt of the title compound (104 mg, 32%) as a fluffy white solid; ¹H NMR D₆-DMSO) 12.40 (br s, 1H), 8.25 (d, 1H), 8.20 (br s, 1H), 8.00 (br s, 2H), 7.85 (d, 1H), 7.75 (s, 1H), 7.65 (br s, 1H), 7.05 (d, 1H), 7.05 (br s, 4H), 5.00 (dd, 1H), 4.60 (q, 1H), 4.30 (d, 1H), 4.25 (d, 2H), 3.85 (s, 3H), 3.85 (dd, 1H), 3.70 (dd, 1H), 3.10 (q, 2H), 3.00 (s, 3H), 2.70 (m, 1H), 2.50 (m, 1H), 2.10 (m, 1H), 1.90 (m, 1H), 1.65 (m, 1H), 1.35 (m, 2H), 1.00 (d, 3H), 0.90 (d, 3H); FAB-MS: [M+H₂O+H] = 623.

### Example 75

### cyclo-(D-Val-NMeArg-Gly-Asp-3-aminomethyl-4-methylbenzoic acid)

The title compound was prepare by the general solution-phase procedure described above for cyclo-(D-Val-NMeArg-Gly-Asp-Mamb). The cyclic peptide (210 mg, 0.25 mmol) was deprotected with excess HF in the presence of anisole as scavenger. Purification was accomplished by reversed-phase HPLC on a preparative LiChrospher® RP-18 column (5 cm) using a 2.3% / minute gradient of 22 to 90% acetonitrile containing 0.1% trifluoroacetic acid to give the TFA salt of the title compound (75 mg, 42%) as a fluffy white solid; ¹H NMR (D₆-DMSO) 12.30 (br s, 1H), 8.85 (d, 1H), 8.55 (d, 1H), 8.30 (t, 1H), 7.75 (d, 1H), 7.55 (m, 2H), 7.40 (s, 1H), 7.20 (s, 1H), 7.00 (br s, 4H), 5.20 (dd, 1H), 4.55 (q, 1H), 4.45 (dd, 1H), 4.30 (m, 2H), 4.05 (dd, 1H), 3.60 (d, 1H), 3.10 (q, 2H), 3.00 (s, 3H), 2.70 (dd, 1H), 2.50 (m, 1H), 2.25 (s, 3H), 2.10 (m, 2H), 1.60 (m, 1H), 1.35 (m, 2H), 1.10 (d, 3H), 0.90 (d, 3H); FAB-MS: [M+H] = 589.

### Example 76

### cyclo-(D-Val-NMeArg-Gly-Asp-3-aminomethyl-6-chlorobenzoic acid)

The title compound was prepare by the general solution-phase procedure described above for cyclo-(D-Val-NMeArg-Gly-Asp-Mamb), except that 4,4'-dinitrobenzophenone oxime was employed. The cyclic peptide (550 mg, 0.65 mmol) was deprotected with excess HF in the presence of anisole as scavenger. Purification was accomplished by reversed-phase HPLC on a preparative Vydac® C18 column (2.5 cm) using a 0.8% / minute gradient of 10 to 38% acetonitrile containing 0.1% trifluoroacetic acid to give the TFA salt of the title compound (254 mg, 54%) as a fluffy white solid; ¹H NMR (D₆-DMSO) 12.30 (br s, 1H), 9.05 (d, 1H), 8.45 (m, 2H), 7.50 (t, 1H), 7.35 (d, 1H), 7.30 (m, 2H), 7.10 (s, 1H), 7.05 (br s, 4H), 5.15 (dd, 1H), 4.45 (dd, 1H), 4.40 (q, 2H), 4.05 (dt, 2H), 3.55 (dd, 1H), 3.15 (q, 2H), 3.10 (s, 3H), 2.70 (dd, 1H), 2.50 (m, 1H), 2.05 (m, 2H), 1.65 (m, 1H), 1.35 (m, 2H), 1.10 (d, 3H), 0.90 (d, 3H); FAB-MS: [M+H] = 609.

### Example 77

### cyclo-(D-Val-NMeArg-Gly-Asp-3-aminomethyl-6-iodobenzoic acid)

The title compound was prepare by the general solution-phase procedure described above for cyclo-(D-Val-NMeArg-Gly-Asp-Mamb), except that 4,4'-dinitrobenzophenone oxime was employed. The cyclic peptide (490 mg, 0.52 mmol) was deprotected with excess HF in the presence of anisole as scavenger. Purification was accomplished by reversed-phase HPLC on a preparative Vydac® C18 column (2.5 cm) using a 0.8% / minute gradient of 10 to 38% acetonitrile containing 0.1% trifluoroacetic acid to give the TFA salt of the title compound (194 mg, 46%) as a fluffy white solid; ¹H NMR (D₆-DMSO) 12.30 (br s, 1H), 9.00 (d, 1H); 8.40 (m, 2H), 7.70 (d, 1H), 7.50 (m, 1H), 7.30 (m, 1H), 7.05 (d, 1H), 7.00 (s, 1H), 7.00 (br s, 4H), 5.15 (dd, 1H), 4.40 (d, 1H), 4.40 (q, 2H), 4.0 (m, 2H), 3.55 (dd, 1H), 3.15 (q, 2H), 3.10 (s, 3H), 2.70 (dd, 1H), 2.50 (m, 1H), 2.05 (m, 2H), 1.65 (m, 1H), 1.35 (m, 2H), 1.15 (d, 3H), 0.90 (d, 3H); FAB-MS: [M+H] = 701.

### Example 78

### cyclo-(D-val-NMeArg-Gly-Asp-3-aminomethyl-6-methoxybenzoic acid)

The title compound was prepare by the general solution-phase procedure described above for cyclo-(D-Val-NMeArg-Gly-Asp-Mamb), except that 4,4'-dinitrobenzophenone oxime was employed. The cyclic peptide (256 mg, 0.30 mmol) was deprotected with excess HF in the presence of anisole as scavenger. Purification was accomplished by reversed-phase HPLC on a preparative Vydac® C18 column (2.5 cm) using a 0.8% / minute gradient of 10 to 38% acetonitrile containing 0.1% trifluoroacetic acid to give the TFA salt of the title compound (137 mg, 63%) as a fluffy white solid; ¹H NMR (D₆-DMSO) 8.45 (d, 1H), 8.40 (d, 1H), 8.30 (t, 1H), 7.65 (d, 1H), 7.50 (t, 1H), 7.40 (s, 1H), 7.35 (d, 1H), 7.05 (d, 1H), 7.00 (br s, 4H), 5.20 (dd, 1H), 4.55 (dd, 1H), 4.50 (q, 1H), 4.35 (dd, 1H), 4.25 (dd, 1H), 3.95 (dd, 1H), 3.90 (s, 3H), 3.55 (d, 1H), 3.10 (q, 2H), 3.00 (s, 3H), 2.70 (dd, 1H), 2.50 (m, 1H), 2.05 (m, 2H), 1.60 (m, 1H), 1.35 (m, 2H), 1.10 (d, 3H), 0.95 (d, 3H); FAB-MS: [M+H] = 605.

### Example 79

### cyclo-(D-Val-NMeArg-Gly-Asp-3-aminomethyl-6-methylbenzoic acid)

The title compound was prepare by the general solution-phase procedure described above for cyclo-(D-Val-NMeArg-Gly-Asp-Mamb), except that 4,4'-dinitrobenzophenone oxime was employed. The cyclic peptide (230 mg, 0.28 mmol) was deprotected with excess HF in the presence of anisole as scavenger. Purification was accomplished by reversed-phase HPLC on a preparative Vydac® C18 column (2.5 cm) using a 0.8% / minute gradient of 10 to 38% acetonitrile containing 0.1% trifluoroacetic acid to give the TFA salt of the title compound (54 mg, 27%) as a fluffy white solid; ¹H NMR (D₆-DMSO) 12.30 (br s, 1H), 8.80 (d, 1H), 8.40 (d, 1H), 8.30 (t, 1H), 7.45 (m, 2H), 7.15 (q, 2H), 7.00 (s, 1H), 7.00 (br s, 4H), 5.15 (dd, 1H), 4.45 (m, 3H), 4.05 (m, 2H), 3.55 (dd, 1H), 3.10 (q, 2H), 3.05 (s, 3H), 2.70 (dd, 1H), 2.50 (m, 1H), 2.30 (s, 3H), 2.05 (m, 2H), 1.60 (m, 1H), 1.35 (m, 2H), 1.05 (d, 3H), 0.90 (d, 3H); FAB-MS: [M+H] = 589.

### Utility

The compounds of this invention possess antiplatelet efficacy, as evidenced by their activity in standard platelet aggregation assays or platelet fibrinogen binding assays, as described below. A compound is considered to be active in these assays if it has an IC50 value of less than about 1 mM. Platelet aggregation and fibrinogen binding assays which may used to demonstrate the antiplatelet activity of the compounds of the invention are described below.

Platelet Aggregation Assay: Venous blood was obtained from the arm of a healthy human donor who was drug-free and aspirin-free for at least two weeks prior to blood collection. Blood was collected into 10 ml citrated Vacutainer tubes. The blood was centrifuged for 15 minutes at 150 x g at room temperature, and platelet-rich plasma (PRP) was removed. The remaining blood was centrifuged for 15 minutes at 1500 x g at room temperature, and platelet-poor plasma (PPP) was removed. Samples were assayed on a aggregometer (PAP-4 Platelet Aggregation Profiler), using PPP as the blank (100% transmittance). 200 µl of PRP was added to each micro test tube, and transmittance was set to 0%. 20 µl of various agonists (ADP, collagen, arachidonate, epinephrine, thrombin) were added to each tube, and the aggregation profiles were plotted (% transmittance versus time). The results were expressed as % inhibition of agonist-induced platelet aggregation. For the IC₅₀ evaluation, the test compounds were added at various concentrations prior to the activation of the platelets.

Platelet-Fibrinogen Binding Assay: Binding of ¹²⁵I-fibrinogen to platelets was performed as described by Bennett et al. (1983) Proc. Natl. Acad. Sci. USA 80: 2417-2422, with some modifications as described below. Human PRP (h-PRP) was applied to a Sepharose® column for the purification of platelet fractions. Aliquots of platelets (5 X 10⁸ cells) along with 1 mM calcium chloride were added to removable 96 well plates prior to the activation of the human gel purified platelets (h-GPP). Activation of the human gel purified platelets was achieved using ADP, collagen, arachidonate, epinephrine, and/or thrombin in the presence of the ligand, ¹²⁵I-fibrinogen. The ¹²⁵I-fibrinogen bound to the activated, platelets was separated from the free form by centrifugation and then counted on a gamma counter. For an IC₅₀ evaluation, the test compounds were added at various concentrations prior to the activation of the platelets.

### Dosage and Formulation

The compounds of this invention can be administered by any means that produces contact of the active agent with the agent's site of action, glycoprotein IIb/IIIa (GPIIb/IIIa), in the body of a mammal. They can be administered by any conventional means available for use in conjunction with pharmaceuticals, either as individual therapeutic agents or in a combination of therapeutic agents, such as a second antiplatelet agent such as aspirin or ticlopidine which are agonist-specific. They can be administered alone, but generally administered with a pharmaceutical carrier selected on the basis of the chosen route of administration and standard pharmaceutical practice.

The dosage administered will, of course, vary depending upon known factors, such as the pharmacodynamic characteristics of the particular agent and its mode and route of administration; the age, health and weight of the recipient; the nature and extent of the symptoms; the kind of concurrent treatment; the frequency of treatment; and the effect desired. A daily dosage of active ingredient can be expected to be 0.01 to 10 milligrams per kilogram of body weight.

Dosage forms (compositions suitable for administration contain from 1 milligram to 100 milligrams of active ingredient per unit. In these pharmaceutical compositions the active ingredient will ordinarily be present in an amount of 0.5-95% by weight based on the total weight of the composition.

The active ingredient can be administered orally in solid dosage forms, such as capsules, tablets, and powders, or in liquid dosage forms, such as elixirs, syrups, and suspensions. It can also be administered parenterally, in sterile liquid dosage forms.

Gelatin capsules contain the active ingredient and powdered carriers, such as lactose, starch, cellulose derivatives, magnesium stearate and stearic acid. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl- or propyl-paraben, and chlorobutanol.

Suitable pharmaceutical carriers are described in Remington's Pharmaceutical Sciences, Mack Publishing Company, a standard reference text in this field.

Useful pharmaceutical dosage-forms for administration of the compounds of this invention can be illustrated as follows:

### Capsules

A large number of unit capsules are prepared by filling standard two-piece hard gelatin capsules each with 100 milligrams of powdered active ingredient, 150 milligrams of lactose, 50 milligrams of cellulose, and 6 milligrams magnesium stearate.

### Soft Gelatin Capsules

A mixture of active ingredient in a digest able oil such as soybean oil, cottonseed oil or olive oil is prepared and injected by means of a positive displacement pump into gelatin to form soft gelatin capsules containing 100 milligrams of the active ingredient. The capsules are washed and dried.

### Tablets

A large number of tablets are prepared by conventional procedures so that the dosage unit was 100 milligrams of active ingredient, 0.2 milligrams of colloidal silicon dioxide, 5 milligrams of magnesium stearate, 275 milligrams of microcrystalline cellulose, 11 milligrams of starch and 98.8 milligrams of lactose. Appropriate coatings may be applied to increase palatability or delay absorption.

The Tables below set forth representative compounds of the present invention.

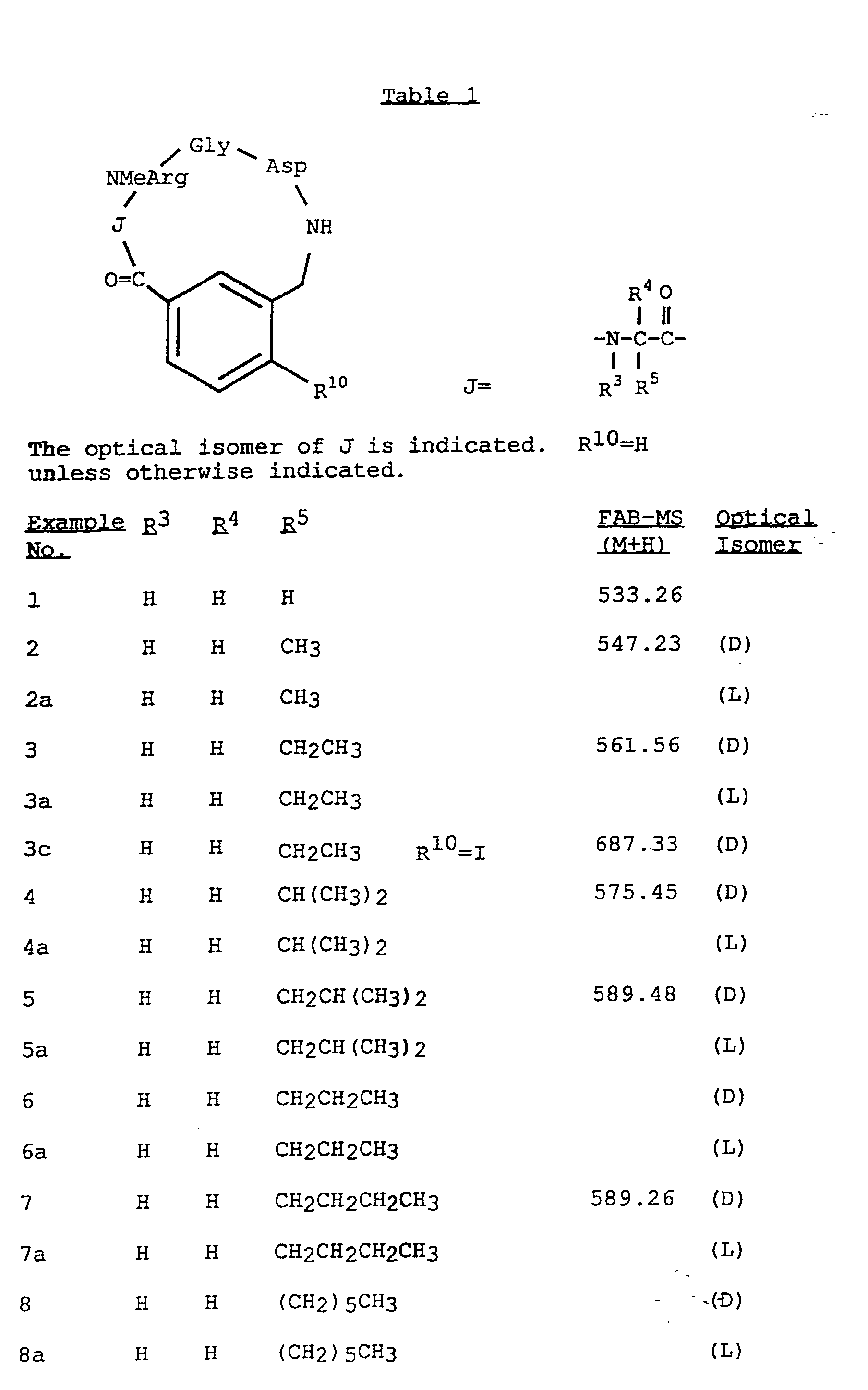

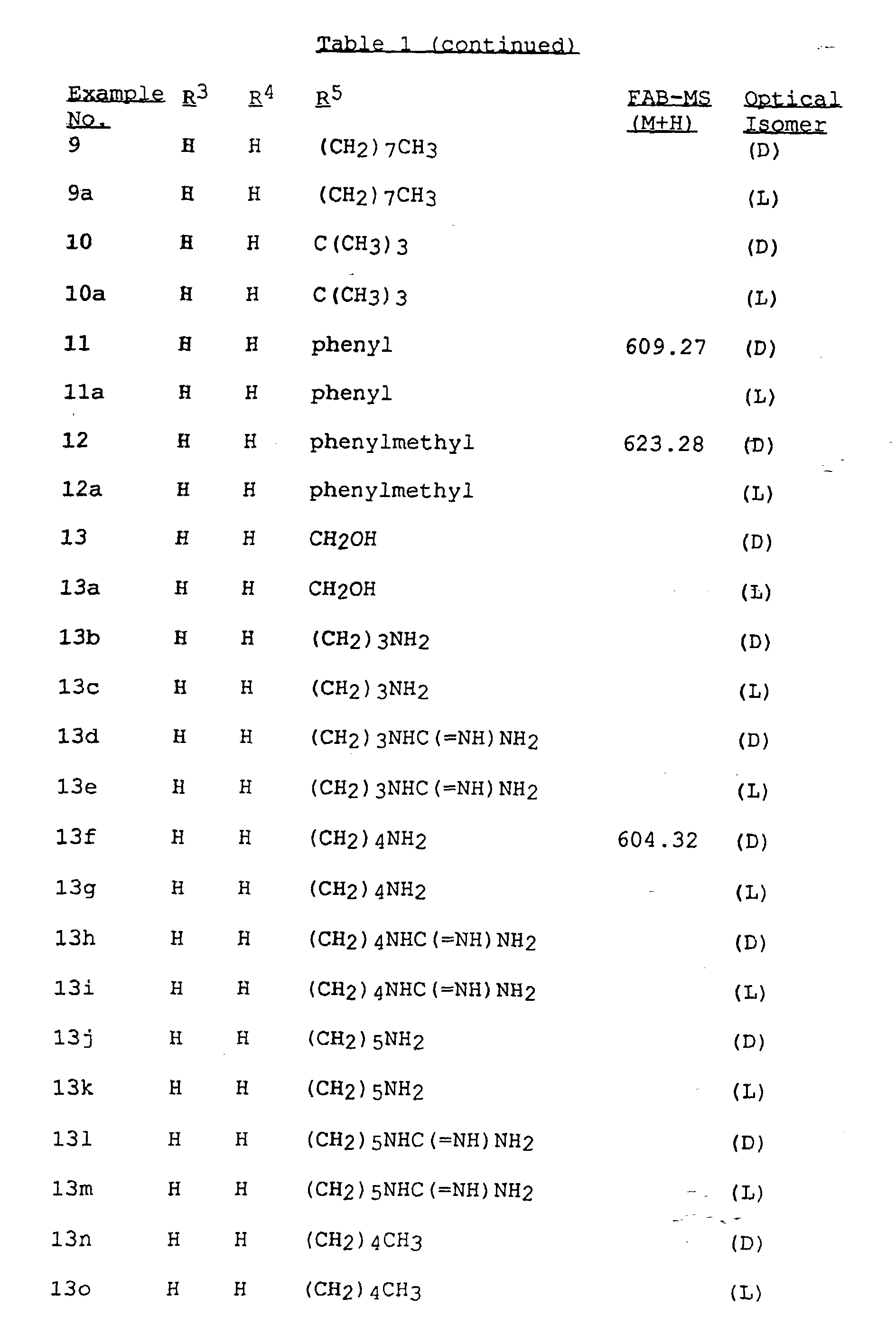

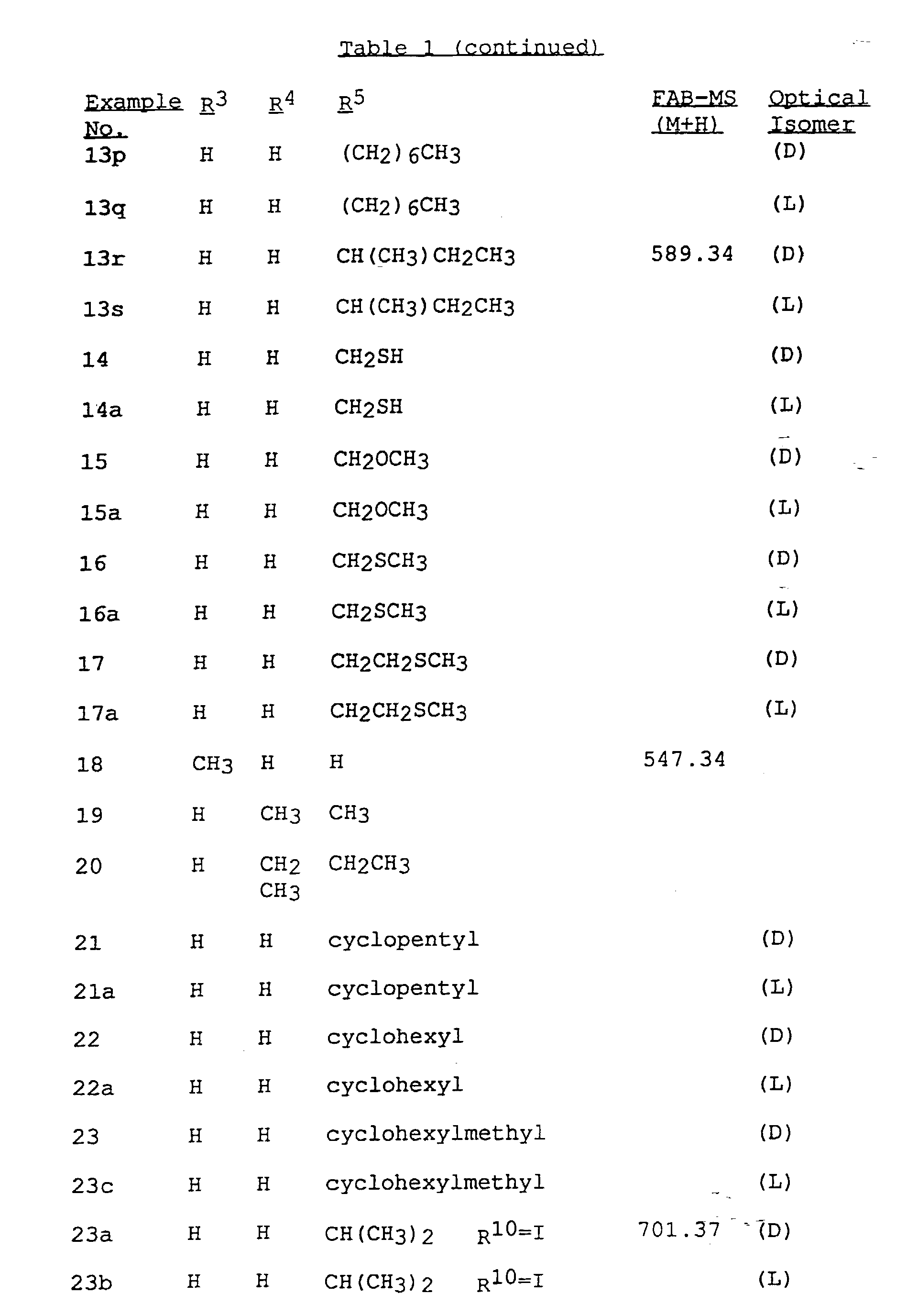

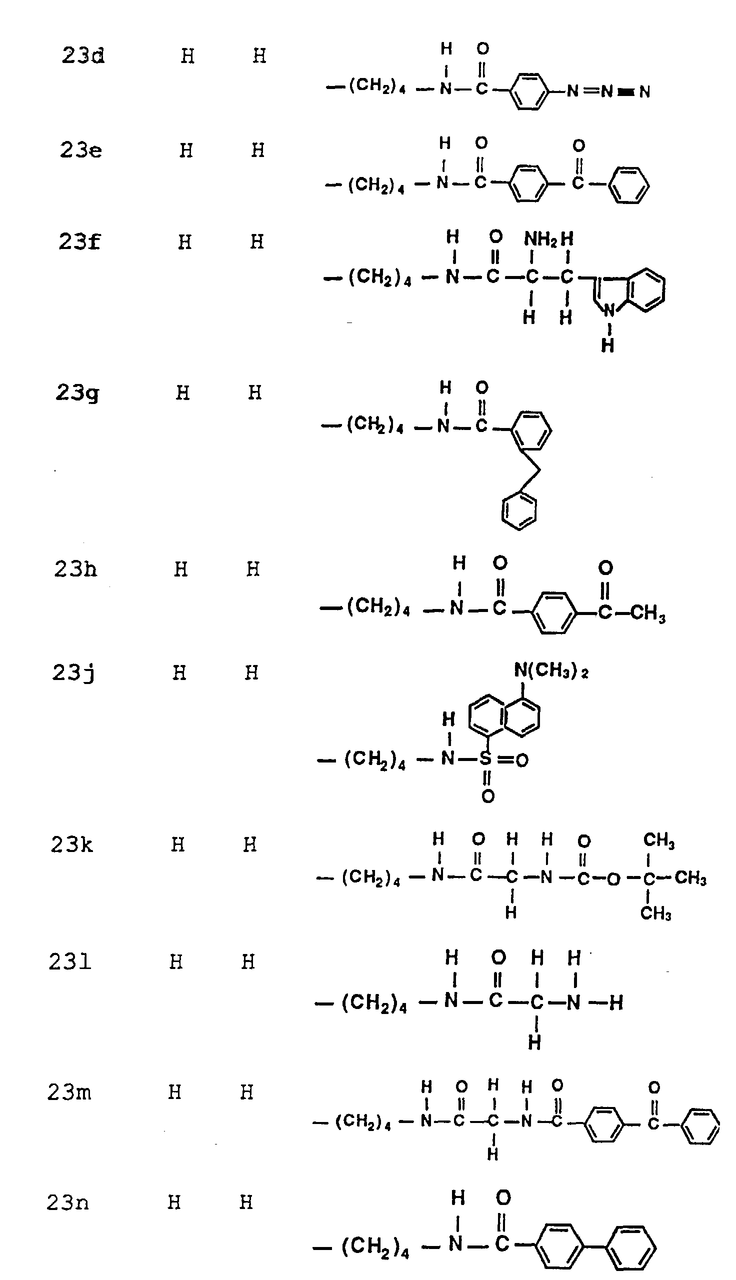

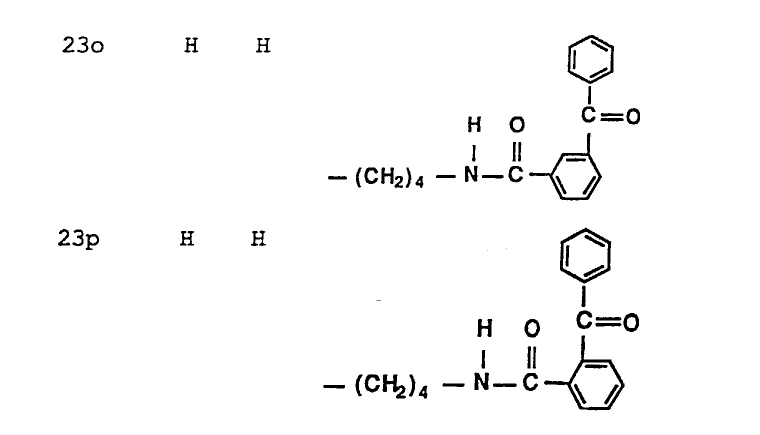

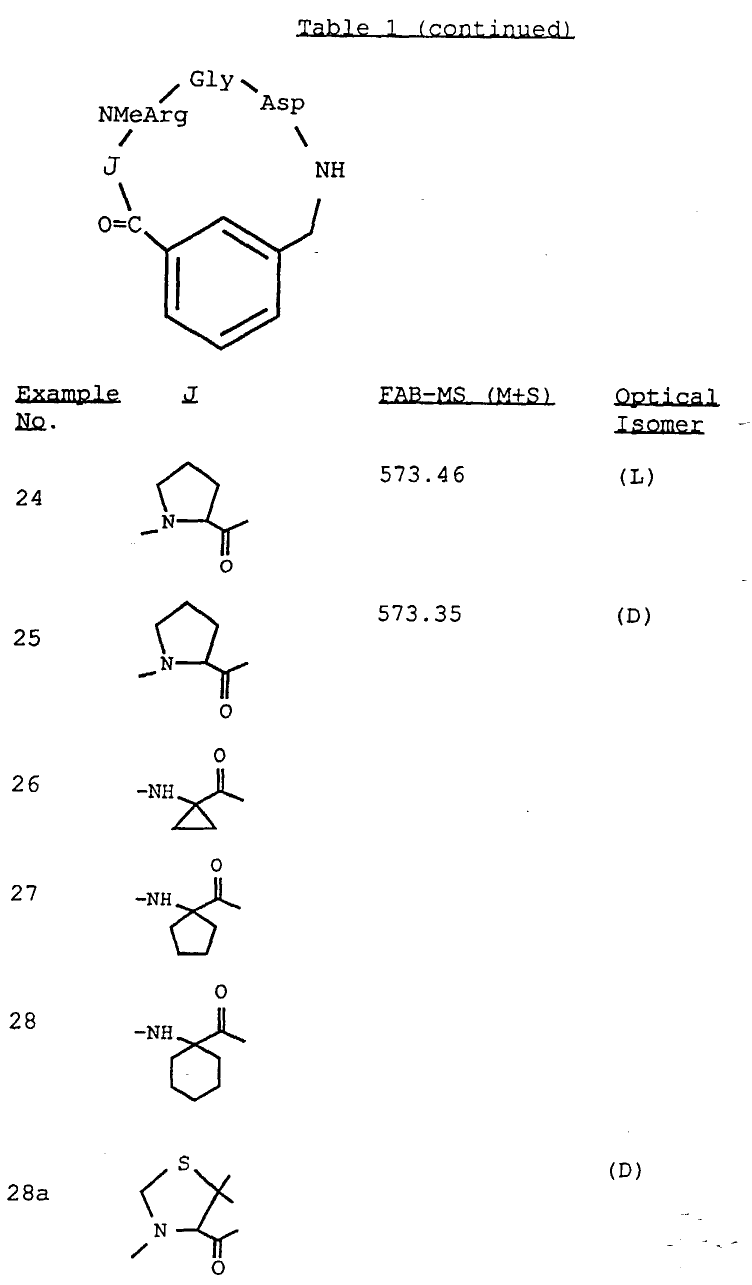

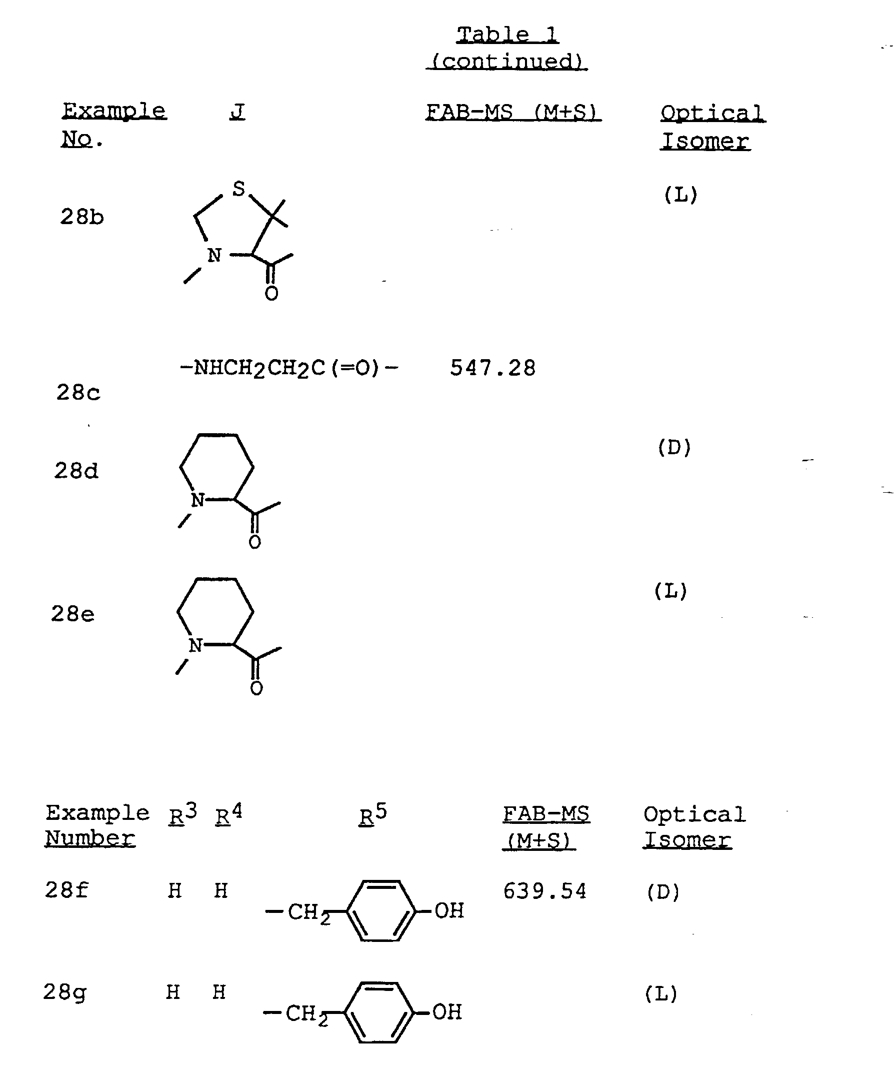

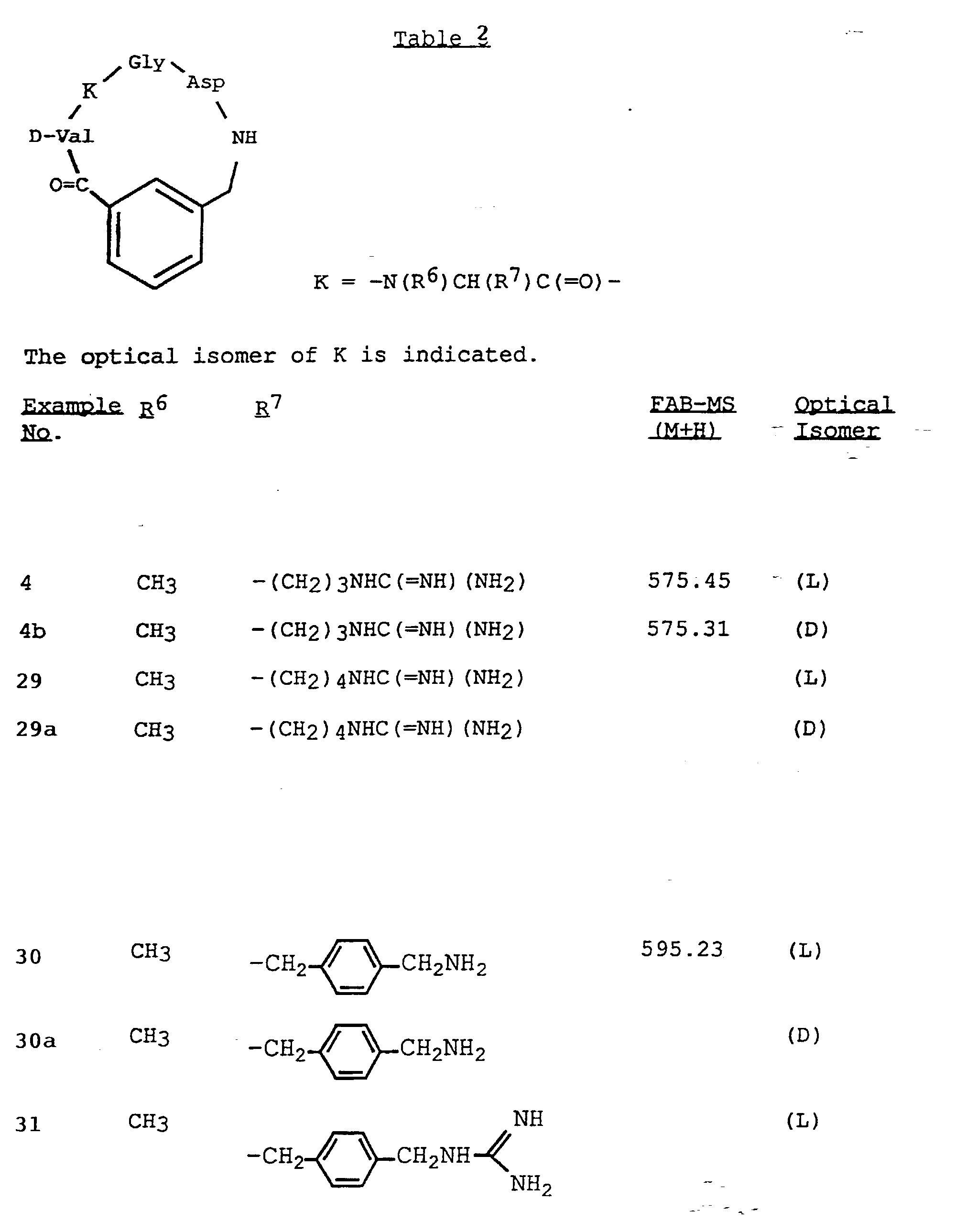

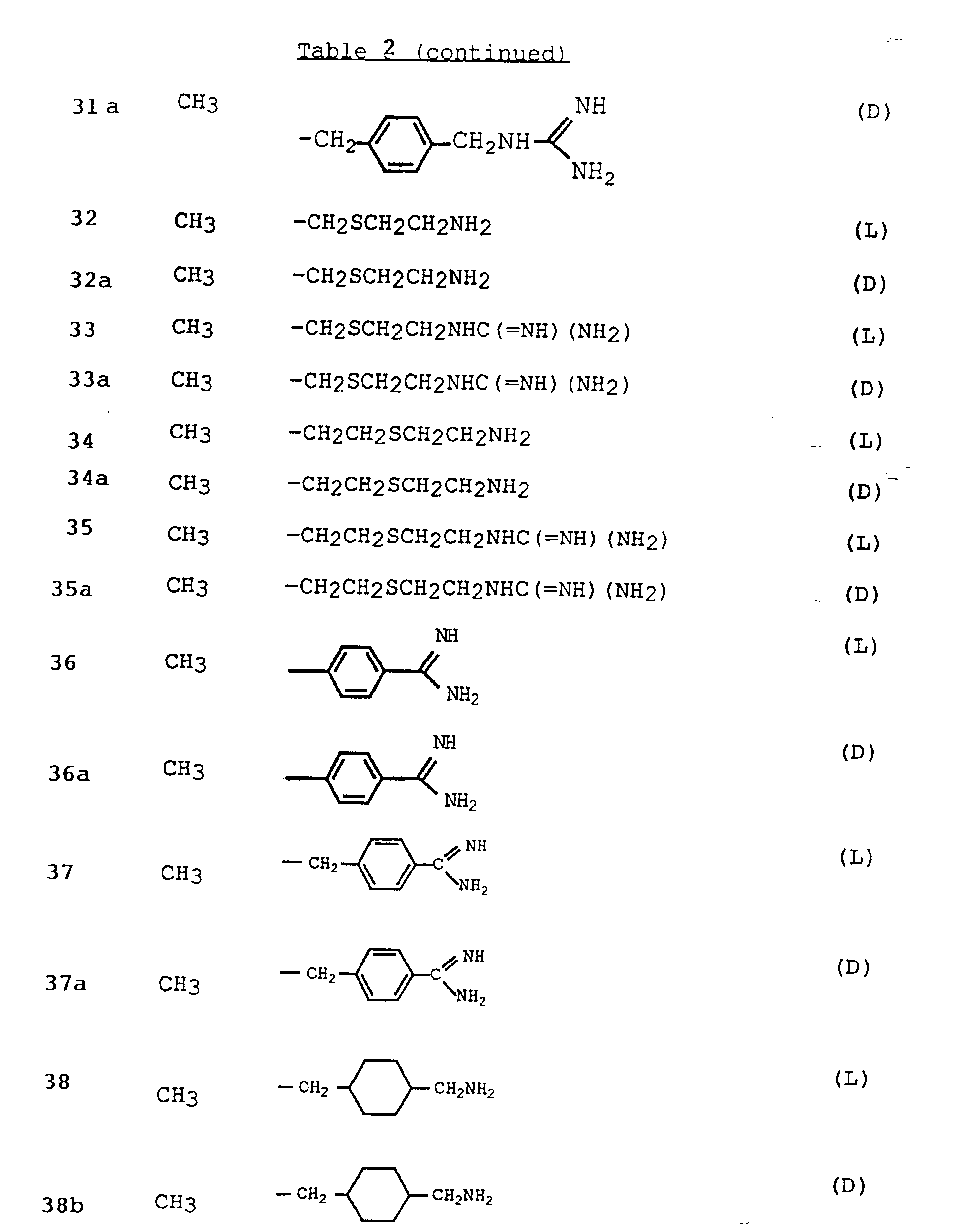

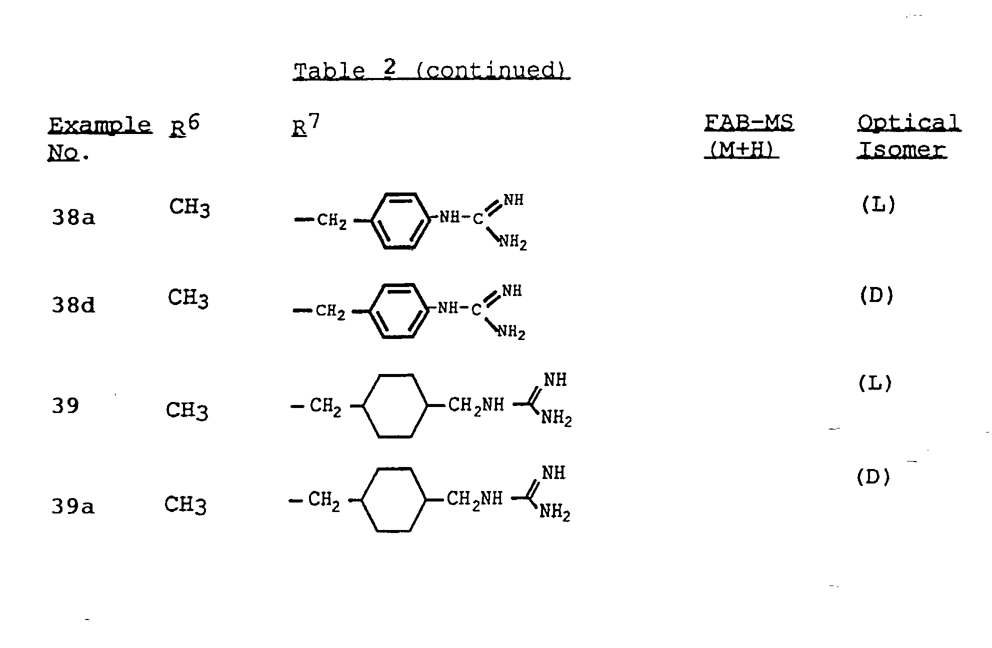

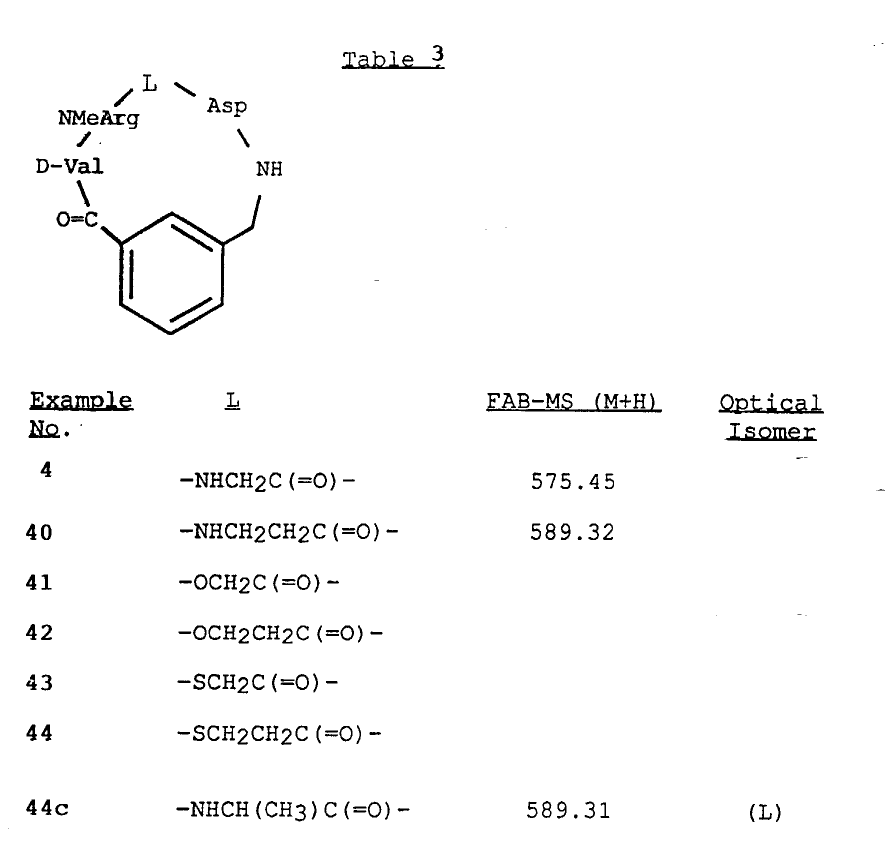

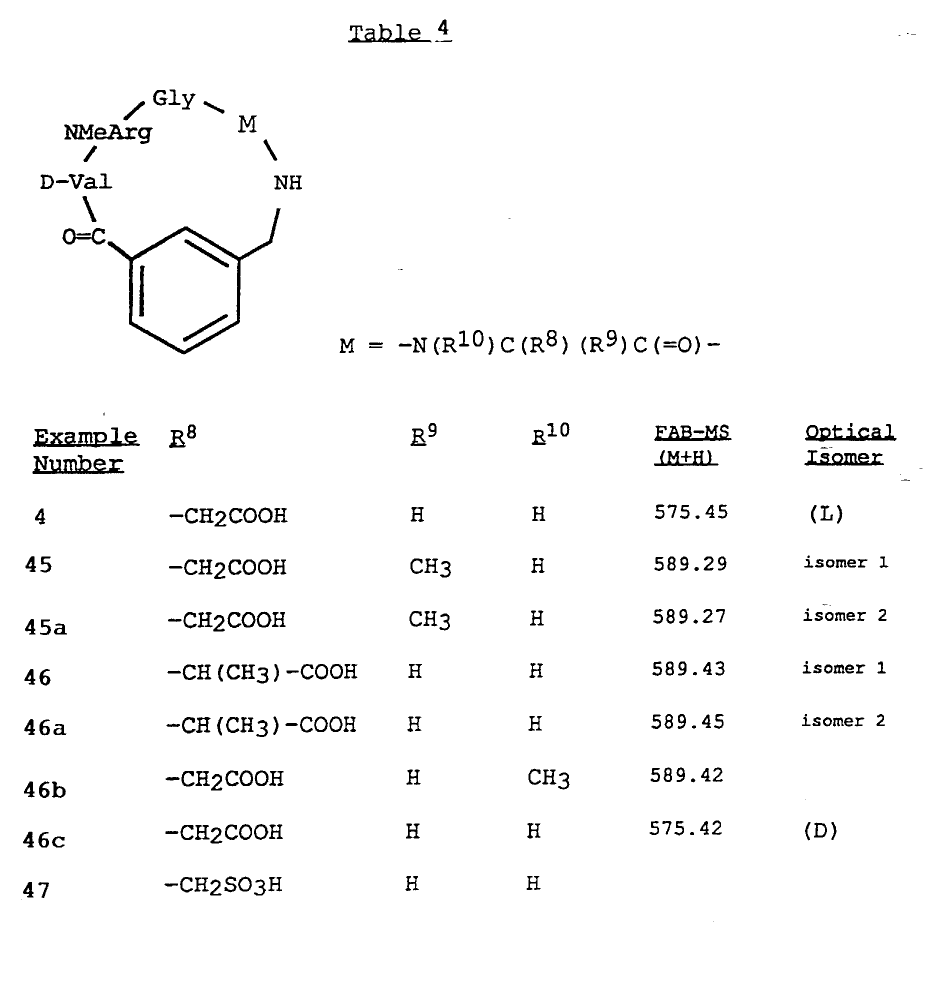

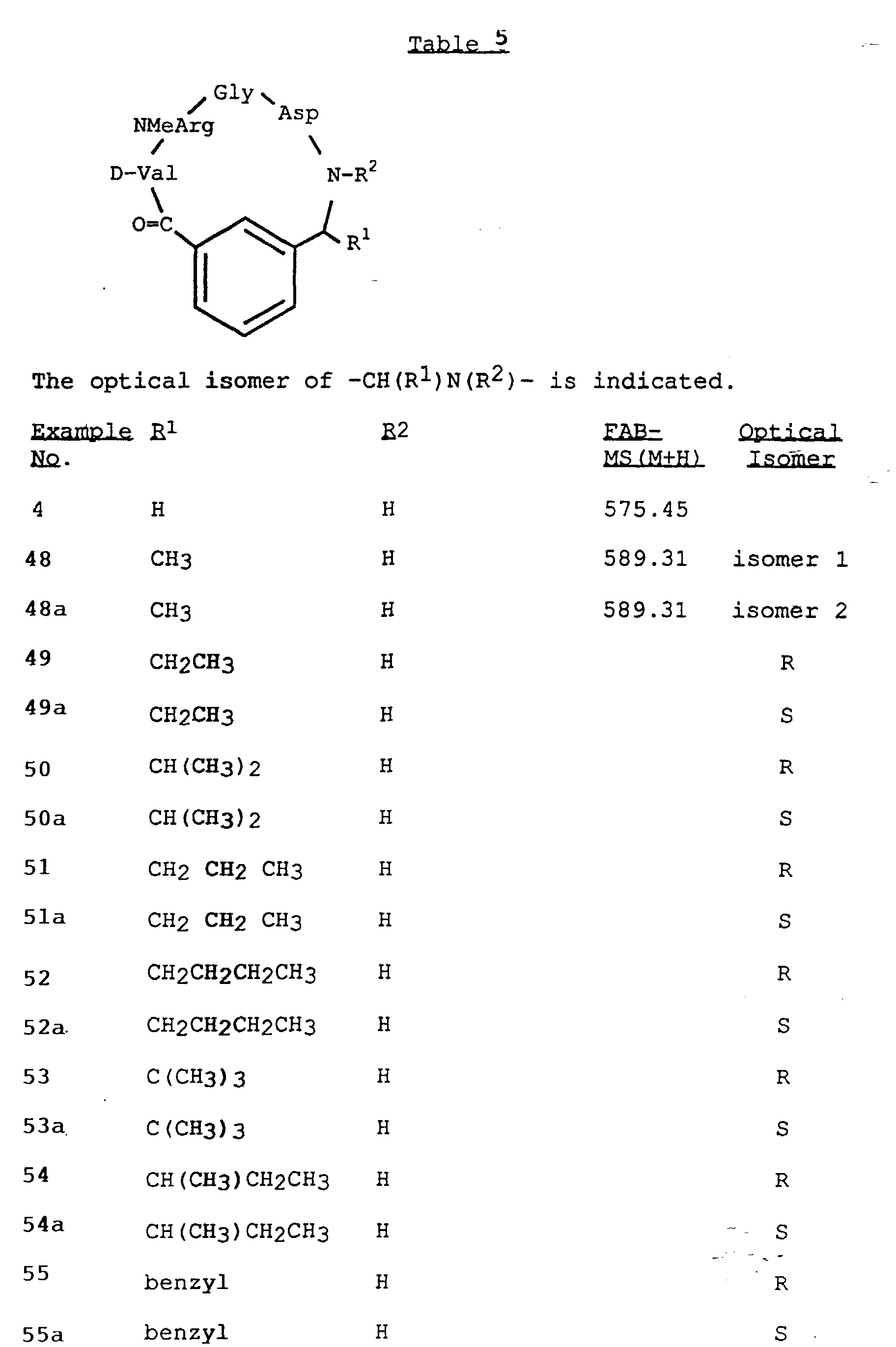

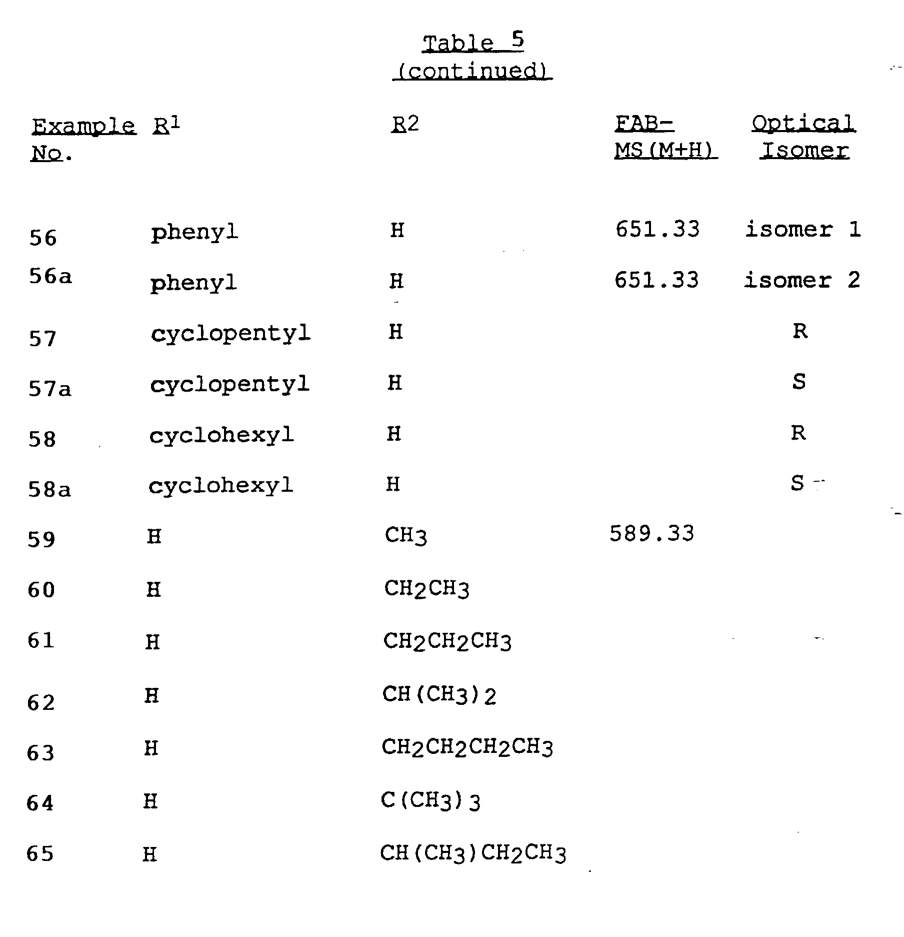

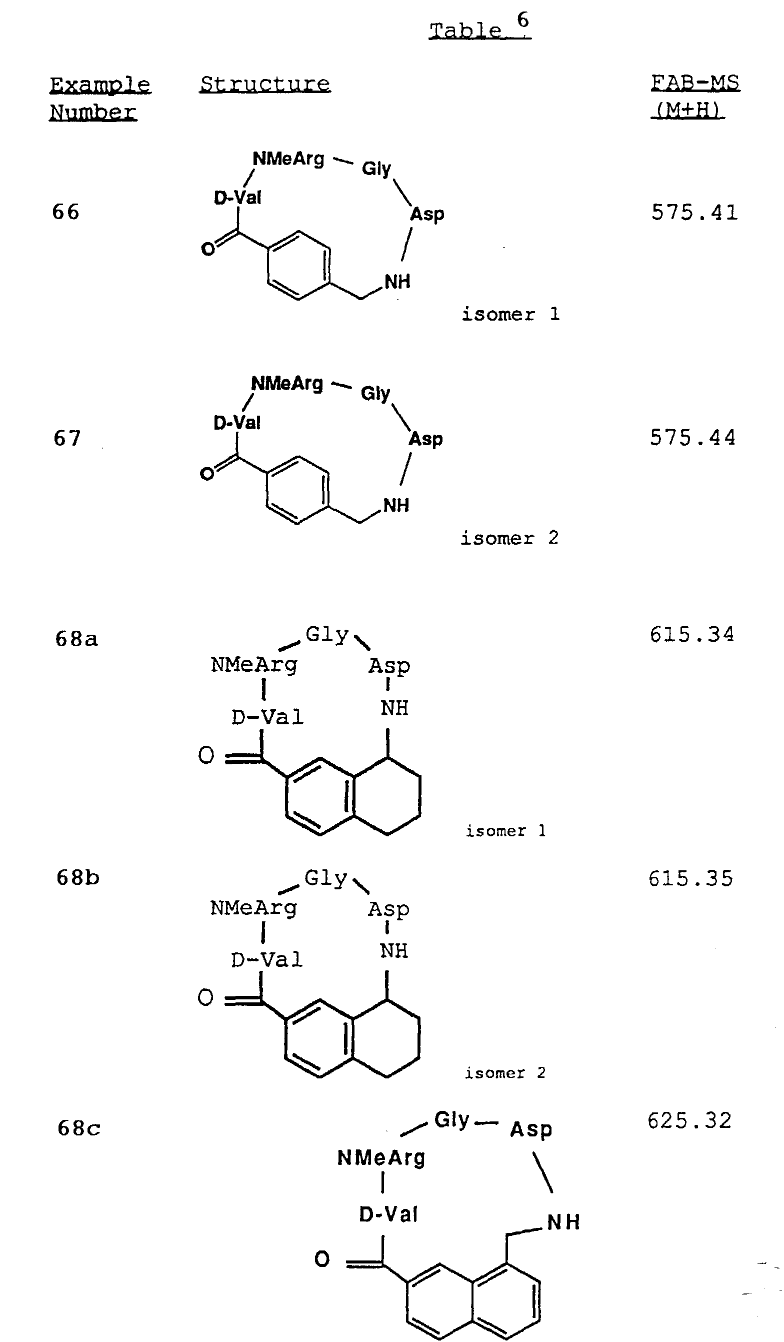

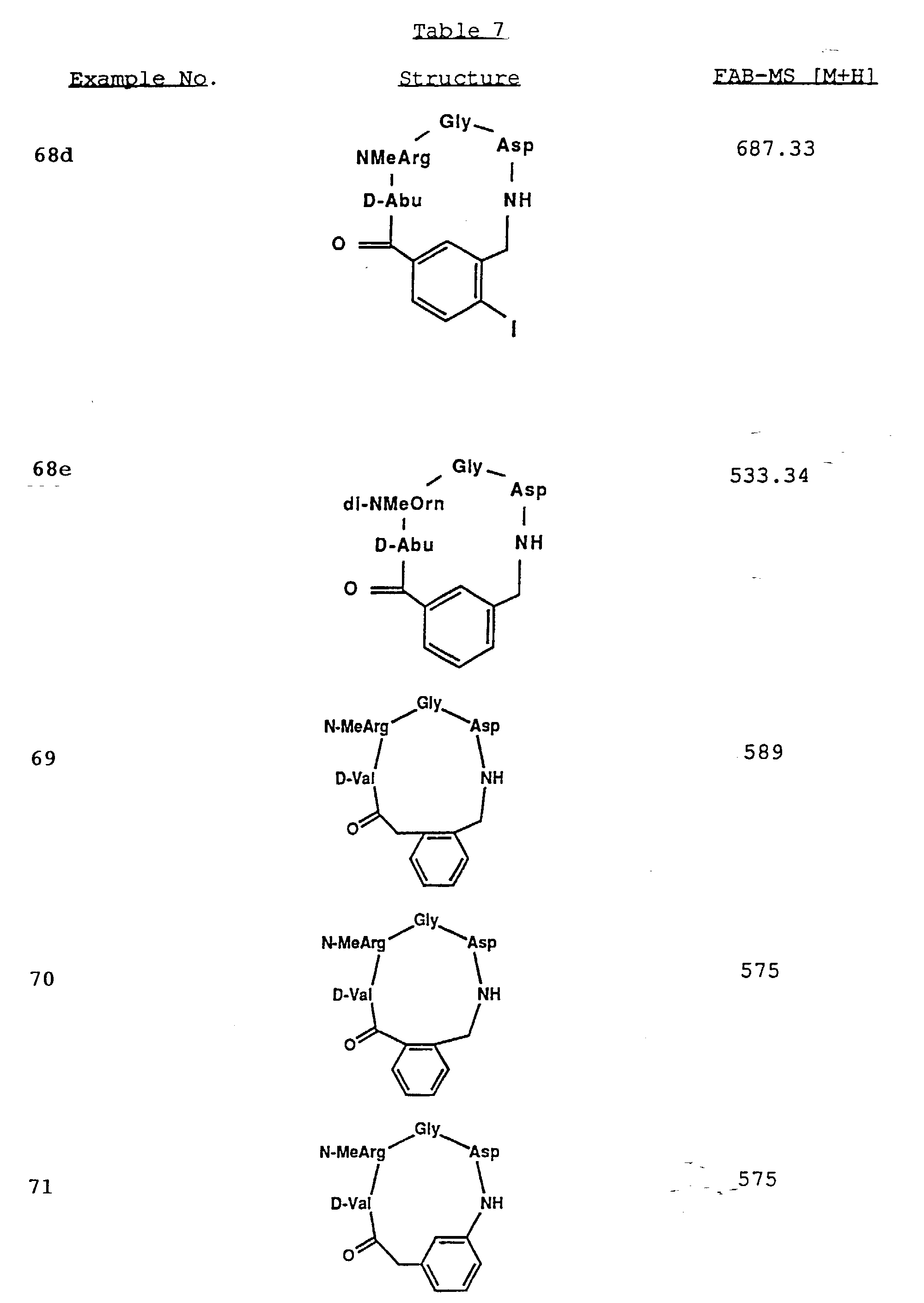

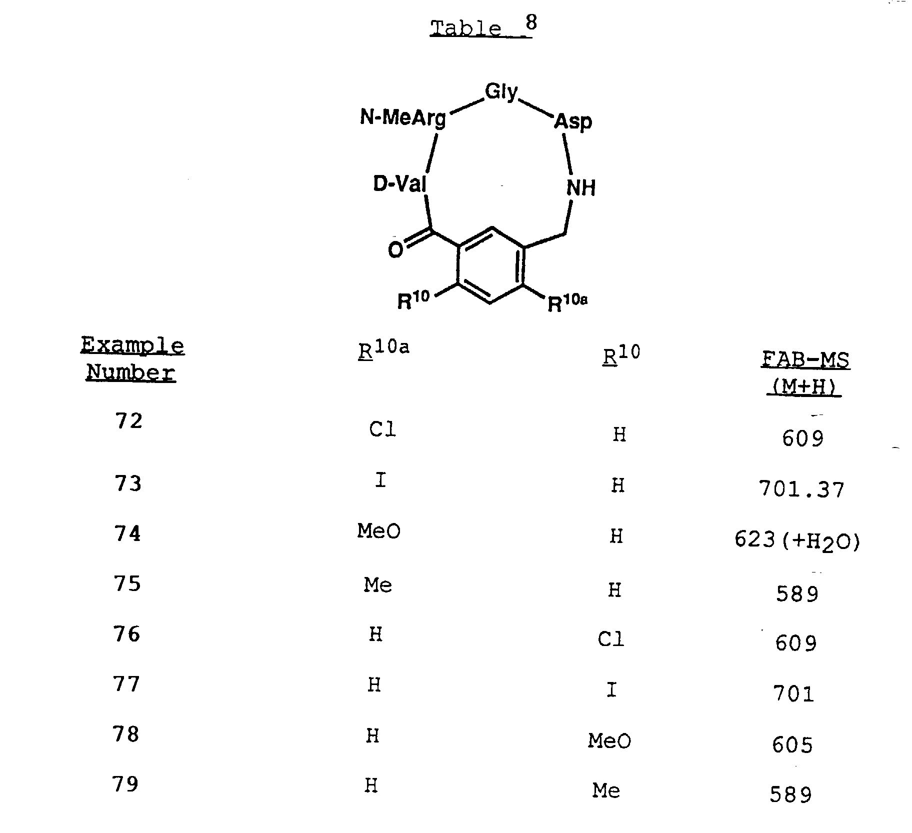

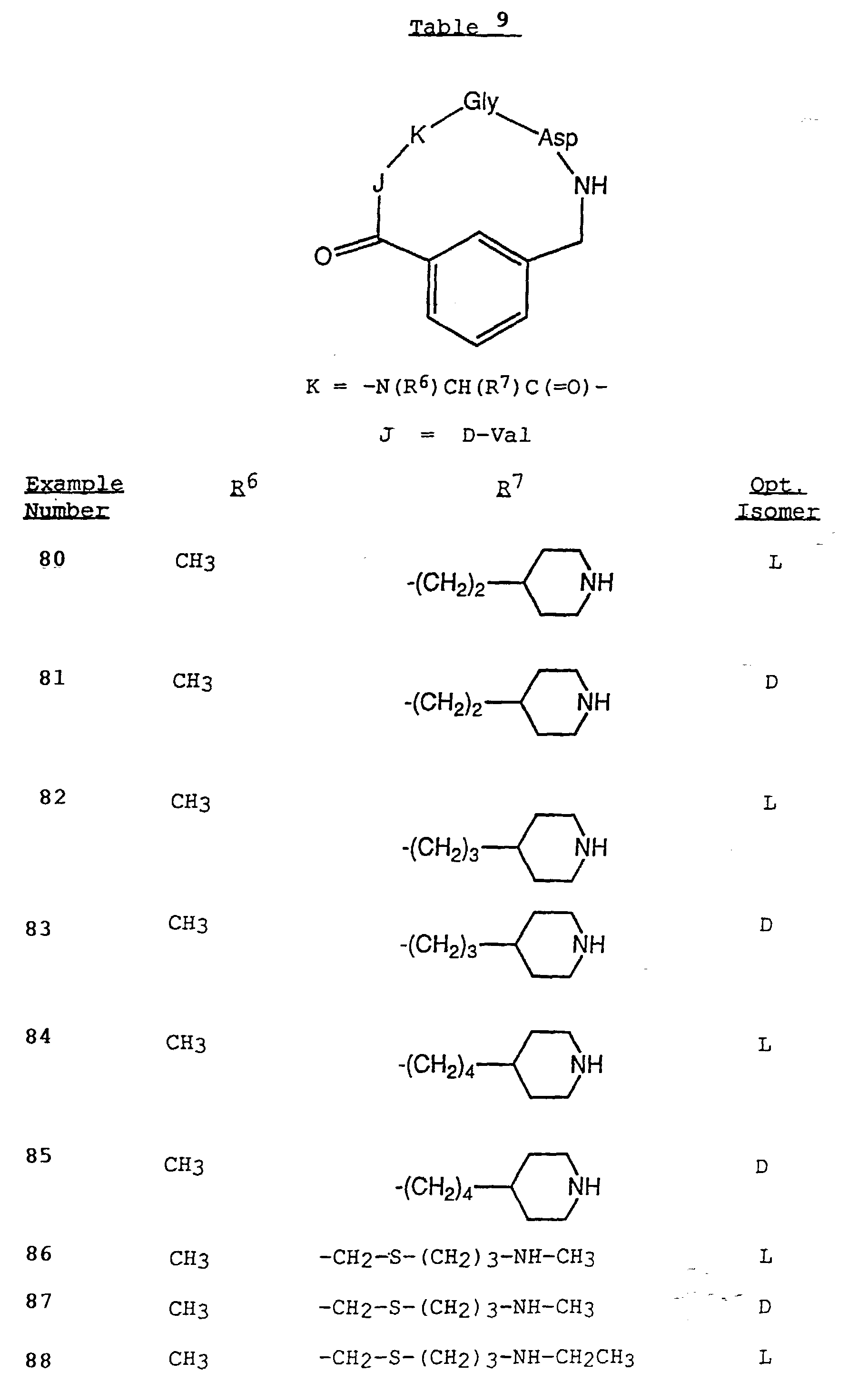

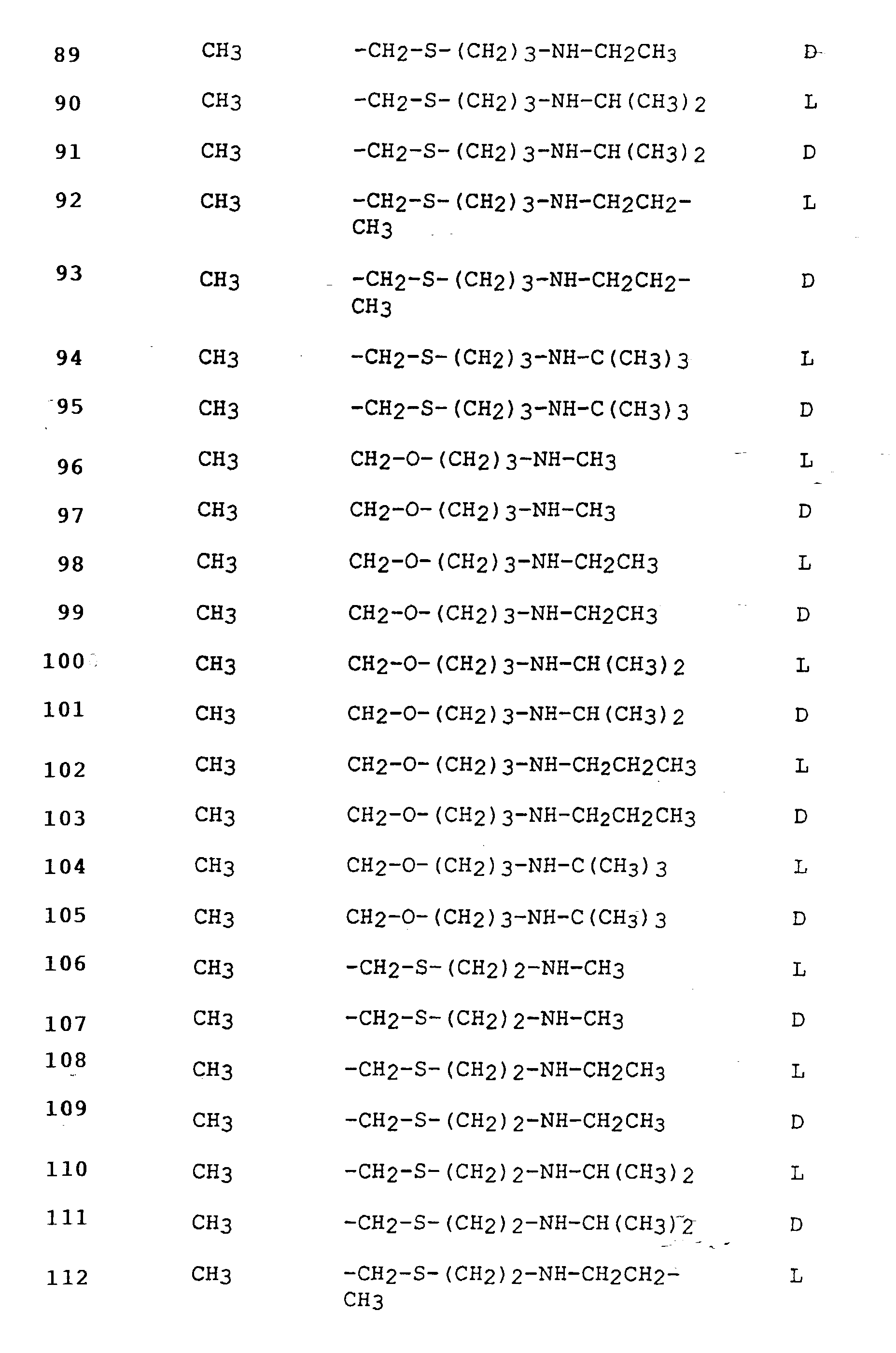

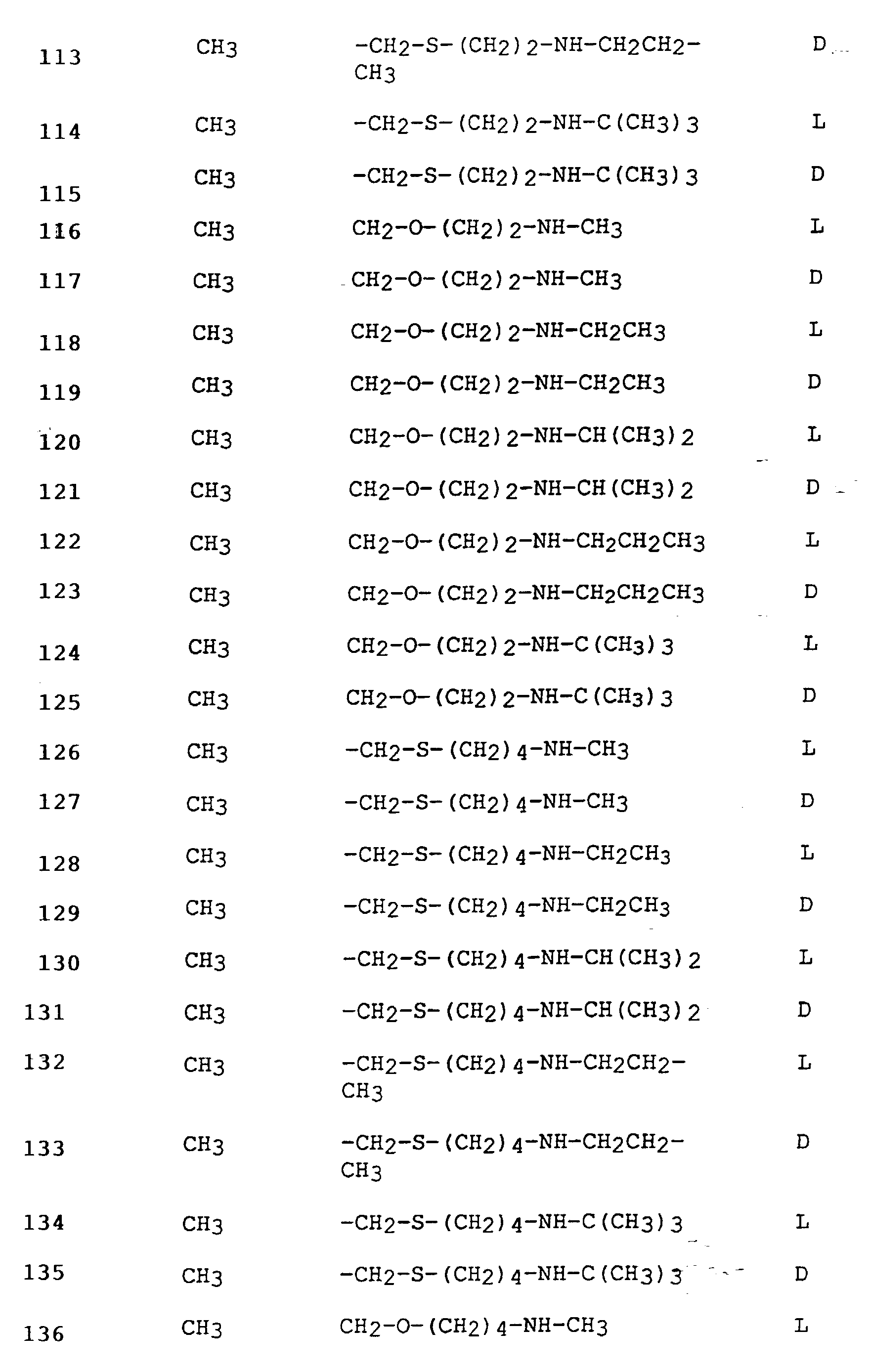

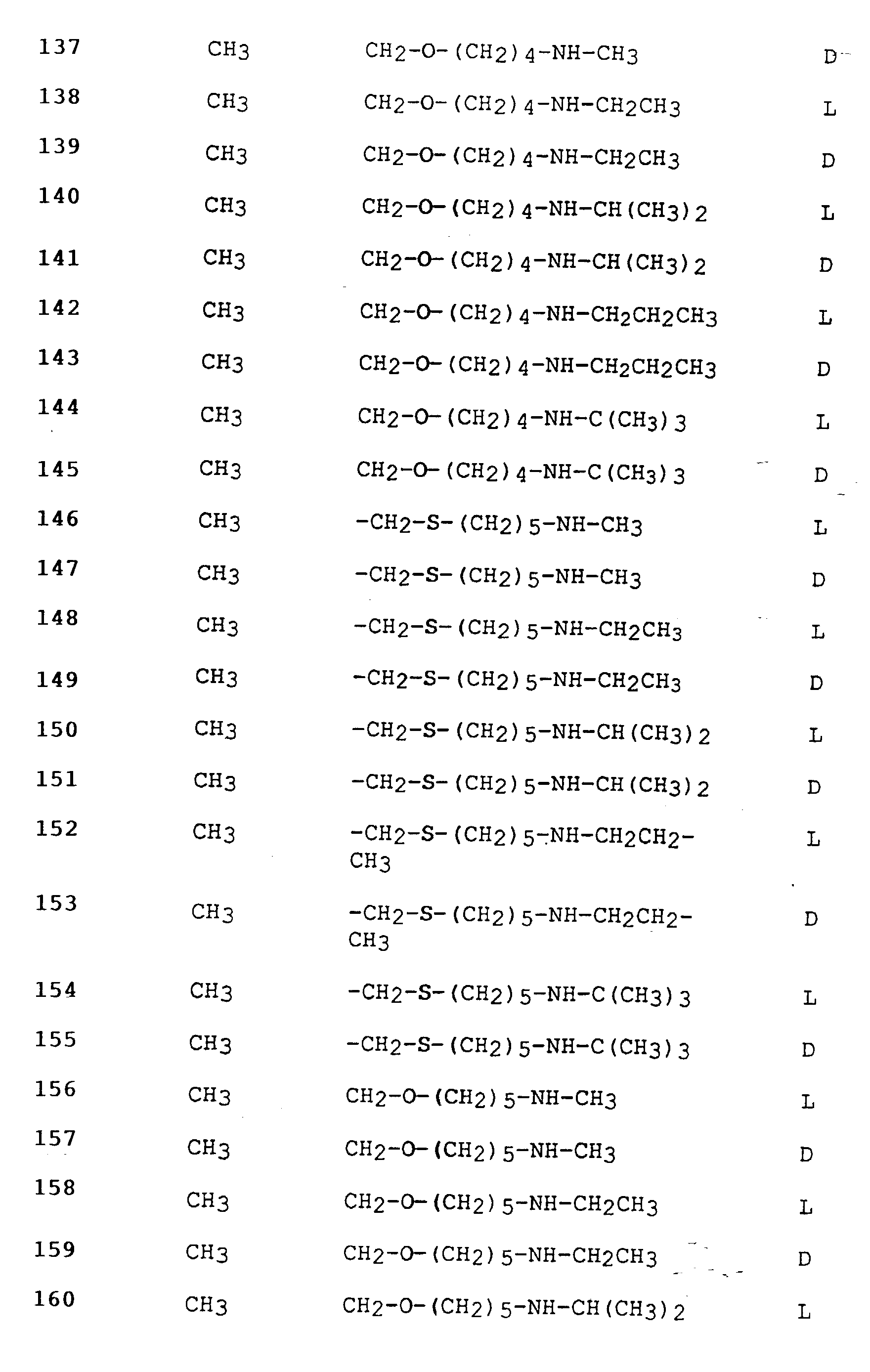

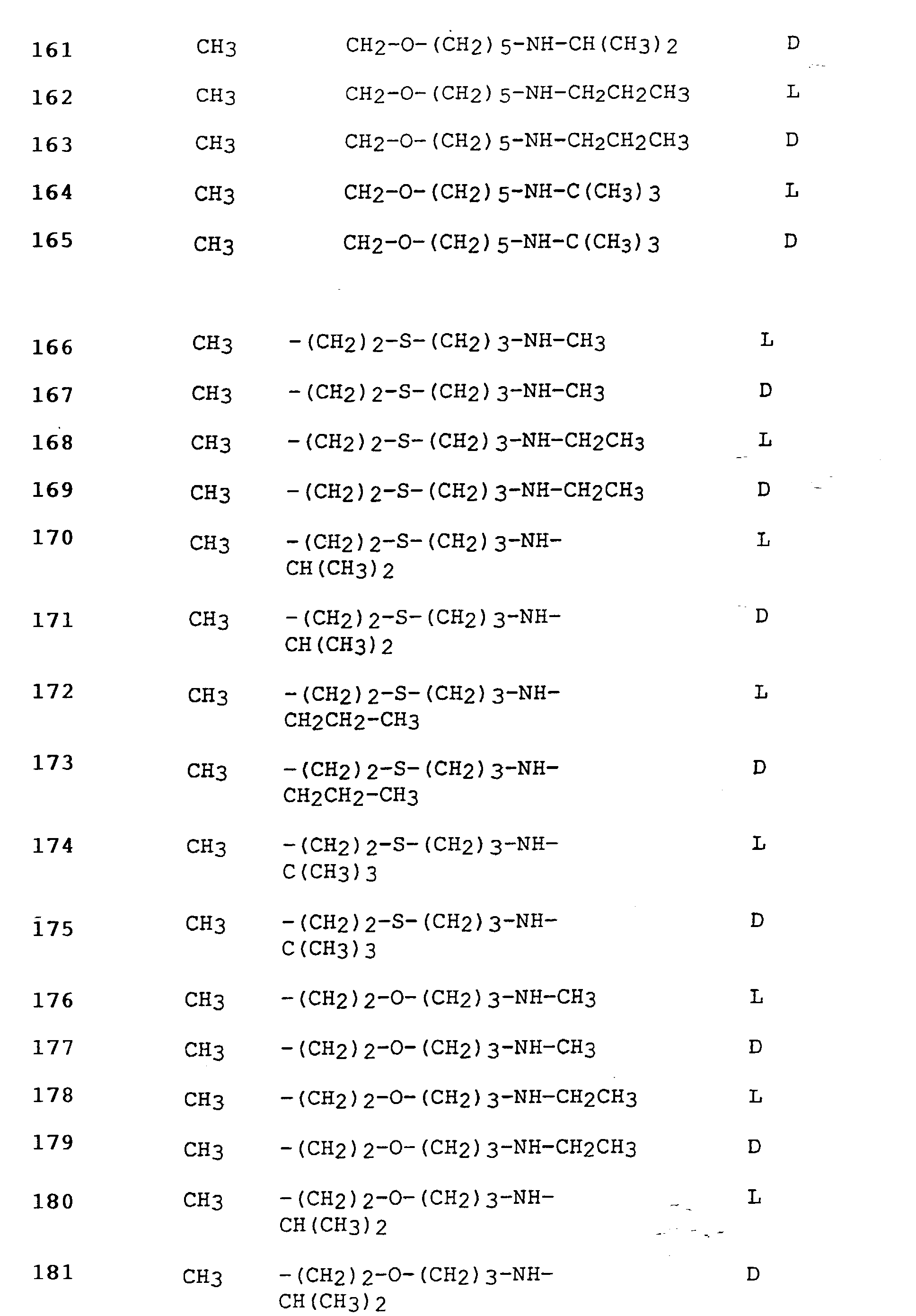

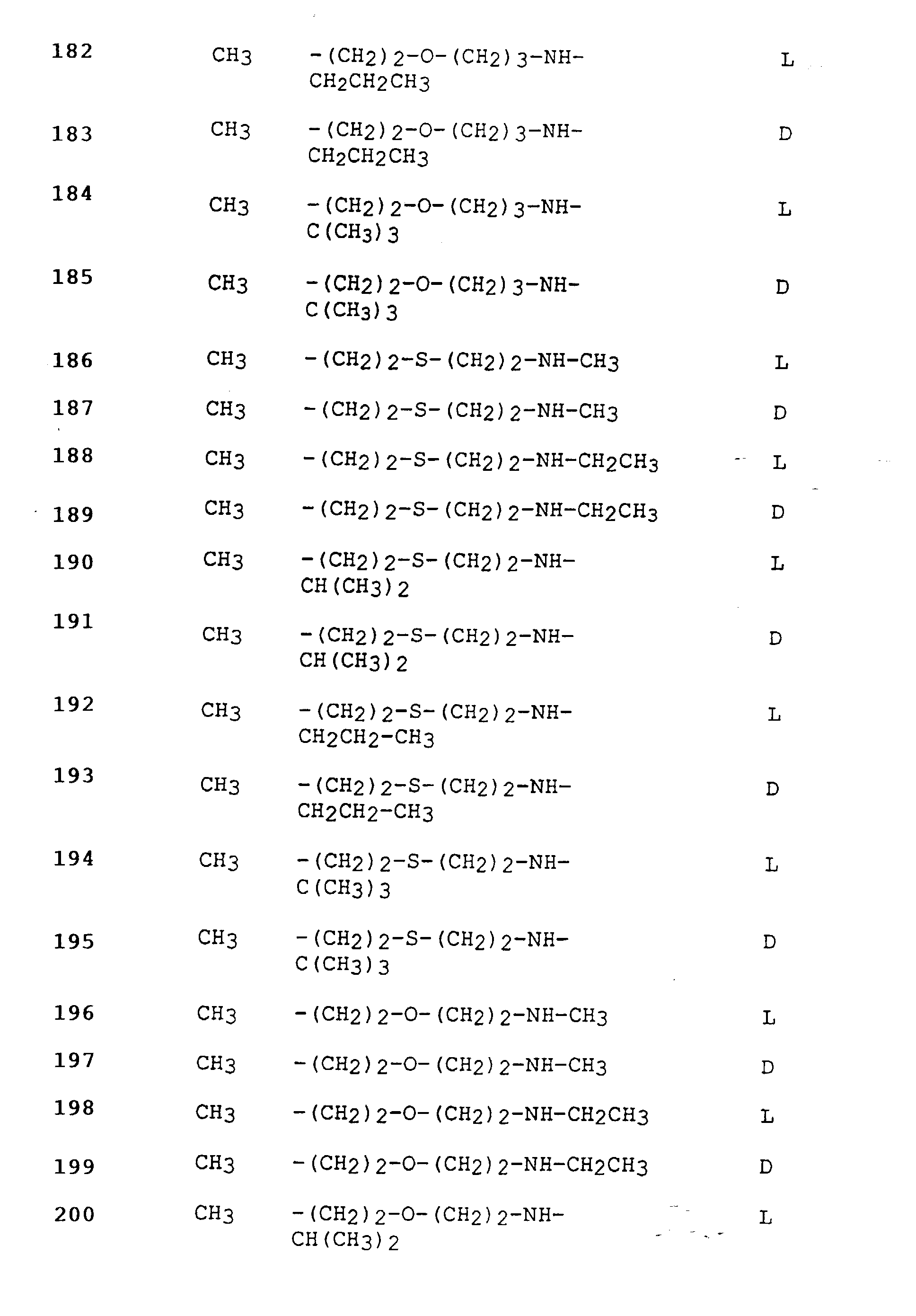

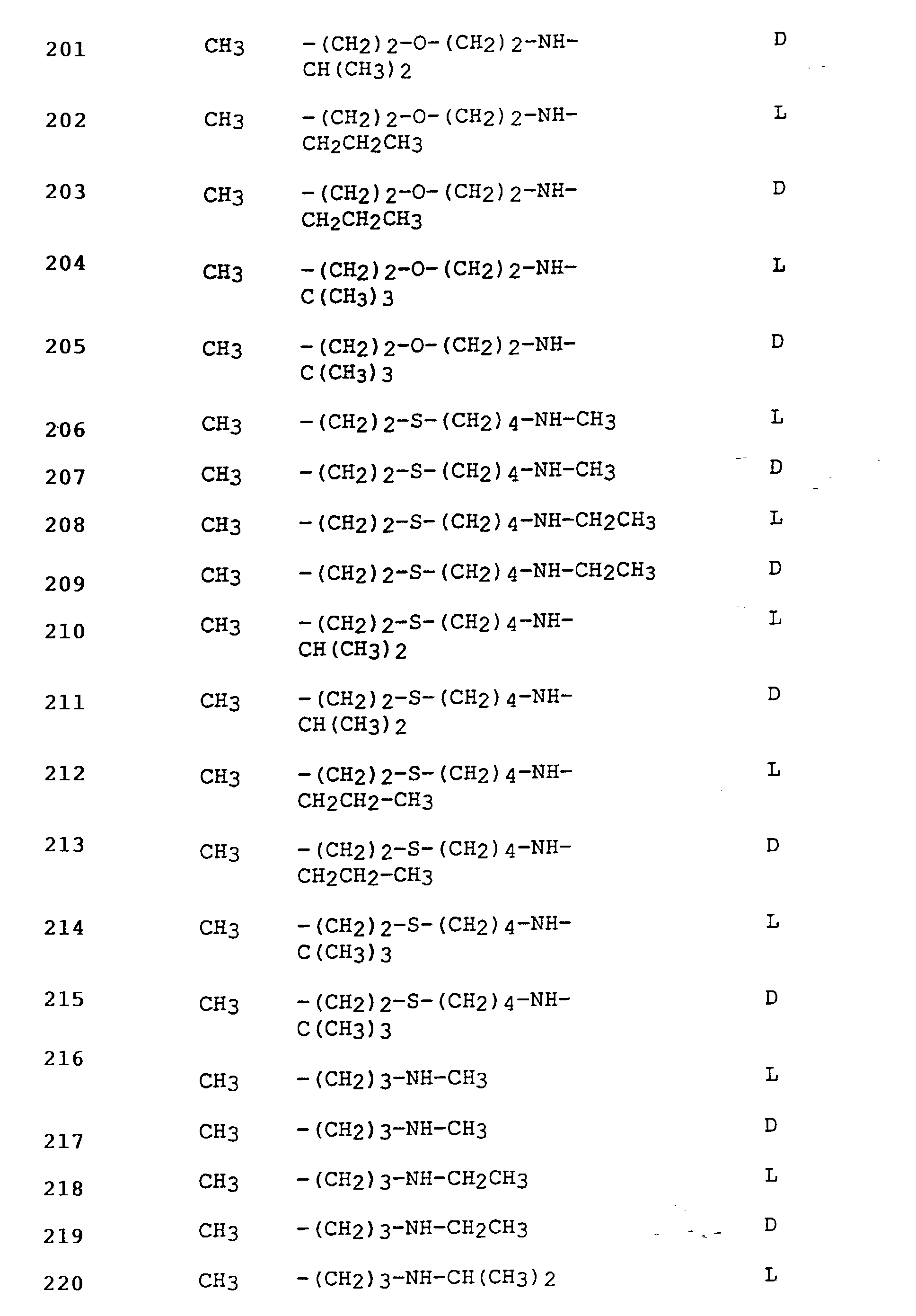

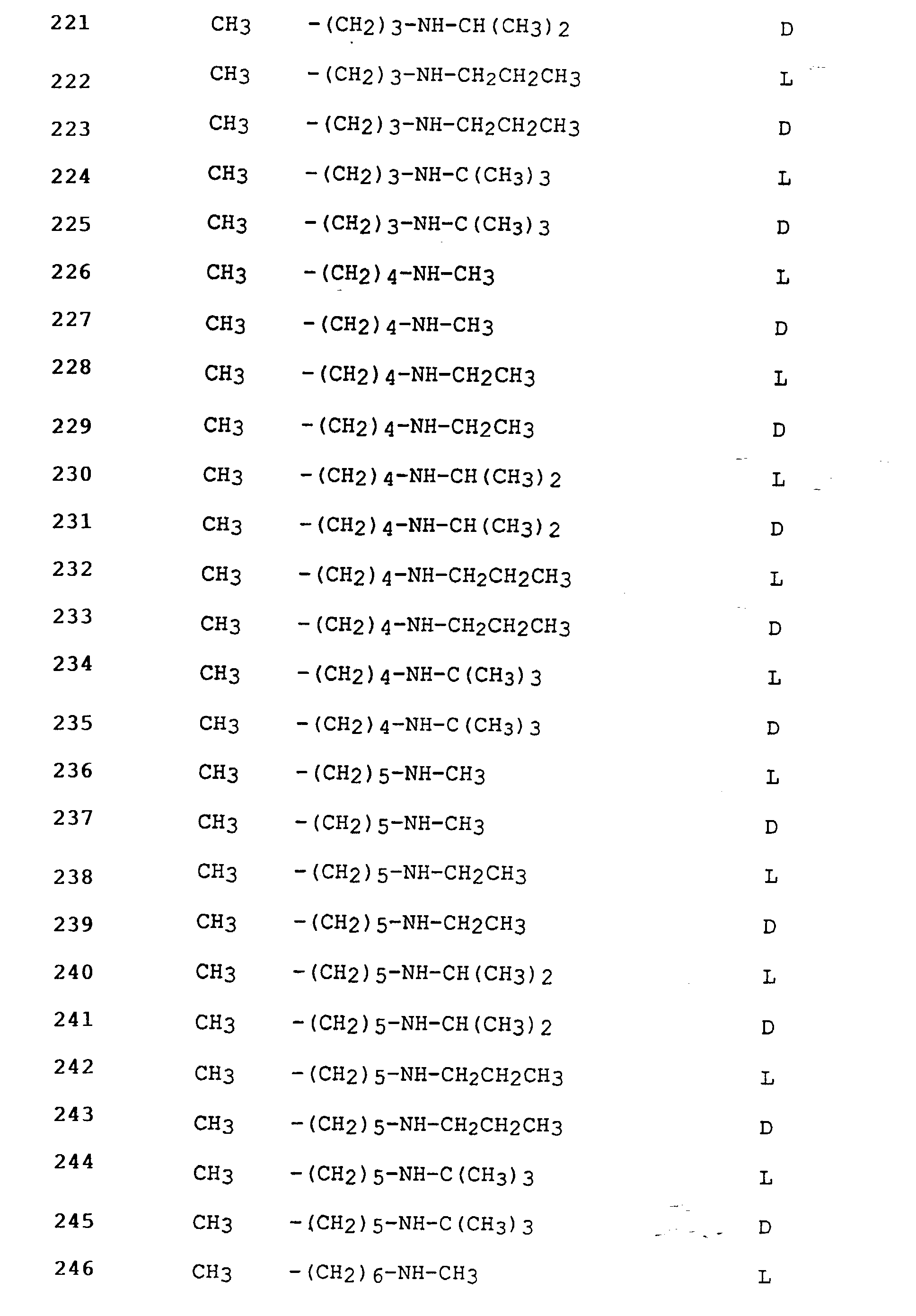

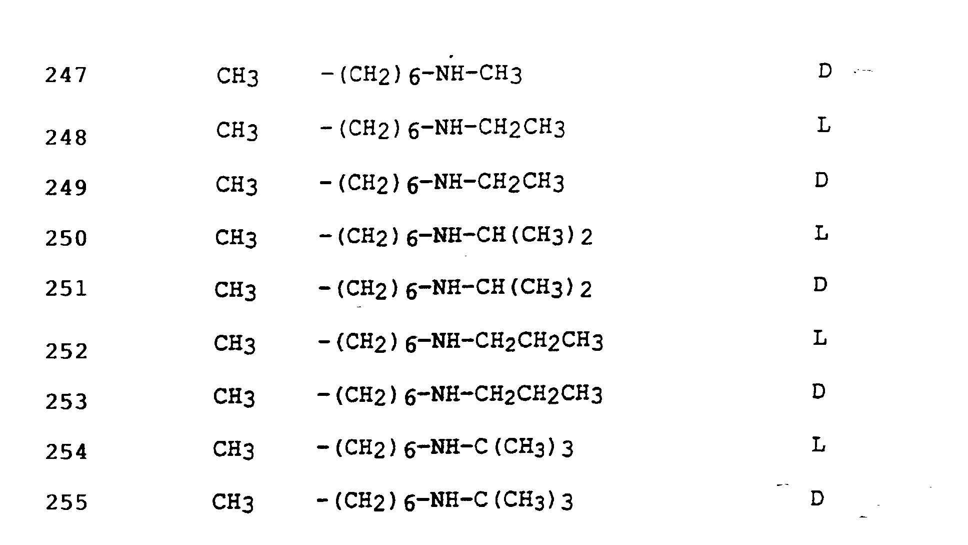

Example Numbers 256-431 have the same structure as Example Numbers 80-255, respectively, except that J = D-Abu rather than D-Val.

Example Numbers 432-607 have the same structure as Example Numbers 80-255, respectively, except that J = D-Phg rather than D-Val.

Example Numbers 608-783 have the same structure as Example Numbers 80-255, respectively, except that J = D-Pro rather than D-Val.

Example Numbers 784-959 have the same structure as Example Numbers 80-255, respectively, except that J = D-Nle, rather than D-Val.

Example Numbers 960-1135 have the same structure as Example Numbers 80-255, respectively, except that J = D-Norvaline, rather than D-Val.

## Claims (Claims for the following Contracting State(s): AT, BE, CH, LI, DE, DK, FR, GB, IE, IT, LU, MC, NL, SE)

1. A compound of the formula (I): 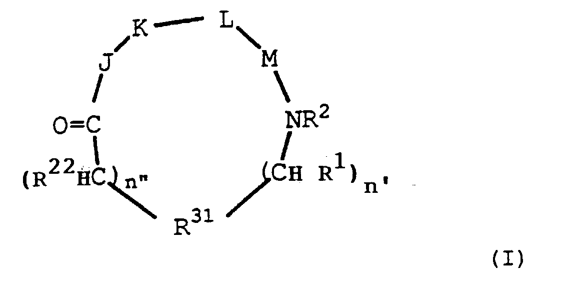 or a pharmaceutically acceptable salt or prodrug form thereof wherein:
R³¹ is
(a) a 6 membered aromatic carbocyclic ring of the formula 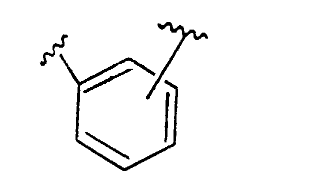 wherein said carbocyclic ring is substituted independently with 0-2 R¹⁰, or
(b) a 10 membered bicyclic carboxylic ring of the formula 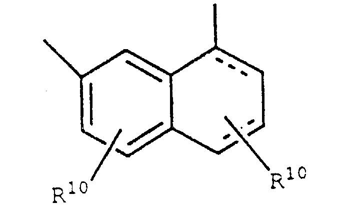 wherein the dashed bond may be a single or double bond;
n" is 0 or 1;
n' is 0 or 1;
R¹ and R²² are independently selected from the following groups:
hydrogen,
C₁-C₈ alkyl substituted with 0-2 R¹¹,
C₂-C₈ alkenyl substituted with 0-2 R¹¹,
C₂-C₈ alkynyl substituted with 0-2 R¹¹,
C₃-C₈ cycloalkyl substituted with 0-2 R¹¹,
C₆-C₁₀ bicycloalkyl substituted with 0-2 R¹¹,
aryl substituted with 0-2 R¹²,
a heterocylic ring system substituted with 0-2 R¹², composed of 5-10 atoms including 1-3 N, S, or O heteroatoms, =O, F, Cl, Br, I, -CN, -CO₂R¹³, -C(=O)R¹³, -C(=O)NR¹³R¹⁴, -CHO, -CH₂OR¹³, -OC(=O)R¹³, -OC(=O)OR¹³, -OR¹³, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -SR¹³, -S(=O)R^{13a}, -SO₂NR¹³R¹⁴, -CH₂NR¹³R¹⁴, -NR¹³R¹⁴, -NHC(=NH)NHR¹³, -C(=NH)NHR¹³,NO₂;
R¹¹ is selected from one or more of the following:
=O, F, Cl, Br, I, -CF₃, -CN, -CO₂R¹³, -C(=O)R¹³, -C(=O)NR¹³R¹⁴, -CH₂OR¹³, -OC(=O)R¹³, -OC(=O)OR¹³, -OR¹³, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -SR¹³, -S(=O)R^{13a},
-SO₂NR¹³R¹⁴, -CH₂NR¹³R¹⁴, -NR¹³R¹⁴, -NHC(=NH)NHR¹³, -C(=NH)NHR¹³, =NOR¹⁴, NO₂,
C₁-C₅ alkyl, C₂-C₄ alkenyl, C₃-C₆ cycloalkyl, C₃-C₆ cycloalkylmethyl, C₂-C₆ alkoxyalkyl, C₁-C₄ alkyl (substituted with -NR¹³R¹⁴, -CF₃, NO₂, -SO₂R¹³, or -S(=O)R^{13a})
aryl substituted with 0-2 R¹²,
a heterocylic ring system substituted with 0-2 R¹², composed of 5-10 atoms including 1-3 nitrogen, oxygen, or sulfur heteroatoms;
R¹² is selected from one or more of the following:
phenyl, benzyl, phenethyl, phenoxy, benzyloxy, halogen, hydroxy, nitro, cyano, C₁-C₅ alkyl, C₃-C₆ cycloalkyl, C₃-C₆ cycloalkylmethyl, C₇-C₁₀ arylalkyl, C₁-C₄ alkoxyl -CO₂R¹³, hydroxamic acid, hydrazide, oxime, boronic acid, sulfonamide, formyl, C₃-C₆ cycloalkoxy, -OC(=O)R¹³, -C(=O)R¹³,-OC (=O)OR¹³, -OR¹³, -CH₂OR¹³, -NR¹³R¹⁴, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -S(=O)R^{13a}, -SR¹³, -SO₂NR¹³R¹⁴, C₂-C₆ alkoxyalkyl, C₁-C₄ hydroxyalkyl, methylenedioxy, ethylenedioxy,
R¹³ is H, C₁-C₇ alkyl, aryl, -(C₁-C₆ alkyl)aryl, or C₃-C₆ alkoxyalkyl;
R^{13a} is C₁-C₇ alkyl, aryl, -(C₁-C₆ alkyl)aryl, or C₃-C₆ alkoxyalkyl;
R¹⁴ is OH, H, C₁-C₄ alkyl, or benzyl;
R² is H or C₁-C₈ alkyl;
R¹⁰ is selected from one or more of the following:
phenyl, benzyl, phenethyl, phenoxy, benzyloxy, halogen, hydroxy, nitro, cyano, C₁-C₅ alkyl, C₃-C₆ cycloalkyl, C₃-C₆ cycloalkylmethyl, C₇-C₁₀ arylalkyl, C₁-C₄ alkoxyl -CO₂R¹³, -C(=O)NHOR¹³, -C(=O)NHNR¹³R¹⁴, oxime, boronic acid, C₃-C₆ cycloalkoxy, -OC(=O)R¹³, -C(=O)R¹³,-OC(=O)OR¹³, -OR¹³, -Ch₂OR¹³, -NR¹³R¹⁴, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -S(=O)R^{13a}, -SR¹³, -SO₂NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -OCH₂CO₂H, 2-(1-morpholino)ethoxy, C₁-C₄ alkyl (substituted with -NR¹³R¹⁴, -CF_{3,} NO₂, or -S(=O)R^{13a});
J is β-Ala or an _{L}-isomer or _{D}-isomer amino acid of structure -N(R³)C(R⁴) (R⁵)C(=O)-, wherein:
R³ is H or CH₃
R⁴ is H or C₁-C₃ alkyl;
R⁵ is H, C₁-C₈ alkyl, C₃-C₆ cycloalkyl, C₃-C₆ cycloalkylmethyl, C₁-C₆ cycloalkylethyl, phenyl, phenylmethyl, CH₂OH, CH₂SH, CH₂OCH₃, CH₂SCH₃, CH₂CH₂SCH₃, (CH₂)ₛNH₂, (CH₂)ₛNHC(=NH) (NH₂), (CH₂)ₛNHR¹⁶, where s = 3-5;
R³ and R⁵ can also be taken together to form -(CH₂)ₜ- (t = 2-4) or -CH₂SC(CH₃)₂-; or
R⁴ and R⁵ can also be taken together to form -(CH₂)ᵤ-, where u = 2-5;
R¹⁶ is an amino protecting group;
K is a _{D}-isomer or _{L}-isomer amino acid of structure -N(R⁶)CH(R⁷)C(=O) -, wherein:
R⁶ is C₁-C₈ alkyl;
R⁷ is selected from:
-(C₁-C₇ alkyl)X; 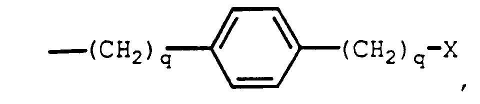 where q is independently 0,1; 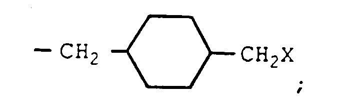 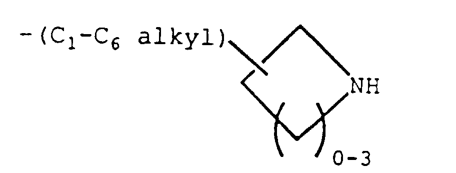 -(CH₂)ₘO-(C₁-C₄ alkyl)-X, where m = 1,2;
-(CH₂)ₘS-(C₁-C₄ alkyl)-X, where m = 1,2; and
X is selected from: 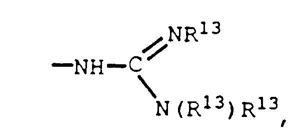 -N(R¹³)R¹³, -C(=NH)(NH₂), -SC(NH)-NH₂, wherein R¹³ is as defined above;
L is -Y(CH₂)ᵥC(=O)-, wherein:
Y is NH, N(C₁-C₃ alkyl), O, or S; and v = 1,2;
M is a _{D}-isomer or _{L}-isomer amino acid of structure -NR¹⁷-CH(R⁸) C(=O)-, wherein :
R¹⁷ is H, C₁-C₃ alkyl;
R⁸ is -CH₂CO₂R¹³, -CH₂SO₃R^{13a}, -CH(CH₃)CO₂R¹³, -SO₂NR¹³R¹⁴, -CH₂-boronic acid, or -CH₂-tetrazole.

2. The compound of claim 1, wherein:
R³¹ is selected from: 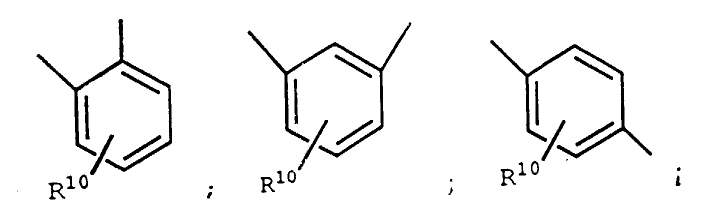 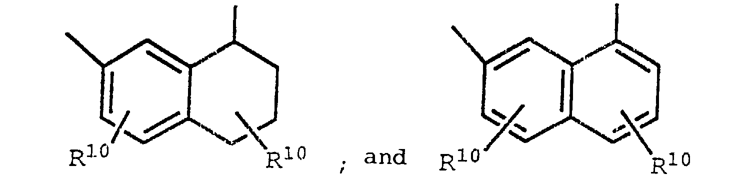 wherein R³¹ may be substituted independently with 0-2 R¹⁰;
R¹ and R²² are independently selected from H, C₁-C₄ alkyl, phenyl, benzyl, phenyl-(C₂-C₄) alkyl, C₁-C₄ alkoxy;
R¹⁰ is H, C₁-C₈ alkyl, phenyl, halogen, or C₁-C₄ alkoxy;
K is an _{L}-isomer amino acid of structure -N (R⁶)CH(R⁷)C(=O)-, wherein:
R⁶ is C₁-C₈ alkyl;
R⁷ is - (CH₂)ₚNHC(=NH) (NH₂), where p = 3-5; 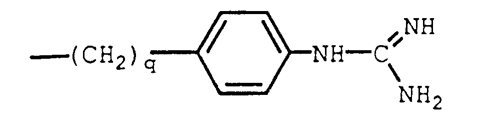 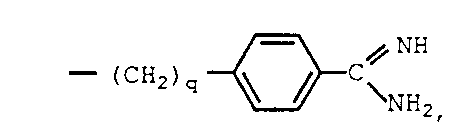 where q = 0,1;
-(CH₂)ᵣX, where r = 4-6; 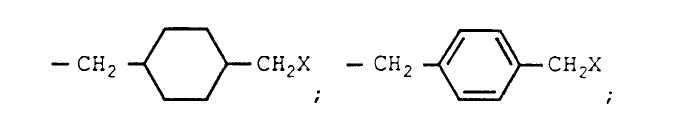 -(CH₂)ₘS(CH₂)₂X, where m = 1,2;
-(C₃-C₇ alkyl)-N-(C₁-C₆ alkyl) 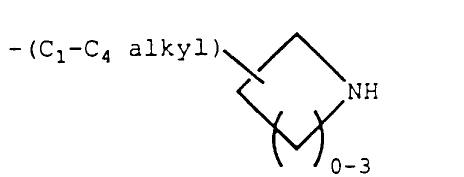 -(CH₂)ₘ-O-(C₁-C₄ alkyl)-NH-(C₁-C₆ alkyl), where m = 1,2;
-(CH₂)ₘ-S-(C₁-C₄ alkyl)-NH-(C₁-C₆ alkyl), where m = 1,2; and
X is -NH₂ or -NHC(=NH)(NH₂); or
L is -Y(CH₂)ᵥC(=O) -, wherein:
Y is NH, O, or S; and v = 1,2;
M is an _{L}-isomer amino acid of structure -NH-CH(R⁸)C(=O)-, wherein:
R⁸ is -CH₂CO₂H, or -CH(CH₃)CO₂H.

3. The compound of Claim 2 of the formula (II): 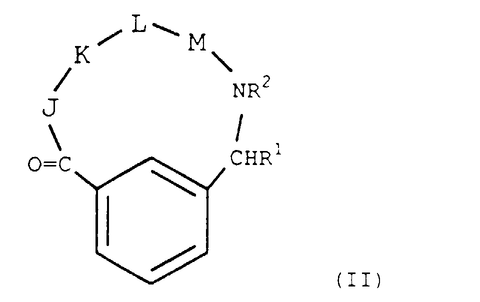 wherein:
R¹ is H, C₁-C₄ alkyl, phenyl, benzyl, or phenyl-(C₂-C₄)alkyl;
R² is H or methyl;
J is β-Ala or an _{L}-isomer or _{D}-isomer amino acid of structure -N(R³)C(R⁴)(R⁵)C(=O)-, wherein:
R³ is H or CH₃;
R⁴ is H or C₁-C₃ alkyl;
R⁵ is H, C₁-C₈ alkyl, C₃-C₆ cycloalkyl, C₃-C₆ cycloalkylmethyl, C₁-C₆ cycloalkylethyl, phenyl, phenylmethyl, CH₂OH, CH₂SH, CH₂OCH₃, CH₂SCH₃, CH₂CH₂SCH₃, (CH₂)ₛNH₂ or (CH₂)ₛNHC(=NH)(NH₂), where s = 3-5; or
R³ and R⁵ can be taken together to form -CH₂CH₂CH₂-; or
R⁴ and R⁵ can be taken together to form -(CH₂)ᵤ-, where u = 2-5;
K is an _{L}-isomer amino acid of structure -N(R⁶)CH(R⁷)C(=O)-, wherein:
R⁶ is C₁-C₈ alkyl;
R⁷ is -(CH₂)ₚNHC(=NH)(NH₂), where p = 3-5; 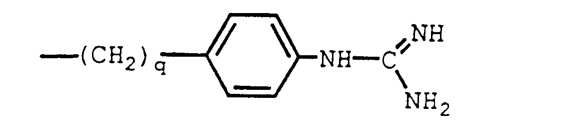 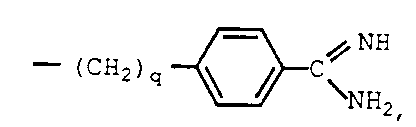 where q = 0,1;
-(CH₂)ᵣX,where r = 4-6; 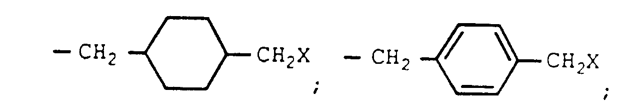 -(CH₂)ₘS(CH₂)₂X,where m = 1,2;
-(CH₂)ₘ-O-(C₁-C₄ alkyl)-NH-(C₁-C₆ alkyl), where m = 1,2;
-(CH₂)ₘ-S-(C₁-C₄ alkyl)-NH-(C₁-C₆ alkyl), where m = 1,2; and
-(C₃-C₇ alkyl)-N-(C₁-C₆ alkyl) 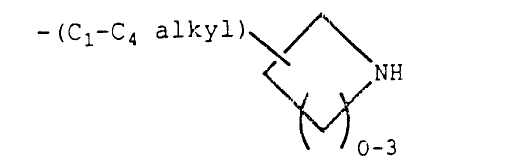
X is -NH₂ or -NHC(=NH)(NH₂),provided that X is not NH₂ when r = 4.

4. The compound of claim 3 wherein
J is β-Ala or an _{L}-isomer or _{D}-isomer amino acid of structure -N(R³)C(R⁴) (R⁵)C(=O)-, wherein:
R³ is H or CH₃;
R⁴ is H;
R⁵ is H, C₁-C₈ alkyl, C₃-C₆ cycloalkyl, C₃-C₆ cycloalkylmethyl, C₁-C6₄ cycloalkylethyl, phenyl, phenylmethyl, CH₂OH, CH₂SH, CH₂OCH₃, CH₂SCH₃, CH₂CH₂SCH₃, (CH₂)ₛNH₂ or (CH₂)ₛNHC(=NH)(NH₂), where s = 3, 4, 5; or
R³ and R⁵ can be taken together to form -CH₂CH₂CH₂-;
K is an _{L}-isomer amino acid of structure -N(CH₃) CH(R⁷)C(=O), wherein :
R⁷ is-(CH₂)ₚNHC(=NH) (NH₂), where p = 3-4; 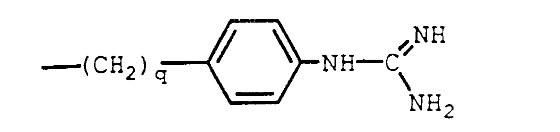 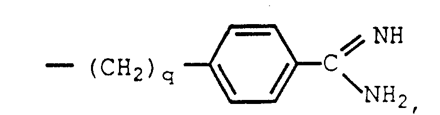 where q = 0,1;
-(CH₂)ᵣX,where r = 4-6; 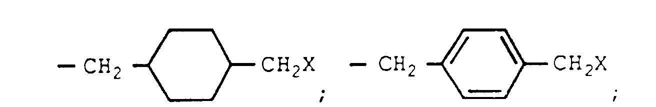 -(CH₂)ₘS(CH₂)₂X, where m = 1,2;
-(C₄-C₇ alkyl)-N-(C₁-C₆ alkyl) 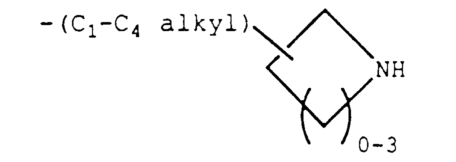 -(CH₂)ₘ-O-(C₁-C₄ alkyl)-NH-(C₁-C₆ alkyl), where m = 1,2;
- (CH₂)ₘ-S-(C₁-C₄ alkyl)-NH-(C₁-C₆ alkyl), where m = 1,2; and
X is -NH₂ or -NHC(=NH) (NH₂), provided that X is not -NH₂ when r = 4;
L is -YCH₂C(=O)-, wherein:
Y is NH or O;
M is an L-isomer amino acid of structure -NH-CH(CH₂CO₂H)C(=O)-.

5. The compound of claims 3 and 4 wherein:
R¹ is H;
R² is H;
J is β-Ala or an _{L}-isomer or _{D}-isomer amino acid of formula -N(R³)CH(R⁵)C(=O)-, wherein:
R³ is Hand R⁵ is H, CH₃, CH₂CH₃, CH(CH₃)₂, CH(CH₃)CH₂CH₃, CH₂CH₂CH₃, CH₂CH₂CH₂CH₃, CH₂CH₂SCH₃, CH₂CH(CH₃)₂, (CH₂)₄NH₂; or
R³ is CH₃ and R⁵ is H; or
R³ and R⁵ can be taken together to form -CH₂CH₂CH₂-.
K is an _{L}-isomer amino acid of formula -N(CH₃)CH(R⁷)C(=O)-, wherein:
R⁷ is- (CH₂)₃NHC(=NH)(NH₂);
L is -NHCH₂C(=O)-; and
M is an _{L}-isomer amino acid of formula -NHCH(CH₂COOH)C(=O)-.

6. The compound of claim 2 of the formula (III) 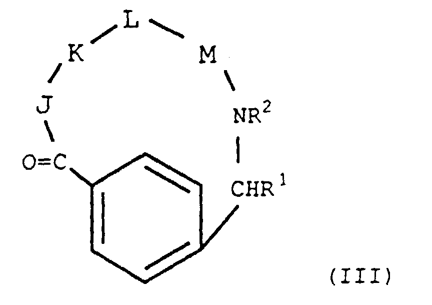 wherein:
R¹ is H, C₁-C₄ alkyl, phenyl, benzyl, or phenyl-(C₂-C₄)alkyl;
R² is H or methyl;
J is β-Ala or an _{L}-isomer or _{D}-isomer amino acid of structure -N(R³)C(R⁴)(R⁵)C =O)-, wherein:
R³ is H or CH₃;
R⁴ is H or C₁-C₃ alkyl;
R⁵ is H, C₁-C₈ alkyl, C₃-C₆ cycloalkyl, C₃-C₆ cycloalkylmethyl, C₁-C₆ cycloalkylethyl, phenyl , phenylmethyl, CH₂OH, CH₂SH, CH₂OCH₃, CH₂SCH₃, CH₂CH₂SCH₃, (CH₂)ₛNH₂ or (CH₂)ₛNHC(=NH)(NH₂), where s = 3-5; or
R³ and R⁵ can be taken together to form -CH₂CH₂CH₂-; or
R⁴ and R⁵ can be taken together to form -(CH₂)ᵤ-, where u = 2-5;
K is an _{L}-isomer amino acid of structure -N(R⁶)CH(R⁷)C(=O)-, wherein:
R⁶ is C₁-C₈ alkyl;
R⁷ is -(CH₂)ₚNHC(=NH) (NH₂), where p 3-4 ; 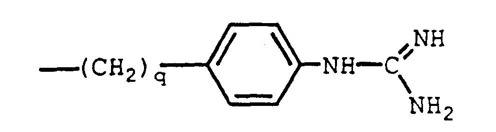 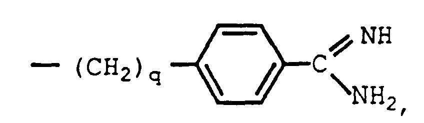 where q = 0,1;
-(CH₂)ᵣX,where r = 4-6; 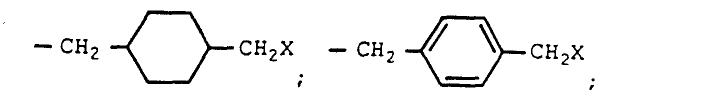 -(CH₂)ₘS(CH₂)₂X, where m = 1,2;
-(C₃-C₇ alkyl)-N-(C₁-C₆ alkyl) 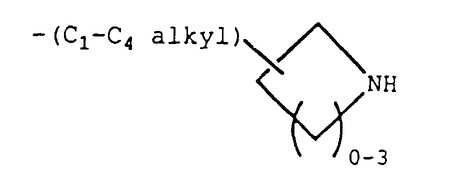 -(CH₂)ₘ-O-(C₁-C₄ alkyl)-NH-(C₁-C₆ alkyl), where m = 1,2;
-(CH₂)ₘ-S-(C₁-C₄ alkyl)-NH-(C₁-C₆ alkyl), where m = 1,2; and
X is -NH₂ or -NHC(=NH) (NH₂), provided that X is not NH₂ when r = 4.

7. The compound of claim 1 which is selected from: the compound of formula (II): 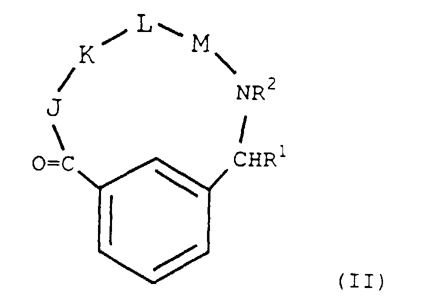 wherein R¹ and R² are H; J is _{D}-Val; K is NMeArg; L is Gly; and M is Asp;
the compound of formula II) wherein R¹ and R² are H; J is _{D}-2-aminobutyric acid; K is NMeArg; L is Gly; and M is Asp;
the compound of formula (II) wherein R¹ and R² are H; J is _{D}-Leu; K is NMeArg; L is Gly; and M is Asp;
the compound of formula (II) wherein R¹ and R² are H; J is _{D}-Ala; K is NMeArg; L is Gly; and M is Asp;
the compound of formula (II) wherein R¹ and R² are H; J is Gly; K is NMeArg; L is Gly; and M is Asp;
the compound of formula (II) wherein R¹ and R² are H; J is D-Pro; K is NMeArg; L is Gly; and M is Asp;
the compound of formula (II) wherein R¹ and R² are H; J is _{D}-Lys; K is NMeArg; L is Gly; and M is Asp;
the compound of formula (II) wherein R¹ and R² are H; J is β-Ala; K is NMeArg; L is Gly; and M is Asp;
the compound of formula (II) wherein R¹ and R² are H; J is _{D}-Val; K is NMeArg; L is β-Ala; and M is Asp;
the compound of formula (II) wherein R¹ and R² are H; J is Pro; K is NMeArg; L is Gly; and M is Asp;
the compound of formula (II) wherein R¹ and R² are H; J is NMeGly; K is NMeArg; L is Gly; and M is Asp;
the compound of formula (II) wherein R¹ is methyl (isomer 1); R² are H; J is _{D}-Val; K is NMeArg; L is Gly; and M is Asp;
the compound of formula (II) wherein R¹ is methyl (isomer 2); R² are H; J is _{D}-Val; K is NMeArg; L is Gly; and M is Asp;
the compound of formula (II) wherein R¹ is phenyl (isomer 1); R² are H; J is D-Val; K is NMeArg; L is Gly; and M is Asp;
the compound of formula (II) wherein R¹ is phenyl (isomer 2); R² are H; J is _{D}-Val; K is NMeArg; L is Gly; and M is Asp;
the compound of formula (II) wherein R¹ and R² are H; J is D-Val; K is D-NMeArg; L is Gly; and M is Asp;
the compound of formula (II) wherein R¹ and R² are H; J is D-Nle; K is NMeArg; L is Gly; and M is Asp;
the compound of formula (II) wherein R¹ and R² are H; J is D-Phg; K is NMeArg; L is Gly; and M is Asp;
the compound of formula (II) wherein R¹ and R² are H; J is D-Ile; K is NMeArg; L is Gly; and M is Asp;
the compound of formula (II) wherein R¹ and R² are H; J is D-Phe; K is NMeArg; L is Gly; and M is Asp;
the compound of formula (II) wherein R¹ and R² are H; J is NMeGly; K is NMeArg; L is Gly; and M is Asp;
the compound of formula (II) wherein R¹ and R² are H; J is D-Nle; K is NMeArg; L is Gly; and M is Asp;
the compound of formula (II) wherein R¹ and R² are H; J is D-Tyr; K is NMeArg; L is Gly; and M is Asp;
the compound of formula (II) wherein R¹ and R² are H; J is D-Val; K is NMeAmf; L is Gly; and M is Asp;
the compound of formula (II) wherein R¹ and R² are H; J is D-Val; K is NMeArg; L is Ala; and M is Asp;
the compound of formula (II) wherein R¹ and R² are H; J is D-Val; K is NMeArg; L is Gly; and M is α-MeAsp;
the compound of formula (II) wherein R¹ and R² are H; J is D-Val;; K is NMeArg; L is Gly; and M is β-MeAsp;
the compound of formula (II) wherein R¹ and R² are H; J is D-Val; K is NMeArg; L is Gly; and M is NMeAsp;
the compound of formula (II) wherein R¹ and R² are H; J is D-Val; K is NMeArg; L is Gly; and M is D-Asp;
the compound of formula (II) wherein R¹ is H; R² is CH₃; J is D-Val; K is NMeArg; L is Gly; and M is Asp;
the compound of formula (II) wherein R¹ and R² are H; J is D-Abu; K is di-NMeOrn; L is Gly; and M is Asp;
the compound of formula (III): 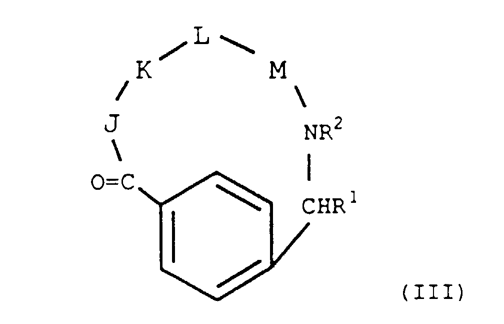 wherein R1 and R2 are H; J is _{D}-Val; K is NMeArg; L is Gly; and M is Asp; the compound of formula (V): 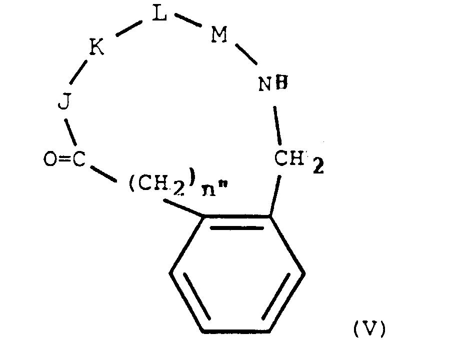 wherein n"=1; J is D-Val; K is NMeArg; L is Gly; and M is Asp;
the compound of formula (V) wherein n"=0; J is D-Val; K is NMeArg; L is Gly; and M is Asp;
the compound of formula (VI): 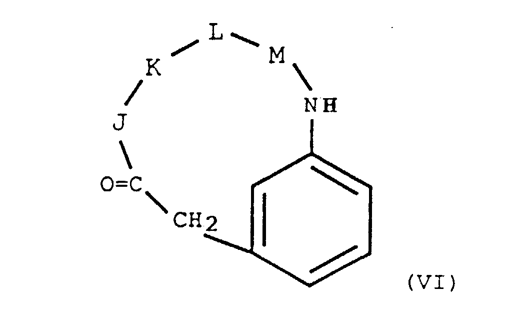 wherein
J is D-Val; K is NMeArg; L is Gly; and M is Asp;
the compound of formula (VII): 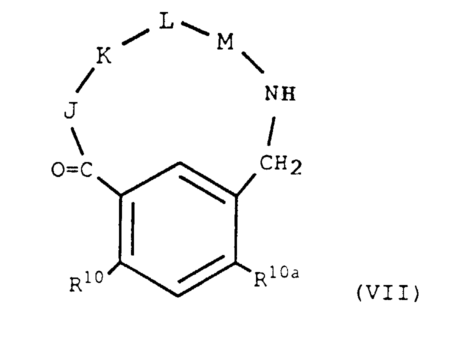 wherein R¹⁰ is H; R^{10a} is Cl; J is D-Val; K is NMeArg; L is Gly; and M is Asp;
the compound of formula (VII) wherein R¹⁰ is H; R^{10a} is I; J is D-Val; K is NMeArg; L is Gly; and M is Asp;
the compound of formula (VII) wherein R¹⁰ is H; R^{10a} is I; J is D-Abu; K is NMeArg; L is Gly; and M is Asp;
the compound of formula (VII) wherein R¹⁰ is H; R^{10a} is OMe; J is D-Val; K is NMeArg; L is Gly; and M is Asp;
the compound of formula (VII) wherein R¹⁰ is H; R^{10a} is Me; J is D-Val; K is NMeArg; L is Gly; and M is Asp;
the compound of formula (VII) wherein R^{10a} is H; R¹⁰ is Cl; J is D-Val; K is NMeArg; L is Gly; and M is Asp;
the compound of formula (VII) wherein R^{10a} is H; R¹⁰ is MeO; J is D-Val; K is NMeArg; L is Gly; and M is Asp;
the compound of formula (VII) wherein R^{10a} is H; R¹⁰ is Me; J is D-Val; K is NMeArg; L is Gly; and M is Asp;
the compound of formula (VIII) : 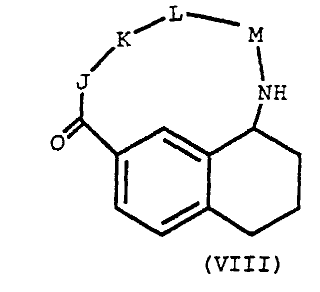 wherein J is D-Val; K is NMeArg; L is Gly; and M is Asp;
the compound of formula (IX) : 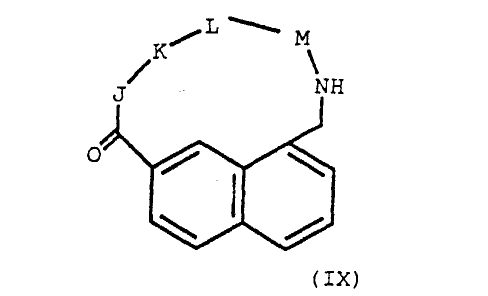 wherein J is D-Val; K is NMeArg; L is Gly; and M is Asp.

8. The compound of claim 7 which is selected from: the compound of formula (II) wherein R¹ and R² are H; J is D-Val; K is NMeArg; L is Gly; and M is Asp;
the compound of formula (II) wherein R¹ and R² are H; J is D-2-aminobutyric acid; K is NMeArg; L is Gly; and M is Asp;
the compound of formula (II) wherein R¹ and R² are H; J is D-Leu; K is NMeArg; L is Gly; and M is Asp;
the compound of formula (II) wherein R¹ and R² are H; J is D-Ala; K is NMeArg; L is Gly; and M is Asp;
the compound of formula (II) wherein R¹ and R² are H; J is Gly; K is NMeArg; L is Gly; and M is Asp;
the compound of formula (II) wherein R¹ and R² are H; J is D-Pro; K is NMeArg; L is Gly; and M is Asp;
the compound of formula (II) wherein R¹ and R² are H; J is D-Lys; K is NMeArg; L is Gly; and M is Asp;
the compound of formula (II) wherein R¹ and R² are H; J is β-Ala; K is NMeArg; L is Gly; and M is Asp;
the compound of formula (II) wherein R¹ and R² are H; J is NMeGly; K is NMeArg; L is Gly; and M is Asp;
the compound of formula (II) wherein R¹ is methyl (isomer 1); R² are H; J is D-Val; K is NMeArg; L is Gly; and M is Asp;
the compound of formula (II) wherein R¹ is methyl (isomer 2); R² are H; J is D-Val; K is NMeArg; L is Gly; and M is Asp;
the compound of formula (II) wherein R¹ is phenyl (isomer 1); R² are H; J is D-Val; K is NMeArg; L is Gly; and M is Asp;
the compound of formula (II) wherein R¹ and R² are H; J is D-Val; K is D-NMeArg; L is Gly; and M is Asp;
the compound of formula (II) wherein R¹ and R² are H; J is D-Nle; K is NMeArg; L is Gly; and M is Asp;
the compound of formula (II) wherein R¹ and R² are H; J is D-Phg; K is NMeArg; L is Gly; and M is Asp;
the compound of formula (II) wherein R¹ and R² are H; J is D-Phe; K is NMeArg; L is Gly; and M is Asp;
the compound of formula (II) wherein R¹ and R² are H; J is D-Ile; K is NMeArg; L is Gly; and M is Asp;
the compound of formula (III) wherein R¹ and R² are H; J is D-Val; K is NMeArg; L is Gly; and M is Asp;
the compound of formula (V) wherein n" = 1; J is D-Val; K is NMeArg; L is Gly and M is Asp;
the compound of formula (V) wherein n" = 0; J is D-Val; K is NMeArg; L is Gly and M is Asp;
the compound of formula (VI) wherein J is D-Val; K is NMeArg; L is Gly; and M is Asp;
the compound of formula (VII) wherein R¹⁰ is H; R^{10a} is Cl; J is D-Val; K is NMeArg; L is Gly; and M is Asp;
the compound of formula (VII) wherein R¹⁰ is H; R^{10a} is I; J is D-Val; K is NMeArg; L is Gly; and M is Asp;
the compound of formula (VII) wherein R10 is H; R^{10a} is I; J is D-Abu; K is NMeArg; L is Gly; and M is Asp;
the compound of formula (VII) wherein R10 is H; R^{10a} is Me; J is D-Val; K is NMeArg; L is Gly; and M is Asp;
the compound of formula (VII) wherein R^{10a} is H; R¹⁰ is Cl; J is D-Val; K is NMeArg; L is Gly; and M is Asp;
the compound of formula (VII) wherein R^{10a} is H; R¹⁰ is MeO; J is D-Val; K is NMeArg; L is Gly; and M is Asp;
the compound of formula (VII) wherein R^{10a} is H; R¹⁰ is Me; J is D-Val; K is NMeArg; L is Gly; and M is Asp;
the compound of formula (II) wherein R¹ and R² are H; J is D-Tyr; K is NMeArg; L is Gly; and M is Asp;
the compound of formula (II) wherein R¹ and R² are H; J is D-Val; K is NMeAmf; L is Gly; and M is Asp;
the compound of formula (II) wherein R¹ and R² are H; J is D-Val;; K is NMeArg; L is Gly; and M is bMeAsp;
the compound of formula (II) wherein R¹ is H; R² is CH₃; J is D-Val; K is NMeArg; L is Gly; and M is Asp;
the compound of formula (III) wherein R¹ and R² are H; J is D-Val; K is NMeArg; L is Gly; and M is Asp;
the compound of formula (VIII) wherein J is D-Val; K is NMeArg; L is Gly; and M is Asp.

9. The compound of Claim 8 selected from:
the compound of formula (II) wherein R¹ and R² are H; J is D-Val; K is NMeArg; L is Gly; and M is Asp;
the compound of formula (II) wherein R¹ and R² are H; J is D-2-aminobutyric acid; K is NMeArg; L is Gly; and M is Asp;
the compound of formula (II) wherein R¹ and R² are H; J is D-Leu; K is NMeArg; L is Gly; and M is Asp;
the compound of formula (II) wherein R¹ and R² are H; J is D-Ala; K is NMeArg; L is Gly; and M is Asp;
the compound of formula (II) wherein R¹ and R² are H; J is D-Pro; K is NMeArg; L is Gly; and M is Asp;
the compound of formula (II) wherein R¹ and R² are H; J is D-Lys; K is NMeArg; L is Gly; and M is Asp;
the compound of formula (II) wherein R¹ and R² are H; J is D-Nle; K is NMeArg; L is Gly; and M is Asp;
the compound of formula (II) wherein R¹ and R² are H; J is D-Phe; K is NMeArg; L is Gly; and M is Asp;
the compound of formula (II) wherein R¹ and R² are H; J is D-Ile; K is NMeArg; L is Gly; and M is Asp;
the compound of formula (V) wherein n" = 1; J is D-Val; K is NMeArg; L is Gly; and M is Asp;
the compound of formula (VI) wherein J is D-Val; K is NMeArg; L is Gly; and M is Asp;
the compound of formula (VII) wherein R¹⁰ is H; R^{10a} is Cl; J is D-Val; K is NMeArg; L is Gly; and M is Asp;
the compound of formula (VII) wherein R¹⁰ is H; R^{10a} is I; J is D-Val; K is NMeArg; L is Gly; and M is Asp;
the compound of formula (VII) wherein R¹⁰ is H; R^{10a} is Me; J is D-Val; K is NMeArg; L is Gly; and M is Asp;
the compound of formula (VII) wherein R^{10a} is H; R¹⁰ is Cl; J is D-Val; K is NMeArg; L is Gly; and M is Asp;
the compound of formula (VII) wherein R^{10a} is H; R¹⁰ is MeO; J is D-Val; K is NMeArg; L is Gly; and M is Asp;
the compound of formula (VII) wherein R^{10a} is H; R¹⁰ is Me; J is D-Val; K is NMeArg; L is Gly; and M is Asp;
the compound of formula (II) wherein R¹ and R² are H; J is D-Val;; K is NMeArg; L is Gly; and M is bMeAsp.

10. A compound of formula (I): 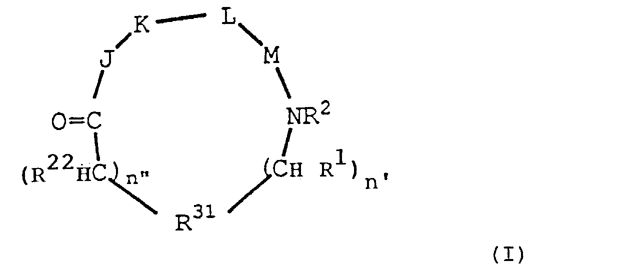 or a pharmaceutically acceptable salt or prodrug form thereof wherein:
R³¹ is selected from: 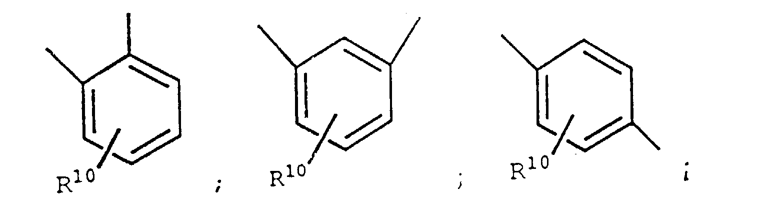 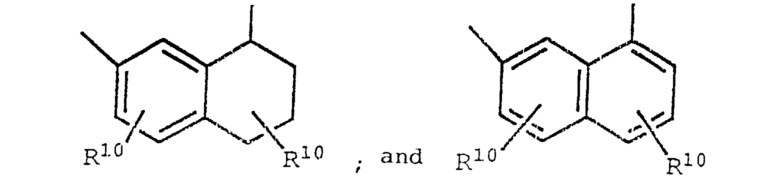 wherein R³¹ may be substituted independently with 0-2 R¹⁰;
n" is 0 or 1;
n' is 0 or 1;
R¹ and R²² are independently selected from H, C₁-C₄ alkyl, phenyl, benzyl, phenyl- (C₂-C₄)alkyl, C₁-C₄ alkoxy;
R² is H or C₁-C₈ alkyl;
R¹⁰ is H, C₁-C₈ alkyl, phenyl, halogen, or C₁-C₄ alkoxy;
J is β-Ala or an _{L}-isomer or _{D}-isomer amino acid of structure -N(R³)C(R⁴)(R⁵)C(=O)-, wherein:
R³ is H or CH₃
R⁴ is H or C₁-C₃ alkyl;
R⁵ is H, C₁-C₈ alkyl, C₃-C₆ cycloalkyl, C₃-C₆ cycloalkylmethyl, C₁-C₆ cycloalkylethyl, phenyl, phenylmethyl, CH₂OH, CH₂SH, CH₂OCH₃, CH₂SCH₃, CH₂CH₂SCH₃, (CH₂)ₛNH₂, (CH₂)ₛNHC(=NH)(NH₂), (CH₂)ₛNHR¹⁶, where s = 3-5;
R³ and R⁵ can also be taken together to form -(CH₂)ₜ- (t = 2-4) or -CH₂SC(CH₃)₂-; or
R⁴ and R⁵ can also be taken together to form -(CH₂)ᵤ-, where u = 2-5;
R¹⁶ is an amino protecting group
K is an _{L}-isomer amino acid of structure -N(R⁶)CH(R⁷)C(=O)-, wherein:
R⁶ is C₁-C₈ alkyl;
R⁷ is -(CH₂)ₚNHC(=NH)(NH₂), where p = 3-5; 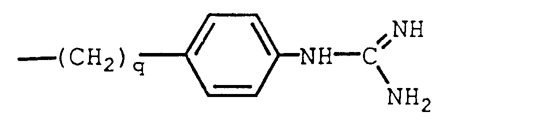 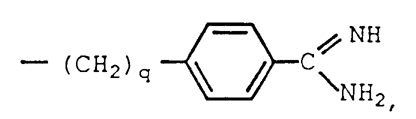 where q = 0,1;
-(CH₂)ᵣX,where r = 4-6; 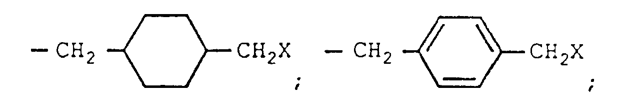 -(CH₂)ₘS(CH₂)₂X, where m = 1,2;
-(C₃-C₇ alkyl)-N-(C₁-C₆ alkyl) 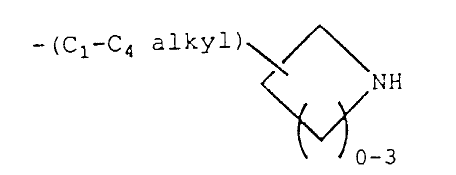 -(CH₂)ₘ-O-(C₁-C₄ alkyl)-N-(C₁-C₆ alkyl), where m = 1,2;
-(CH₂)ₘ-S-(C₁-C₄ alkyl)-NH-(C₁-C₆ alkyl), where m = 1,2; and
X is -NH₂ or -NHC(=NH)(NH₂);
L is -Y(CH₂)ᵥC(=O)-, wherein:
Y is NH, O, or S; and v = 1,2;
M is an _{L}-isomer amino acid of structure -NH-CH (CH₂SO₃H)C(=O)-.

11. Use of a compound according to any of claims 1-10 for preparing a medicament for the treatment of thromboembolic disorders.

12. A pharmaceutical composition comprising a pharmaceutically acceptable carrier and a therapeutically effective amount of a compound according to any of claims 1-10.

## Claims (Claims for the following Contracting State(s): GR)

1. A compound of the formula (I): 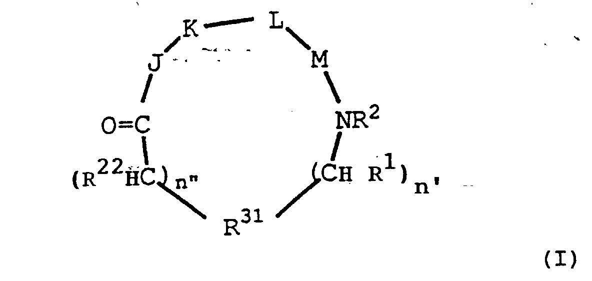 or a pharmaceutically acceptable salt or prodrug form thereof wherein:
R³¹ is
(a) a 6 membered aromatic carbocyclic ring of the formula 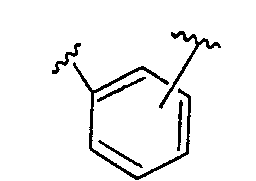 wherein said carbocyclic ring is substituted independently with 0-2 R¹⁰, or
(b) a 10 membered bicyclic carboxylic ring of the formula 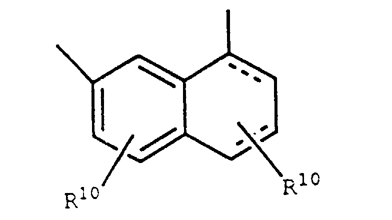 wherein the dashed bond may be a single or double bond;
n" is 0 or 1;
n' is 0 or 1;
R¹ and R²² are independently selected from the following groups:
hydrogen, C₁-C₈ alkyl substituted with 0-2 R¹¹,
C₂-C₈ alkenyl substituted with 0-2 R¹¹,
C₂-C₈ alkynyl substituted with 0-2 R¹¹,
C₃-C₈ cycloalkyl substituted with 0-2 R¹¹,
C₆-C₁₀ bicycloalkyl substituted with 0-2 R¹¹,
aryl substituted with 0-2 R¹²,
a heterocylic ring system substituted with 0-2 R¹², composed of 5-10 atoms including 1-3 N, S, or O heteroatoms,
=O, F, Cl, Br, I, -CN, -CO₂R¹³, -C(=O)R¹³, -C(=O)NR¹³R¹⁴, -CHO, -CH₂OR¹³, -OC(=O)R¹³, -OC(=O)OR¹³, -OR¹³, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -SR¹³, S(=O)R^{13a}, -SO₂NR¹³R¹⁴, -CH₂NR¹³R¹⁴, -NR¹³R¹⁴, -NHC(=NH)NHR¹³, -C(=NH)NHR¹³,NO₂;
R¹¹ is selected from one or more of the following:
=O, F, Cl, Br, I, -CF₃, -CN, -CO₂R¹³, -C(=O)R¹³, -C(=O)NR¹³R¹⁴, -CH₂OR¹³, -OC(=O)R¹³, -OC(=O)OR¹³, -OR¹³, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -SR¹³, -S(=O)R^{13a},
-SO₂NR¹³R¹⁴, -CH₂NR¹³R¹⁴, -NR¹³R¹⁴, -NHC(=NH)NHR¹³, -C(=NH)NHR¹³, =NOR¹⁴, NO₂,
C₁-C₅ alkyl, C₂-C₄ alkenyl, C₃-C₆ cycloalkyl, C₃-C₆ cycloalkylmethyl, C₂-C₆ alkoxyalkyl, C₁-C₄ alkyl (substituted with -NR¹³R¹⁴, -CF₃, NO₂, -SO₂R¹³, or -S(=O)R^{13a})
aryl substituted with 0-2 R¹²,
a heterocylic ring system substituted with 0-2 R¹², composed of 5-10 atoms including 1-3 nitrogen, oxygen, or sulfur heteroatoms;
R¹² is selected from one or more of the following:
phenyl, benzyl, phenethyl, phenoxy, benzyloxy, halogen, hydroxy, nitro, cyano, C₁-C₅ alkyl, C₃-C₆ cycloalkyl, C₃-C₆ cycloalkylmethyl, C₇-C₁₀ arylalkyl, C₁-C₄ alkoxyl -CO₂R¹³, hydroxamic acid, hydrazide, oxime, boronic acid, sulfonamide, formyl, C₃-C₆ cycloalkoxy, -OC(=O)R¹³, -C(=O)R¹³,-OC(=O)OR¹³, -OR¹³, -CH₂OR¹³, -NR¹³R¹⁴, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -S(=O)R^{13a}, -SR¹³, -SO₂NR¹³R¹⁴, C₂-C₆ alkoxyalkyl, C₁-C₄ hydroxyalkyl, methylenedioxy, ethylenedioxy,
R¹³ is H, C₁-C₇ alkyl, aryl, -(C₁-C₆ alkyl)aryl, or C₃-C₆ alkoxyalkyl;
R^{13a} is C₁-C₇ alkyl, aryl, -(C₁-C₆ alkyl)aryl, or C₃-C₆ alkoxyalkyl;
R¹⁴ is OH, H, C₁-C₄ alkyl, or benzyl;
R² is H or C₁-C₈ alkyl;
R¹⁰ is selected from one or more of the following:
phenyl, benzyl, phenethyl, phenoxy, benzyloxy, halogen, hydroxy, nitro, cyano, C₁-C₅ alkyl, C₃-C₆ cycloalkyl, C₃-C₆ cycloalkylmethyl, C₇-C₁₀ arylalkyl, C₁-C₄ alkoxyl -CO₂R¹³, -C(=O)NHOR¹³, -C(=O)NHNR¹³R¹⁴, oxime, boronic acid, C₃-C₆ cycloalkoxy, -OC(=O)R¹³, -C(=O)R¹³, -OC(=O)OR¹³, -OR¹³, -Ch₂OR¹³, -NR¹³R¹⁴, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -S(=O)R^{13a}, -SR¹³, -SO₂NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C1-C4 alkylcarbonylamino, -OCH₂CO₂H, 2-(1-morpholino)ethoxy, C₁-C₄ alkyl (substituted with -NR¹³R¹⁴, -CF₃, NO₂, or -S(=O)R^{13a});
J is β-Ala or an _{L}-isomer or _{D}-isomer amino acid of structure -N(R³)C(R⁴) (R⁵)C(=O)-, wherein:
R³ is H or CH₃
R⁴ is H or C₁-C₃ alkyl;
R⁵ is H, C₁-C₈ alkyl, C₃-C₆ cycloalkyl, C₃-C₆ cycloalkylmethyl, C₁-C₆ cycloalkylethyl, phenyl, phenylmethyl, CH₂OH, CH₂SH, CH₂OCH₃, CH₂SCH₃, CH₂CH₂SCH₃, (CH₂)ₛNH₂, (CH₂)_{S}NHC(=NH)(NH₂), (CH₂)ₛNHR¹⁶, where s = 3-5;
R³ and R⁵ can also be taken together to form -(CH₂)ₜ- (t = 2-4) or -CH₂SC(CH₃)₂-; or
R⁴ and R⁵ can also be taken together to form -(CH₂)ᵤ-, where u = 2-5;
R¹⁶ is an amino protecting group;
K is a _{D}-isomer or _{L}-isomer amino acid of structure -N(R⁶)CH(R⁷)C(=O)-, wherein:
R⁶ is C₁-C₈ alkyl;
R⁷ is selected from:
- (C₁-C₇ alkyl)X; 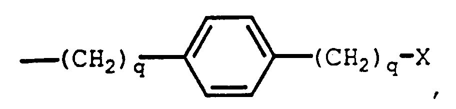 where q is independently 0,1; 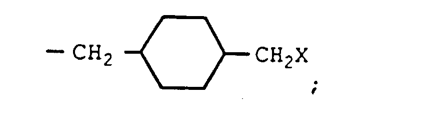 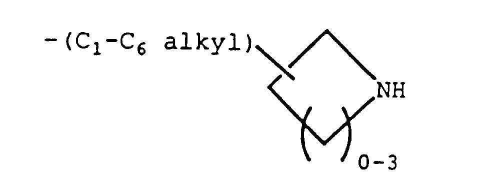 - (CH₂)ₘO-(C₁-C₄ alkyl)-X, where m = 1,2;
-(CH₂)ₘS-(C₁-C₄ alkyl)-X, where m = 1,2; and
X is selected from: 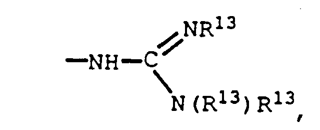 -N(R¹³)R¹³, -C(=NH)(NH₂), -SC(NH)-NH₂, wherein R¹³ is as defined above;
L is -Y(CH₂)ᵥC(=O)-, wherein:
Y is NH, N(C₁-C₃ alkyl), O, or S; and v = 1,2;
M is a _{D}-isomer or _{L}-isomer amino acid of structure -NR¹⁷-CH(R⁸)C(=O) -, wherein:
R¹⁷ is H, C₁-C₃ alkyl;
R⁸ is -CH₂CO₂R¹³,-CH₂SO₃R^{13a}, -CH(CH₃)CO₂R¹³, -SO₂NR¹³R¹⁴, -CH₂-boronic acid, or -CH₂-tetrazole.

2. The compound of claim 1, wherein:
R³¹ is selected from: 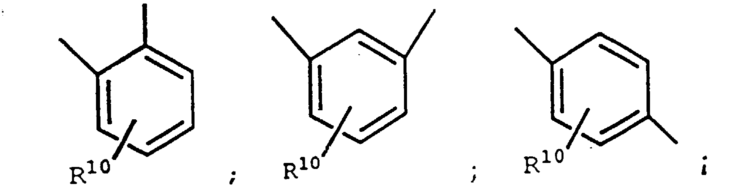 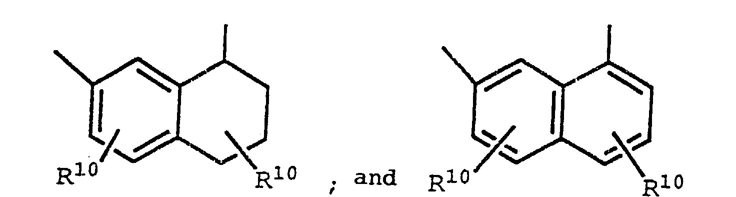 wherein R³¹ may be substituted independently with 0-2 R¹⁰; R¹ and R²² are independently selected from H, C₁-C₄ alkyl, phenyl, benzyl, phenyl-(C₂-C₄)alkyl, C₁-C₄ alkoxy;
R¹⁰ is H, C₁-C₈ alkyl, phenyl, halogen, or C₁-C₄ alkoxy;
K is an _{L}-isomer amino acid of structure -N(R⁶)CH(R⁷)C(=O)-, wherein:
R⁶ is C₁-C₈ alkyl;
R⁷ is- (CH₂)ₚNHC(=NH)(NH₂), where p = 3-5; 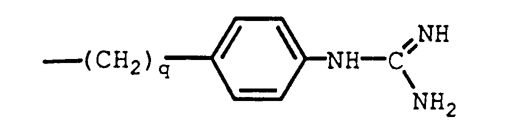 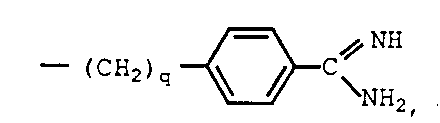 where q = 0,1;
-(CH₂)ᵣX,where r = 4-6; 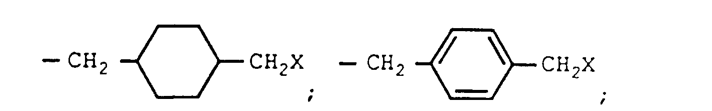 -(CH₂)ₘS(CH₂)₂X, where m = 1,2;
-(C₃-C₇ alkyl)-N-(C₁-C₆ alkyl) 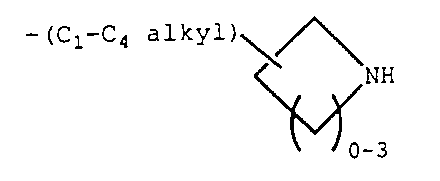 -(CH₂)ₘ-O-(C₁-C₄ alkyl)-NH-(C₁-C₆ alkyl), where m = 1,2;
-(CH₂)ₘ-S-(C₁-C₄ alkyl)-NH-(C₁-C₆ alkyl), where m = 1,2; and
X is -NH₂ or -NHC(=NH)(NH₂); or
L is -Y(CH₂)ᵥC(=O)-, wherein:
Y is NH, O, or S; and v = 1,2;
M is an _{L}-isomer amino acid of structure -NH-CH(R⁸)C(=O)-, wherein:
R⁸ is -CH₂CO₂H, or -CH(CH₃)CO₂H.

3. The compound of Claim 2 of the formula (II): 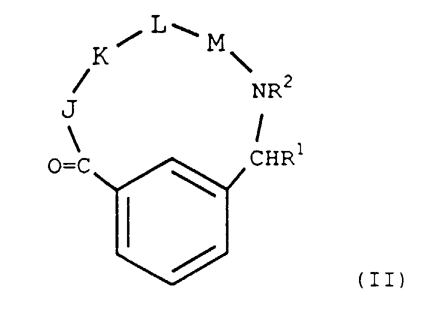 wherein:
R¹ is H, C₁-C₄ alkyl, phenyl, benzyl, or phenyl-(C₂-C₄)alkyl;
R² is H or methyl;
J is β-Ala or an _{L}-isomer or _{D}-isomer amino acid of structure -N(R³)C(R⁴) (R⁵)C(=O)-, wherein:
R³ is H or CH₃;
R⁴ is H or C₁-C₃ alkyl;
R⁵ is H, C₁-C₈ alkyl, C₃-C₆ cycloalkyl, C₃-C₆ cycloalkylmethyl, C₁-C₆ cycloalkylethyl, phenyl, phenylmethyl, CH₂OH, CH₂SH, CH₂OCH₃, CH₂SCH₃, CH₂CH₂SCH₃, (CH₂)ₛNH₂ or (CH₂)ₛNHC(=NH)(NH₂), where s = 3-5; or
R³ and R⁵ can be taken together to form -CH₂CH₂CH₂-; or
R⁴ and R⁵ can be taken together to form -(CH₂)ᵤ-, where u = 2-5;
K is an _{L}-isomer amino acid of structure -N(R⁶)CH(R⁷)C(=O)-, wherein:
R⁶ is C₁-C₈ alkyl;
R⁷ is -(CH₂)ₚNHC(=NH)(NH₂), where p = 3-5; 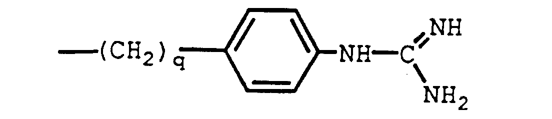 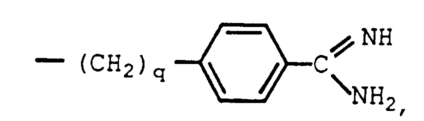 where q = 0,1;
-(CH₂)ᵣX,where r = 4-6; 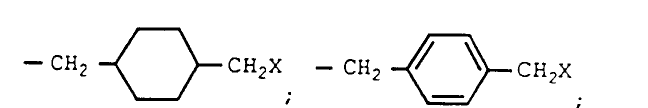 - (CH₂)ₘS(CH₂)₂X, where m = 1,2;
-(CH₂)ₘ-O-(C₁-C₄alkyl)-NH-(C₁-C₆ alkyl), where m = 1,2;
-(CH₂)ₘ-S-(C₁-C₄ alkyl)-NH-(C₁-C₆ alkyl), where m = 1,2; and
-(C₃-C₇ alkyl)-N-(C₁-C₆ alkyl) 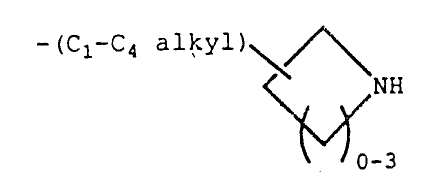
X is -NH₂ or -NHC(=NH) (NH₂), provided that X is not NH₂ when r = 4.

4. The compound of claim 3 wherein
J is β-Ala or an _{L}-isomer or _{D}-isomer amino acid of structure -N(R³)C(R⁴) (R⁵)C(=O)-, wherein:
R³ is H or CH₃;
R⁴ is H;
R⁵ is H, C₁-C₈ alkyl, C₃-C₆ cycloalkyl, C₃-C₆ cycloalkylmethyl, C₁-C6₄ cycloalkylethyl, phenyl, phenylmethyl, CH₂OH, CH₂SH, CH₂OCH₃, CH₂SCH₃, CH₂CH₂SCH₃, (CH₂)ₛNH₂ or (CH₂)ₛNHC(=NH) (NH₂), where s = 3, 4, 5; or
R³ and R⁵ can be taken together to form -CH₂CH₂CH₂-;
K is an _{L}-isomer amino acid of structure -N(CH₃) CH(R⁷⁴)C(=O), wherein:
R⁷ is -(CH₂)ₚNHC(=NH)(NH₂), where p = 3-4 ; 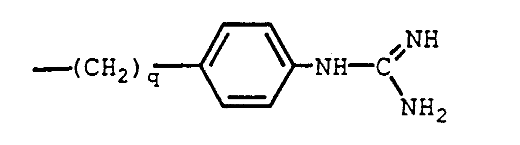 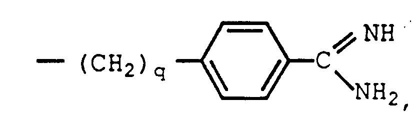 where q = 0,1;
-(CH₂)ᵣX, where r = 4-6; 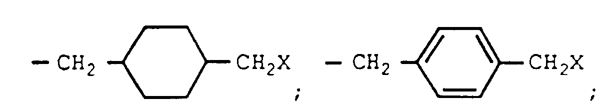 -(CH₂)ₘS(CH₂)₂X, where m = 1,2;
-(C₄-C₇ alkyl)-N-(C₁-C₆ alkyl) 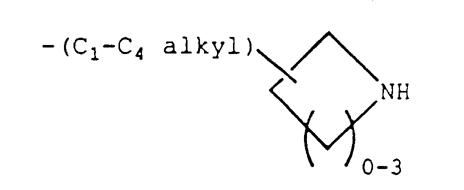 -(CH₂)ₘ-O-(C₁-C₄ alkyl) -NH-(C₁-C₆ alkyl), where m = 1,2;
-(CH₂)ₘ-S-(C₁-C₄ alkyl)-NH-(C₁-C₆ alkyl), where m = 1,2; and
X is -NH₂ or -NHC(=NH) (NH₂), provided that X is not -NH₂ when r = 4;
L is -YCH₂C(=O)-, wherein:
Y is NH or O;
M is an L-isomer amino acid of structure -NH-CH(CH₂CO₂H)C(=O)-.

5. The compound of claims 3 and 4 wherein:
R¹ is H;
R² is H;
J is β-Ala or an _{L}-isomer or _{D}-isomer amino acid of formula -N(R³)CH(R⁵)C (=O)-, wherein:
R³ is H and R⁵ is H, CH₃, CH₂CH₃, CH(CH₃)₂, CH(CH₃)CH₂CH₃, CH₂CH₂CH₃, CH₂CH₂CH₂CH₃, CH₂CH₂SCH₃, CH₂CH(CH₃)₂, (CH₂)₄NH₂; or
R³ is CH₃ and R⁵ is H; or
R³ and R⁵ can be taken together to form -CH₂CH₂CH₂-.
K is an _{L}-isomer amino acid of formula -N(CH₃)CH(R⁷)C(=O)-, wherein:
R⁷ is -(CH₂)₃NHC(=NH) (NH₂);
L is -NHCH₂C(=O)-; and
M is an _{L}-isomer amino acid of formula -NHCH(CH₂COOH)C (=O)-.

6. The compound of claim 2 of the formula (III) 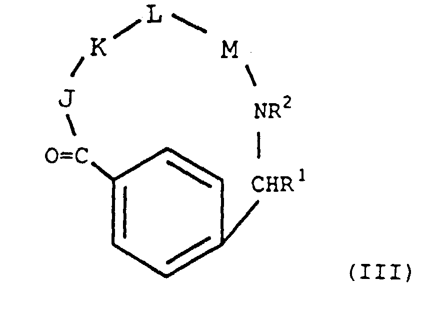 wherein:
R¹ is H, C₁-C₄ alkyl, phenyl, benzyl, or phenyl- (C₂-C₄) alkyl;
R² is H or methyl;
J is β-Ala or an _{L}-isomer or _{D}-isomer amino acid of structure -N(R³)C(R⁴)(R⁵)C(=O)-, wherein:
R³ is H or CH₃;
R⁴ is H or C₁-C₃ alkyl;
R⁵ is H, C₁-C₈ alkyl, C₃-C₆ cycloalkyl, C₃-C₆ cycloalkylmethyl, C₁-C₆ cycloalkylethyl, phenyl , phenylmethyl, CH₂OH, CH₂SH, CH₂OCH₃, CH₂SCH₃, CH₂CH₂SCH₃, (CH₂)ₛNH₂ or (CH₂)ₛNHC(=NH)(NH₂), where s = 3-5; or
R³ and R⁵ can be taken together to form -CH₂CH₂CH₂-; or
R⁴ and R⁵ can be taken together to form -(CH₂)ᵤ-,
where u = 2-5;
K is an _{L}-isomer amino acid of structure -N(R⁶)CH(R⁷)C(=O)-, wherein:
R⁶ is C₁-C₈ alkyl;
R⁷ is -(CH₂)ₚNHC(=NH)(NH₂), where p = 3-4 ; 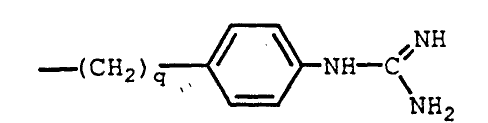 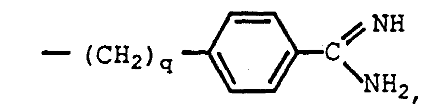 where q = 0,1;
-(CH₂)ᵣX,where r = 4-6; 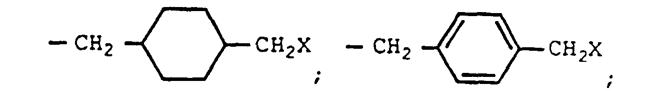 -(CH₂)ₘS(CH₂)₂X, where m = 1,2;
-(C₃-C₇ alkyl)-N-(C₁-C₆ alkyl) 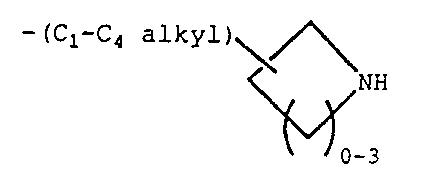 - (CH₂)ₘ-O-(C₁-C₄ alkyl)-NH-(C₁-C₆ alkyl), where m = 1,2;
-(CH₂)ₘ-S-(C₁-C₄ alkyl)-NH-(C₁-C₆ alkyl), where m = 1,2; and
X is -NH₂ or -NHC(=NH) (NH₂), provided that X is not NH₂ when r = 4.

7. The compound of claim 1 which is selected from: the compound of formula (II): 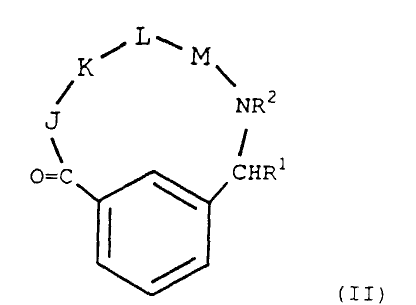 wherein R¹ and R² are H; J is D-Val; K is NMeArg; L is Gly; and M is Asp;
the compound of formula II) wherein R¹ and R² are H; J is D-2-aminobutyric acid; K is NMeArg; L is Gly; and M is Asp;
the compound of formula (II) wherein R¹ and R² are H; J is D-Leu; K is NMeArg; L is Gly; and M is Asp;
the compound of formula (II) wherein R¹ and R² are H; J is D-Ala; K is NMeArg; L is Gly; and M is Asp;
the compound of formula (II) wherein R¹ and R² are H; J is Gly; K is NMeArg; L is Gly; and M is Asp;
the compound of formula (II) wherein R¹ and R² are H; J is D-Pro; K is NMeArg; L is Gly; and M is Asp;
the compound of formula (II) wherein R¹ and R² are H; J is D-Lys; K is NMeArg; L is Gly; and M is Asp;
the compound of formula (II) wherein R¹ and R² are H; J is β-Ala; K is NMeArg; L is Gly; and M is Asp;
the compound of formula (II) wherein R¹ and R² are H; J is D-Val; K is NMeArg; L is β-Ala; and M is Asp;
the compound of formula (II) wherein R¹ and R² are H; J is Pro; K is NMeArg; L is Gly; and M is Asp;
the compound of formula (II) wherein R¹ and R² are H; J is NMeGly; K is NMeArg; L is Gly; and M is Asp;
the compound of formula (II) wherein R¹ is methyl (isomer 1); R² are ; J is D-Val; K is NMeArg; L is Gly; and M is Asp;
the compound of formula (II) wherein R¹ is methyl (isomer 2); R² are H; J is D-Val; K is NMeArg; L is Gly; and M is Asp;
the compound of formula (II) wherein R¹ is phenyl (isomer 1); R² are H; J is D-Val; K is NMeArg; L is Gly; and M is Asp;
the compound of formula (II) wherein R¹ is phenyl (isomer 2); R² are H; J is D-Val; K is NMeArg; L is Gly; and M is Asp;
the compound of formula (II) wherein R¹ and R² are H; J is D-Val; K is D-NMeArg; L is Gly; and M is Asp;
the compound of formula (II) wherein R¹ and R² are H; J is D-Nle; K is NMeArg; L is Gly; and M is Asp;
the compound of formula (II) wherein R¹ and R² are H; J is D-Phg; K is NMeArg; L is Gly; and M is Asp;.
the compound of formula (II) wherein R¹ and R² are H; J is D-Ile; K is NMeArg; L is Gly; and M is Asp;
the compound of formula (II) wherein R¹ and R² are H; J is D-Phe; K is NMeArg; L is Gly; and M is Asp;
the compound of formula (II) wherein R¹ and R² are H; J is NMeGly; K is NMeArg; L is Gly; and M is Asp;
the compound of formula (II) wherein R¹ and R² are H; J is D-Nle; K is NMeArg; L is Gly; and M is Asp;
the compound of formula (II) wherein R¹ and R² are H; J is D-Tyr; K is NMeArg; L is Gly; and M is Asp;
the compound of formula (II) wherein R¹ and R² are H; J is D-Val; K is NMeAmf; L is Gly; and M is Asp;
the compound of formula (II) wherein R¹ and R² are H; J is D-Val; K is NMeArg; L is Ala; and M is Asp;
the compound of formula (II) wherein R¹ and R² are H; J is D-Val; K is NMeArg; L is Gly; and M is α-MeAsp;
the compound of formula (II) wherein R¹ and R² are H; J is D-Val;; K is NMeArg; L is Gly; and M is β-MeAsp;
the compound of formula (II) wherein R¹ and R² are H; J is D-Val; K is NMeArg; L is Gly; and M is NMeAsp;
the compound of formula (II) wherein R¹ and R2 are H; J is D-Val; K is NMeArg; L is Gly; and M is D-Asp;
the compound of formula (II) wherein R¹ is H; R² is CH₃; J is D-Val; K is NMeArg; L is Gly; and M is Asp;
the compound of formula (II) wherein R¹ and R2 are H; J is D-Abu; K is di-NMeOrn; L is Gly; and M is Asp;
the compound of formula (III): 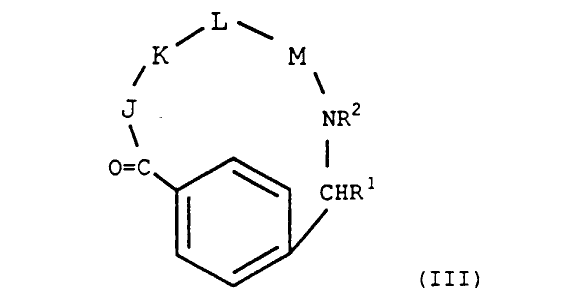 wherein R1 and R2 are H; J is D-Val; K is NMeArg; L is Gly; and M is Asp;
the compound of formula (V): 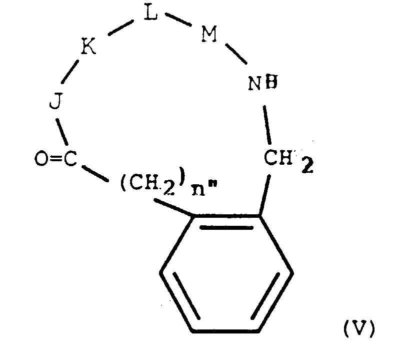 wherein n"=1; J is D-Val; K is NMeArg; L is Gly; and M is Asp;
the compound of formula (V) wherein n"=0; J is D-Val; K is NMeArg; L is Gly; and M is Asp;
the compound of formula (VI): 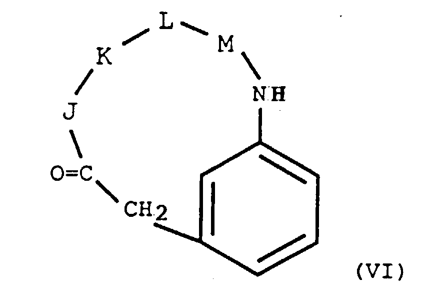 wherein J is D-Val; K is NMeArg; L is Gly; and M is Asp;
the compound of formula (VII): 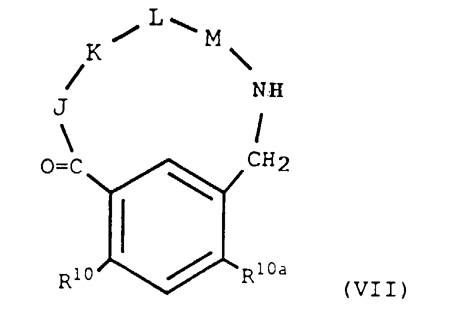 wherein R¹⁰ is H; R^{10a} is Cl; J is D-Val; K is NMeArg; L is Gly; and M is Asp;
the compound of formula (VII) wherein R¹⁰ is H; R^{10a} is I; J is D-Val; K is NMeArg; L is Gly; and M is Asp;
the compound of formula (VII) wherein R¹⁰ is H; R^{10a} is I; J is D-Abu; K is NMeArg; L is Gly; and M is Asp;
the compound of formula (VII) wherein R¹⁰ is H; R^{10a} is OMe; J is D-Val; K is NMeArg; L is Gly; and M is Asp;
the compound of formula (VII) wherein R¹⁰ is H; R^{10a} is Me; J is D-Val; K is NMeArg; L is Gly; and M is Asp;
the compound of formula (VII) wherein R^{10a} is H; R¹⁰ is Cl; J is D-Val; K is NMeArg; L is Gly; and M is Asp;
the compound of formula (VII) wherein R^{10a} is H; R¹⁰ is MeO; J is D-Val; K is NMeArg; L is Gly; and M is Asp;
the compound of formula (VII) wherein R^{10a} is H; R¹⁰ is Me; J is D-Val; K is NMeArg; L is Gly; and M is Asp;
the compound of formula (VIII) : 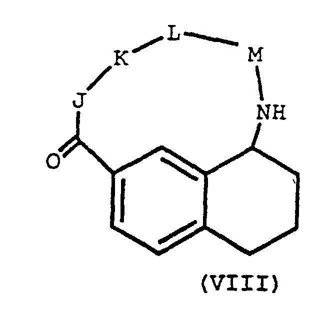 wherein J is D-Val; K is NMeArg; L is Gly; and M is Asp;
the compound of formula (IX) : 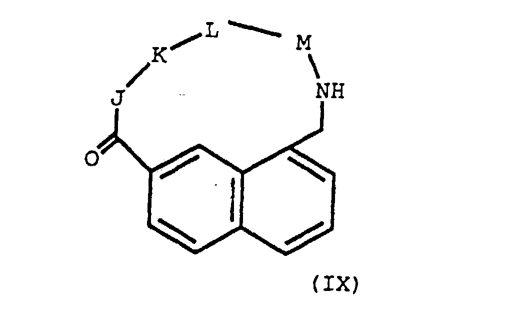 wherein J is D-Val; K is NMeArg; L is Gly; and M is Asp.

8. The compound of claim 7 which is selected from:
the compound of formula (II) wherein R¹ and R² are H; J is D-Val; K is NMeArg; L is Gly; and M is Asp;
the compound of formula (II) wherein R¹ and R² are H; J is D-2-aminobutyric acid; K is NMeArg; L is Gly; and M is Asp;
the compound of formula (II) wherein R¹ and R² are H; J is D-Leu; K is NMeArg; L is Gly; and M is Asp;
the compound of formula (II) wherein R¹ and R² are H; j is D-Ala; K is NMeArg; L is Gly; and M is Asp;
the compound of formula (II) wherein R¹ and R² are H; J is Gly; K is NMeArg; L is Gly; and M is Asp;
the compound of formula (II) wherein R¹ and R² are H; J is D-Pro; K is NMeArg; L is Gly; and M is Asp;
the compound of formula (II) wherein R¹ and R² are H; J is D-Lys; K is NMeArg; L is Gly; and M is Asp;
the compound of formula (II) wherein R¹ and R² are H; J is β-Ala; K is NMeArg; L is Gly; and M is Asp;
the compound of formula (II) wherein R¹ and R² are H; J is NMeGly; K is NMeArg; L is Gly; and M is Asp;
the compound of formula (II) wherein R¹ is methyl (isomer 1); R² are H; J is D-Val; K is NMeArg; L is Gly; and M is Asp;
the compound of formula (II) wherein R¹ is methyl (isomer 2); R² are H; J is D-Val; K is NMeArg; L is Gly; and M is Asp;
the compound of formula (II) wherein R¹ is phenyl (isomer 1); R² are H; J is D-Val; K is NMeArg; L is Gly; and M is Asp;
the compound of formula (II) wherein R¹ and R² are H; J is D-Val; K is D-NMeArg; L is Gly; and M is Asp;
the compound of formula (II) wherein R¹ and R² are H; J is D-Nle; K is NMeArg; L is Gly; and M is Asp;
the compound of formula (II) wherein R¹ and R² are H; J is D-Phg; K is NMeArg; L is Gly; and M is Asp;
the compound of formula (II) wherein R¹ and R² are H; J is D-Phe; K is NMeArg; L is Gly; and M is Asp;
the compound of formula (II) wherein R¹ and R² are H; J is D-Ile; K is NMeArg; L is Gly; and M is Asp;
the compound of formula (III) wherein R¹ and R² are H; J is D-Val; K is NMeArg; L is Gly; and M is Asp;
the compound of formula (V) wherein n" = 1; J is D-Val; K is NMeArg; L is Gly and M is Asp;
the compound of formula (V) wherein n" = 0; J is D-Val; K is NMeArg; L is Gly and M is Asp;
the compound of formula (VI) wherein J is D-Val; K is NMeArg; L is Gly; and M is Asp;
the compound of formula (VII) wherein R¹⁰ is H; R^{10a} is Cl; J is D-Val; K is NMeArg; L is Gly; and M is Asp;
the compound of formula (VII) wherein R¹⁰ is H; R^{10a} is I; J is D-Val; K is NMeArg; L is Gly; and M is Asp;
the compound of formula (VII) wherein R¹⁰ is H; R^{10a} is I; J is D-Abu; K is NMeArg; L is Gly; and M is Asp;
the compound of formula (VII) wherein R¹⁰ is H; R^{10a} is Me; J is D-Val; K is NMeArg; L is Gly; and M is Asp;
the compound of formula (VII) wherein R^{10a} is H; R¹⁰ is Cl; J is D-Val; K is NMeArg; L is Gly; and M is Asp;
the compound of formula (VII) wherein R^{10a} is H; R¹⁰ is MeO; J is D-Val; K is NMeArg; L is Gly; and M is Asp;
the compound of formula (VII) wherein R^{10a} is H; R¹⁰ is Me; J is D-Val; K is NMeArg; L is Gly; and M is Asp;
the compound of formula (II) wherein R¹ and R² are H; J is D-Tyr; K is NMeArg; L is Gly; and M is Asp;
the compound of formula (II) wherein R¹ and R² are H; J is D-Val; K is NMeAmf; L is Gly; and M is Asp;
the compound of formula (II) wherein R¹ and R² are H; J is D-Val;; K is NMeArg; L is Gly; and M is bMeAsp;
10 the compound of formula (II) wherein R¹ is H; R² is CH₃; J is D-Val; K is NMeArg; L is Gly; and M is Asp;
the compound of formula (III) wherein R¹ and R² are H; J is D-Val; K is NMeArg; L is Gly; and M is Asp;
the compound of formula (VIII) wherein J is D-Val; K is NMeArg; L is Gly; and M is Asp.

9. The compound of Claim 8 selected from:
the compound of formula (II) wherein R¹ and R² are H; J is D-Val; K is NMeArg; L is Gly; and M is Asp;
the compound of formula (II) wherein R¹ and R² are H; J is D-2-aminobutyric acid; K is NMeArg; L is Gly; and M is Asp;
the compound of formula (II) wherein R¹ and R² are H; J is D-Leu; K is NMeArg; L is Gly; and M is Asp;
the compound of formula (II) wherein R¹ and R² are H; J is D-Ala; K is NMeArg; L is Gly; and M is Asp;
the compound of formula (II) wherein R¹ and R² are H; J is D-Pro; K is NMeArg; L is Gly; and M is Asp;
the compound of formula (II) wherein R¹ and R² are H; J is D-Lys; K is NMeArg.; L is Gly; and M is Asp;
the compound of formula (II) wherein R¹ and R² are H; J is D-Nle; K is NMeArg; L is Gly; and M is Asp;
the compound of formula (II) wherein R¹ and R² are H; J is D-Phe; K is NMeArg; L is Gly; and M is Asp;
the compound of formula (II) wherein R¹ and R² are H; J is D-Ile; K is NMeArg; L is Gly; and M is Asp;
the compound of formula (V) wherein n" = 1; J is D-Val; K is NMeArg; L is Gly; and M is Asp;
the compound of formula (VI) wherein J is D-Val; K is NMeArg; L is Gly; and M is Asp;
the compound of formula (VII) wherein R¹⁰ is H; R^{10a} is Cl; J is D-Val; K is NMeArg; L is Gly; and M is Asp;
the compound of formula (VII) wherein R¹⁰ is H; R^{10a} is I; J is D-Val; K is NMeArg; L is Gly; and M is Asp;
the compound of formula (VII) wherein R¹⁰ is H; R^{10a} is Me; J is D-Val; K is NMeArg; L is Gly; and M is Asp;
the compound of formula (VII) wherein R^{10a} is H; R¹⁰ is Cl; J is D-Val; K is NMeArg; L is Gly; and M is Asp;
the compound of formula (VII) wherein R^{10a} is H; R¹⁰ is MeO; J is D-Val; K is NMeArg; L is Gly; and M is Asp;
the compound of formula (VII) wherein R^{10a} is H; R¹⁰ is Me; J is D-Val; K is NMeArg; L is Gly; and M is Asp;
the compound of formula (II) wherein R¹ and R² are H; J is D-Val;; K is NMeArg; L is Gly; and M is bMeAsp,

10. A compound of formula (I): 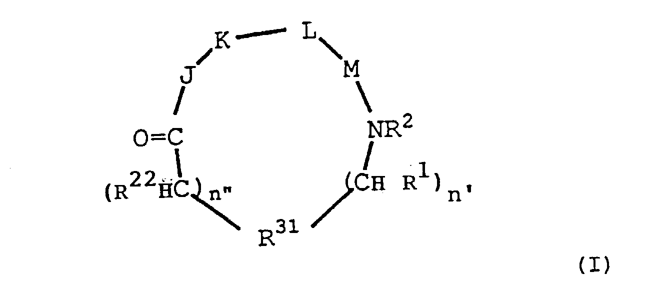 or a pharmaceutically acceptable salt or prodrug form thereof wherein:
R³¹ is selected from: 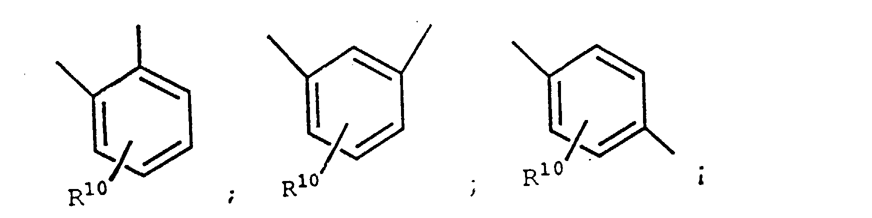 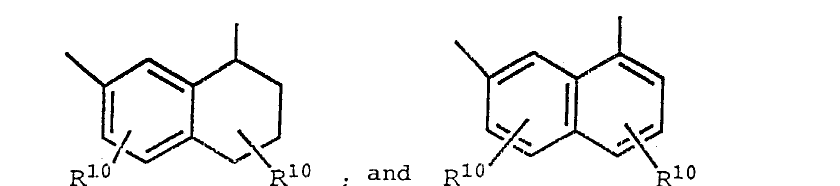 wherein R³¹ may be substituted independently with 0-2 R¹⁰;
n" is 0 or 1;
n' is 0 or 1;
R¹ and R²² are independently selected from H, C₁-C₄ alkyl, phenyl, benzyl, phenyl-(C₂-C₄)alkyl, C₁-C₄ alkoxy;
R² is H or C₁-C₈ alkyl;
R¹⁰ is H, C₁-C₈ alkyl, phenyl, halogen, or C₁-C₄ alkoxy;
J is β-Ala or an _{L}-isomer or _{D}-isomer amino acid of structure -N(R³-)C(R⁴)(R⁵)C(=O)-, wherein:
R³ is H or CH₃
R⁴ is H or C₁-C₃ alkyl;
R⁵ is H, C₁-C₈ alkyl, C₃-C₆ cycloalkyl, C₃-C₆ cycloalkylmethyl, C₁-C₆ cycloalkylethyl, phenyl, phenylmethyl, CH₂OH, CH₂SH, CH₂OCH₃, CH₂SCH₃, CH₂CH₂SCH₃, (CH₂)ₛNH₂, (CH₂)ₛNHC(=NH)(NH₂), (CH₂)ₛNHR¹⁶, where s = 3-5;
R³ and R⁵ can also be taken together to form -(CH₂)ₜ- (t = 2-4) or -CH₂SC(CH₃)₂-; or
R⁴ and R⁵ can also be taken together to form -(CH₂)ᵤ-, where u = 2-5;
R¹⁶ is an amino protecting group
K is an _{L}-isomer amino acid of structure -N(R⁶)CH(R⁷)C(=O)-, wherein:
R⁶ is C₁-C₈ alkyl;
R⁷ is -(CH₂)ₚNHC(=NH) (NH₂), where p = 3-5; 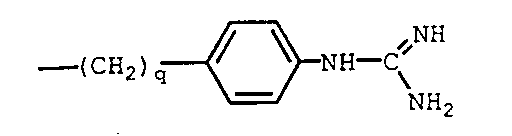 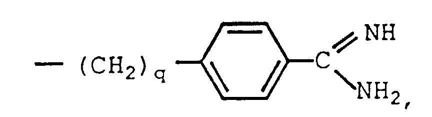 where q = 0,1;
-(CH₂)ᵣX,where r = 4-6; 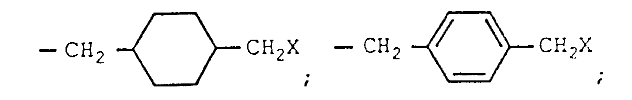 -(CH₂)ₘS(CH₂)₂X, where m = 1,2;
-(C₃-C₇ alkyl)-N-(C₁-C₆ alkyl) 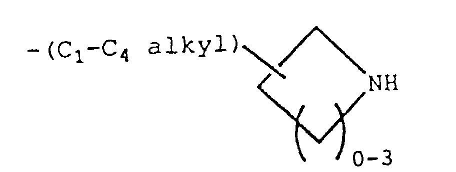 -(CH₂)ₘ-O-(C₁-C₄ alkyl)-NH-(C₁-C₆ alkyl), where m = 1,2;
-(CH₂)ₘ-S-(C₁-C₄ alkyl)-NH-(C₁-C₆ alkyl), where m = 1,2; and
X is -NH₂ or -NHC(=NH)(NH₂);
L is -Y(CH₂)ᵥC(=O)-, wherein:
Y is NH, O, or S; and v = 1,2;
M is an _{L}-isomer amino acid of structure -NH-CH (CH₂SO₃H)C(=O)-.

11. Use of a compound according to any of claims 1-10 for preparing a medicament for the treatment of thromboembolic disorders.

12. A process for preparing a pharmaceutical composition comprising a pharmaceutically acceptable carrier and a therapeutically effective amount of a compound according to any of claims 1-10, which process comprises joining said compound to the carrier.

## Claims (Claims for the following Contracting State(s): ES)

1. A process for preparing a compound of the formula (I): 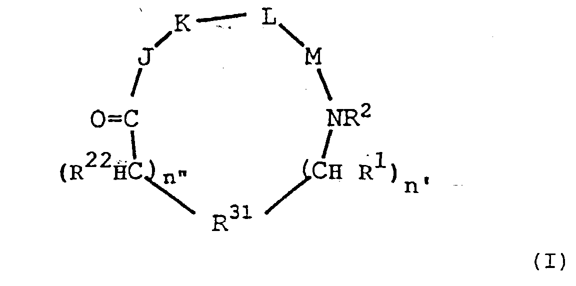 or a pharmaceutically acceptable salt or prodrug form thereof wherein:
R³¹ is
(a) a 6 membered aromatic carbocyclic ring of the formula 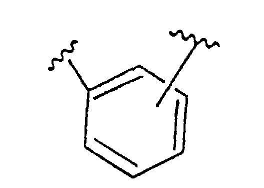 wherein said carbocyclic ring is substituted independently with 0-2 R¹⁰, or
(b) a 10 membered bicyclic carboxylic ring of the formula 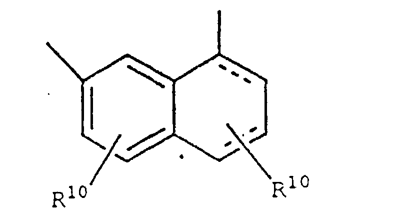 wherein the dashed bond may be a single or double bond;
n" is 0 or 1;
n' is 0 or 1;
R¹ and R²² are independently selected from the following groups:
hydrogen, C₁-C₈ alkyl substituted with 0-2 R¹¹,
C₂-C₈ alkenyl substituted with 0-2 R¹¹,
C₂-C₈ alkynyl substituted with 0-2 R¹¹;
C₃-C₈ cycloalkyl substituted with 0-2 R¹¹,
C₆-C₁₀ bicycloalkyl substituted with 0-2 R¹¹,
aryl substituted with 0-2 R¹²,
a heterocylic ring system substituted with 0-2 R¹², composed of 5-10 atoms including 1-3 N, S, or O heteroatoms, =O, F, Cl, Br, I, -CN, -CO₂R¹³, -C(=O)R¹³,
-C(=O)NR¹³R¹⁴, -CHO, -CH₂OR¹³, -OC(=O)R¹³,
-OC(=O)OR¹³, -OR¹³, -OC(=O)NR¹³R¹⁴, -NR¹⁴C(=O)R¹³,
-NR¹⁴C(=O)OR¹³, -NR¹³C(=O)NR¹³R¹⁴, -NR¹⁴SO₂NR¹³R¹⁴,
-NR¹⁴SO₂R^{13a}, -SO₃H, -SO₂R^{13a}, -SR¹³, -S(=O)R^{13a},
-SO₂NR¹³R¹⁴, -CH₂NR¹³R¹⁴, -NR¹³R¹⁴, -NHC(=NH)NHR¹³,
-C(=NH)NHR¹³,NO₂;
R¹¹ is selected from one or more of the following:
=O, F, Cl, Br, I, -CF₃, -CN, -CO₂R¹³, -C(=O)R¹³, -C(=O)NR¹³R¹⁴, -CH₂OR¹³, -OC(=O)R¹³, -OC(=O)OR¹³, -OR¹³, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -SR¹³, -S(=O)R^{13a},
-SO₂NR¹³R¹⁴, -CH₂NR¹³R¹⁴, -NR¹³R¹⁴, -NHC(=NH)NHR¹³, -C(=NH)NHR¹³, =NOR¹⁴, NO₂,
C₁-C₅ alkyl, C₂-C₄ alkenyl, C₃-C₆ cycloalkyl, C₃-C₆ cycloalkylmethyl, C₂-C₆ alkoxyalkyl, C₁-C₄ alkyl (substituted with -NR¹³R¹⁴, -CF₃, NO₂, -SO₂R¹³, or -S(=O)R^{13a})
aryl substituted with 0-2 R¹²,
a heterocylic ring system substituted with 0-2 R¹², composed of 5-10 atoms including 1-3 nitrogen, oxygen, or sulfur heteroatoms;
R¹² is selected from one or more of the following:
phenyl, benzyl, phenethyl, phenoxy, benzyloxy, halogen, hydroxy, nitro, cyano, C₁-C₅ alkyl, C₃-C₆-cycloalkyl, C₃-C₆ cycloalkylmethyl, C₇-C₁₀ arylalkyl, C₁-C₄ alkoxyl -CO₂R¹³, hydroxamic acid, hydrazide, oxime, boronic acid, sulfonamide, formyl, C₃-C₆ cycloalkoxy, -OC(=O)R¹³, -C(=O)R¹³, -OC(=O)OR¹³, -OR¹³, -CH₂OR¹³, -NR¹³R¹⁴ -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -S(=O)R^{13a}, -SR¹³, -SO₂NR¹³R¹⁴, C₂-C₆ alkoxyalkyl, C₁-C₄ hydroxyalkyl, methylenedioxy, ethylenedioxy,
R¹³ is H, C₁-C₇ alkyl, aryl, -(C₁-C₆ alkyl)aryl, or C₃-C₆ alkoxyalkyl;
R^{13a} is C₁-C₇ alkyl, aryl, -(C₁-C₆ alkyl)aryl, or C₃-C₆ alkoxyalkyl;
R¹⁴ is OH, H, C₁-C₄ alkyl, or benzyl;
R² is H or C₁-C₈ alkyl;
R¹⁰ is selected from one or more of the following:
phenyl, benzyl, phenethyl, phenoxy, benzyloxy, halogen, hydroxy, nitro, cyano, C₁-C₅ alkyl, C₃-C₆ cycloalkyl, C₃-C₆ cycloalkylmethyl, C₇-C₁₀ arylalkyl, C₁-C₄ alkoxyl -CO₂R¹³, -C(=O)NHOR¹³, -C(=O)NHNR¹³R¹⁴, oxime, boronic acid, C₃-C₆ cycloalkoxy, -OC(=O)R¹³, -C (=O)R¹³,-OC(=O)OR¹³, -OR¹³, -CH₂OR¹³, -NR¹³R¹⁴, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H,- -SO₂R^{13a}, -S(=O)R^{13a}, -SR¹³, -SO₂NR¹³R¹⁴, C₂-C₆ alkoxyalkyl, C₁-C₄ hydroxyalkyl, methylenedioxy, ethylenedioxy, C₁-C₄ haloalkyl, C₁-C₄ haloalkoxy, C₁-C₄ alkoxycarbonyl, C₁-C₄ alkylcarbonyloxy, C₁-C₄ alkylcarbonyl, C₁-C₄ alkylcarbonylamino, -OCH₂CO₂H; 2-(1-morpholino)ethoxy, C₁-C₄ alkyl (substituted with -NR¹³R¹⁴, -CF₃, NO₂, or -S(=O)R^{13a});
J is β-Ala or an _{L}-isomer or _{D}-isomer amino acid of structure -N(R³)C(R⁴) (R⁵)C(=O)-, wherein:
R³ is H or CH₃
R⁴ is H or C₁-C₃ alkyl;
R⁵ is H, C₁-C₈ alkyl, C₃-C₆ cycloalkyl, C₃-C₆ cycloalkylmethyl, C₁-C₆ cycloalkylethyl, phenyl, phenylmethyl, CH₂OH, CH₂SH, CH₂OCH_{3,} CH₂SCH₃, CH₂CH₂SCH₃, (CH₂)ₛNH₂, (CH₂)ₛNHC(=NH)(NH₂), (CH₂)ₛNHR¹⁶, where s = 3-5;
R³ and R⁵ can also be taken together to form -(CH₂)ₜ- (t = 2-4) or -CH₂SC(CH₃)₂-; or
R⁴ and R⁵ can also be taken together to form -(CH₂)ᵤ-, where u = 2-5;
R¹⁶ is an amino protecting group;
K is a _{D}-isomer or _{L}-isomer amino acid of structure -N(R⁶)CH(R⁷)C(=O)-, wherein:
R⁶ is C₁-C₈ alkyl;
R⁷ is selected from:
-(C₁-C₇ alkyl)X; 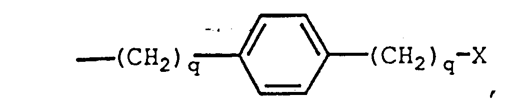 where q is
independently 0,1; 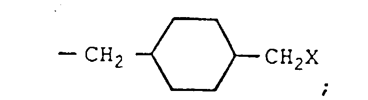 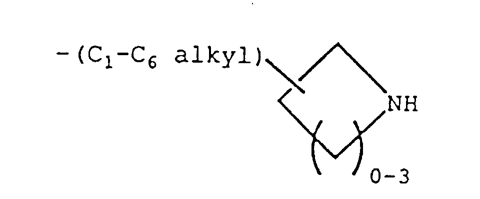 -(CH₂)ₘO-(C₁-C₄ alkyl)-X, where m = 1,2;
-(CH₂)ₘS-(C₁-C₄alkyl)-X, where m = 1,2; and
X is selected from: 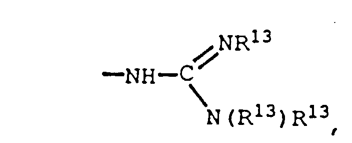 -N(R¹³)R¹³,-C(=NH)(NH₂), -SC(NH)-NH₂, wherein R¹³ is as defined above;
L is -Y(CH₂)ᵥC(=O)-, wherein:
Y is NH, N(C₁-C₃ alkyl), O, Or S; and v = 1,2;
M is a _{D}-isomer or _{L}-isomer amino acid of structure -NR¹⁷-CH(R⁸)C(=O)-, wherein:
R¹⁷ is H, C₁-C₃ alkyl;
R⁸ is -CH₂CO₂R¹³,-CH₂SO₃R^{13a}, -CH(CH₃)CO₂R¹³, -SO₂NR¹³R¹⁴, CH₂-boronic acid, or -CH₂-tetrazole;
which process comprises cyclizing a linear precursor peptide.

2. The process of claim 1, wherein:
R³¹ is selected from: 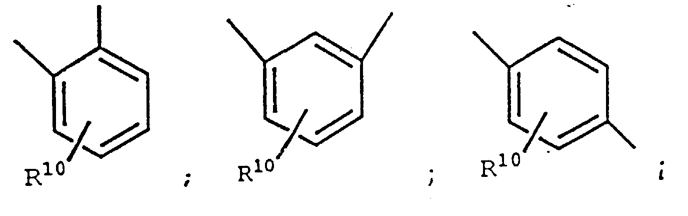 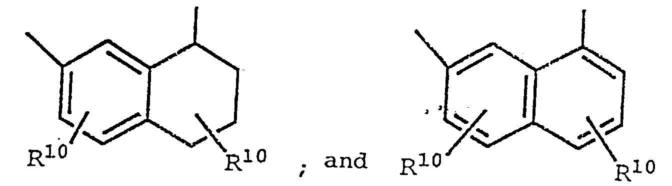 wherein R³¹ may be substituted independently with 0-2 R¹⁰;
R¹ and R²² are independently selected from H, C₁-C₄ alkyl, phenyl, benzyl, phenyl- (C₂-C₄) alkyl, C₁-C₄ alkoxy;
R¹⁰ is H, C₁-C₈ alkyl, phenyl, halogen, or C₁-C₄ alkoxy;
K is an _{L}-isomer amino acid of structure -N(R⁶)CH(R⁷)C(=O)-, wherein:
R⁶ is C₁-C₈ alkyl;
R⁷ is -(CH₂)ₚNHC(=NH) (NH₂), where p = 3-5; 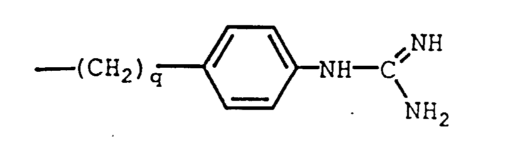 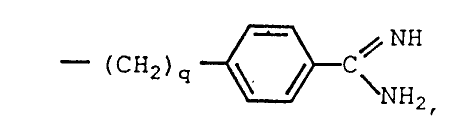 where q = 0,1;
-(CH₂)ᵣX,where r = 4-6; 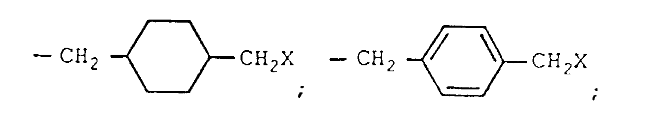 -(CH₂)ₘS(CH₂)₂X, where m = 1,2;
-(C₃-C₇ alkyl)-N-(C₁-C₆ alkyl) 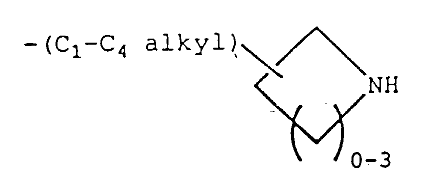 -(CH₂)ₘ-O-(C₁-C₄alkyl)-NH-(C₁-C₆ alkyl), where m = 1,2;
-(CH₂)ₘ-S-(C₁-C₄alkyl)-NH-(C₁-C₆ alkyl), where m = 1,2; and
X is -NH₂ or -NHC(=NH) (NH₂); for
L is -Y(CH₂)ᵥC(=O)-, wherein:
Y is NH, O, or S; and v = 1,2;
M is an _{L}-isomer amino acid of structure -NH-CH(R⁸)C(=O)-, wherein:
R⁸ is -CH₂CO₂H, or -CH(CH₃)CO₂H.

3. The process of claim 2 wherein the prepard compound has the formula (II): 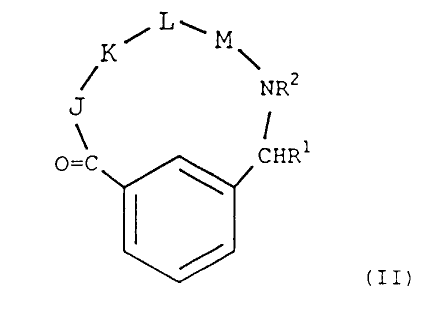 wherein:
R¹ is H, C₁-C₄ alkyl, phenyl, benzyl, or phenyl-(C₂-C₄)alkyl;
R² is H or methyl;
J is β-Ala or an _{L}-isomer or _{D}-isomer amino acid of structure -N(R³)C(R⁴)(R⁵)C(=O)-, wherein:
R³ is H or CH₃;
R⁴ is H or C₁-C₃ alkyl;
R⁵ is H, C₁-C₈ alkyl, C₃-C₆ cycloalkyl, C₃-C₆ cycloalkylmethyl, C₁-C₆ cycloalkylethyl, phenyl, phenylmethyl, CH₂OH, CH₂SH, CH₂OCH₃, CH₂SCH₃, CH₂CH₂SCH₃, (CH₂)ₛNH₂ or (CH₂)ₛNHC(=NH)(NH₂), where s = 3-5; or
R³ and R⁵ can be taken together to form -CH₂CH₂CH₂-; or
R⁴ and R⁵ can be taken together to form -(CH₂)ᵤ-,
where u = 2-5;
K is an _{L}-isomer amino acid of structure -N(R⁶)CH(R⁷)C(=O) -, wherein:
R⁶ is C₁-C₈ alkyl;
R⁷ is -(CH₂)ₚNHC(=NH)(NH₂), where p = 3-5; 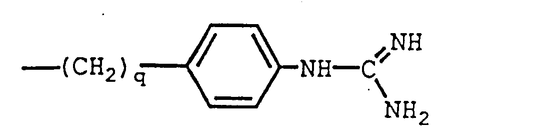 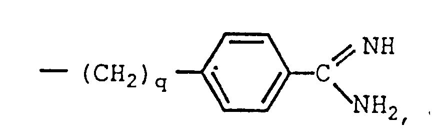 where q = 0,1;
-(CH₂)ᵣX,where r = 4-6; 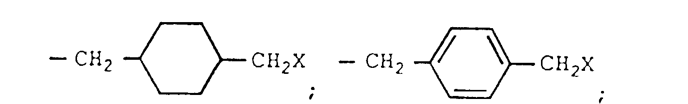 -(CH₂)ₘS(CH₂)₂X, where m = 1,2;
-(CH₂)ₘ-O-(C₁-C₄ alkyl)-NH-(C₁-C₆ alkyl), where m = 1,2;
-(CH₂)ₘ-S-(C₁-C₄ alkyl)-NH-(C₁-C₆ alkyl), where m = 1,2; and
-(C₃-C₇ alkyl)-N-(C₁-C₆ alkyl) 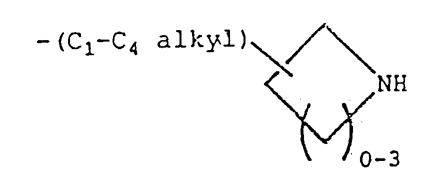
X is -NH₂ or -NHC(=NH) (NH₂), provided that X is not NH₂ when r = 4.

4. The process of claim 3 wherein
J is β-Ala or an _{L}-isomer or _{D}-isomer amino acid of structure -N(R³)C(R⁴) (R⁵)C(=O)-, wherein:
R³ is H or CH₃;
R⁴ is H;
R⁵ is H, C₁-C₈ alkyl, C₃-C₆ cycloalkyl C₃-C₆ cycloalkylmethyl, C₁-C6₄ cycloalkylethyl, phenyl, phenylmethyl, CH₂OH, CH₂SH, CH₂OCH₃, CH₂SCH₃, CH₂CH₂SCH₃, (CH₂)ₛNH₂ or (CH₂)ₛNHC(=NH) (NH₂), where s = 3, 4, 5; or
R³ and R⁵ can be taken together to form -CH₂CH₂CH₂-;
K is an _{L}-isomer amino acid of structure -N(CH₃) CH(R⁷)C (=O), wherein:
R⁷ is -(CH₂)ₚNHC(=NH)(NH₂),- where p = 3-4 ; 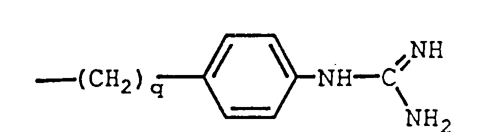 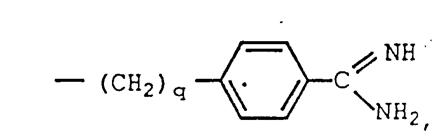 where q = 0,1;
-(CH₂)ᵣX,where r = 4-6; 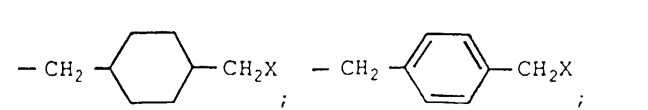 -(CH₂)ₘS(CH₂)₂X, where m = 1,2;
-(C₄-C₇alkyl)-N-(C₁-C₆ alkyl) 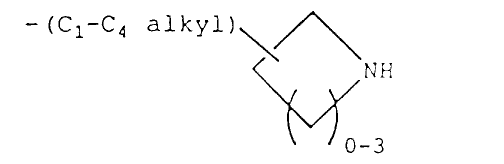 -(CH₂)ₘ-O-(C₁-C₄ alkyl)-NH-(C₁-C₆ alkyl), where m = 1,2;
-(CH₂)ₘ-S-(C₁-C₄ alkyl) -NH- (C₁-C₆ alkyl), where m = 1,2; and
X is -NH₂ or -NHC(=NH) (NH₂), provided that X is not -NH₂ when r = 4;
L is -YCH₂C(=O)-, wherein:
Y is NH or O;
M is an _{L}-isomer amino acid of structure -NH-CH(CH₂CO₂H)C(=O)-.

5. The process of claims 3 and 4 wherein:
R¹ is H;
R² is H;
J is β-Ala or an _{L}-isomer or _{D}-isomer amino acid of formula -N (R³)CH(R⁵)C(=O)-, wherein:
R³ is H and R⁵ is H, CH₃, CH₂CH₃, CH(CH₃)₂, CH(CH₃)CH₂CH₃, CH₂CH₂CH₃, CH₂CH₂CH₂CH₃, CH₂CH₂SCH₃, CH₂CH (CH₃)₂, (CH₂)₄NH₂; or
R³ is CH₃ and R⁵ is H; or
R³ and R⁵ can be taken together to form -CH₂CH₂CH₂-.
K is an _{L}-isomer amino acid of formula -N(CH₃)CH(R⁷)C(=O)-, wherein:
R⁷ is-(CH₂)₃NHC(=NH)(NH₂);
L is -NHCH₂C(=O)-; and
M is an _{L}-isomer amino acid of formula -NHCH(CH₂COOH)C (=O)-.

6. The process of claim 2 wherein the prepared compound has the formula (III) 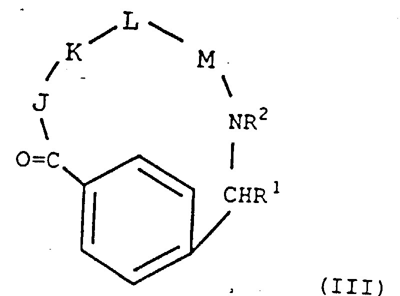 wherein:
R¹ is H, C₁-C₄ alkyl, phenyl, benzyl, or phenyl-(C₂-C₄)alkyl;
R² is H or methyl;
J is β-Ala or an _{L}-isomer or _{D}-isomer amino acid of structure -N(R³)C(R⁴)(R⁵)C(=O) -, wherein:
R³ is H or CH₃;
R⁴ is H or C₁-C₃ alkyl;
R⁵ is H, C₁-C₈ alkyl, C₃-C₆ cycloalkyl, C₃-C₆ cycloalkylmethyl, C₁-C₆ cycloalkylethyl, phenyl, phenylmethyl, CH₂OH, CH₂SH, CH₂OCH₃, CH₂SCH₃, CH₂CH₂SCH₃, (CH₂)ₛNH₂ or (CH₂)ₛNHC(=NH)(NH₂), where s = 3-5; or
R³ and R⁵ can be taken together to form -CH₂CH₂CH₂-; or
R⁴ and R⁵ can be taken together to form -(CH₂)ᵤ-, where u = 2-5;
K is an _{L}-isomer amino acid of structure -N(R⁶)CH(R⁷)C(=O)-, wherein:
R⁶ is C₁-C₈ alkyl;
R⁷ is -(CH₂)ₚNHC(=NH)(NH₂), where p = 3-4 ; 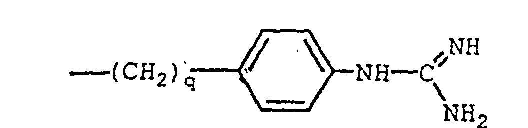 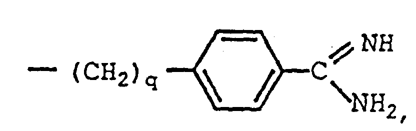 where q = 0,1;
-(CH₂)ᵣX,where r = 4-6; 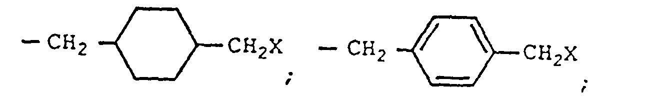 - (CH₂)ₘS(CH₂)₂X; where m = 1,2;
- (C₃-C₇ alkyl)-N-(C₁-C₆ alkyl) 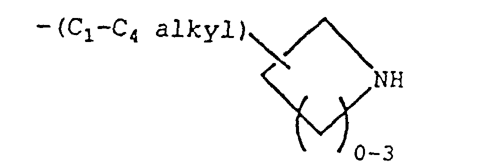 -(CH₂)ₘ-O-(C₁-C₄ alkyl)-NH-(C₁-C₆ alkyl), where m = 1,2;
-(CH₂)ₘ-S-(C₁-C₄ alkyl)-NH-(C₁-C₆ alkyl), where m = 1,2; and
X is -NH₂ or -NHC(=NH) (NH₂), provided that X is not NH₂ when r = 4.

7. The process of claim 1 wherein the prepared compound is selected from the compound of formula (II): 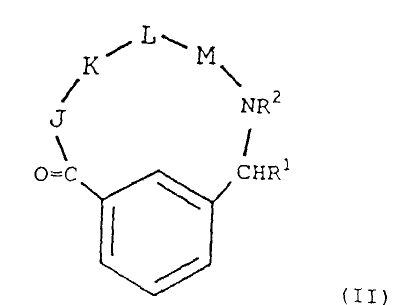 wherein R¹ and R² are H; J is D-Val; K is NMeArg; L is Gly; and M is Asp;
the compound of formula II) wherein R¹ and R² are H; J is D-2-aminobutyric acid; K is NMeArg; L is Gly; and M is Asp;
the compound of formula (II) wherein R¹ and R² are H; J is D-Leu; K is NMeArg; L is Gly; and M is Asp;
the compound of formula (II) wherein R¹ and R² are H; J is D-Ala; K is NMeArg; L is Gly; and M is Asp;
the compound of formula (II) wherein R¹ and R² are H; J is Gly; K is NMeArg; L is Gly; and M is Asp;
the compound of formula (II) wherein R¹ and R² are H; J is D-Pro; K is NMeArg; L is Gly; and M is Asp;
the compound of formula (II) wherein R¹ and R² are H; J is D-Lys; K is NMeArg; L is Gly; and M is Asp;
the compound of formula (II) wherein R¹ and R² are H; J is β-Ala; K is NMeArg; L is Gly; and M is Asp;
the compound of formula (II) wherein R¹ and R² are H; J is D-Val; K is NMeArg; L is β-Ala; and M is Asp;
the compound of formula (II) wherein R¹ and R² are H; J is Pro; K is NMeArg; L is Gly; and M is Asp;
the compound of formula (II) wherein R¹ and R² are H; J is NMeGly; K is NmeArg; L is Gly; and M is Asp;
the compound of formula (II) wherein R¹ is methyl (isomer 1); R² are H; J is D-Val; K is NMeArg; L is Gly; and M is Asp;
the compound of formula (II) wherein R¹ is methyl (isomer 2); R² are H; J is D-Val; K is NMeArg; L is Gly; and M is Asp;
the compound of formula (II) wherein R¹ is phenyl (isomer 1); R² are H; J is D-Val; K is NMeArg; L is Gly; and M is Asp;
the compound of formula (II) wherein R¹ is phenyl (isomer 2); R² are H; J is D-Val; K is NMeArg; L is Gly; and M is Asp;
the compound of formula (II) wherein R¹ and R² are H; J is D-Val; K is D-NMeArg; L is Gly; and M is Asp;
the compound of formula (II) wherein R¹ and R² are H; J is D-Nle; K is NMeArg; L is Gly; and M is Asp;
the compound of formula (II) wherein R¹ and R² are H; J is D-Phg; K is NMeArg; L is Gly; and M is Asp;.
the compound of formula (II) wherein R¹ and R² are H; J is D-Ile; K is NMeArg; L is Gly; and M is Asp;
the compound of formula (II) wherein R¹ and R² are H; J is D-Phe; K is NMeArg; L is Gly; and M is Asp;
the compound of formula (II) wherein R¹ and R² are H; J is NMeGly; K is NMeArg; L is Gly; and M is Asp;
the compound of formula (II) wherein R¹ and R² are H; J is D-Nle; K is NMeArg; L is Gly; and M is Asp;
the compound of formula (II) wherein R¹ and R² are H; J is D-Tyr; K is NMeArg; L is Gly; and M is Asp;
the compound of formula (II) wherein R¹ and R² are H; J is D-Val; K is NMeAmf; L is Gly; and M is Asp;
the compound of formula (II) wherein R¹ and R² are H; J is D-Val; K is NMeArg; L is Ala; and M is Asp;
the compound of formula (II) wherein R¹ and R² are H, J is D-Val; K is NMeArg; L is Gly; and M is α-MeAsp;
the compound of formula (II) wherein R¹ and R² are H; J is D-Val;; K is NMeArg; L is Gly; and M is β-MeAsp;
the compound of formula (II) wherein R¹ and R² are H; J is D-Val; K is NMeArg; L is Gly; and M is NMeAsp;
the compound of formula (II) wherein R¹ and R² are H; J is D-Val; K is NMeArg; L is Gly; and M is D-Asp;
the compound of formula (II) wherein R¹ is H; R² is CH₃; J is D-Val; K is NMeArg; L is Gly; and M is Asp;
the compound of formula (II) wherein R¹ and R² are H; J is D-Abu; K is di-NMeOrn; L is Gly; and M is Asp;
the compound of formula (III): 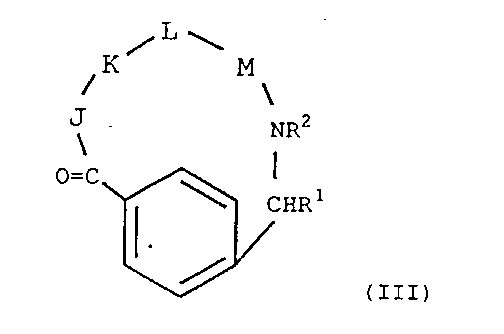 wherein R1 and R2 are H; J is D-Val; K is NMeArg; L is Gly; and M is Asp;
the compound of formula (V): 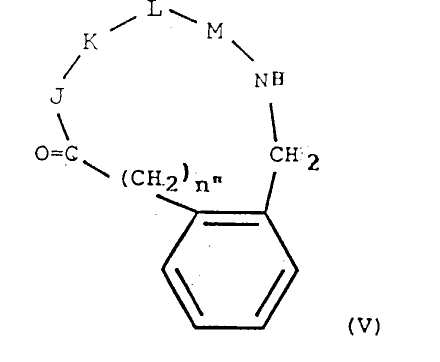 wherein n"=1; J is D-Val; K is NMeArg; L is Gly; and M is Asp;
the compound of formula (V) wherein n"=0; J is D-Val; K is NMeArg; L is Gly; and M is Asp;
the compound of formula (VI): 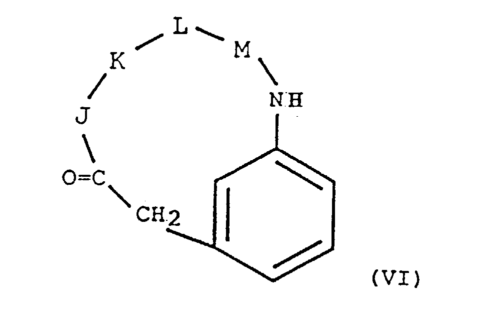 wherein J is D-Val; K is NMeArg; L is Gly; and M is Asp;
the compound of formula (VII): 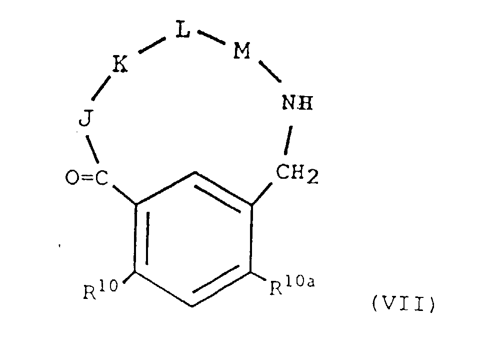 wherein R¹⁰ is H; R^{10a} is Cl; J is D-Val; K is NMeArg; L is Gly; and M is Asp;
the compound of formula (VII) wherein R¹⁰ is H; R^{10a} is I; J is D-Val; K is NMeArg; L is Gly; and M is Asp;
the compound of formula (VII) wherein R¹⁰ is H; R^{10a} is I; J is D-Abu; K is NMeArg; L is Gly; and M is Asp;
the compound of formula (VII) wherein R¹⁰ is H; R^{10a} is OMe; J is D-Val; K is NMeArg; L is Gly; and M is Asp;
the compound of formula (VII) wherein R¹⁰ is H; R^{10a} is Me; J is D-Val; K is NMeArg; L is Gly; and M is Asp;
the compound of formula (VII) wherein R^{10a} is H; R¹⁰ is Cl; J is D-Val; K is NMeArg; L is Gly; and M is Asp;
the compound of formula (VII) wherein R^{10a} is H; R¹⁰ is MeO; J is D-Val; K is NMeArg; L is Gly; and M is Asp;
the compound of formula (VII) wherein R^{10a} is H; R¹⁰ is Me; J is D-Val; K is NMeArg; L is Gly; and M is Asp;
the compound of formula (VIII): 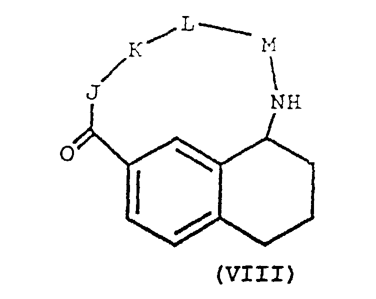 wherein J is D-Val; K is NMeArg; L is Gly; and M is Asp;
the compound of formula (IX): 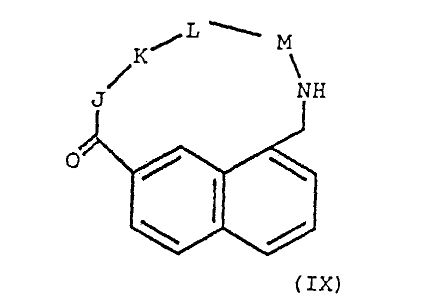 wherein J is D-Val; K is NMeArg; L is Gly; and M is Asp.

8. The process of claim 7 wherein the prepared compound is selected from:
the compound of formula (II) wherein R¹ and R² are H; J is D-Val; K is NMeArg; L is Gly; and M is Asp;
the compound of formula (II) wherein R¹ and R² are H; J is D-2-aminobutyric acid; K is NMeArg; L is Gly; and M is Asp;
the compound of formula (II) wherein R¹ and R² are H; J is D-Leu; K is NMeArg; L is Gly; and M is Asp;
the compound of formula (II) wherein R¹ and R² are H; J is D-Ala; K is NMeArg; L is Gly; and M is Asp;
the compound of formula (II) wherein R¹ and R² are H; J is Gly; K is NMeArg; L is Gly; and M is Asp;
the compound of formula (II) wherein R¹ and R2 are H; J is D-Pro; K is NMeArg; L is Gly; and M is Asp;
the compound of formula (II) wherein R¹ and R² are H; J is D-Lys; K is NMeArg; L is Gly; and M is Asp;
the compound of formula (II) wherein R¹ and R² are H; J is β-Ala; K is NMeArg; L is Gly; and M is Asp;
the compound of formula (II) wherein R¹ and R² are H; J is NMeGly; K is NMeArg; L is Gly; and M is Asp;
the compound of formula (II) wherein R¹ is methyl (isomer 1); R² are H; J is D-Val; K is NMeArg; L is Gly; and M is Asp;
the compound of formula (II) wherein R¹ is methyl (isomer 2); R² are H; J is D-Val; K is NMeArg; L is Gly; and M is Asp;
the compound of formula (II) wherein R¹ is phenyl (isomer 1); R² are H; J is D-Val; K is NMeArg; L is Gly; and M is Asp;
the compound of formula (II) wherein R¹ and R² are H; J is D-Val; K is D-NMeArg; L is Gly; and M is Asp;
the compound of formula (II) wherein R1 and R2 are H; J is D-Nle; K is NMeArg; L is Gly; and M is Asp;
the compound of formula (II) wherein R¹ and R² are H; J is D-Phg; K is NMeArg; L is Gly; and M is Asp;
the compound of formula (II) wherein R¹ and R² are H; J is D-Phe; K is NMeArg; L is Gly; and M is Asp;
the compound of formula (II) wherein R¹ and R² are H; J is D-Ile; K is NMeArg; L is Gly; and M is Asp;
the compound of formula (III) wherein R¹ and R² are H; J is D-Val; K is NMeArg; L is Gly; and M is Asp;
the compound of formula (V) wherein n" = 1; J is D-Val; K is NMeArg; L is Gly and M is Asp;
the compound of formula (V) wherein n" = 0; J is D-Val; K is NMeArg; L is Gly and M is Asp;
the compound of formula (VI) wherein J is D-Val; K is NMeArg; L is Gly; and M is Asp;
the compound of formula (VII) wherein R¹⁰ is H; R^{10a} is Cl; J is D-Val; K is NMeArg; L is Gly; and M is Asp;
the compound of formula (VII) wherein R¹⁰ is H; R^{10a} is I; J is D-Val; K is NMeArg; L is Gly; and M is Asp;
the compound of formula (VII) wherein R¹⁰ is H; R^{10a} is I; J is D-Abu; K is NMeArg; L is Gly; and M is Asp;
the compound of formula (VII) wherein R¹⁰ is H; R^{10a} is Me; J is D-Val; K is NMeArg; L is Gly; and M is Asp;
the compound of formula (VII) wherein R^{10a} is H; R¹⁰ is Cl; J is D-Val; K is NMeArg; L is Gly; and M is Asp;
the compound of formula (VII) wherein R^{10a} is H; R¹⁰ is MeO; J is D-Val; K is NMeArg; L is Gly; and M is Asp;
the compound of formula (VII) wherein R¹⁰a is H; R¹⁰ is Me; J is D-Val; K is NMeArg; L is Gly; and M is Asp;
the compound of formula (II) wherein R¹ and R² are H; J is D-Tyr; K is NMeArg; L is Gly; and M is Asp;
the compound of formula (II) wherein R¹ and R² are H; J is D-Val; K is NMeAmf; L is Gly; and M is Asp;
the compound of formula (II) wherein R¹ and R² are H; J is D-Val;; K is NMeArg; L is Gly; and M is bMeAsp;
the compound of formula (II) wherein R¹ is H; R² is CH₃; J is D-Val; K is NMeArg; L is Gly; and M is Asp;
the compound of formula (III) wherein R¹ and R² are H; J is D-Val; K is NMeArg; L is Gly; and M is Asp;
the compound of formula (VIII) wherein J is D-Val; K is NMeArg; L is Gly; and M is Asp.

9. The process of claim 8 wherein the prepared compound is selected from:
the compound of formula (II) wherein R¹ and R² are H; J is D-Val; K is NMeArg; L is Gly; and M is Asp;
the compound of formula (II) wherein R¹ and R² are H; J is D-2-aminobutyric acid; K is NMeArg; L is Gly; and M is Asp;
the compound of formula (II) wherein R¹ and R² are H; J is D-Leu; K is NMeArg; L is Gly; and M is Asp;
the compound of formula (II) wherein R¹ and R² are H; J is D-Ala; K is NMeArg; L is Gly; and M is Asp;
the compound of formula (II) wherein R¹ and R² are H; J is D-Pro; K is NMeArg; is Gly; and M is Asp;
the compound of formula (II) wherein R¹ and R² are H; J is D-Lys; K is NMeArg; L is Gly; and M is Asp;
the compound of formula (II) wherein R¹ and R² are H; J is D-Nle; K is NMeArg; L is Gly; and M is Asp;
the compound of formula (II) wherein R¹ and R² are H; J is D-Phe; K is NMeArg; L is Gly; and M is Asp;
the compound of formula (II) wherein R¹ and R² are H; J is D-Ile; K is NMeArg; L is Gly; and M is Asp;
the compound of formula (V) wherein n" = 1; J is D-Val; K is NMeArg; L is Gly; and M is Asp;
the compound of formula (VI) wherein J is D-Val; K is NMeArg; L is Gly; and M is Asp;
the compound of formula (VII) wherein R¹⁰ is H; R^{10a} is Cl; J is D-Val; K is NMeArg; L is Gly; and M is Asp;
the compound of formula (VII) wherein R¹⁰ is H; R^{10a} is I; J is D-Val; K is NMeArg; L is Gly; and M is Asp;
the compound of formula (VII) wherein R¹⁰ is H; R^{10a} is Me; J is D-Val; K is NMeArg; L is Gly; and M is Asp;
the compound of formula (VII) wherein R^{10a} is H; R¹⁰ is Cl; J is D-Val; K is NMeArg; L is Gly; and M is Asp;
the compound of formula (VII) wherein R^{10a} is H; R¹⁰ is MeO; J is D-Val; K is NMeArg; L is Gly; and M is Asp;
the compound of formula (VII) wherein R^{10a} is H; R¹⁰ is Me; J is D-Val; K is NMeArg; L is Gly; and M is Asp;
the compound of formula (II) wherein R¹ and R² are H; J is D-Val;; K is NMeArg; L is Gly; and M is bMeAsp.

10. The process for preparing a compound of formula (I): 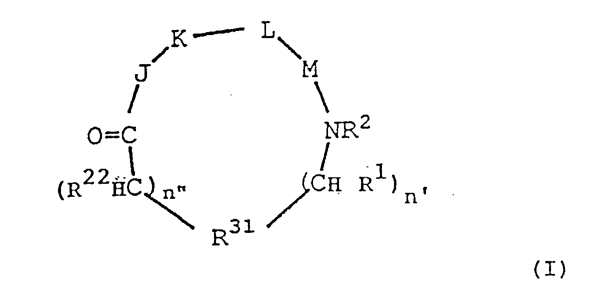 or a pharmaceutically acceptable salt or prodrug form thereof wherein:
R³¹ is selected from: 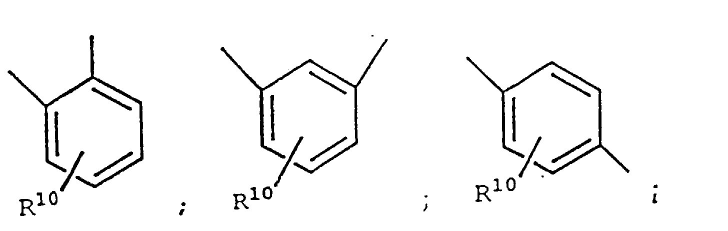 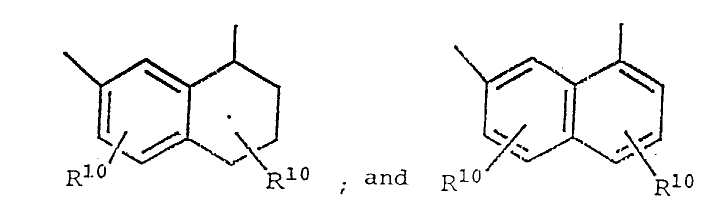 wherein R³¹ may be substituted independently with 0-2 R¹⁰;
n" is 0 or 1;
n' is 0 or 1;
R¹ and R²² are independently selected from H, C₁-C₄ alkyl, phenyl, benzyl, phenyl- (C₂-C₄) alkyl, C₁-C₄ alkoxy;
R² is H or C₁-C₈ alkyl;
R¹⁰ is H, C₁-C₈ alkyl, phenyl, halogen, or C₁-C₄ alkoxy;
J is β-Ala or an _{L}-isomer or _{D}-isomer amino acid of structure -N(R³)O(R⁴)(R⁵)C =O)-, wherein:
R³ is H or CH₃
R⁴ is H or C₁-C₃ alkyl;
R⁵ is H, C₁-C₈ alkyl, C₃-C₆ cycloalkyl, C₃-C₆ cycloalkylmethyl, C₁-C₆ cycloalkylethyl, phenyl, phenylmethyl, CH₂OH, CH₂SH, CH₂OCH₃, CH₂SCH₃, CH₂CH₂SCH₃, (CH₂)ₛNH₂, (CH₂)ₛNHC (=NH) (NH₂), (CH₂)ₛNHR¹⁶, where s = 3-5;
R³ and R⁵ can also be taken together to form -(CH₂)ₜ- (t = 2-4) or CH₂SC(CH₃)₂-; or
R⁴ and R⁵ can also be taken together to form -(CH₂)ᵤ-, where u = 2-5;
R¹⁶ is an amino protecting group
K is an _{L}-isomer amino acid of structure -N(R⁶)CH(R⁷)C(=O)-, wherein:
R⁶ is C₁-C₈ alkyl;
R⁷ is -(CH₂)ₚNHC(=NH)(NH₂), where p = 3-5; 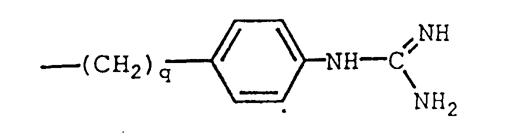 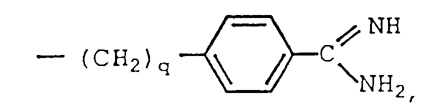 where q = 0,1;
-(CH₂)ᵣX,where r = 4-6; 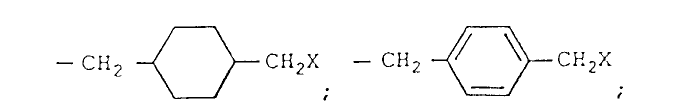 -(CH₂)ₘS(CH₂)₂X, where m = 1,2;
-(C₃-C₇ alkyl)-N-(C₁-C₆ alkyl) 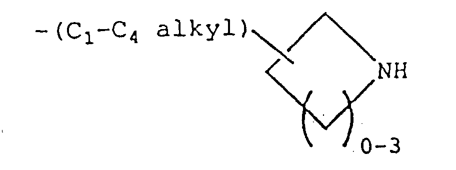 -(CH₂)ₘ-O-(C₁-C₄ alkyl)-NH-(C₁-C₆ alkyl), where m = 1,2;
-(CH₂)ₘ-S-(C₁-C₄ alkyl)-NH(C₁-C₆ alkyl), where m = 1,2; and
X is -NH₂ or -NHC(=NH) (NH₂);
L is -Y(CH₂)ᵥC(=O)-, wherein:
Y is NH, O, or S; and v = 1,2;
M is an _{L}-isomer amino acid of structure -NH-CH (CH₂SO₂H))C(=O)-;
which process comprises cyclizing a linear precursor peptide.

11. A process for preparing a medicament for the treatment of thromboembolic disorders comprising the use of a compound prepared according to any of claims 1-10.

12. A process for preparing a pharmaceutical composition comprising a pharmaceutically acceptable carrier and a therapeutically effective amount of a compound prepared according to any of claims 1-10, which process comprises joining said compound to the carrier.

## Patentansprüche (Patentansprüche für folgende(n) Vertragsstaat(en): AT, BE, CH, LI, DE, DK, FR, GB, IE, IT, LU, MC, NL, SE)

1. Verbindung der Formel (I): 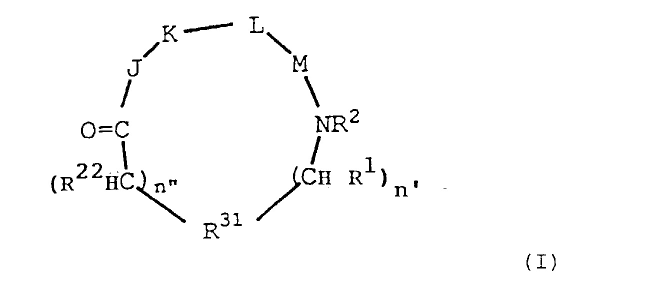 oder ein pharmazeutisch annehmbares Salz oder eine pharmazeutisch annehmbare Medikamentenvorstufe davon, wobei
R³¹
(a) ein sechsgliedriger aromatischer carbocyclischer Ring der Formel 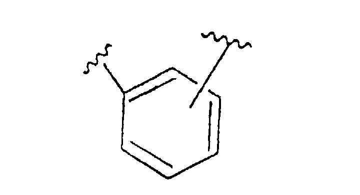 wobei der carbocyclische Ring unabhängig mit 0-2 R¹⁰ substituiert ist, oder
(b) ein zehngliedriger bicyclischer carbocyclischer Ring der Formel 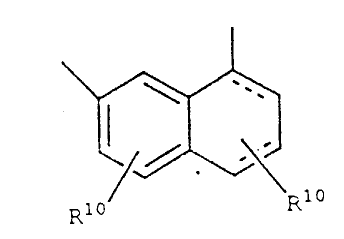 ist, wobei die gestrichelte Bindung eine Einfach- oder Doppelbindung sein kann;
n" 0 oder 1 ist;
n' 0 oder 1 ist;
R¹ und R²² unabhängig aus den folgenden Gruppen ausgewählt sind:
Wasserstoff, C₁-C₈-Alkyl, das mit 0-2 R¹¹ substituiert ist,
C₂-C₈-Alkenyl, das mit 0-2 R¹¹ substituiert ist,
C₂-C₈-Alkinyl, das mit 0-2 R¹¹ substituiert ist,
C₃-C₈-Cycloalkyl, das mit 0-2 R¹¹ substituiert ist,
C₆-C₁₀-Bicycloalkyl, das mit 0-2 R¹¹ substituiert ist,
Aryl, das mit 0-2 R¹² substituiert ist,
ein heterocyclisches Ringsystem, das mit 0-2 R¹² substituiert ist und aus 5-10 Atomen einschließlich 1-3 N-, S-oder O-Heteroatomen besteht,
=O, F, Cl, Br, I, -CN, -CO₂R¹³, -C(=O)R¹³, -C(=O)NR¹³R¹⁴, -CHO, -CH₂OR¹³, -OC(=O)R¹³, -OC(=O)OR¹³, -OR¹³, -OC(=O)NR¹³R¹⁴, -NR¹⁴C (=O) R¹³, -NR¹⁴C (=O) OR¹³, -NR¹³C(=O)NR¹³R¹⁴, NR¹⁴SO₂NR¹³R¹⁴, -NR¹⁴SO₂R^{13a}, -SO₃H, -SO₂R^{13a}, -SR¹³, -S(=O)R^{13a}, -SO₂NR¹³R¹⁴, -CH₂NR¹³R¹⁴, -NR¹³R¹⁴, -NHC(=NH)NHR¹³, -C(=NH)NHR¹³, NO₂;
R¹¹ aus einem oder mehreren der folgenden Gruppen ausgewählt ist:
=O, F, Cl, Br, I, -CF₃, -CN, -CO₂R¹³, -C(=O)R¹³, -C(=O)NR¹³R¹⁴, -CH₂OR¹³, -OC(=O)R¹³, -OC(=O)OR¹³, -OR¹³, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -SR¹³, -S(=O)R^{13a}, -SO₂NR¹³R¹⁴, -CH₂NR¹³R¹⁴, -NR¹³R¹⁴, -NHC(=NH)NHR¹³, -C(=NH)NHR¹³, =NOR¹⁴, NO₂;
C₁-C₅-Alkyl, C₂-C₄-Alkenyl, C₃-C₆-Cycloalkyl, C₃-C₆-cycloalkylmethyl, C₂-C₆-Alkoxyalkyl, C₁-C₄-Alkyl (substituiert mit -NR¹³R¹⁴, -CF₃, NO₂, -SO₂R¹³ oder -S(=O)R^{13a})
Aryl, das mit 0-2 R¹² substituiert ist,
ein heterocyclisches Ringsystem, das mit 0-2 R¹² substituiert ist und aus 5-10 Atomen einschließlich 1-3 Stickstoff-, Schwefel- oder Sauerstoff-Heteroatomen besteht;
R¹² aus einem oder mehreren der folgenden Gruppen ausgewählt ist:
Phenyl, Benzyl, Phenethyl, Phenoxy, Benzyloxy, Halogen, Hydroxy, Nitro, Cyan, C₁-C₅-Alkyl, C₃-C₆-Cycloalkyl, C₃-C₆-Cycloalkylmethyl, C₇-C₁₀-Arylalkyl, C₁-C₄-Alkoxy, -CO₂R¹³, Hydroxamsäure, Hydrazid, Oxim, Boronsäure, Sulfonamid, Formyl, C₃-C₆-Cycloalkoxy, -OC(=O)R¹³, -C(=O)R¹³, -OC(=O)OR¹³, -OR¹³, -CH₂OR¹³, -NR¹³R¹⁴, -OC(=O)NR¹³R¹⁴, -NR¹⁴C(=O)R¹³, -NR¹⁴C(=O)OR¹³, -NR¹³C(=O) NR¹³R¹⁴, -NR¹⁴SO₂NR¹³R¹⁴, -NR¹⁴SO₂R^{13a}, -SO₃H, -SO₂R^{13a}, -S(=O)R^{13a}, -SR¹³, -SO₂NR¹³R¹⁴, C₂-C₆-Alkoxyalkyl, C₁-C₄-Hydroxyalkyl, Methylendioxy, Ethylendioxy;
R¹³ H, C₁-C₇-Alkyl, Aryl, -(C₁-C₆-alkyl)-aryl oder C₃-C₆-Alkoxyalkyl ist;
R^{13a} C₁-C₇-Alkyl, Aryl, -(C₁-C₆-alkyl)-aryl oder C₃-C₆-Alkoxyalkyl ist;
R¹⁴ OH, H, C₁-C₄-Alkyl oder Benzyl ist;
R² H oder C₁-C₈-Alkyl ist;
R¹⁰ aus einem oder mehreren der folgenden Gruppen ausgewählt ist:
Phenyl, Benzyl, Phenethyl, Phenoxy, Benzyloxy, Halogen, Hydroxy, Nitro, Cyan, C₁-C₅-Alkyl, C₃-C₆-Cycloalkyl, C₃-C₆-Cycloalkylmethyl, C₇-C₁₀-Arylalkyl, C₁-C₄-Alkoxy, -CO₂R¹³, -C(=O)NHOR¹³, -C(=O)NHNR¹³R¹⁴, Oxim, Boronsäure, C₃-C₆-Cycloalkoxy, -OC(=O)R¹³, -C(=O)R¹³, -OC(=O)OR¹³, -OR¹³, -CH₂OR¹³, -NR¹³R¹⁴, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -S(=O)R^{13a}, -SR¹³, -SO₂NR¹³R¹⁴, C₂-C₆-Alkoxyalkyl, C₁-C₄-Hydroxyalkyl, Methylendioxy, Ethylendioxy, C₁-C₄-Halogenalkyl, C₁-C₄-Halogenalkoxy, C₁-C₄-Alkoxycarbonyl, C₁-C₄-Alkylcarbonyloxy, C₁-C₄-Alkylcarbonyl, C₁-C₄-Alkylcarbonylamino, -OCH₂CO₂H, 2-(1-Morpholino)ethoxy, C₁-C₄-Alkyl (substituiert mit -NR¹³R¹⁴, -CF₃, NO₂ oder -S(=O)R^{13a});
J β-Ala oder eine _{L}-Isomer- oder _{D}-Isomer-Aminosäure der Struktur -N(R³)C(R⁴) (R⁵)C(=O)- ist, wobei
R³ H oder CH₃ ist;
R⁴ H oder C₁-C₃-Alkyl ist;
R⁵ H, C₁-C₈-Alkyl, C₃-C₆-Cycloalkyl, C₃-C₆-Cycloalkylmethyl, C₁-C₆-Cycloalkylethyl, Phenyl, Phenylmethyl, CH₂OH, CH₂SH, CH₂OCH₃, CH₂SCH₃, CH₂CH₂SCH₃, (CH₂)ₛNH₂, (CH₂)ₛNHC(=NH) (NH₂), (CH₂)ₛNHR¹⁶ ist, wobei s 3 bis 5 ist;
R³ und R⁵ auch unter Bildung von -(CH₂)ₜ- (t = 2-4) oder -CH₂SC(CH₃)₂- zusammengefaßt sein können; oder
R⁴ und R⁵ unter Bildung von -(CH₂)ᵤ-, wobei u = 2-5, ebenfalls zusammengefaßt sein können;
R¹⁶ eine Aminschutzgruppe ist;
K eine _{D}-Isomer- oder _{L}-Isomer-Aminosäure der Struktur -N(R⁶)CH(R⁷)C (=O) - ist, wobei
R⁶ C₁-C₈-Alkyl ist;
R⁷ ausgewählt ist aus:
-(C₁-C₇-Alkyl)X; 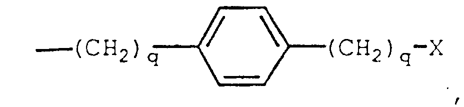 wobei q unabhängig 0, 1 ist; 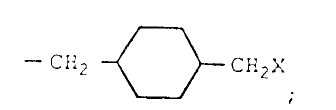 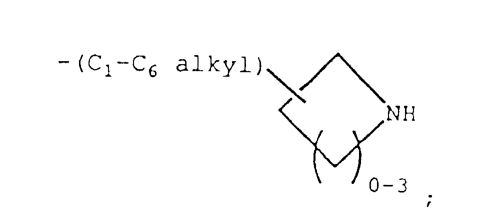 -(CH₂)ₘO-(C₁-C₄-Alkyl)-X, wobei m = 1, 2; -(CH₂)ₘS-(C₁-C₄-Alkyl) -X, wobei m = 1, 2; und
X ausgewählt ist aus: 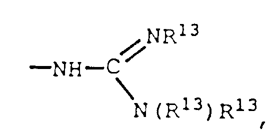 -N(R¹³)R¹³, -C(=NH)(NH₂), -SC(NH)-NH₂, wobei R¹³ wie oben definiert ist;
L -Y(CH₂)ᵥC(=O)- ist, wobei
Y NH, N(C₁-C₃-Alkyl), O oder S ist und v = 1, 2;
M eine _{D}-Isomer- oder _{L}-Isomer-Aminosäure der Struktur NR¹⁷-CH(R⁸)C(-O)- ist, wobei
R¹⁷ H, C₁-C₃-Alkyl ist;
R⁸ -CH₂CO₂R¹³, -CH₂SO₃R^{13a}, -CH(CH₃)CO₂R¹³, -SO₂NR¹³R¹⁴, -CH₂-Boronsäure oder -CH₂-Tetrazol ist.

2. Verbindung gemäß Anspruch 1, wobei
R³¹ ausgewählt ist aus 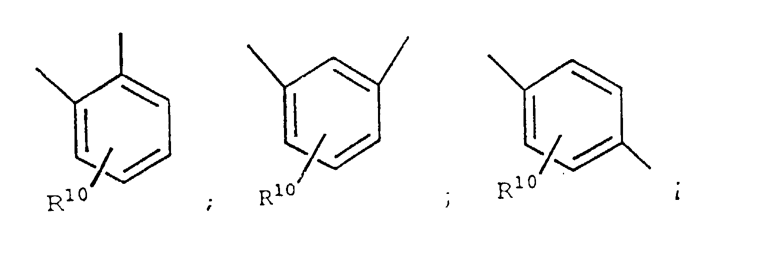 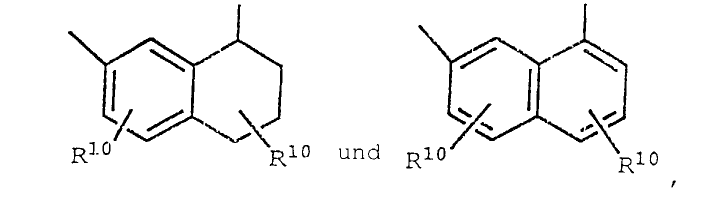 wobei R³¹ unabhängig mit 0-2 R¹⁰ substituiert sein kann;
R¹ und R²² unabhängig aus H, C₁-C₄-Alkyl, Phenyl, Benzyl, Phenyl-(C₂-C₄)-alkyl, C₁-C₄-Alkoxy ausgewählt sind;
R¹⁰ H, C₁-C₈-Alkyl, Phenyl, Halogen oder C₁-C₄-Alkoxy ist;
K eine _{L}-Isomer-Aminosäure der Struktur -N(R⁶)CH(R⁷)C(=O)-ist, wobei
R⁶ C₁-C₈-Alkyl ist;
R⁷ folgendes ist:
-(CH₂)ₚNHC(=NH)(NH₂), wobei p = 3-5; 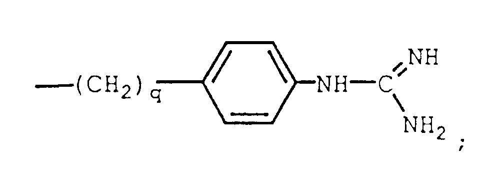 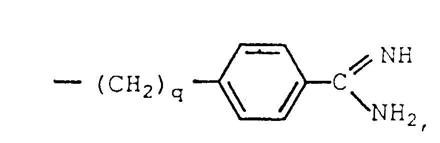 wobei q = 0, 1;
-(CH₂)ᵣX, wobei r = 4-6; 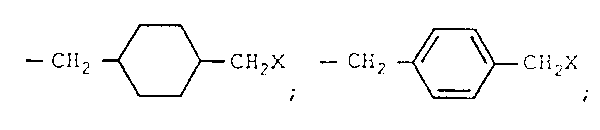 -(CH₂)ₘS(CH₂)₂X, wobei m = 1, 2;
- (C₃-C₇-Alkyl) -N-(C₁-C₆-alkyl) ; 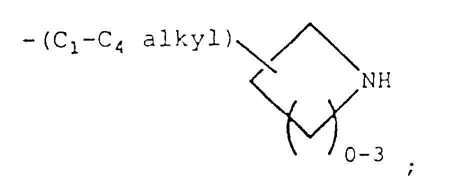 -(CH₂)ₘ-O-(C₁-C₄-Alkyl)-NH-(C₁-C₆-alkyl), wobei m = 1, 2;
-(CH₂)ₘ-S-(C₁-C₄-Alkyl)-NH-(C₁-C₆-alkyl), wobei m = 1, 2; und
X NH₂ oder -NHC(=NH)(NH₂) ist;
oder
L -Y(CH₂)ᵥC(=O) - ist, wobei
Y NH, O oder S ist und v = 1, 2;
M eine _{L}-Isomer-Aminosäure der Struktur -NH-CH(R⁸)C(=O)-ist, wobei
R⁸ -CH₂CO₂H oder -CH(CH₃)CO₂H ist.

3. Verbindung gemäß Anspruch 2 der Formel (II): 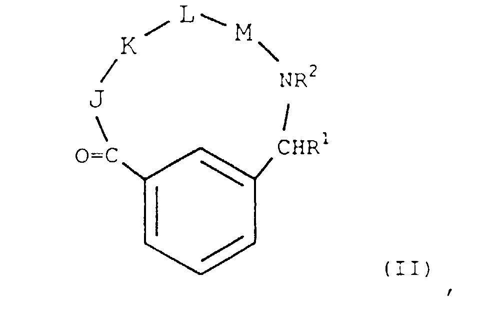 wobei
R¹ H, C₁-C₄-Alkyl, Phenyl, Benzyl oder Phenyl-(C₂-C₄)-alkyl ist;
R² H oder Methyl ist;
J β-Ala oder eine _{L}-Isomer- oder _{D}-Isomer-Aminosäure der Struktur -N(R³)C(R⁴) (R⁵)C(=O)- ist, wobei
R³ H oder CH₃ ist;
R⁴ H oder C₁-C₃-Alkyl ist;
R⁵ H, C₁-C₈-Alkyl, C₃-C₆-Cycloalkyl, C₃-C₆-Cycloalkylmethyl, C₁-C₆-Cycloalkylethyl, Phenyl, Phenylmethyl, CH₂OH, CH₂SH, CH₂OCH₃, CH₂SCH₃, CH₂CH₂SCH₃, (CH₂)ₛNH₂ oder (CH₂)ₛNHC(=NH)(NH₂) ist, wobei s = 3-5; oder
R³ und R⁵ unter Bildung von -CH₂CH₂CH₂- zusammengefaßt sein können; oder
R⁴ und R⁵ unter Bildung von -(CH₂)ᵤ-, wobei u = 2-5, zusammengefaßt sein können;
K eine _{L}-Isomer-Aminosäure der Struktur -N(R⁶)CH(R⁷)C(=O)-ist, wobei
R⁶ C₁-C₈-Alkyl ist;
R⁷ folgendes ist:
-(CH₂)ₚNHC(=NH) (NH₂), wobei p = 3-5; 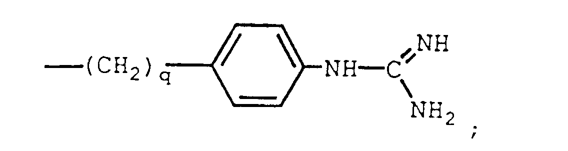 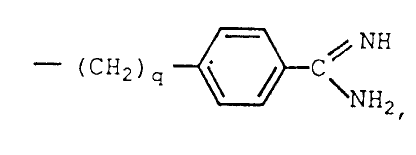 wobei q = 0, 1;
- (CH₂)ᵣX, wobei r = 4-6; 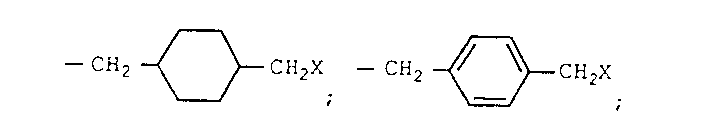 -(CH₂)ₘS(CH₂)₂X,wobei m = 1, 2;
-(CH₂)ₘ-O-(C₁-C₄-Alkyl)-NH-(C₁-C₆-alkyl), wobei m = 1, 2;
-(CH₂)ₘ-S-(C₁-C₄-Alkyl)-NH-(C₁-C₆-alkyl), wobei m = 1, 2; und
-(C₃-C₇-Alkyl)-N-(C₁-C₆-alkyl); 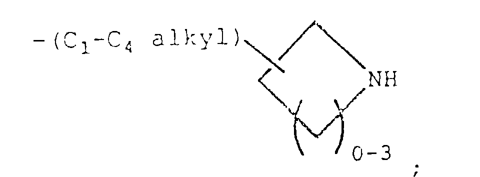
X -NH₂ oder -NHC(=NH) (NH₂) ist, mit der Maßgabe, daß X nicht NH₂ ist, wenn r = 4.

4. Verbindung gemäß Anspruch 3, wobei
J β-Ala oder eine _{L}-Isomer- oder _{D}-Isomer-Aminosäure der Struktur -N(R³)C(R⁴) (R⁵)C(=O)- ist, wobei
R³ H oder CH₃ ist;
R⁴ H ist;
R⁵ H, C₁-C₈-Alkyl, C₃-C₆-Cycloalkyl, C₃-C₆-Cycloalkylmethyl, C₁-C₆-Cycloalkylethyl, Phenyl, Phenylmethyl, CH₂OH, CH₂SH, CH₂OCH₃, CH₂SCH₃, CH₂CH₂SCH₃, (CH₂)ₛNH₂ oder (CH₂)ₛNHC(=NH)(NH₂) ist, wobei s = 3, 4, 5; oder
R³ und R⁵ unter Bildung von -CH₂CH₂CH₂- zusammengefaßt sein können;
K eine _{L}-Isomer-Aminosäure der Struktur -N(CH₃)CH(R⁷)C(=O)- ist, wobei
R⁷ folgendes ist:
-(CH₂)ₚNHC(=NH) (NH₂), wobei p = 3-4 ; 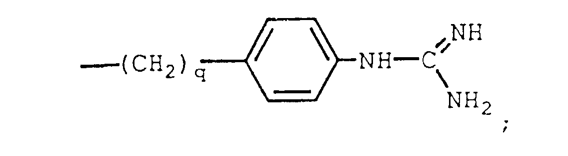 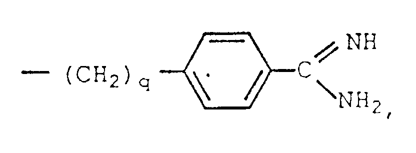 wobei q = 0, 1;
-(CH₂)ᵣX, wobei r = 4-6; 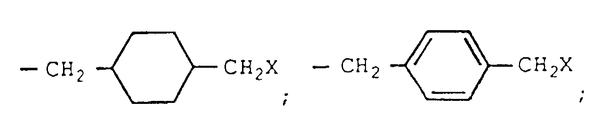 - (CH₂)ₘS(CH₂)₂X, wobei m = 1, 2;
- (C₄-C₇-Alkyl) -N-(C₁-C₆-alkyl) ; 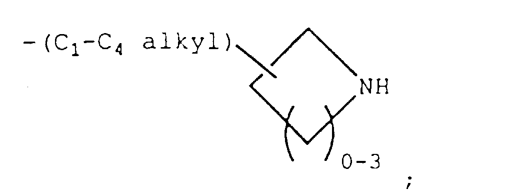 - (CH₂)ₘ-O-(C₁-C₄-Alkyl)-NH-(C₁-C₆-alkyl), wobei m = 1, 2;
-(CH₂)ₘ-S-(C₁-C₄-Alkyl)-NH-(C₁-C₆-alkyl), wobei m = 1, 2; und
X -NH₂ oder -NHC(=NH)(NH₂) ist, mit der Maßgabe, daß X nicht -NH₂ ist, wenn r = 4;
L -YCH₂C(=O)- ist, wobei
Y NH oder O ist;
M eine _{L}-Isomer-Aminosäure der Struktur -NH-CH(CH₂CO₂H)C(=O)- ist.

5. Verbindung gemäß Anspruch 3 und 4, wobei
R¹ H ist;
R² H ist;
J β-Ala oder eine _{L}-Isomer- oder _{D}-Isomer-Aminosäure der Struktur -N(R³)CH(R⁵)C(=O)- ist, wobei
R³ H ist und R⁵ H, CH₃, CH₂CH₃, CH(CH₃)₂, CH(CH₃) CH₂CH₃, CH₂CH₂CH₃, CH₂CH₂CH₂CH₃, CH₂CH₂SCH₃, CH₂CH (CH₃)₂, (CH₂)₄NH₂ ist; oder
R³ CH₃ ist und R⁵ H ist; oder
R³ und R⁵ unter Bildung von -CH₂CH₂CH₂- zusammengefaßt sein können;
K eine _{L}-Isomer-Aminosäure der Formel -N(CH₃)CH(R⁷)-C(=O)-ist, wobei
R⁷ -(CH₂)₃NHC(=NH)(NH₂) ist;
L -NHCH₂C(=O)- ist; und
M eine _{L}-Isomer-Aminosäure der Formel -NHCH(CH₂COOH)C(=O)-ist.

6. Verbindung gemäß Anspruch 2 der Formel (III): 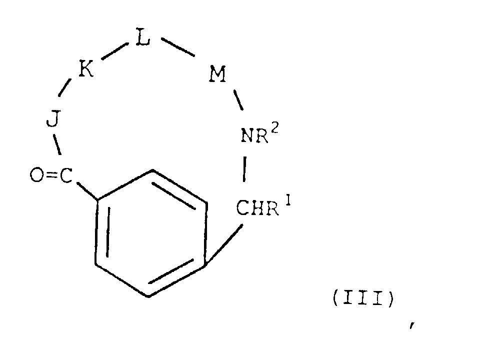 wobei
R¹ H, C₁-C₄-Alky[, Phenyl, Benzyl oder Phenyl-(C₂-C₄)-alkyl ist;
R² H oder Methyl ist;
J β-Ala oder eine _{L}-Isomer- oder _{D}-Isomer-Aminosäure der Struktur -N(R³)C(R⁴)(R⁵)C(=O)- ist, wobei
R³ H oder CH₃ ist;
R⁴ H oder C₁-C₃-Alkyl ist;
R⁵ H, C₁-C₈-Alkyl, C₃-C₆-Cycloalkyl, C₃-C₆-Cycloalkylmethyl, C₁-C₆-Cycloalkylethyl, Phenyl, Phenylmethyl, CH₂OH, CH₂SH, CH₂OCH₃, CH₂SCH₃, CH₂CH₂SCH₃, (CH₂)ₛNH₂ oder (CH₂)ₛNHC(=NH)(NH₂) ist, wobei s = 3-5; oder
R³ und R⁵ unter Bildung von -CH₂CH₂CH₂- zusammengefaßt sein können; oder
R⁴ und R⁵ unter Bildung von -(CH₂)ᵤ-, wobei u = 2-5, zusammengefaßt sein können;
K eine _{L}-Isomer-Aminosäure der Struktur -N(R⁶)CH(R⁷)C(=O)-ist, wobei
R⁶ C₁-C₈-Alkyl ist;
R⁷ folgendes ist:
-(CH₂)ₚNHC(NH)(NH₂), wobei p = 3-4 ; 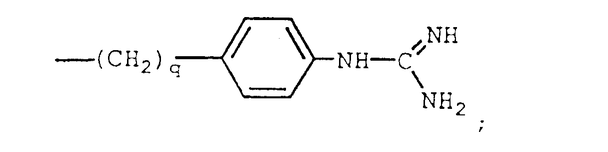 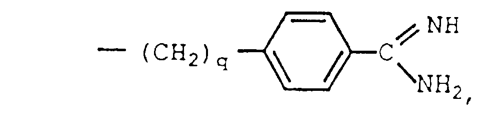 wobei q = 0, 1;
-(CH₂)ᵣX,wobei r = 4-6; 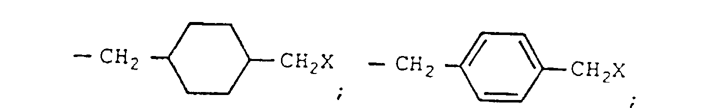 -(CH₂)ₘS(CH₂)₂X,wobei m = 1, 2;
- (C₃-C₇-Alkyl) -N-(C₁-C₆-alkyl) ; 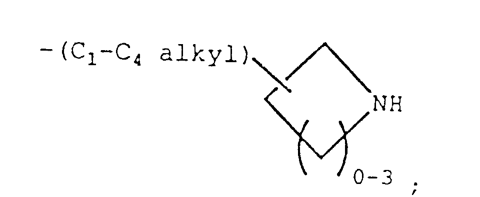 -(CH₂)ₘ-O-(C₁-C₄-Alkyl)-NH-(C₁-C₆-alkyl), wobei m = 1, 2;
-(CH₂)ₘ-S-(C₁-C₄-Alkyl)-NH-(C₁-C₆-alkyl), wobei m = 1, 2; und
X -NH₂ oder -NHC(=NH) (NH₂) ist, mit der Maßgabe, daß X nicht NH₂ ist, wenn r = 4.

7. Verbindung gemäß Anspruch 1, die ausgewählt ist aus:
der Verbindung der Formel (II): 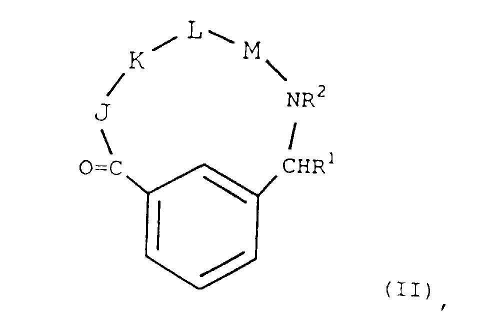 wobei R¹ und R² H sind, J _{D}-Val ist, K NMeArg ist, L Gly ist und M Asp ist;
der Verbindung der Formel (II), wobei R¹ und R² H sind, J _{D}-2-Aminobuttersäure ist, K NMeArg ist, L Gly ist und M Asp ist;
der Verbindung der Formel (II), wobei R¹ und R² H sind, J _{D}-Leu ist, K NMeArg ist, L Gly ist und M Asp ist;
der Verbindung der Formel (II), wobei R¹ und R² H sind, J _{D}-Ala ist, K NMeArg ist, L Gly ist und M Asp ist;
der Verbindung der Formel (II), wobei R¹ und R² H sind, J Gly ist, K NMeArg ist, L Gly ist und M Asp ist;
der Verbindung der Formel (II), wobei R¹ und R² H sind, J _{D}-Pro ist, K NMeArg ist, L Gly ist und M Asp ist;
der Verbindung der Formel (II), wobei R¹ und R² H sind, J _{D}-Lys ist, K NMeArg ist, L Gly ist und M Asp ist;
der Verbindung der Formel (II), wobei R¹ und R² H sind, J β-Ala ist, K NMeArg ist, L Gly ist und M Asp ist;
der Verbindung der Formel (II), wobei R¹ und R² H sind, J _{D}-Val ist, K NMeArg ist, L β-Ala ist und M Asp ist;
der Verbindung der Formel (II), wobei R¹ und R² H sind, J Pro ist, K NMeArg Ist, L Gly ist und M Asp ist;
der Verbindung der Formel (II), wobei R¹ und R² H sind, J NMeGly ist, K NMeArg ist, L Gly ist und M Asp ist;
der Verbindung der Formel (II), wobei R¹ Methyl ist (Isomer 1), R² H ist, J _{D}-Val ist, K NMeArg ist, L Gly ist und M Asp ist;
der Verbindung der Formel (II), wobei R¹ Methyl ist (Isomer 2), R² H ist, J _{D}-Val ist, K NMeArg ist, L Gly ist und M Asp ist;
der Verbindung der Formel (II), wobei R¹ Phenyl ist (Isomer 1), R² H ist, J _{D}-Val ist, K NMeArg ist, L Gly ist und M Asp ist;
der Verbindung der Formel (II), wobei R¹ Phenyl ist (Isomer 2), R² H ist, J _{D}-Val ist, K NMeArg ist, L Gly ist und M Asp ist;
der Verbindung der Formel (II), wobei R¹ und R² H sind, J _{D}-Val ist, K _{D}-NMeArg ist, L Gly ist und M Asp ist;
der Verbindung der Formel (II), wobei R¹ und R² H sind, J _{D}-Nle ist, K NMeArg ist, L Gly ist und M Asp ist;
der Verbindung der Formel (II), wobei R¹ und R² H sind, J _{D}-Phg ist, K NMeArg ist, L Gly ist und M Asp ist;
der Verbindung der Formel (II), wobei R¹ und R² H sind, J _{D}-Ile ist, K NMeArg ist, L Gly ist und M Asp ist;
der Verbindung der Formel (II), wobei R¹ und R² H sind, J _{D}-Phe ist, K NMeArg ist, L Gly ist und M Asp ist;
der Verbindung der Formel (II), wobei R¹ und R² H sind, J _{D}-NMeGly ist, K NMeArg ist, L Gly ist und M Asp ist;
der Verbindung der Formel (II), wobei R¹ und R² H sind, J _{D}-Nle ist, K NMeArg ist, L Gly ist und M Asp ist;
der Verbindung der Formel (II), wobei R¹ und R² H sind, J _{D}-Tyr ist, K NMeArg ist, L Gly ist und M Asp ist;
der Verbindung der Formel (II), wobei R¹ und R² H sind, J _{D}-Val ist, K NMeAmf ist, L Gly ist und M Asp ist;
der Verbindung der Formel (II), wobei R¹ und R² H sind, J _{D}-Val ist, K NMeArg ist, L Ala ist und M Asp ist;
der Verbindung der Formel (II), wobei R¹ und R² H sind, J _{D}-Val ist, K NMeArg ist, L Gly ist und M α-MeAsp ist;
der Verbindung der Formel (II), wobei R¹ und R² H sind, J _{D}-Val ist, K NMeArg ist, L Gly ist und M β-MeAsp ist;
der Verbindung der Formel (II), wobei R¹ und R² H sind, J _{D}-Val ist, K NMeArg ist, L Gly ist und M NMeAsp ist;
der Verbindung der Formel (II), wobei R¹ und R² H sind, J _{D}-Val ist, K NMeArg ist, L Gly ist und M _{D}-Asp ist;
der Verbindung der Formel (II), wobei R¹ H ist, R² CH₃ ist, J _{D}-Val ist, K NMeArg ist, L Gly ist und M Asp ist;
der Verbindung der Formel (II), wobei R¹ und R² H sind, J _{D}-Abu ist, K Di-NMeOrn ist, L Gly ist und M Asp ist;
der Verbindung der Formel (III) : 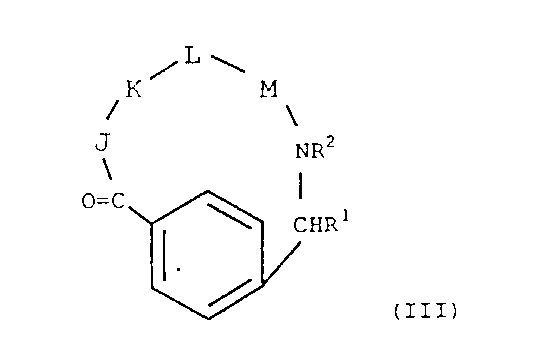 wobei R¹ und R² H sind, J _{D}-Val ist, K NMeArg ist, L Gly ist und M Asp ist;
der Verbindung der Formel (V): 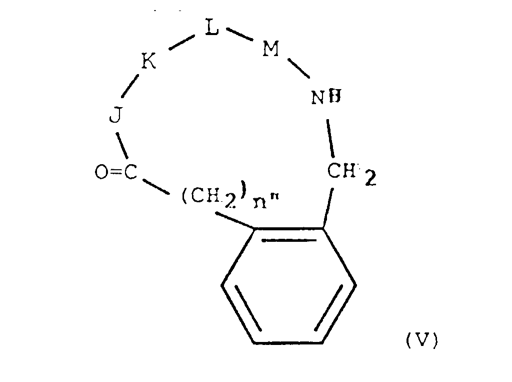 wobei n" = 1, J _{D}-Val ist, K NMeArg ist, L Gly ist und M Asp ist;
der Verbindung der Formel (V), wobei n" = 0, _{D}-Val ist, K NMeArg ist, L Gly ist und M Asp ist;
der Verbindung der Formel (VI): 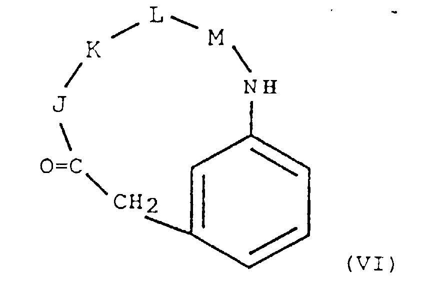 wobei J _{D}-Val ist, K NMeArg ist, L Gly ist und M Asp ist;
der Verbindung der Formel (VII): 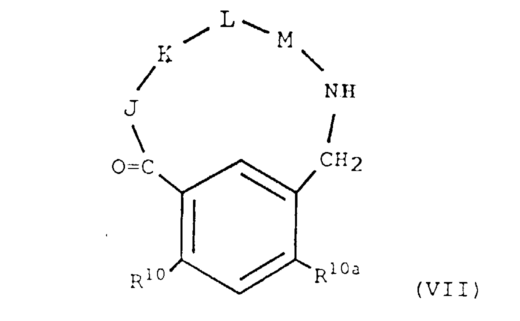 wobei R¹⁰ H ist, R^{10a} Cl ist, J _{D}-Val ist, K NMeArg ist, L Gly ist und M Asp ist;
der Verbindung der Formel (VII), wobei R¹⁰ H ist, R^{10a} I ist, J _{D}-Val ist, K NMeArg ist, L Gly ist und M Asp ist;
der Verbindung der Formel (VII), wobei R¹⁰ H ist, R^{10a} I ist, J _{D}-Abu ist, K NMeArg ist, L Gly ist und M Asp ist;
der Verbindung der Formel (VII), wobei R¹⁰ H ist, R^{10a} OMe ist, J _{D}-Val ist, K NMeArg ist, L Gly ist und M Asp ist;
der Verbindung der Formel (VII), wobei R¹⁰ H ist, R^{10a} Me ist, J _{D}-Val ist, K NMeArg ist, L Gly ist und M Asp ist;
der Verbindung der Formel (VII), wobei R^{10a} H ist, R¹⁰ Cl ist, J _{D}-Val ist, K NMeArg ist, L Gly ist und M Asp ist;
der Verbindung der Formel (VII), wobei R^{10a} H ist, R¹⁰ MeO ist, J _{D}-Val ist, K NMeArg ist, L Gly ist und M Asp ist;
der Verbindung der Formel (VII), wobei R^{10a} H ist, R¹⁰ Me ist, J _{D}-Val ist, K NMeArg ist, L Gly ist und M Asp ist;
der Verbindung der Formel (VIII) : 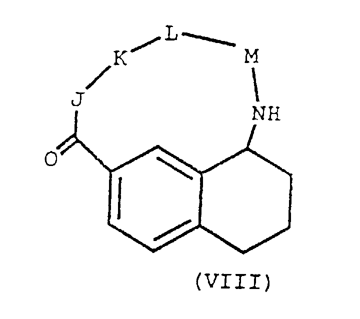 wobei J _{D}-Val ist, K NMeArg ist, L Gly ist und M Asp ist;
der Verbindung der Formel (IX) : 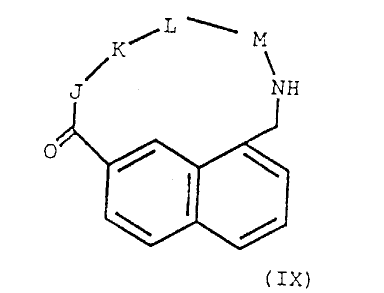 wobei J _{D}-Val ist, K NMeArg ist, L Gly ist und M Asp ist.

8. Verbindung gemäß Anspruch 7, die ausgewählt ist aus:
der Verbindung der Formel (II), wobei R¹ und R² H sind, J _{D}-Val ist, K NMeArg ist, L Gly ist und M Asp ist;
der Verbindung der Formel (II), wobei R¹ und R² H sind, J _{D}-2-Aminobuttersäure ist, K NMeArg ist, L Gly ist und M Asp ist;
der Verbindung der Formel (II), wobei R¹ und R² H sind, J _{D}-Leu ist, K NMeArg ist, L Gly ist und M Asp ist;
der Verbindung der Formel (II), wobei R¹ und R² H sind, J _{D}-Ala ist, K NMeArg ist, L Gly ist und M Asp ist;
der Verbindung der Formel (II), wobei R¹ und R² H sind, J Gly ist, K NMeArg ist, L Gly ist und M Asp ist;
der Verbindung der Formel (II), wobei R¹ und R² H sind, J _{D}-Pro ist, K NMeArg ist, L Gly ist und M Asp ist;
der Verbindung der Formel (II), wobei R¹ und R² H sind, J _{D}-Lys ist, K NMeArg ist, L Gly ist und M Asp ist;
der Verbindung der Formel (II), wobei R¹ und R² H sind, J β-Ala ist, K NMeArg ist, L Gly ist und M Asp ist;
der Verbindung der Formel (II), wobei R¹ und R² H sind, J NMeGly ist, K NMeArg ist, L Gly ist und M Asp ist;
der Verbindung der Formel (II), wobei R¹ Methyl ist (Isomer 1), R² H ist, J _{D}-Val ist, K NMeArg ist, L Gly ist und M Asp ist;
der Verbindung der Formel (II), wobei R¹ Methyl ist (Isomer 2), R² H ist, J _{D}-Val ist, K NMeArg ist, L Gly ist und M Asp ist;
der Verbindung der Formei (II), wobei R¹ Phenyl ist (Isomer 1), R² H ist, J _{D}-Val ist, K NMeArg ist, L Gly ist und M Asp ist;
der Verbindung der Formel (II), wobei R¹ und R² H sind, J _{D}-Val ist, K _{D}-NMeArg ist, L Gly ist und M Asp ist;
der Verbindung der Formel (II), wobei R¹ und R² H sind, J _{D}-Nle ist, K NMeArg ist, L Gly ist und M Asp ist;
der Verbindung der Formel (II), wobei R¹ und R² H sind, J _{D}-Phg ist, K NMeArg ist, L Gly ist und M Asp ist;
der Verbindung der Formel (II), wobei R¹ und R² H sind, J _{D}-Phe ist, K NMeArg ist, L Gly ist und M Asp ist;
der Verbindung der Formel (II), wobei R¹ und R² H sind, J _{D}-Ile ist, K NMeArg ist, L Gly ist und M Asp ist;
der Verbindung der Formel (III), wobei R¹ und R² H sind, J _{D}-Val ist, K NMeArg ist, L Gly ist und M Asp ist;
der Verbindung der Formel (V), wobei n" = 1, J _{D}-Val ist, K NMeArg ist, L Gly ist und M Asp ist;
der Verbindung der Formel (V), wobei n" = 0, J _{D}-Val ist, K NMeArg ist, L Gly ist und M Asp ist;
der Verbindung der Formel (VI), wobei J _{D}-Val ist, K NMeArg ist, L Gly ist und M Asp ist;
der Verbindung der Formel (VII), wobei R¹⁰ H ist, R^{10a} C₁ ist, J _{D}-Val ist, K NMeArg ist, L Gly ist und M Asp ist;
der Verbindung der Formal (VII), wobei R¹⁰ H ist, R^{10a} I ist, J _{D}-Val ist, K NMeArg ist, L Gly ist und M Asp ist;
der Verbindung der Formel (VII), wobei R¹⁰ H ist, R^{10a} I ist, J _{D}-Abu ist, K NMeArg ist, L Gly ist und M Asp ist;
der Verbindung der Formel (VII), wobei R¹⁰ H ist, R^{10a} Me ist, J _{D}-Val ist, K NMeArg ist, L Gly ist und M Asp ist;
der Verbindung der Formel (VII), wobei R^{10a} H ist, R¹⁰ C₁ ist, J _{D}-Val ist, K NMeArg ist, L Gly ist und M Asp ist;
der Verbindung der Formel (VII), wobei R^{10a} H ist, R¹⁰ MeO ist, J _{D}-Val ist, K NMeArg ist, L Gly ist und M Asp ist;
der Verbindung der Formel (VII), wobei R^{10a} H ist, R¹⁰ Me ist, J _{D}-Val ist, K NMeArg ist, L Gly ist und M Asp ist;
der Verbindung der Formel (II), wobei R¹ und R² H sind, J _{D}-Tyr ist, K NMeArg ist, L Gly ist und M Asp ist;
der Verbindung der Formel (II), wobei R¹ und R² H sind, J _{D}-Val ist, K NMeAmf ist, L Gly ist und M Asp ist;
der Verbindung der Formel (II), wobei R¹ und R² H sind, J _{D}-Val ist, K NMeArg ist, L Gly ist und M β-MeAsp ist;
der Verbindung der Formel (II), wobei R¹ H ist, R² CH₃ ist, J _{D}-Val ist, K NMeArg ist, L Gly ist und M Asp ist;
der Verbindung der Formel (III), wobei R¹ und R² H sind, J _{D}-Val ist, K NMeArg ist, L Gly ist und M Asp ist;
der Verbindung der Formel (VIII), wobei J _{D}-Val ist, K NMeArg ist, L Gly ist und M Asp ist.

9. Verbindung gemäß Anspruch 8, die ausgewählt ist aus:
der Verbindung der Formel (II), wobei R¹ und R² H sind, J _{D}-Val ist, K NMeArg ist, L Gly ist und M Asp ist;
der Verbindung der Formel (II), wobei R¹ und R² H sind, J _{D}-2-Aminobuttersäure ist, K NMeArg ist, L Gly ist und M Asp ist;
der Verbindung der Formel (II), wobei R¹ und R² H sind, J _{D}-Leu ist, K NMeArg ist, L Gly ist und M Asp ist;
der Verbindung der Formel (II), wobei R¹ und R² H sind, J _{D}-Ala ist, K NMeArg ist, L Gly ist und M Asp ist;
der Verbindung der Formel (II), wobei R¹ und R² H sind, J _{D}-Pro ist, K NMeArg ist, L Gly ist und M Asp ist;
der Verbindung der Formel (II), wobei R¹ und R² H sind, J _{D}-Lys ist, K NMeArg ist, L Gly ist und M Asp ist;
der Verbindung der Formel (II), wobei R¹ und R² H sind, J _{D}-Nle ist, K NMeArg ist, L Gly ist und M Asp ist;
der Verbindung der Formel (II), wobei R¹ und R² H sind, J _{D}-Phe ist, K NMeArg ist, L Gly ist und M Asp ist;
der Verbindung der Formel (II), wobei R¹ und R² H sind, J _{D}-Ile ist, K NMeArg ist, L Gly ist und M Asp ist;
der Verbindung der Formel (V), wobei n" = 1, J _{D}-Val ist, K NMeArg ist, L Gly ist und M Asp ist;
der Verbindung der Formel (VI), wobei J _{D}-Val ist, K NMeArg ist, L Gly ist und M Asp ist;
der Verbindung der Formel (VII), wobei R¹⁰ H ist, R^{10a} C₁ ist, J _{D}-Val ist, K NMeArg ist, L Gly ist und M Asp ist;
der Verbindung der Formel (VII), wobei R¹⁰ H ist, R^{10a} I ist, J _{D}-Val ist, K NMeArg ist, L Gly ist und M Asp ist;
der Verbindung der Formel (VII), wobei R¹⁰ H ist, R^{10a} Me ist, J _{D}-Val ist, K NMeArg ist, L Gly ist und M Asp ist;
der Verbindung der Formel (VII), wobei R^{10a} H ist, R¹⁰ Cl ist, J _{D}-Val ist, K NMeArg ist, L Gly ist und M Asp ist;
der Verbindung der Formel (VII), wobei R^{10a} H ist, R¹⁰ MeO ist, J _{D}-Val ist, K NMeArg ist, L Gly ist und M Asp ist;
der Verbindung der Formel (VII), wobei R^{10a} H ist, R¹⁰ Me ist, J _{D}-Val ist, K NMeArg ist, L Gly ist und M Asp ist;
der Verbindung der Formel (II), wobei R¹ und R² H sind, J _{D}-Val ist, K NMeArg ist, L Gly ist und M β-MeAsp ist.

10. Verbindung der Formel (I): 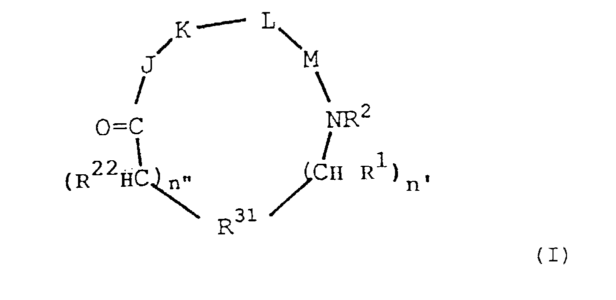 oder ein pharmazeutisch annehmbares Salz oder eine pharmazeutisch annehmbare Medikamentenvorstufe davon, wobei
R³¹ ausgewählt ist aus 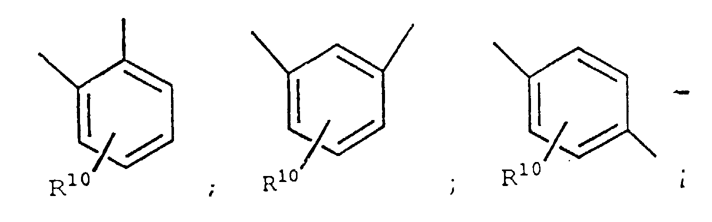 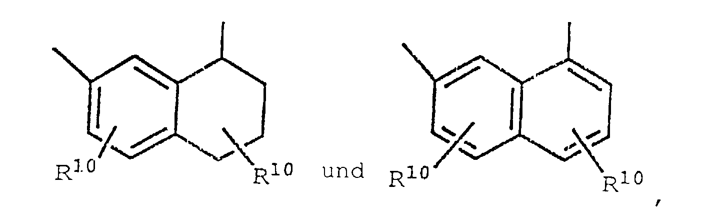 wobei R³¹ unabhängig mit 0-2 R¹⁰ substituiert sein kann;
n" 0 oder 1 ist;
n' 0 oder 1 ist;
R¹ und R²² unabhängig aus H, C₁-C₄-Alkyl, Phenyl, Benzyl, Phenyl-(C₂-C₄)-alkyl, C₁-C₄-Alkoxy ausgewählt sind;
R² H oder C₁-C₈-Alkyl ist;
R¹⁰ H, C₁-C₈-Alkyl, Phenyl, Halogen oder C₁-C₄-Alkoxy ist;
J β-Ala oder eine _{L}-Isomer- oder O-Isomer-Aminosäure der Struktur -N(R³)C(R⁴) (R⁵)C(=O)- ist, wobei
R² H oder CH₃ ist;
R² H oder C₁-C₃-Alkyl ist;
R⁵ H, C₁-C₈-Alkyl, C₃-C₆-Cycloalkyl, C₃-C₆-Cycloalkylmethyl, C₁-C₆-Cycloalkylethyl, Phenyl, Phenylmethyl, CH₂OH, CH₂SH, CH₂OCH₃, CH₂SCH₃, CH₂CH₂SCH₃, (CH₂)ₛNH₂, (CH₂)ₛNHC (=NH) (NH₂), (CH₂)ₛNHR¹⁶ ist, wobei s = 3-5;
R³ und R⁵ auch unter Bildung von -(CH₂)ₜ- (t = 2-4) oder -CH₂SC(CH₃)₂- zusammengefaßt sein können; oder
R⁴ und R⁵ unter Bildung von -(CH₂)ᵤ-, wobei u = 2-5, ebenfalls zusammengefaßt sein können;
R¹⁶ eine Aminschutzgruppe ist;
K eine _{L}-Isomer-Aminosäure der Struktur -N(R⁶)CH(R⁷)C(=O)-ist, wobei
R⁶ C₁-C₈-Alkyl ist;
R⁷ folgendes ist:
-(CH₂)ₚNHC(=NH)(NH₂), wobei p = 3-5; 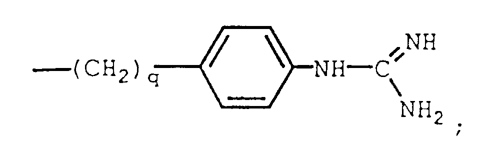 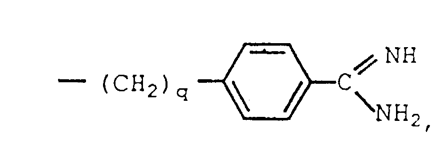 wobei q = 0, 1;
-(CH₂)ᵣX, wobei r 4-6; 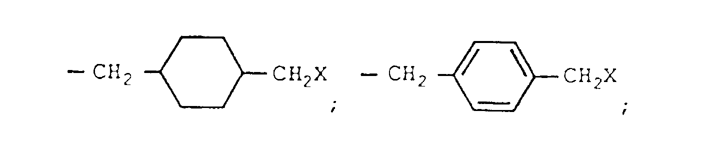 -(CH₂)ₘS(CH₂)₂X,wobei m = 1, 2;
-(C₃-C₇-Alkyl) -N-(C₁-C₆-alkyl); 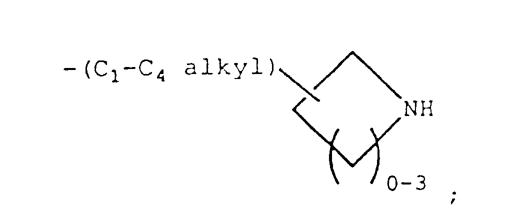 -(CH₂)ₘ-O-(C₁-C₄-Alkyl)-NH-(C₁-C₆-alkyl), wobei m = 1, 2;
-(CH₂)ₘ-S-(C₁-C₄-Alkyl)-NH-(C₁-C₆-alkyl), wobei m = 1, 2; und
X NH₂ oder -NHC(=NH)(NH₂) ist;
L -Y(CH₂)ᵥC(=O)- ist, wobei
Y NH, O oder S ist und v = 1, 2;
M eine _{L}-Isomer-Aminosäure der Struktur -NH-CH(CH₂SO₃H)C(=O)- ist.

11. Verwendung einer Verbindung gemäß einem der Ansprüche 1 bis 10 zur Herstellung eines Medikaments zur Behandlung von thromboembolischen Störungen.

12. Pharmazeutische Zusammensetzung, die einen pharmazeutisch annehmbaren Träger und eine therapeutisch wirksame Menge einer Verbindung gemäß einem der Ansprüche 1 bis 10 umfaßt.

## Patentansprüche (Patentansprüche für folgende(n) Vertragsstaat(en): GR)

1. Verbindung der Formel (I): 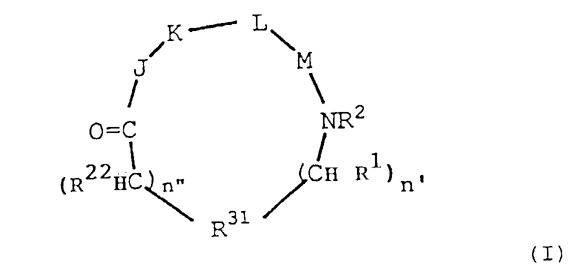 oder ein pharmazeutisch annehmbares Salz oder eine pharmazeutisch annehmbare Medikamentenvorstufe davon, wobei
R³¹
(a) ein sechsgliedriger aromatischer carbocyclischer Ring der Formel 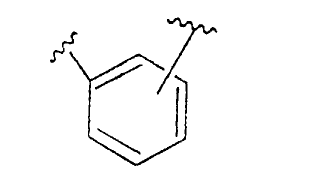 wobei der carbocyclische Ring unabhängig mit 0-2 R¹⁰ substituiert ist, oder
(b) ein zehngliedriger bicyclischer carbocyclischer Ring der Formel 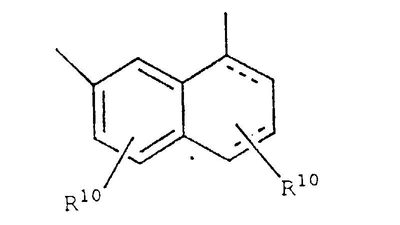 ist, wobei die gestrichelte Bindung eine Einfach- oder Doppelbindung sein kann;
n" 0 oder 1 ist;
n' 0 oder 1 ist;
R¹ und R²² unabhängig aus den folgenden Gruppen ausgewählt sind:
Wasserstoff,
C₁-C₈-Alkyl, das mit 0-2 R¹¹ substituiert ist,
C₂-C₈-Alkenyl, das mit 0-2 R¹¹ substituiert ist,
C₂-C₈-Alkinyl, das mit 0-2 R¹¹ substituiert ist,
C₃-C₈-Cycloalkyl, das mit 0-2 R¹¹ substituiert ist,
C₆-C₁₀-Bicycloalkyl, das mit 0-2 R¹¹ substituiert ist
Aryl, das mit 0-2 R¹² substituiert ist,
ein heterocyclisches Ringsystem, das mit 0-2 R¹² substituiert ist und aus 5-10 Atomen einschließlich 1-3 N-, S-oder O-Heteroatomen besteht,
=O, F, Cl, Br, I, -CN, -CO₂R¹³, -C(=O)R¹³, -C(=O)NR¹³R¹⁴, -CHO, -CH₂OR¹³, -OC(=O)R¹³, -OC(=O)OR¹³, -OR¹³, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -SR¹³, -S(=O)R^{13a}, -SO₂NR¹³R¹⁴, -CH₂NR¹³R¹⁴, -NR¹³R¹⁴, -NHC(=NH)NHR¹³, -C(=NH)NHR¹³, NO₂;
R¹¹ aus einem oder mehreren der folgenden Gruppen ausgewählt ist:
=O, F, Cl, Br, I, -CF₃, -CN, -CO₂R¹³, -C(=O)R¹³, -C(=O)NR¹³R¹⁴, -CH₂OR¹³, -OC(=O)R¹³, -OC(=O)OR¹³, -OR¹³, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -SR¹³, -S(=O)R^{13a}, -SO₂NR¹³R¹⁴, -CH₂NR¹³R¹⁴, -NR¹³R¹⁴, -NHC(=NH)NHR¹³, -C(=NH)NHR¹³, =NOR¹⁴, NO₂;
C₁-C₅-Alkyl, C₂-C₄-Alkenyl, C₃-C₆-Cycloalkyl, C₃-C₆-Cycloalkylmethyl, C₂-C₆-Alkoxyalkyl, C₁-C₄-Alkyl (substituiert mit -NR¹³R¹⁴, -CF₃, NO₂, -SO₂R¹³ oder -S(=O)R^{13a})
Aryl, das mit 0-2 R¹² substituiert ist,
ein heterocyclisches Ringsystem, das mit 0-2 R¹² substituiert ist und aus 5-10 Atomen einschließlich 1-3 Stickstoff-, Schwefel- oder Sauerstoff-Heteroatomen besteht;
R¹² aus einem oder mehreren der folgenden Gruppen ausgewählt ist:
Phenyl, Benzyl, Phenethyl, Phenoxy, Benzyloxy, Halogen, Hydroxy, Nitro, Cyan, C₁-C₅-Alkyl, C₃-C₆-Cycloalkyl, C₃-C₆-Cycloalkylmethyl, C₇-C₁₀-Arylalkyl, C₁-C₄-Alkoxy, -CO₂R¹³, Hydroxamsäure, Hydrazid, Oxim, Boronsäure, Sulfonamid, Formyl, C₃-C₆-Cycloalkoxy, -OC(=O)R¹³, -C(=O)R¹³, -OC(=O)OR¹³, -OR¹³, -CH₂OR¹³, -NR¹³R¹⁴, -OC(=O)NR¹³R¹⁴, -NR¹⁴C(=O)R¹³, NR¹⁴C(=O)OR¹³, NR¹³C(=O)NR¹³R¹⁴, -NR¹⁴SO₂NR¹³R¹⁴, -NR¹⁴SO₂R^{13a}, -SO₃H, -SO₂R^{13a}, -S(=O)R^{13a}, -SR¹³, -SO₂NR¹³R¹⁴, C₂-C₆-Alkoxyalkyl, C₁-C₄-Hydroxyalkyl, Methylendioxy, Ethylendioxy;
R¹³ H, C₁-C₇-Alkyl, Aryl,-(C₁-C₆-alkyl)-aryl oder C₃-C₆-Alkoxyalkyl ist;
R^{13a} C₁-C₇-Alkyl, Aryl, -(C₁-C₆-alkyl)-aryl oder C₃-C₆-Alkoxyalkyl ist;
R¹⁴ OH, H, C₁-C₄-Alkyl oder Benzyl ist;
R² H oder C₁-C₈-Alkyl ist;
R¹⁰ aus einem oder mehreren der folgenden Gruppen ausgewählt ist:
Phenyl, Benzyl, Phenethyl, Phenoxy, Benzyloxy, Halogen, Hydroxy, Nitro, Cyan, C₁-C₅-Alkyl, C₃-C₆-Cycloalkyl, C₃-C₆-Cycloalkylmethyl, C₇-C₁₀-Arylalkyl, C₁-C₄-Alkoxy, -CO₂R¹³, -C(=O)NHOR¹³, -C(=O)NHNR¹³R¹⁴, Oxim, Boronsäure, C₃-C₆-Cycloalkoxy, -OC(=O)R¹³, -C(=O)R¹³, -OC(=O)OR¹³, -OR¹³, -CH₂OR¹³, -NR¹³R¹⁴, -OC(=O)NR¹³R¹⁴, -NR¹⁴C(=O)R¹³, -NR¹⁴C(=O)OR¹³, -NR¹³O(=O) NR¹³R¹⁴, -NR¹⁴SO₂NR¹³R¹⁴, -NR¹⁴SO₂R^{13a}, -SO₃H, -SO₂R^{13a}, -S(=O)R^{13a}, -SR¹³, -SO₂NR¹³R¹⁴, C₂-C₆-Alkoxyalkyl, C₁-C₄-Hydroxyalkyl, Methylendioxy, Ethylendioxy, C₁-C₄-Halogenalkyl, C₁-C₄-Halogenalkoxy, C₁-C₄-Alkoxycarbonyl, C₁-C₄-Alkylcarbonyloxy, C₁-C₄-Alkylcarbonyl, C₁-C₄-Alkylcarbonylamino, -OCH₂CO₂H, 2-(1-Morpholino)ethoxy, C₁-C₄-Alkyl (substituiert mit -NR¹³R¹⁴, -CF₃, NO₂ oder -S(=O)R^{13a});
J β-Ala oder eine _{L}-Isomer- oder _{D}-Isomer-Aminosäure der Struktur -N(R³)C(R⁴) (R⁵)C(=O)- ist, wobei
R³ H oder CH₃ ist;
R⁴ H oder C₁-C₃-Alkyl ist;
R⁵ H, C₁-C₈-Alkyl, C₃-C₆-Cycloalkyl, C₃-C₆-Cycloalkylmethyl, C₁-C₆-Cycloalkylethyl, Phenyl, Phenylmethyl, CH₂OH, CH₂SH, CH₂OCH₃, CH₂SCH₃, CH₂CH₂SCH₃, (CH₂)ₛNH₂, (CH₂)ₛNHO(-NH) (NH₂), (CH₂)ₛNHR¹⁶ ist, wobei s 3 bis 5 ist;
R³ und R⁵ auch unter Bildung von -(CH₂)ₜ- (t = 2-4) oder -CH₂SC(CH₃)₂- zusammengefaßt sein können; oder
R⁴ und R⁵ unter Bildung von -(CH₂)ᵤ-, wobei u = 2-5, ebenfalls zusammengefaßt sein können;
R¹⁶ eine Aminschutzgruppe ist;
K eine _{D}-Isomer- oder _{L}-Isomer-Aminosäure der Struktur -N(R⁶)CH(R⁷)C(=O)- ist, wobei
R⁶ C₁-C₈-Alkyl ist;
R⁷ ausgewählt ist aus:
-(C₁-C₇-Alkyl)X; 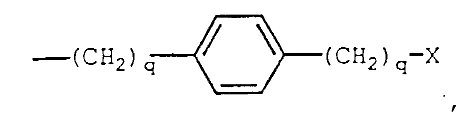 wobei q unabhängig 0, 1 ist; 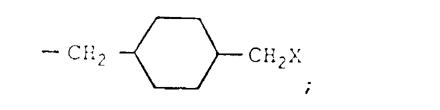 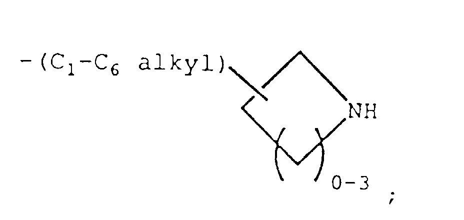 - (CH₂)ₘO-(C₁-C₄-Alkyl)-X, wobei m = 1, 2;
-(CH₂)ₘS-(C₁-C₄-Alkyl)-X, wobei m = 1, 2; und
X ausgewählt ist aus: 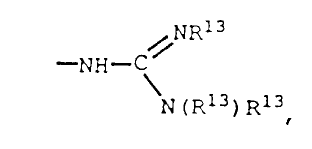 -N(R¹³)R¹³, -C(=NH)(NH₂), -SC(NH)-NH₂, wobei R¹³ wie oben definiert ist;
L -Y(CH₂)ᵥC(=O)- ist, wobei
Y NH, N(C₁-C₃-Alkyl), O oder S ist und v = 1, 2;
M eine _{D}-Isomer- oder _{L}-Isomer-Aminosäure der Struktur -NR¹⁷-CH (R⁸) C(=O)- ist, wobei
R¹⁷ H, C₁-C₃-Alkyl ist;
R⁸ -CH₂CO₂R¹³, -CH₂SO₃R^{13a}, -CH(CH₃)CO₂R¹³, -SO₂NR¹³R¹⁴, -CH₂-Boronsäure oder -CH₂-Tetrazol ist.

2. Verbindung gemäß Anspruch 1, wobei
R³¹ ausgewählt ist aus 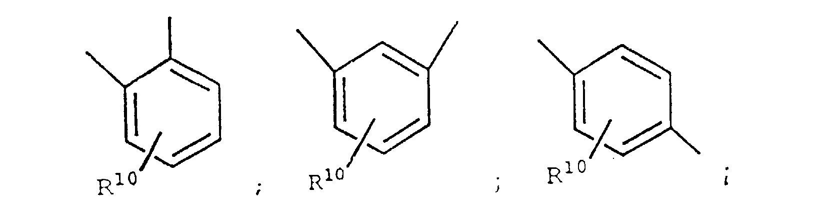 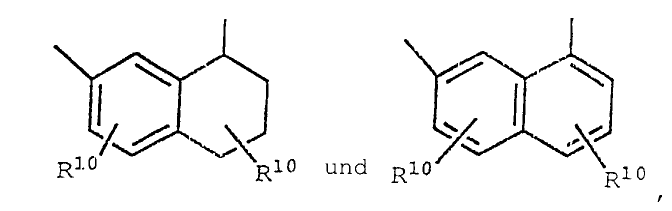 wobei R³¹ unabhängig mit 0-2 R¹⁰ substituiert sein kann;
R¹ und R²² unabhängig aus H, C₁-C₄-Alkyl, Phenyl, Benzyl, Phenyl-(C₂-C₄)-alkyl, C₁-C₄-Alkoxy ausgewählt sind;
R¹⁰ H, C₁-C₈-Alkyl, Phenyl, Halogen oder C₁-C₄-Alkoxy ist;
K eine _{L}-Isomer-Aminosäure der Struktur -N(R⁶) CH(R⁷) C(=O)-ist, wobei
R⁶ C₁-C₈-Alkyl ist;
R⁷ folgendes ist:
-(CH₂)ₚNHC(=NH)(NH₂), wobei p = 3-5; 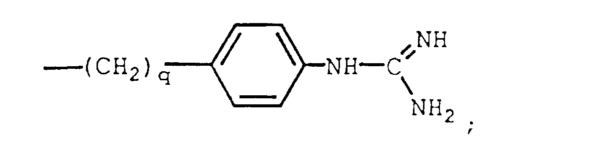 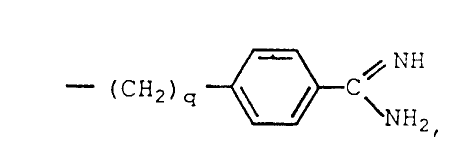 wobei q = 0, 1;
-(CH₂)ᵣX, wobei r 4-6; 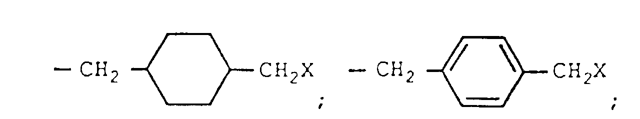 -(CH₂)ₘS(CH₂)₂X,wobei m = 1, 2;
- (C₃-C₇-Alkyl ) -N-(C₁-C₆-alkyl); 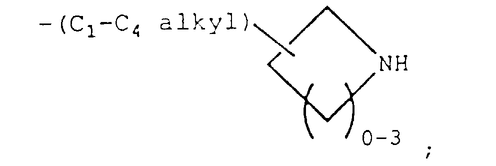 - (CH₂)ₘ-O- (C₁-C₄-Alkyl) -NH-(C₁-C₆-alkyl), wobei m = 1, 2;
-(CH₂)ₘ-S-(C₁-C₄-Alkyl)-NH-(C₁-C₆-alkyl), wobei m = 1, 2; und
X NH₂ oder -NHC(=NH) (NH₂) ist;
oder
L -Y(CH₂)ᵥC(=O)- ist, wobei
Y NH, 0 oder S ist und v = 1, 2;
M eine _{L}-Isomer-Aminosäure der Struktur -NH-CH(R⁸)C(=O)-ist, wobei
R⁸ -CH₂CO₂H oder -CH(CH₃)CO₂H ist.

3. Verbindung gemäls Anspruch 2 der Formel (II): 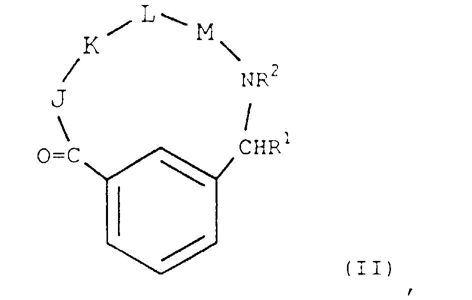 wobei
R¹ H, C₁-C₄-Alkyl, Phenyl, Benzyl oder Phenyl-(C₂-C₄)-alkyl ist;
R² H oder Methyl ist;
J β-Ala oder eine _{L}-Isomer- oder _{D}-Isomer-Aminosäure der Struktur -N(R³)C(R⁴) (R⁵)C(=O)- ist, wobei
R³ H oder CH₃ ist;
R⁴ H oder C₁-C₃-Alkyl ist;
R⁵ H, C₁-C₈-Alkyl, C₃-C₆-Cycloalkyl, C₃-C₆-Cycloalkylmethyl, C₁-C₆-Cycloalkyl.ethyl, Phenyl, Phenylmethyl, CH₂OH, CH₂SH, CH₂OCH₃, CH₂SCH₃, CH₂CH₂SCH₃, (OH₂)ₛNH₂ oder (CH₂)ₛNHC(=NH)(NH₂) ist, wobei s = 3-5; oder
R³ und R⁵ unter Bildung von -CH₂CH₂CH₂- zusammengefaßt sein können; oder
R⁴ und R⁵ unter Bildung von -(CH₂)ᵤ-, wobei u = 2-5, zusammengefaßt sein können;
K eine _{L}-Isomer-Aminosäure der Struktur -N(R⁶)CH(R⁷)C(=O)-ist, wobei
R⁶ C₁-C₈-Alkyl ist;
R⁷ folgendes ist:
-(CH₂)ₚNHC(=NH)(NH₂), wobei p = 3-5; 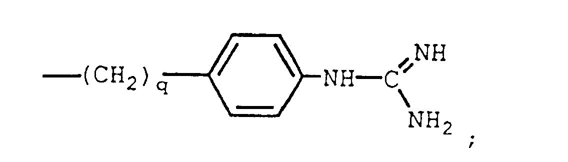 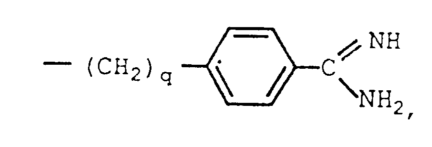 wobei q = 0, 1;
-(CH₂)ᵣX, wobei r = 4-6; 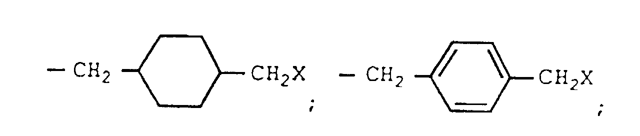 -(CH₂)ₘS(CH₂)₂X,wobei m = 1, 2;
-(CH₂)ₘ-O-(C₁-C₄-Alkyl)-NH-(C₁-C₆-alkyl),wobei m = 1, 2;
-(CH₂)ₘ-S-(C₁-C₄-Alkyl)-NH-(C₁-C₆-alkyl), wobei m = 1, 2; und
- (C₃-C₇-Alkyl) -N-(C₁-C₆-alkyl); 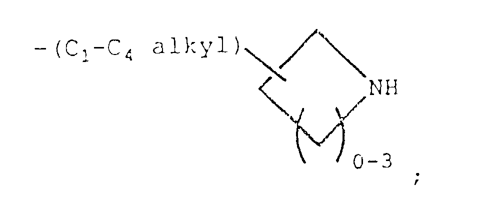
X -NH₂ oder -NHC(=NH)(NH₂)ist, mit der Maßgabe, daß X nicht NH₂ ist, wenn r = 4.

4. Verbindung gemäß Anspruch 3, wobei
J β-Ala oder eine _{L}-Isomer- oder _{D}-Isomer-Aminosäure der Struktur -N(R³)C(R⁴)(R⁵)C(=O)- ist, wobei
R³ H oder CH₃ ist;
R⁴ H ist;
R⁵ H, C₁-C₈-Alkyl, C₃-C₆-Cycloalkyl, C₃-C₆-Cycloalkylmethyl, C₁-C₆-Cycloalkylethyl, Phenyl, Phenylmethyl, CH₂OH, CH₂SH, CH₂OCH₃, CH₂SCH₃, CH₂CH₂SCH₃, (CH₂)ₛNH₂ oder (CH₂)ₛNHC(=NH)(NH₂) ist, wobei s = 3, 4, 5; oder
R³ und R⁵ unter Bildung von -CH₂CH₂CH₂- zusammengefaßt sein können;
K eine _{L}-Isomer-Aminosäure der Struktur -N(CH₃)CH(R⁷)C(=O)- ist, wobei
R⁷ folgendes ist:
-(CH₂)ₚNHC(=NH)(NH₂), wobei p = 3-4 ; 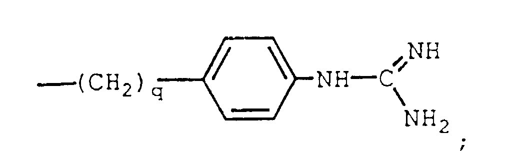 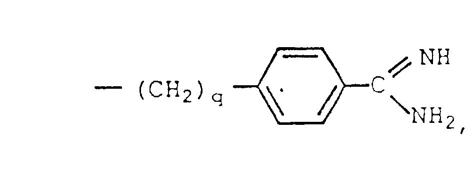 wobei q = 0, 1;
- (CH₂)ᵣX, wobei r = 4-6; 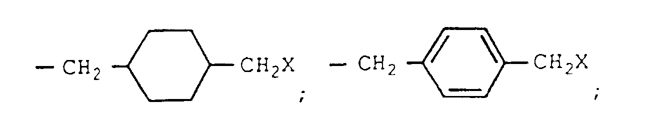 -(CH₂)ₘS(CH₂)₂X, wobei m = 1, 2;
- (C₄-C₇-Alkyl) -N-(C₁-C₆-alkyl) ; 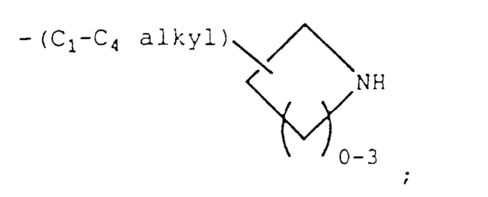 -(CH₂)ₘ-O-(C₁-C₄-Alkyl)-NH-(C₁-C₆-alkyl), wobei m = 1, 2;
-(CH₂)ₘ-S-(C₁-C₄-Alkyl)-NH-(C₁-C₆-alkyl), wobei m = 1, 2; und
X -NH₂ oder -NHC(=NH) (NH₂) ist, mit der Maßgabe, daß X nicht -NH₂ ist, wenn r = 4;
L -YCH₂C(=O)- ist, wobei
Y NH oder O ist;
M eine _{L}-Isomer-Aminosäure der Struktur -NH-CH(CH₂CO₂H)C(=O)- ist.

5. Verbindung gemäß Anspruch 3 und 4, wobei
R² H ist;
R² H ist;
J β-Ala oder eine _{L}-Isomer- oder _{D}-Isomer-Aminosäure der Struktur -N(R³)CH(R⁵)C(=O)- ist, wobei
R³ H ist und R⁵ H, CH₃, CH₂CH₃, CH(CH₃)₂, CH(CH₃)CH₂CH₃, CH₂CH₂CH₃, CH₂CH₂CH₂CH₃, CH₂CH₂SCH₃, CH₂CH(CH₃)₂, (CH₂)₄NH₂ ist; oder
R³ CH₃ ist und R⁵ H ist; oder
R³ und R⁵ unter Bildung von -CH₂CH₂CH₂- zusammengefaßt sein können;
K eine _{L}-Isomer-Aminosäure der Formel -N(CH₃)CH(R⁷)-C(=O)-ist, wobei
R⁷ -(CH₂)₃NHC(=NH) (NH₂) ist;
L -NHCH₂C(=O)- ist; und
M eine _{L}-Isomer-Aminosäure der Formel -NHCH(CH₂COOH)C(=O)-ist.

6. Verbindung gemäß Anspruch 2 der Formel (III): 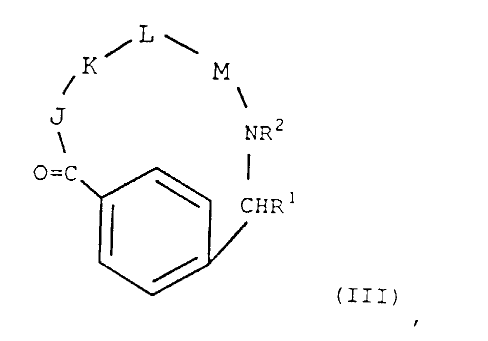 wobei
R¹ H, C₁-C₄-Alkyl, Phenyl, Benzyl oder Phenyl-(C₂-C₄)-alkyl ist;
R² H oder Methyl ist;
J β-Ala oder eine _{L}-Isomer- oder _{D}-Isomer-Aminosäure der Struktur -N(R³)C(R⁴) (R⁵)C(=O)- ist, wobei
R³ H oder CH₃ ist;
R⁴ H oder C₁-C₃-Alkyl ist;
R⁵ H, C₁-C₈-Alkyl, C₃-C₆-Cycloalkyl, C₃-C₆-Cycloalkylmethyl, C₁-C₆-Cycloalkylethyl, Phenyl, Phenylmethyl, CH₂OH, CH₂SH, CH₂OCH₃, CH₂SCH₃, CH₂CH₂SCH₃, (CH₂)ₛNH₂ oder (CH₂)ₛNHC(=NH) (NH₂) ist, wobei s = 3-5; oder
R³ und R⁵ unter Bildung von -CH₂CH₂CH₂- zusammengefaßt sein können; oder
R⁴ und R⁵ unter Bildung von -(CH₂)ᵤ-, wobei u = 2-5, zusammengefaßt sein können;
K eine _{L}-Isomer-Aminosäure der Struktur -N(R⁶)CH(R⁷)C(=O)-ist, wobei
R⁶ C₁-C₈-Alkyl ist;
R⁷ folgendes ist:
-(CH₂)ₚNHC(=NH) (NH₂), wobei p = 3-4 ; 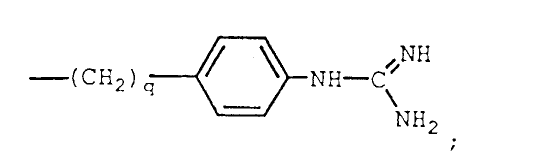 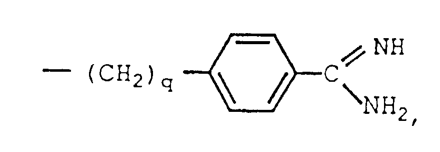 wobei q = 0, 1;
-(CH₂)ᵣX, wobei r = 4-6; 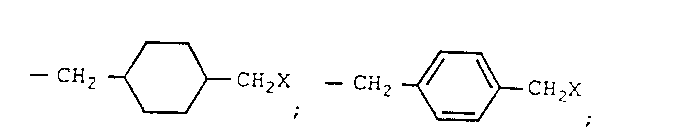 -(CH₂)ₘS(CH₂)₂X,wobei m = 1, 2;
- (C₃-C₇-Alkyl) -N-(C₁-C₆-alkyl) ; 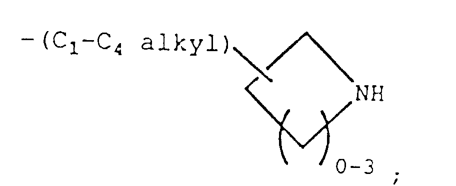 - (CH₂)ₘ-O-(C₁-C₄-Alkyl)-NH-(C₁-C₆-alkyl), wobei m = 1, 2;
-(CH₂)ₘ-S-(C₁-C₄-Alkyl)-NH-(C₁-C₆-alkyl), wobei m = 1, 2; und
X -NH₂ oder -NHC(=NH) (NH₂) ist, mit der Maßgabe, daß X nicht NH₂ ist, wenn r = 4.

7. Verbindung gemäß Anspruch 1, die ausgewählt ist aus:
der Verbindung der Formel (II): 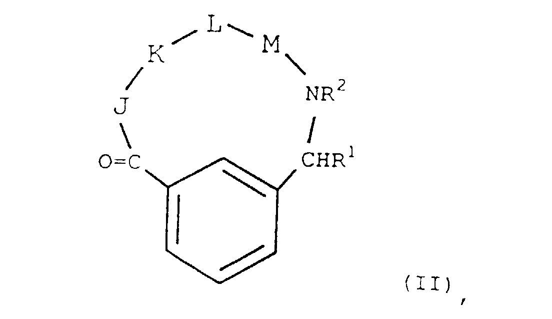 wobei R¹ und R² H sind, J _{D}-Val ist, K NMeArg ist, L Gly ist und M Asp ist;
der Verbindung der Formel (II), wobei R¹ und R² H sind, J _{D}-2-Aminobuttersäure ist, K NMeArg ist, L Gly ist und M Asp ist;
der Verbindung der Formel (II), wobei R und R² H sind, J _{D}-Leu ist, K NMeArg ist, L Gly ist und M Asp ist;
der Verbindung der Formel (II), wobei R¹ und R² H sind, J _{D}-Ala ist, K NMeArg ist, L Gly ist und M Asp ist;
der Verbindung der Formel (II), wobei R¹ und R² H sind, J Gly ist, K NMeArg ist, L Gly ist und M Asp ist;
der Verbindung der Formel (II), wobei R¹ und R² H sind, J _{D}-Pro ist, K NMeArg ist, L Gly ist und M Asp ist;
der Verbindung der Formel (II), wobei R¹ und R² H sind, J _{D}-Lys ist, K NMeArg ist, L Gly ist und M Asp ist;
der Verbindung der Formel (II), wobei R¹ und R² H sind, J β-Ala ist, K NMeArg ist, L Gly ist und M Asp ist;
der Verbindung der Formel (II), wobei R¹ und R² H sind, J _{D}-Val ist, K NMeArg ist, L β-Ala ist und M Asp ist;
der Verbindung der Formel (II), wobei R¹ und R² H sind, J Pro ist, K NMeArg ist, L Gly ist und M Asp ist;
der Verbindung der Formel (II), wobei R¹ und R² H sind, J NMeGly ist, K NMeArg ist, L Gly ist und M Asp ist;
der Verbindung der Formel (II), wobei R¹ Methyl ist (Isomer 1), R² H ist, J _{D}-Val ist, K NMeArg ist, L Gly ist und M Asp ist;
der Verbindung der Formel (II), wobei R¹ Methyl ist (Isomer 2), R² H ist, J _{D}-Val ist, K NMeArg ist, L Gly ist und M Asp ist;
der Verbindung der Formel (II), wobei R¹ Phenyl ist (Isomer 1), R² H ist, J _{D}-Val ist, K NMeArg ist, L Gly ist und M Asp ist;
der Verbindung der Formel (II), wobei R¹ Phenyl ist (Isomer 2), R² H ist, J _{D}-Val ist, K NMeArg ist, L Gly ist und M Asp ist;
der Verbindung der Formel (II), wobei R¹ und R² H sind, J _{D}-Val ist, K _{D}-NMeArg ist, L Gly ist und M Asp ist;
der Verbindung der Formel (II), wobei R¹ und R² H sind, J _{D}-Nle ist, K NMeArg ist, L Gly ist und M Asp ist;
der Verbindung der Formel (II), wobei R¹ und R² H sind, J _{D}-Phg ist, K NMeArg ist, L Gly ist und M Asp ist;
der Verbindung der Formel (II), wobei R¹ und R² H sind, J _{D}-Ile ist, K NMeArg ist, L Gly ist und M Asp ist;
der Verbindung der Formel (II), wobei R¹ und R² H sind, J _{D}-Phe ist, K NMeArg ist, L, Gly ist und M Asp ist;
der Verbindung der Formel (II), wobei R¹ und R² H sind, J _{D}-NMeGly ist, K NMeArg ist, L Gly ist und M Asp ist;
der Verbindung der Formel (II), wobei R¹ und R² H sind, J _{D}-Nle ist, K NMeArg ist, L Gly ist und M Asp ist;
der Verbindung der Formel (II), wobei R¹ und R² H sind, J _{D}-Tyr ist, K NMeArg ist, L Gly ist und M Asp ist;
der Verbindung der Formel (II), wobei R¹ und R² H sind, J _{D}-Val ist, K NMeAmf ist, L Gly ist und M Asp ist;
der Verbindung der Formel (II), wobei R¹ und R² H sind, J _{D}-Val ist, K NMeArg ist, L Ala ist und M Asp ist;
der Verbindung der Formel (II), wobei R¹ und R² H sind, J _{D}-Val ist, K NMeArg ist, L Gly ist und M α-MeAsp ist;
der Verbindung der Formel (II), wobei R¹ und R² H sind, J _{D}-Val ist, K NMeArg ist, L Gly ist und M β-MeAsp ist;
der Verbindung der Formel (II), wobei R¹ und R² H sind, J _{D}-Val ist, K NMeArg ist, L Gly ist und M NMeAsp ist;
der Verbindung der Formel (II), wobei R¹ und R² H sind, J _{D}-Val ist, K NMeArg ist, L Gly ist und M _{D}-Asp ist;
der Verbindung der Formel (II), wobei R¹ H ist, R² CH₃ ist, J _{D}-Val ist, K NMeArg ist, L Gly ist und M Asp ist;
der Verbindung der Formel (II), wobei R¹ und R² H sind, J _{D}-Abu ist, K Di-NMeOrn ist, L Gly ist und M Asp ist;
der Verbindung der Formel (III): 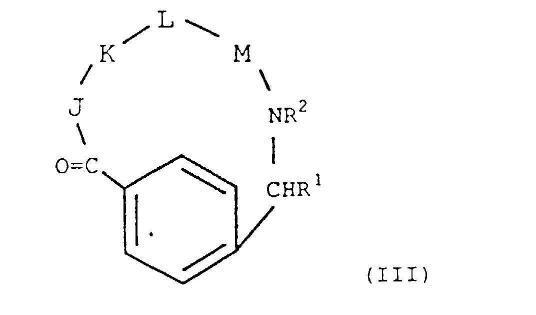 wobei R¹ und R² H sind, J _{D}-Val ist, K NMeArg ist, L Gly ist und M Asp ist;
der Verbindung der Formel (V): 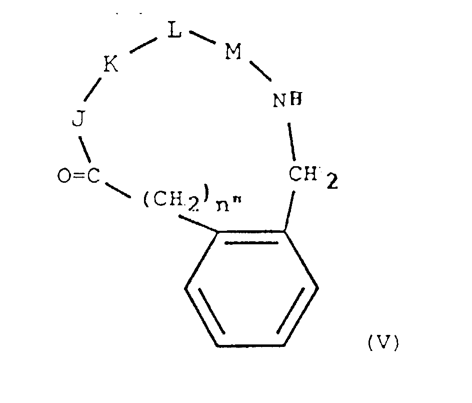 wobei n" = 1, J _{D}-Val ist, K NMeArg ist, L Gly ist und M Asp ist;
der Verbindung der Formel (V), wobei n" = 0, J _{D}-Val ist, K NMeArg ist, L Gly ist und M Asp ist;
der Verbindung der Formel (VI): 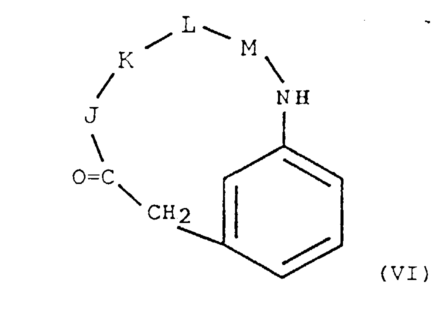 wobei J _{D}-Val ist, K NMeArg ist, L Gly ist und M Asp ist;
der Verbindung der Formel (VII): 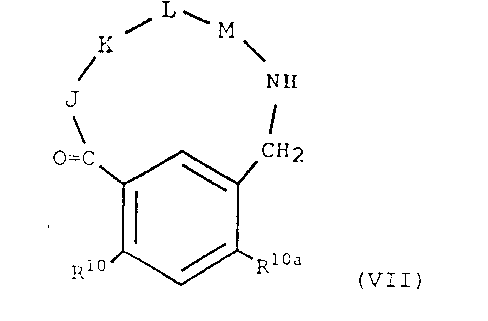 wobei R¹⁰ H ist, R^{10a} Cl ist, J _{D}-Val ist, K NMeArg ist, L Gly ist und M Asp ist;
der Verbindung der Formel (VII), wobei R¹⁰ H ist, R^{10a} I ist, J _{D}-Val ist, K NMeArg ist, L Gly ist und M Asp ist;
der Verbindung der Formel (VII), wobei R¹⁰ H ist, R^{10a} I ist, J _{D}-Abu ist, K NMeArg ist, L Gly ist und M Asp ist;
der Verbindung der Formel (VII), wobei R¹⁰ H ist, R^{10a} OMe ist, J _{D}-Val ist, K NMeArg ist, L Gly ist und M Asp ist;
der Verbindung Tier Formel (VII), wobei R¹⁰ H ist, R^{10a} Me ist, J _{D}-Val ist, K NMeArg ist, L Gly ist und Asp ist;
der Verbindung der Formel (VII), wobei R^{10a} H ist, R¹⁰ Cl ist, J _{D}-Val ist, K NMeArg ist, L Gly ist und M Asp ist;
der Verbindung der Formel (VII), wobei R^{10a} H ist, R¹⁰ MeO ist, J _{D}-Val ist, K NMeArg ist, L Gly ist und M Asp ist;
der Verbindung der Formel (VII), wobei R^{10a} H ist, R¹⁰ Me ist, J _{D}-Val ist, K NMeArg ist, L Gly ist und M Asp ist;
der Verbindung der Formel (VIII) : 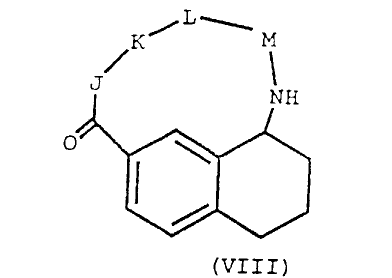 wobei J _{D}-Val ist, K NMeArg ist, L Gly ist und M Asp ist;
der Verbindung der Formel (IX) : 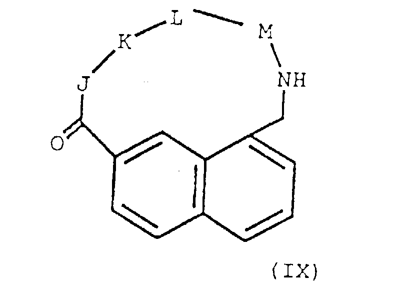 wobei J _{D}-Val ist, K NMeArg ist, L Gly ist und M Asp ist.

8. Verbindung gemäß Anspruch 7, die ausgewählt ist aus:
der Verbindung der Formel (II), wobei R¹ und R² H sind, J _{D}-Val ist, K NMeArg ist, L Gly ist und M Asp ist;
der Verbindung der Formel (II), wobei R¹ und R² H sind, J _{D}-2-Aminobuttersäure ist, K NMeArg ist, L Gly ist und M Asp ist;
der Verbindung der Formel (II), wobei R¹ und R² H sind, J _{D}-Leu ist, K NMeArg ist, L Gly ist und M Asp ist;
der Verbindung der Formel (II), wobei R¹ und R² H sind, J _{D}-Ala ist, K NMeArg ist, L Gly ist und M Asp ist;
der Verbindung der Formel (II), wobei R¹ und R² H sind, J Gly ist, K NMeArg ist, L Gly ist und M Asp ist;
der Verbindung der Formel (II), wobei R¹ und R² H sind, J _{D}-Pro ist, K NMeArg ist, L Gly ist und M Asp ist;
der Verbindung der Formel (II), wobei R¹ und R² H sind, J _{D}-Lys ist, K NMeArg ist, L Gly ist und M Asp ist;
der Verbindung der Formel (II), wobei R¹ und R² H sind, J β-Ala ist, K NMeArg ist, L Gly ist und M Asp ist;
der Verbindung der Formel (II), wobei R¹ und R² H sind, J NMeGly ist, K NMeArg ist, L Gly ist und M Asp ist;
der Verbindung der Formel (II), wobei R¹ Methyl ist (Isomer 1), R² H ist, J _{D}-Val ist, K NMeArg ist, L Gly ist und M Asp ist;
der Verbindung der Formel (II), wobei R¹ Methyl ist (Isomer 2), R² H ist, J _{D}-Val ist, K NMeArg ist, L Gly ist und M Asp ist;
der Verbindung der Formel (II), wobei R¹ Phenyl ist (Isomer 1), R² H ist, J _{D}-Val ist, K NMeArg ist, L Gly ist und M Asp ist;
der Verbindung der Formel (II), wobei R¹ und R² H sind, J _{D}-Val ist, K _{D}-NMeArg ist, L Gly ist und M Asp ist;
der Verbindung der Formel (II), wobei R¹ und R² H sind, J _{D}-Nle ist, K NMeArg ist, L Gly ist und M Asp ist;
der Verbindung der Formel (II), wobei R¹ und R² H sind, J _{D}-Phg ist, K NMeArg ist, L Gly ist und M Asp ist;
der Verbindung der Formel (II), wobei R¹ und R² H sind, J _{D}-Phe ist, K NMeArg ist, L Gly ist und M Asp ist;
der Verbindung der Formel (II), wobei R¹ und R² H sind, J _{D}-Ile ist, K NMeArg ist, L Gly ist und M Asp ist;
der Verbindung der Formel (III), wobei R¹ und R² H sind, J _{D}-Val ist, K NMeArg ist, L Gly ist und M Asp ist;
der Verbindung der Formel (V), wobei n" = 1, J _{D}-Val ist, K NMeArg ist, L Gly ist und M Asp ist;
der Verbindung der Formel (V), wobei n" = 0, J _{D}-Val ist, K NMeArg ist, L Gly ist und M Asp ist;
der Verbindung der Formel (VI), wobei J _{D}-Val ist, K NMeArg ist, L Gly ist und M Asp ist;
der Verbindung der Formel (VII), wobei R¹⁰ H ist, R^{10a} Cl ist, J _{D}-Val ist, K NMeArg ist, L Gly ist und M Asp ist;
der Verbindung der Formel (VII), wobei R¹⁰ H ist, R^{10a} ist, J _{D}-Val ist, K NMeArg ist, L Gly ist und M Asp ist;
der Verbindung der Formel (VII), wobei R¹ H ist, R^{10a} ist, J _{D}-Abu ist, K NMeArg ist, L Gly ist und M Asp ist;
der Verbindung der Formel (VII), wobei R¹⁰ H ist, R^{10a} Me ist, J _{D}-Val ist, K NMeArg ist, L Gly ist und M Asp ist;
der Verbindung der Formel (VII), wobei R^{10a} H ist, R¹⁰ C₁ ist, J _{D}-Val ist, K NMeArg ist, L Gly ist und M Asp ist;
der Verbindung der Formel (VII), wobei R^{10a} H ist, R¹⁰ MeO ist, J _{D}-Val ist, K NMeArg ist, L Gly ist und M Asp ist;
der Verbindung der Formel (VII), wobei R^{10a} H ist, R¹⁰ Me ist, J _{D}-Val ist, K NMeArg ist, L Gly ist und M Asp ist;
der Verbindung der Formel (II), wobei R¹ und R² H sind, J _{D}-Tyr ist, K NMeArg ist, L Gly ist und M Asp ist;
der Verbindung der Formel (II), wobei R¹ und R² H sind, J _{D}-Val ist, K NMeAmf ist, L Gly ist und M Asp ist;
der Verbindung der Formel (II), wobei R¹ und R² H sind, J _{D}-Val ist, K NMeArg ist, L Gly ist und M β-MeAsp ist;
der Verbindung der Formel (II), wobei R¹ ist, R² CH₃ ist, J _{D}-Val ist, K NMeArg ist, L Gly ist und M Asp ist;
der Verbindung der Formel (III), wobei R¹ und R² H sind, J _{D}-Val ist, K NMeArg ist, L Gly ist und M Asp ist;
der Verbindung der Formel (VIII), wobei J _{D}-Val ist, K NMeArg ist, L Gly ist und M Asp ist.

9. Verbindung gemäß Anspruch 8, die ausgewählt ist aus:
der Verbindung der Formel (II), wobei R¹ und R² H sind, J _{D}-Val ist, K NMeArg ist, L Gly ist und M Asp ist;
der Verbindung der Formel (II), wobei R¹ und R² H sind, J _{D}-2-Aminobuttersäure ist, K NMeArg ist, L Gly ist und M Asp ist;
der Verbindung der Formel (II), wobei R¹ und R² H sind, J _{D}-Leu ist, K NMeArg ist, L Gly ist und M Asp ist;
der Verbindung der Formel (II), wobei R¹ und R² H sind, J _{D}-Ala ist, K NMeArg ist, L Gly ist und M Asp ist;
der Verbindung der Formel (II), wobei R¹ und R² H sind, J _{D}-Pro ist, K NMeArg ist, L Gly ist und M Asp ist;
der Verbindung der Formel (II), wobei R¹ und R² H sind, J _{D}-Lys ist, K NMeArg ist, L Gly ist und M Asp ist;
der Verbindung der Formel (II), wobei R¹ und R² H sind, J _{D}-Nle ist, K NMeArg ist, L Gly ist und M Asp ist;
der Verbindung der Formel (II), wobei R¹ und R² H sind, J _{D}-Phe ist, K NMeArg ist, L Gly ist und M Asp ist;
der Verbindung der Formel (II), wobei R¹ und R² H sind, J _{D}-Ile ist, K NMeArg ist, L Gly ist und M Asp ist;
der Verbindung der Formel (V), wobei n" = 1, J _{D}-Val ist, K NMeArg ist, L Gly ist und M Asp ist;
der Verbindung der Formel (VI), wobei J _{D}-Val ist, K NMeArg ist, L Gly ist und M Asp ist;
der Verbindung der Formel (VII), wobei R¹⁰ H ist, R^{10a} Cl ist, J _{D}-Val ist, K NMeArg ist, L Gly ist und M Asp ist;
der Verbindung der Formel (VII), wobei R¹ H ist, R^{10a} I ist, J _{D}-Val ist, K NMeArg ist, L Gly ist und M Asp ist;
der Verbindung der Formel (VII), wobei R¹⁰ H ist, R^{10a} Me ist, J _{D}-Val ist, K NMeArg ist, L Gly ist und M Asp ist;
der Verbindung der Formel (VII), wobei R^{10a} H ist, R¹⁰ Cl ist, J _{D}-Val ist, K NMeArg ist, L Gly ist und M Asp ist;
der Verbindung der Formel (VII), wobei R^{10a} H ist, R¹⁰ MeO ist, J _{D}-Val ist, K NMeArg ist, L Gly ist und M Asp ist;
der Verbindung der Formel (VII), wobei R^{10a} H ist, R¹⁰ Me ist, J _{D}-Val ist, K NMeArg ist, L Gly ist und M Asp ist;
der Verbindung der Formel (II), wobei R¹ und R² H sind, J _{D}-Val ist, K NMeArg ist, L Gly ist und M β-MeAsp ist.

10. Verbindung der Formel (I): 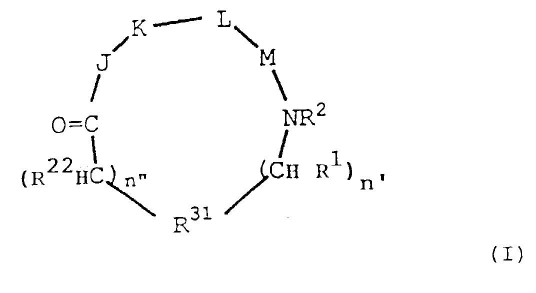 oder ein pharmazeutisch annehmbares Salz oder eine pharmazeutisch annehmbare Medikamentenvorstufe davon, wobei
R³¹ ausgewählt ist aus 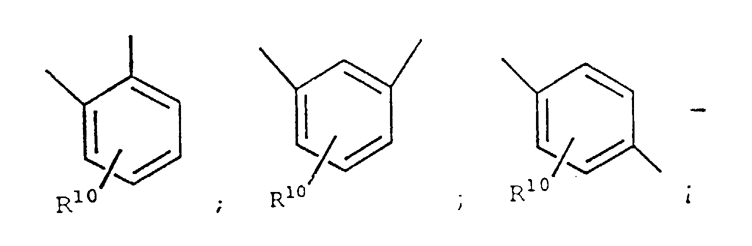 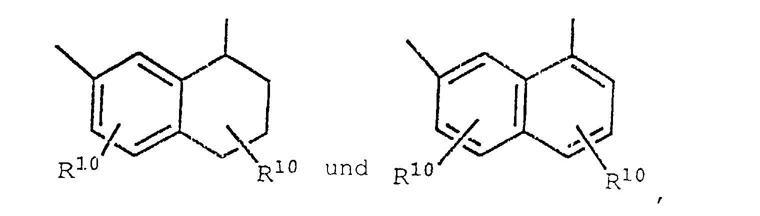 wobei R³¹ unabhängig mit 0-2 R¹⁰ substituiert sein kann;
n" 0 oder 1 ist;
n' 0 oder 1 ist;
R¹ und R²² unabhängig aus H, C₁-C₄-Alkyl, Phenyl, Benzyl, Phenyl-(C₂-C₄)-alkyl, C₁-C₄-Alkoxy ausgewählt sind;
R² H oder C₁-C₈-Alkyl ist;
R¹⁰ H, C₁-C₈-Alkyl, Phenyl, Halogen oder C₁-C₄-Alkoxy ist;
J β-Ala oder eine _{L}-Isomer- oder _{D}-Isomer-Aminosäure der Struktur -N(R³)C(R⁴)(R⁵)C(=O)- ist, wobei
R³ H oder CH₃ ist;
R⁴ H oder C₁-C₃-Alkyl ist;
R⁵ H, C₁-C₈-Alkyl, C₃-C₆-Cycloalkyl, C₃-C₆-Cycloalkylmethyl, C₁-C₆-Cycloalkylethyl, Phenyl, Phenylmethyl, CH₂OH, CH₂SH, CH₂OCH₃, CH₂SCH₃, CH₂CH₂SCH₃, (CH₂)ₛNH₂, (CH₂)ₛNHC(=NH)(NH₂), (CH₂)ₛNHR¹⁶ ist, wobei s = 3-5;
R³ und R⁵ auch unter Bildung von -(CH₂)ₜ- (t = 2-4) oder -CH₂SC(CH₃)₂- zusammengefaßt sein können; oder
R⁴ und R⁵ unter Bildung von -(CH₂)ᵤ-, wobei u = 2-5, ebenfalls zusammengefaßt sein können;
R¹⁶ eine Aminschutzgruppe ist;
K eine _{L}-Isomer-Aminosäure der Struktur -N(R⁶)CH(R⁷)C(=O)-ist, wobei
R⁶ C₁-C₈-Alkyl ist;
R⁷ folgendes ist:
-(CH₂)ₚNHC(=NH)(NH₂), wobei p = 3-5; 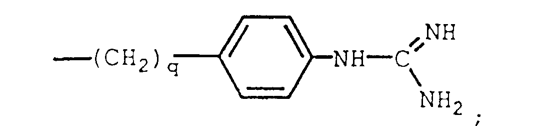 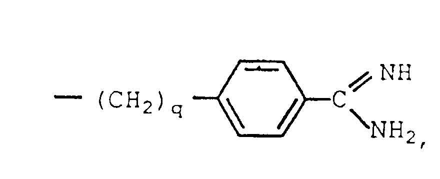 wobei q = 0, 1;
-(CH₂)ᵣX, wobei r = 4-6; 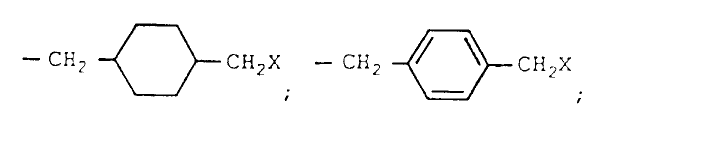 -(CH₂)ₘS(CH₂)₂X, wobei m = 1, 2;
- (C₃-C₇-Alkyl)-N-(C₁-C₆-alkyl ) ; 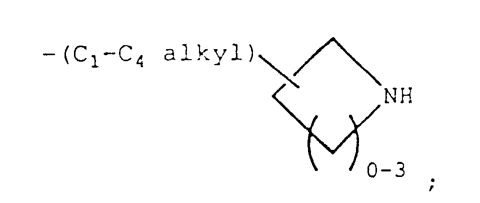 -(CH2)ₘ-O-(C₁-C₄-Alkyl)-NH-(C₁-C₆-alkyl), wobei m = 1, 2;
-(CH₂)ₘ-S-(C₁-C₄-Alkyl)-NH-(C₁-C₆-alkyl), wobei m = 1, 2; und
X NH₂ oder -NHC(=NH) (NH₂) ist;
L -Y(CH₂)ᵥC(=O)-ist, wobei
Y NH, O oder S ist und v = 1, 2;
M eine _{L}-Isomer-Aminosäure der Struktur -NH-CH(CH₂SO₃H)C(=O)- ist.

11. Verwendung einer Verbindung gemäß einem der Ansprüche 1 bis 10 zur Herstellung eines Medikaments zur Behandlung von thromboembolischen Störungen.

12. Verfahren zur Herstellung einer pharmazeutischen Zusammensetzung, die einen pharmazeutisch annehmbaren Träger und eine therapeutisch wirksame Menge einer Verbindung gemäß einem der Ansprüche 1 bis 10 umfaßt, wobei das Verfahren das Hinzufügen der Verbindung zu dem Träger umfaßt.

## Patentansprüche (Patentansprüche für folgende(n) Vertragsstaat(en): GR)

1. Verfahren zur Herstellung einer Verbindung der Formel (I): 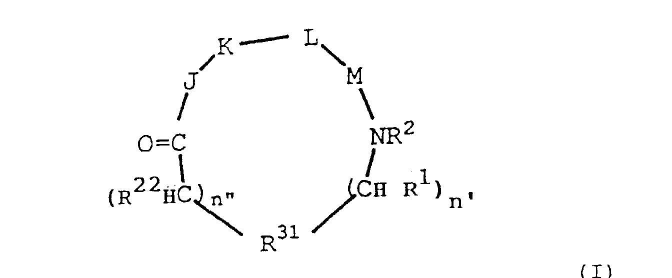 oder eines pharmazeutisch annehmbaren Salzes oder einer pharmazeutisch annehmbaren Medikamentenvorstufe davon, wobei
R³¹
(a) ein sechsgliedriger aromatischer carbocyclischer Ring der Formel 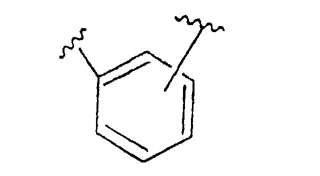 wobei der carbocyclische Ring unabhängig mit 0-2 R¹⁰ substituiert ist, oder
(b) ein zehngliedriger bicyclischer carbocycllscher Ring der Formel 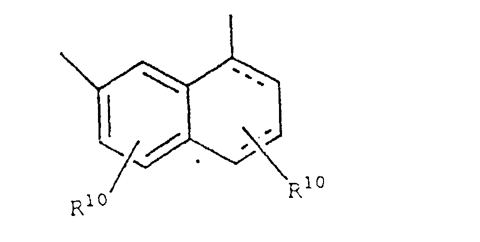 ist, wobei die gestrichelte Bindung eine Einfach- oder Doppelbindung sein kann;
n" 0 oder 1 ist;
n' 0 oder 1 ist;
R¹ und R²² unabhängig aus den folgenden Gruppen ausgewählt sind:
Wasserstoff,
C₁-C₈-Alkyl, das mit 0-2 R¹¹ substituiert ist,
C₂-C₈-Alkenyl, das mit 0-2 R¹¹ substituiert ist,
C₂-C₈-Alkinyl, das mit 0-2 R¹¹ substituiert ist,
C₃-C₈-Cycloalkyl, das mit 0-2 R¹¹ substituiert ist,
C₆-C₁₀-Bicycloalkyl, das mit 0-2 R¹¹ substituiert ist,
Aryl, das mit 0-2 R¹² substituiert ist,
ein heterocyclisches Ringsystem, das mit 0-2 R¹² substituiert ist und aus 5-10 Atomen einschließlich 1-3 N-, S-oder O-Heteroatomen besteht,
=O, F, Cl, Br, I, -CN, -CO₂R¹³, -C(=O)R¹³, -C(=-O)NR¹³R¹⁴, -CHO, -CH₂OR¹³, -OC(=O)R¹³, -OC(=O)OR¹³, -OR¹³, -OC(=O)NR¹³R¹⁴, -NR¹⁴C(=O)R¹³, -NR¹⁴(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -SR¹³, -S(=O)R^{13a}, -SO₂NR¹³R¹⁴, -CH₂NR¹³R¹⁴, -NR¹³R¹⁴, -NHC(=NH)NHR¹³, -C(=NH)NHR¹³, NO₂;
R¹¹ aus einem oder mehreren der folgenden Gruppen ausgewählt ist:
=O, F, Cl, Br, 1, -CF₃, -CN, -CO₂R¹³, -C(=O)R¹³, -C(=O)NR¹³R¹⁴, -CH₂OR¹³, -OC(=O)R¹³, -OC(=O)OR¹³, -OR¹³, C(=O)NR¹³R¹⁴, -CH₂OR¹³, -OC(=O)R¹³, -OC(=O)OR¹³, -OR¹³, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -SR¹³, -S(=O)R^{13a}, -SO₂NR¹³R¹⁴, -CH₂NR¹³R¹⁴, -NR¹³R¹⁴, -NHC(=NH)NHR¹³, -C(=NH)NHR¹³, =NOR¹⁴, NO₂;
C₁-C₅-Alkyl, C₂-C₄-Alkenyl, C₃-C₆-Cycloalkyl, C₃-C₆-Cycloalkylmethyl, C₂-C₆-Alkoxyalkyl, C₁-C₄-Alkyl (substituiert mit -NR¹³R¹⁴, -CF₃, NO₂, -SO₂R¹³ oder -S(=O)R^{13a})
Aryl, das mit 0-2 R¹² substituiert ist,
ein heterocyclisches Ringsystem, das mit 0-2 R¹² substituiert ist und aus 5-10 Atomen einschließlich 1-3 Stickstoff-, Schwefel- oder Sauerstoff-Heteroatomen besteht;
R¹² aus einem oder mehreren der folgenden Gruppen ausgewählt ist:
Phenyl, Benzyl, Phenethyl, Phenoxy, Benzyloxy, Halogen, Hydroxy, Nitro, Cyan, C₁-C₅-Alkyl, C₃-C₆-Cycloalkyl, C₃-C₆-Cycloalkylmethyl, C₇-C₁₀-Arylalkyl, C₁-C₄-Alkoxy, -CO₂R¹³, Hydroxamsäure, Hydrazid, Oxim, Boronsäure, Sulfonamid, Formyl, C₃-C₆-Cycloalkoxy, -OC(=O)R¹³, -O(=O)R¹³, -OC(=O)OR¹³, -OR¹³, -CH₂OR¹³, -NR¹³R¹⁴, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -S(=O)R^{13a}, -SR¹³, -SO₂NR¹³R¹⁴, C₂-C₆-Alkoxyalkyl, C₁-C₄-Hydroxyalkyl, Methylendioxy, Ethylendioxy;
R¹³ H, C₁-C₇-Alkyl, Aryl, -(C₁-C₆-alkyl)-aryl oder C₃-C₆-Alkoxyalkyl ist;
R^{13a} C₁-C₇-Alkyl, Aryl, -(C₁-C₆-alkyl)-aryl oder C₃-C₆-Alkoxyalkyl ist;
R¹⁴ OH, H, C₁-C₄-Alkyl oder Benzyl ist;
R² H oder C₁-C₈-Alkyl ist;
R¹⁰ aus einem oder mehreren der folgenden Gruppen ausgewählt ist:
Phenyl, Benzyl, Phenethyl, Phenoxy, Benzyloxy, Halogen, Hydroxy, Nitro, Cyan, C₁-C₅-Alkyl, C₃-C₆-Cycloalkyl, C₃-C₆-Cycloalkylmethyl, C₇-C₁₀-Arylalkyl, C₁-C₄-Alkoxy, -CO₂R¹³, -C(=O)NHOR¹³, -C(=O) NHNR¹³R¹⁴, Oxim, Boronsäure, C₃-C₆-Cycloalkoxy, -OC(=O)R¹³, -C(=O)R¹³, -OC(=O)OR¹³, -OR¹³, -CH₂OR¹³, -NR¹³R¹⁴, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R ^{13a}, -SO₃H, -SO₂R^{13a}, -S(=O)R^{13a}, -SR¹³, -SO₂NR¹³R¹⁴, C₂-C₆-Alkoxyalkyl, C₁-C₄-Hydroxyalkyl, Methylendioxy, Ethylendioxy, C₁-C₄-Halogenalkyl, C₁-C₄-Halogenalkoxy, C₁-C₄-Alkoxycarbonyl, C₁-C₄-Alkylcarbonyloxy, C₁-C₄-Alkylcarbonyl, C₁-C₄-Alkylcarbonylamino, -OCH₂CO₂H, 2-(1-Morpholino)ethoxy, C₁-C₄-Alkyl (substituiert mit -NR¹³R¹⁴, -CF₃, NO₂ oder -S(=O) R^{13a});
J β-Ala oder eine _{L}-Isomer- oder _{D}-Isomer-Aminosäure der Struktur -N(R³)C(R⁴) (R⁵)C(=O)- ist, wobei
R³ H oder CH₃ ist;
R⁴ H oder C₁-C₃-Alkyl ist;
R⁵ H, C₁-C₈-Alkyl, C₃-C₆-Cycloalkyl, C₃-C₆-Cycloalkylmethyl, C₁-C₆-Cycloalkylethyl, Phenyl, Phenylmethyl, CH₂OH, CH₂SH, CH₂OCH₃, CH₂SCH₃, CH₂CH₂SCH₃, (CH₂)ₛNH₂, (CH₂)ₛNHC(=NH) (NH₂), (CH₂)ₛNHR¹⁶ ist, wobei s 3 bis 5 ist;
R³ und R⁵ auch unter Bildung von -(CH₂)ₜ- (t = 2-4) oder -CH₂SC(CH₃)₂- zusammengefaßt sein können; oder
R⁴ und R⁵ unter Bildung von -(CH₂)ᵤ-, wobei u = 2-5, ebenfalls zusammengefaßt sein können;
R¹⁶ eine Aminschutzgruppe ist;
K eine _{D}-Isomer- oder _{L}-Isomer-Aminosäure der Struktur -N(R⁶)CH(R⁷)C(=O)- ist, wobei
R⁶ C₁-C₈-Alkyl ist;
R⁷ ausgewählt ist aus:
-(C₁-C₇-Alkyl)X; 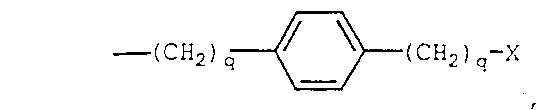 wobei q unabhängig 0, 1 ist; 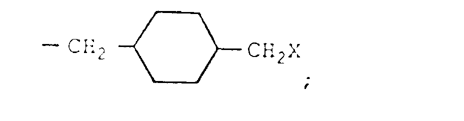 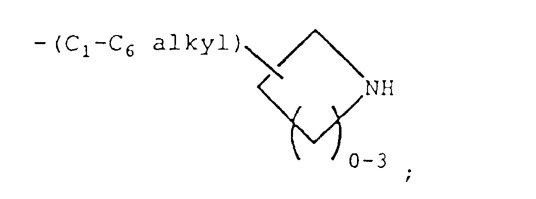 -(CH₂)ₘO-(C₁-C₄-Alkyl)-X, wobei m = 1, 2;
-(CH₂)ₘS-(C₁-C₄-Alkyl)-X, wobei m = 1, 2; und
X ausgewählt ist aus: 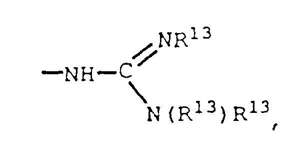 -N(R¹³)R¹³, -C(=NH)(NH₂), -SC(NH)-NH₂, wobei R¹³ wie oben definiert ist;
L -Y(CH₂)ᵥC(=O)- ist, wobei
Y NH, N(C₁-C₃-Alkyl), 0 oder S ist und v = 1, 2;
M eine _{D}-Isomer- oder _{L}-Isomer-Aminosäure der Struktur -NR¹⁷-CH(R⁸)C(=O)- ist, wobei
R¹⁷ H, C₁-C₃-Alkyl ist;
R⁸ -CH₂CO₂R¹³, -CH₂SO₃R^{13a}, -CH(CH₃)CO₂R¹³, -SO₂NR¹³R¹⁴, -CH₂-Boronsäure oder -CH₂-Tetrazol ist;
wobei das Verfahren das Cyclisieren einer linearen Peptidvorstufe umfaßt.

2. Verfahren gemäß Anspruch 1, wobei
R³¹ ausgewählt ist aus 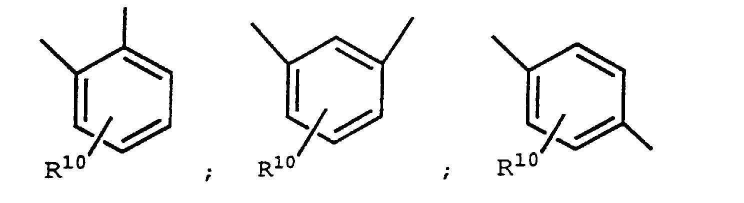 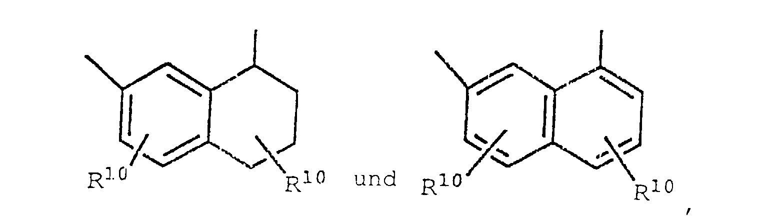 wobei R³¹ unabhängig mit 0-2 R¹⁰ substituiert sein kann;
R¹ und R²² unabhängig aus H, C₁-C₄-Alkyl, Phenyl, Benzyl, Phenyl-(C₂-C₄)-alkyl, C₁-C₄-Alkoxy ausgewählt sind;
R¹⁰ H, C₁-C₈-Alkyl, Phenyl, Halogen oder C₁-C₄-Alkoxy ist;
K eine _{L}-Isomer-Aminosäure der Struktur -N(R⁶)CH(R⁷)C(-O)-ist, wobei
R⁶ C₁-C₈-Alkyl ist;
R⁷ folgendes ist:
-(CH₂)ₚNHC(=NH)(NH₂), wobei p = 3-5; 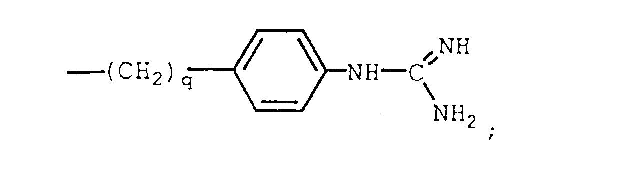 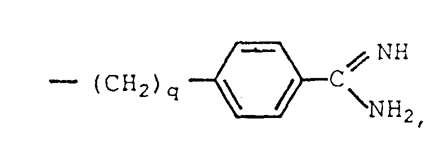 wobei q = 0, 1;
-(CH₂)ᵣX, wobei r = 4-6; 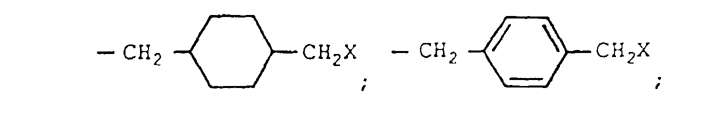 -(CH₂)ₘS(CH₂)₂X, wobei m = 1, 2;
- (C₃-C₇-Alkyl) -N-(C₁-C₆-alkyl) ; 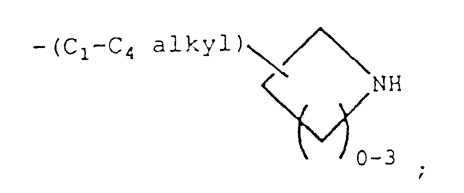 -(CH₂)ₘ-O-(C₁-C₄-Alkyl)-NH-(C₁-C₆-alkyl), wobei m = 1, 2;
-(CH₂)ₘ-S-(C₁-C₄-Alkyl)-NH-(C₁-C₆-alkyl), wobei m = 1, 2; und
X NH₂ oder -NHC(=NH) (NH₂) ist;
oder
L -Y(CH₂)ᵥC(=O)- ist, wobei
Y NH, O oder S ist und v = 1, 2;
M eine _{L}-Isomer-Aminosaure der Struktur -NH-CH(R⁸)C(=O)-ist, wobei
R⁸ -CH₂CO₂H oder -CH(CH₃)CO₂H ist.

3. Verfahren gemäß Anspruch 2, wobei die hergestellte Verbindung die Formel (II) hat: 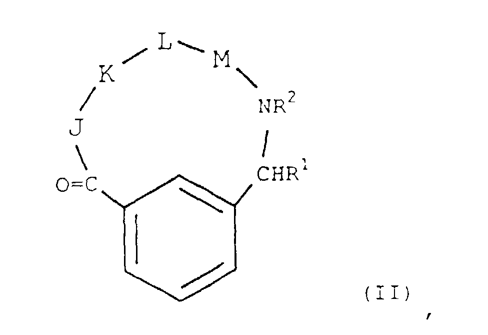 wobei
R¹ H, C₁-C₄-Alkyl, Phenyl, Benzyl oder Phenyl-(C₂-C₄)-alkyl ist;
R² H oder Methyl ist;
J β-Ala oder eine _{L}-Isomer- oder _{D}-Isomer-Aminosäure der Struktur -N(R³)C(R⁴)(R⁵)C(=O)- ist, wobei
R³ H oder CH₃ ist;
R⁴ H oder C₁-C₃-Alkyl ist;
R⁵ H, C₁-C₈-Alkyl, C₃-C₆-Cycloalkyl, C₃-C₆-Cycloalkyl-methyl, C₁-C₆-Cycloalkylethyl, Phenyl, Phenylmethyl, CH₂OH, CH₂SH, CH₂OCH₃, CH₂SCH₃, CH₂CH₂SCH₃, (CH₂)ₛNH₂ oder (CH₂)ₛNHC(=NH) (NH₇) ist, wobei s = 3-5; oder
R³ und R⁵ unter Bildung von -CH₂CH₂CH₂- zusammengefaßt sein können; oder
R⁴ und R⁵ unter Bildung von -(CH₂)ᵤ-, wobei u = 2-5, zusammengefaßt sein können;
K eine _{L}-Isomer-Aminosäure der Struktur -N(R⁶)CH(R⁷)C(=O)-ist, wobei
R⁶ C₁-C₈-Alkyl ist;
R⁷ folgendes ist:
-(CH₂)ₚNHC(=NH)(NH₂), wobei p = 3-5; 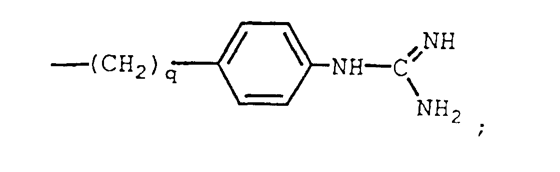 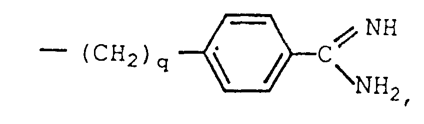 wobei q = 0, 1;
-(CH₂)ᵣX,wobei r 4-6; 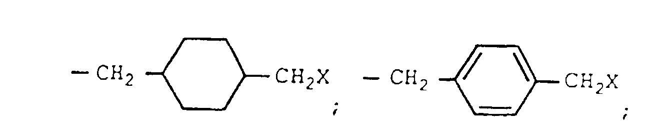 -(CH₂)ₘS(CH₂)₂X,wobei m = 1, 2;
-(CH₂)ₘ-O-(C₁-C₄-Alkyl)-NH-(C₁-C₆-alkyl), wobei m = 1, 2;
-(CH₂)ₘ-S-(C₁-C₄-Alkyl)-NH-(C₁-C₆-alkyl),wobei m = 1, 2; und
- (C₃-C₇-Alkyl) -N-(C₁-C₆-alkyl) ; 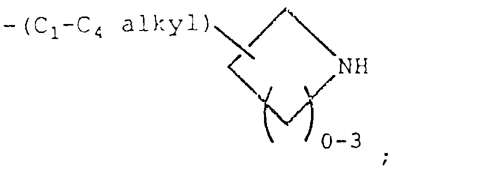
X -NH₂ oder -NHC(=NH) (NH₂) ist, mit der Maßgabe, daß X nicht NH₂ ist, wenn r = 4.

4. Verfahren gemäß Anspruch 3, wobei
J β-Ala oder eine _{L}-Isomer- oder _{D}-Isomer-Aminosäure der Struktur -N(R³)C(R⁴) (R⁵)C(=O)- ist, wobei
R³ H oder CH₃ ist;
R⁴ H ist;
R⁵ H, C₁-C₈-Alkyl, C₃-C₆-Cycloalkyl, C₃-C₆-Cycloalkylmethyl, C₁-C₆-Cycloalkylethyl, Phenyl, Phenylmethyl, CH₂OH, CH₂SH, CH₂OCH₃, CH₂SCH₃, CH₂CH₂SCH₃, (CH₂)ₛNH₂ oder (CH₂)ₛNHC(=NH)(NH₂) ist, wobei s = 3, 4, 5; oder
R² und R⁵ unter Bildung von -CH₂CH₂CH₂- zusammengefaßt sein können;
K eine _{L}-Isomer-Aminosäure der Struktur -N(CH₃)CH(R⁷)C (=O) - ist, wobei
R⁷ folgendes ist:
-(CH₂)ₚNHC(=NH)(NH₂), wobei p 3-4 ; 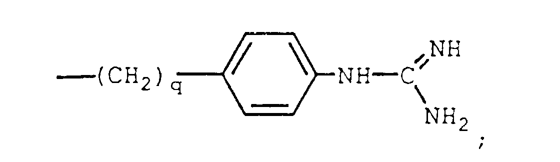 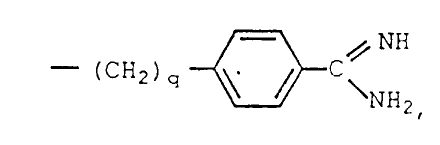 wobei q = 0, 1;
-(CH₂)ᵣX, wobei r 4-6; 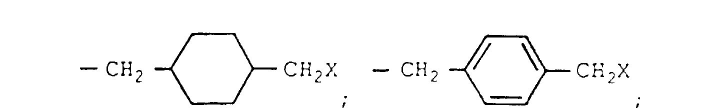 -(CH₂)ₘS(CH₂)₂X, wobei m = 1, 2;
-(C₄-C₇-Alkyl) -N-(C₁-C₆-alkyl); 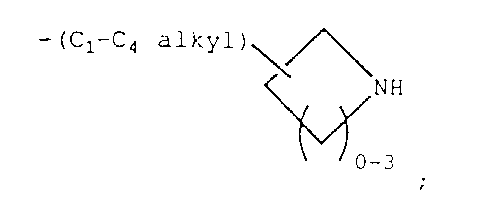 -(CH₂)ₘ-O-(C₁-C₄-Alkyl)-NH-(C₁-C₆-alkyl), wobei m = 1, 2;
-(CH₂)ₘ-S-(C₁-C₄-Alkyl)-NH-(C₁-C₆-alkyl), wobei m = 1, 2; und
X -NH₂ oder -NHC(=NH) (NH₂) ist, mit der Maßgabe, daß X nicht -NH₂ ist, wenn r = 4;
L -YCH₂C(=O)- ist, wobei
Y NH oder O ist;
M eine _{L}-Isomer-Aminosäure der Struktur -NH-CH(CH₂CO₂H)C(=O)- ist.

5. Verfahren gemäß Anspruch 3 und 4, wobei
R¹ H ist;
R² H ist;
J β-Ala oder eine _{L}-Isomer- oder _{D}-Isomer-Aminosäure der Struktur -N(R³)CH(R⁵)C(=O)- ist, wobei
R³ H ist und R⁵ H, CH₃, CH₂CH₃, CH(CH₃)₂, CH(CH₃)CH₂CH₃, CH₂CH₂CH₃, CH₂CH₂CH₂CH₃, CH₂CH₂SCH₃, CH₂CH(CH₃)₂, (CH₂)₄NH₂ ist; oder
R³ CH₃ ist und R⁵ H ist; oder
R³ und R⁵ unter Bildung von -CH₂CH₂CH₂- zusammengefaßt sein können;
K eine _{L}-Isomer-Aminosäure der Formel -N(CH₃)CH(R⁷)-C(=O)-ist, wobei
R⁷ - (CH₂)₃NHC(=NH)(NH₂) ist;
L -NHCH₂C(=O)- ist; und
M eine _{L}-Isomer-Aminosäure der Formel -NHCH(CH₂COOH)C(=O)-ist.

6. Verfahren gemäß Anspruch 2, wobei die hergestellte Verbindung die Formel (III) hat: 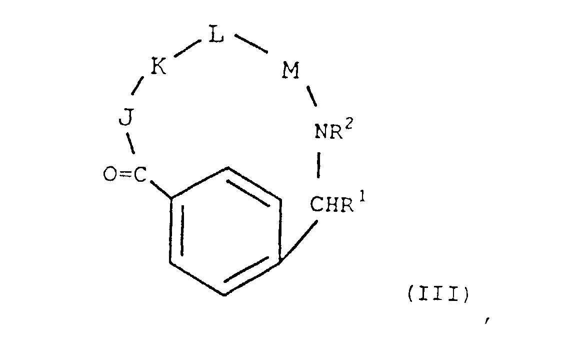 wobei
R¹ H, C₁-C₄-Alkyl, Phenyl, Benzyl oder Phenyl-(C₂-C₄)-alkyl ist;
R² H oder Methyl ist;
J β-Ala oder eine _{L}-Isomer- oder _{D}-Isomer-Aminosäure der Struktur -N(R³)C(R⁴) (R⁵)C(=O)- ist, wobei
R³ H oder CH₃ ist;
R⁴ H oder C₁-C₃-Alkyl ist;
R⁵ H, C₁-C₈-Alkyl, C₃-C₆-Cycloalkyl, C₃-C₆-Cycloalkylmethyl, C₁-C₆-Cycloalkylethyl, Phenyl, Phenylmethyl, CH₂OH, CH₂SH, CH₂OCH₃, CH₂SCH₃, CH₂CH₂SCH₃, (OH₂)ₛNH₂ oder (CH₂)ₛNHC(=NH) (NH₂) ist, wobei s = 3-5; oder
R³ und R⁵ unter Bildung von -CH₂CH₂CH₂- zusammengefaßt sein können; oder
R⁴ und R⁵ unter Bildung von -(CH₂)ᵤ-, wobei u = 2-5, zusammengefaßt sein können;
K eine _{L}-Isomer-Aminosäure der Struktur -N(R⁶)CH(R⁷)C(=O)-ist, wobei
R⁶ C₁-C₈-Alkyl ist;
R⁷ folgendes ist:
-(CH₂)ₚNHC(=NH)(NH₂), wobei p = 3-4 ; 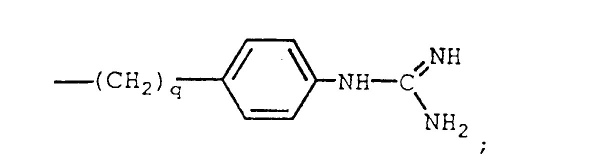 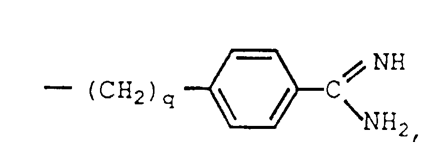 wobei q = 0, 1;
-(CH₂)ᵣX,wobei r = 4-6; 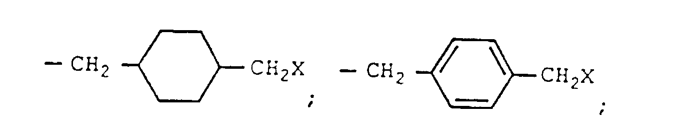 -(CH₂)ₘS(CH₂)₂X, wobei m = 1, 2;
-(C₃-C₇-Alkyl) -N-(C₁-C₆-alkyl) ; 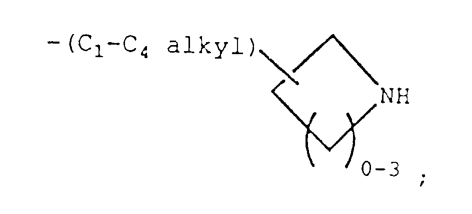 -(CH₂)ₘ-O- (C₁-C₄-Alkyl) -NH-(C₁-C₆-alkyl), wobei m = 1, 2;
-(CH₂)ₘ-S-(C₁-C₄-Alkyl)-NH-(C₁-C₆-alkyl), wobei m = 1, 2; und
X -NH₂ oder -NHC(=NH) (NH₂) ist, mit der Maßgabe, daß X nicht NH₂ ist, wenn r = 4.

7. Verfahren gemäß Anspruch 1, wobei die hergestellte Verbindung ausgewählt ist aus:
der Verbindung der Formel (II): 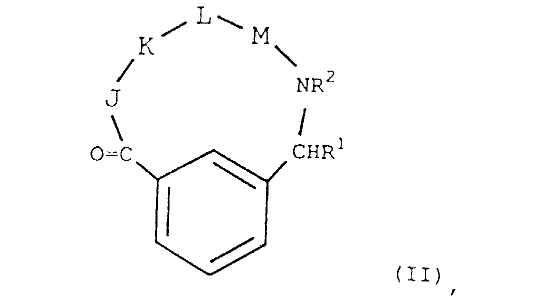 wobei R¹ und R² H sind, J _{D}-Val ist, K NMeArg ist, L Gly ist und M Asp ist;
der Verbindung der Formel (II), wobei R¹ und R² H sind, J _{D}-2-Aminobuttersäure ist, K NMeArg ist, L Gly ist und M Asp ist;
der Verbindung der Formel (II), wobei R¹ und R² H sind, J _{D}-Leu ist, K NMeArg ist, L Gly ist und M Asp ist;
der Verbindung der Formel (II), wobei R¹ und R² H sind, J _{D}-Ala ist, K NMeArg ist, L Gly ist und M Asp ist;
der Verbindung der Formel (II), wobei R¹ und R² H sind, J Gly ist, K NMeArg ist, L Gly ist und M Asp ist;
der Verbindung der Formel (II), wobei R¹ und R² H sind, J _{D}-Pro ist, K NMeArg ist, L Gly ist und M Asp ist;
der Verbindung der Formel (II), wobei R¹ und R² H sind, J _{D}-Lys ist, K NMeArg ist, L Gly ist und M Asp ist;
der Verbindung der Formel (II), wobei R¹ und R² H sind, J β-Ala ist, K NMeArg ist, L Gly ist und M Asp ist;
der Verbindung der Formel (II), wobei R¹ und R² H sind, J _{D}-Val ist, K NMeArg ist, L β-Ala ist und M Asp ist;
der Verbindung der Formel (II), wobei R¹ und R² H sind, J Pro ist, K NMeArg ist, L Gly ist und M Asp ist;
der Verbindung der Formel (II), wobei R¹ und R² H sind, J NMeGly ist, K NMeArg ist, L Gly ist und M Asp ist;
der Verbindung der Formel (II), wobei R¹ Methyl ist (Isomer 1), R² H ist, J _{D}-Val ist, K NMeArg ist, L Gly ist und M Asp ist;
der Verbindung der Formel (II), wobei R¹ Methyl ist (Isomer 2), R² H ist, J _{D}-Val ist, K NMeArg ist, L Gly ist und M Asp ist;
der Verbindung der Formel (II), wobei R¹ Phenyl ist (Isomer 1), R² H ist, J _{D}-Val ist, K NMeArg ist, L Gly ist und M Asp ist;
der Verbindung der Formel (II), wobei R¹ Phenyl ist (Isomer 2), R² H ist, J _{D}-Val ist, K NMeArg ist, L Gly ist und M Asp ist;
der Verbindung der Formel (II), wobei R¹ und R² H sind, J _{D}-Val ist, K _{D}-NMeArg ist, L Gly ist und M Asp ist;
der Verbindung der Formel (II), wobei R¹ und R² H sind, J _{D}-Nle ist, K NMeArg ist, L Gly ist und M Asp ist;
der Verbindung der Formel (II), wobei R¹ und R² H sind, J _{D}-Phg ist, K NMeArg ist, L Gly ist und M Asp ist;
der Verbindung der Formel (II), wobei R¹ und R² H sind, J _{D}-Ile ist, K NMeArg ist, L Gly ist und M Asp ist;
der Verbindung der Formel (II), wobei R¹ und R² H sind, J _{D}-Phe ist, K NMeArg ist, L Gly ist und M Asp ist;
der Verbindung der Formel (II), wobei R¹ und R² H sind, J _{D}-NMeGly ist, K NMeArg ist, L Gly ist und M Asp ist;
der Verbindung der Formel (II), wobei R¹ und R² H sind, J _{D}-Nle ist, K NMeArg ist, L Gly ist und M Asp ist;
der Verbindung der Formel (II), wobei R¹ und R² H sind, J _{D}-Tyr ist, K NMeArg ist, L Gly ist und M Asp ist;
der Verbindung der Formel (II), wobei R¹ und R² H sind, J _{D}-Val ist, K NMeAmf ist, L Gly ist und M Asp ist;
der Verbindung der Formel (II), wobei R¹ und R² H sind, J _{D}-Val ist, K NMeArg ist, L Ala ist und M Asp ist;
der Verbindung der Formel (II), wobei R¹ und R² H sind, J _{D}-Val ist, K NMeArg ist, L Gly ist und M α-MeAsp ist;
der Verbindung der Formel (II), wobei R¹ und R² H sind, J _{D}-Val ist, K NMeArg ist, L Gly ist und M β-MeAsp ist;
der Verbindung der Formel (II), wobei R¹ und R² H sind, J _{D}-Val ist, K NMeArg ist, L Gly ist und M NMeAsp ist; der Verbindung der Formel (II), wobei R¹ und R² H sind, J _{D}-Val ist, K NMeArg ist, L Gly ist und M _{D}-Asp ist;
der Verbindung der Formel (II), wobei R¹ H ist, R² CH₃ ist, J _{D}-Val ist, K NMeArg ist, L Gly ist und M Asp ist;
der Verbindung der Formel (II), wobei R¹ und R² H sind, J _{D}-Abu ist, K Di-NMeOrn ist, L Gly ist und M Asp ist;
der Verbindung der Formel (III): 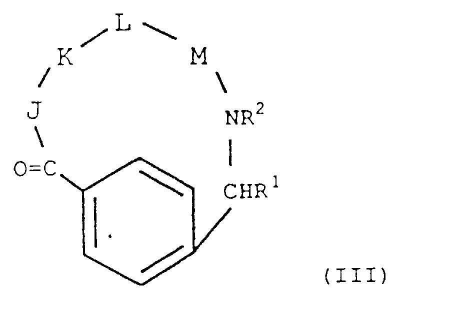 wobei R¹ und R² H sind, J _{D}-Val ist, K NMeArg ist, L Gly ist und M Asp ist;
der Verbindung der Formel (V): 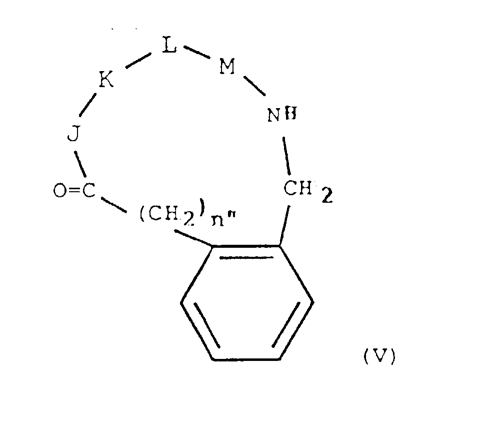 wobei n" = 1, J _{D}-Val ist, K NMeArg ist, L Gly ist und M Asp ist;
der Verbindung der Formel (V), wobei n" = 0, J _{D}-Val ist, K NMeArg ist, L Gly ist und M Asp ist;
der Verbindung der Formel (VI): 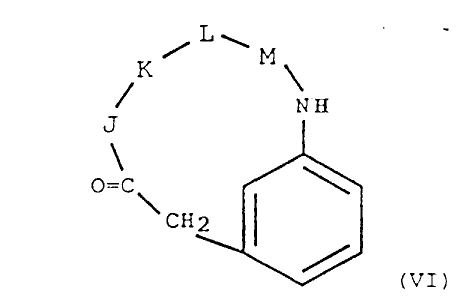 wobei J _{D}-Val ist, K NMeArg ist, L Gly ist und M Asp ist; der Verbindung der Formel (VII): 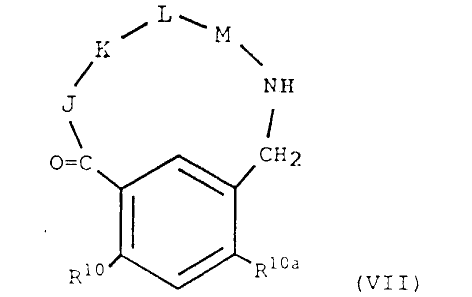 wobei R¹⁰ H ist, R^{10a} Cl ist, J _{D}-Val ist, K NMeArg ist, I Gly ist und M Asp ist;
der Verbindung der Formel (VII), wobei R¹⁰ H ist, R^{10a} I ist, J _{D}-Val ist, K NMeArg ist, L Gly ist und M Asp ist;
der Verbindung der Formel (VII), wobei R¹⁰ H ist, R^{10a} I ist, J _{D}-Abu ist, K NMeArg ist, L Gly ist und M Asp ist;
der Verbindung der Formel (VII), wobei R¹⁰ H ist, R^{10a} OMe ist, J _{D}-Val ist, K NMeArg ist, L Gly ist und M Asp ist;
der Verbindung der Formel (VII), wobei R¹⁰ H ist, R^{10a} Me ist, J _{D}-Val ist, K NMeArg ist, L Gly ist und M Asp ist;
der Verbindung der Formel (VII), wobei R^{10a} H ist, R¹⁰ Cl ist, J _{D}-Val ist, K NMeArg ist, L Gly ist und M Asp ist;
der Verbindung der Formel (VII), wobei R^{10a} H ist, R¹⁰ MeO ist, J _{D}-Val ist, K NMeArg ist, L Gly ist und M Asp ist;
der Verbindung der Formel (VII), wobei R^{10a} H ist, R¹⁰ Me ist, J _{D}-Val ist, K NMeArg ist, L Gly ist und M Asp ist;
der Verbindung der Formel (VIII) : 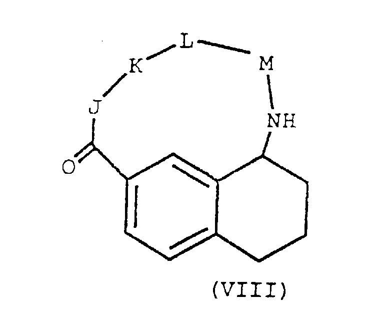 wobei J _{D}-Val ist, K NMeArg ist, L Gly ist und M Asp ist;
der Verbindung der Formel (IX) : 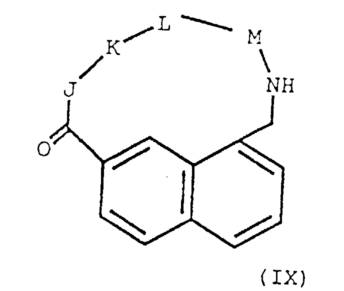 wobei J _{D}-Val ist, K NMeArg ist, L Gly ist und M Asp ist.

8. Verfahren gemäß Anspruch 7, wobei die hergestellte Verbindung ausgewählt ist aus:
der Verbindung der Formel (II), wobei R¹ und R² H sind, J _{D}-Val ist, K NMeArg ist, L Gly ist und M Asp ist;
der Verbindung der Formel (II), wobei R¹ und R² H sind, J _{D}-2-Aminobuttersäure ist, K NMeArg ist, L Gly ist und M Asp ist;
der Verbindung der Formel (II), wobei R¹ und R² H sind, J _{D}-Leu ist, K NMeArg ist, L Gly ist und M Asp ist;
der Verbindung der Formel (II), wobei R¹ und R² H sind, J _{D}-Ala ist, K NMeArg ist, L Gly ist und M Asp ist;
der Verbindung der Formel (II), wobei R¹ und R² H sind, J Gly ist, K NMeArg ist, L Gly ist und M Asp ist;
der Verbindung der Formel (II), wobei R¹ und R² H sind, J _{D}-Pro ist, K NMeArg ist, L Gly ist und M Asp ist
der Verbindung der Formel (II), wobei R¹ und R² H sind, J _{D}-Lys ist, K NMeArg ist, L Gly ist und M Asp ist;
der Verbindung der Formel (II), wobei R¹ und R² H sind, J β-Ala ist, K NMeArg ist, L Gly ist und M Asp ist;
der Verbindung der Formel (II), wobei R¹ und R² H sind, J NMeGly ist, K NMeArg ist, L Gly ist und M Asp ist;
der Verbindung der Formel (II), wobei R¹ Methyl ist (Isomer 1), R² H ist, J _{D}-Val ist, K NMeArg ist, L Gly ist und M Asp ist;
der Verbindung der Formel (II), wobei R¹ Methyl ist (Isomer 2), R² H ist, J _{D}-Val ist, K NMeArg ist, L Gly ist und M Asp ist;
der Verbindung der Formel (II), wobei R¹ Phenyl ist (Isomer 1), R² H ist, J _{D}-Val ist, K NMeArg ist, L Gly ist und M Asp ist;
der Verbindung der Formel (II), wobei R¹ und R² H sind, J _{D}-Val ist, K D-NMeArg ist, L Gly ist und M Asp ist;
der Verbindung der Formel (II), wobei R¹ und R² H sind, J _{D}-Nle ist, K NMeArg ist, L Gly ist und M Asp ist;
der Verbindung der Formel (II), wobei R¹ und R² H sind, J _{D}-Phg ist, K NMeArg ist, L Gly ist und M Asp ist;
der Verbindung der Formel (II), wobei R¹ und R² H sind, J _{D}-Phe ist, K NMeArg ist, L Gly ist und M Asp ist;
der Verbindung der Formel (II), wobei R¹ und R² H sind, J _{D}-Ile ist, K NMeArg ist, L Gly ist und M Asp ist;
der Verbindung der Formel (III), wobei R¹ und R² H sind, J _{D}-Val ist, K NMeArg ist, L Gly ist und M Asp ist;
der Verbindung der Formel (V), wobei n" = 1, J _{D}-Val ist, K NMeArg ist, L Gly ist und M Asp ist;
der Verbindung der Formel (V), wobei n" = 0, J _{D}-Val ist, K NMeArg ist, L Gly ist und M Asp ist;
der Verbindung der Formel (VI), wobei J _{D}-Val ist, K NMeArg ist, L Gly ist und M Asp ist;
der Verbindung der Formel (VII), wobei R¹⁰ H ist, R^{10a} C₁ ist, J _{D}-Val ist, K NMeArg ist, L Gly ist und M Asp ist;
der Verbindung der Formel (VII), wobei R¹⁰ H ist, R^{10a} I ist, J _{D}-Val ist, K NMeArg ist, L Gly ist und M Asp ist;
der Verbindung der Formel (VII), wobei R¹⁰ H ist, R^{10a} I ist, J _{D}-Abu ist, K NMeArg ist, L Gly ist und M Asp ist;
der Verbindung der Formel (VII), wobei R¹⁰ H ist, R^{10a} Me ist, J _{D}-Val ist, K NMeArg ist, L Gly ist und M Asp ist;
der Verbindung der Formel (VII), wobei R^{10a} H ist, R¹⁰ Cl ist, J _{D}-Val ist, K NMeArg ist, L Gly ist und M Asp ist;
der Verbindung der Formel (VII), wobei R^{10a} H ist, R¹⁰ MeO ist, J _{D}-Val ist, K NMeArg ist, L Gly ist und M Asp ist;
der Verbindung der Formel (VII), wobei R^{10a} H ist, R¹⁰ Me ist, J _{D}-Val ist, K NMeArg ist, L Gly ist und M Asp ist;
der Verbindung der Formel (II), wobei R¹ und R² H sind, J _{D}-Tyr ist, K NMeArg ist, L Gly ist und M Asp ist;
der Verbindung der Formel (II), wobei R¹ und R² H sind, J _{D}-Val ist, K NMeAmf ist, L Gly ist und M Asp ist;
der Verbindung der Formel (II), wobei R¹ und R² H sind, J _{D}-Val ist, K NMeArg ist, L Gly ist und M β-MeAsp ist;
der Verbindung der Formel (II), wobei R¹ H ist, R² CH₃ ist, J _{D}-Val ist, K NMeArg ist, L Gly ist und M Asp ist;
der Verbindung der Formel (III), wobei R¹ und R² H sind, J _{D}-Val ist, K NMeArg ist, L Gly ist und M Asp ist;
der Verbindung der Formel (VIII), wobei J _{D}-Val ist, K NMeArg ist, L Gly ist und M Asp ist.

9. Verfahren gemäß Anspruch 8, wobei die hergestellte Verbindung ausgewählt ist aus:
der Verbindung der Formel (II), wobei R¹ und R² H sind, J _{D}-Val ist, K NMeArg ist, L Gly ist und M Asp ist;
der Verbindung der Formel (II), wobei R¹ und R² H sind, J _{D}-2-Aminobuttersäure ist, K NMeArg ist, L Gly ist und M Asp ist;
der Verbindung der Formel (II), wobei R¹ und R² H sind, J _{D}-Leu ist, K NMeArg ist, L Gly ist und M Asp ist;
der Verbindung der Formel (II), wobei R¹ und R² H sind, J _{D}-Ala ist, K NMeArg ist, L Gly ist und M Asp ist;
der Verbindung der Formel (II), wobei R¹ und R² H sind, J _{D}-Pro ist, K NMeArg ist, L Gly ist und M Asp ist;
der Verbindung der Formel (II), wobei R¹ und R² H sind, J _{D}-Lys ist, K NMeArg ist, L Gly ist und M Asp ist;
der Verbindung der Formel (II), wobei R¹ und R² H sind, J _{D}-Nle ist, K NMeArg ist, L Gly ist und M Asp ist;
der Verbindung der Formel (II), wobei R¹ und R² H sind, J _{D}-Phe ist, K NMeArg ist, L Gly ist und M Asp ist;
der Verbindung der Formel (II), wobei R¹ und R² H sind, J _{D}-Ile ist, K NMeArg ist, L Gly ist und M Asp ist;
der Verbindung der Formel (V), wobei n" = 1, J _{D}-Val ist, K NMeArg ist, L Gly ist und M Asp ist;
der Verbindung der Formel (VI), wobei J _{D}-Val ist, K NMeArg ist, L Gly ist und M Asp ist;
der Verbindung der Formel (VII), wobei R¹⁰ H ist, R^{10a} Cl ist, J _{D}-Val ist, K NMeArg ist, L Gly ist und M Asp ist;
der Verbindung der Formel (VII), wobei R¹⁰ H ist, R^{10a} I ist, J _{D}-Val ist, K NMeArg ist, L Gly ist und M Asp ist;
der Verbindung der Formel (VII), wobei R¹⁰ H ist, R^{10a} Me ist, J _{D}-Val ist, K NMeArg ist, L Gly ist und M Asp ist;
der Verbindung der Formel (VII), wobei R^{10a} H ist, R¹⁰ Cl ist, J _{D}-Val ist, K NMeArg ist, L Gly ist und M Asp ist;
der Verbindung der Formel (VII), wobei R^{10a} H ist, R¹⁰ MeO ist, J _{D}-Val ist, K NMeArg ist, L Gly ist und M Asp ist;
der Verbindung der Formel (VII), wobei R^{10a} H ist, R¹⁰ Me ist, J _{D}-Val ist, K NMeArg ist, L Gly ist und M Asp ist;
der Verbindung der Formel (II), wobei R¹ und R² H sind, J _{D}-Val ist, K NMeArg ist, L Gly ist und M β-MeAsp ist.

10. Verfahren zur Herstellung einer Verbindung der Formel (I): 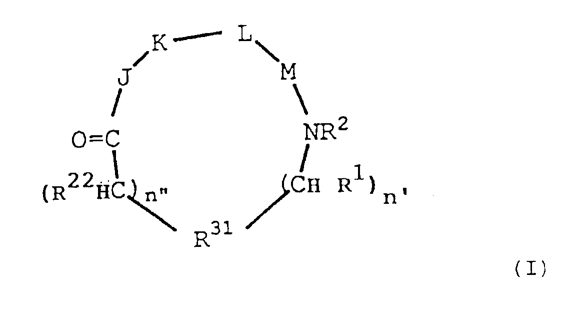 oder eines pharmazeutisch annehmbaren Salzes oder einer pharmazeutisch annehmbaren Medikamentenvorstufe davon, wobei
R³¹ ausgewählt ist aus 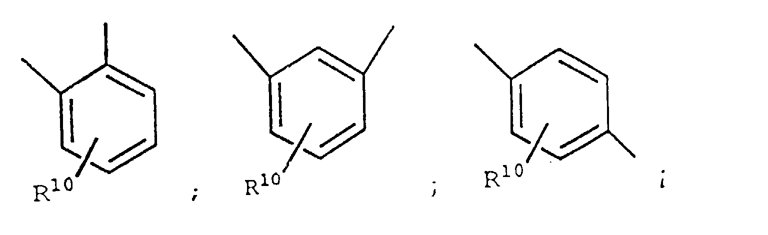 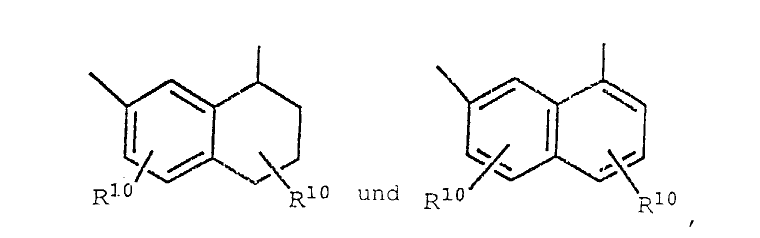 wobei R³¹ unabhängig mit 0-2 R¹⁰ substituiert sein kann;
n" 0 oder 1 ist;
n' 0 oder 1 ist;
R¹ und R²² unabhängig aus H, C₁-C₄-Alkyl, Phenyl, Benzyl, Phenyl-(C₂-C₄)-alkyl, C₁-C₄-Alkoxy ausgewählt sind;
R² H oder C₁-C₈-Alkyl ist;
R¹⁰ H, C₁-C₈-Alkyl, Phenyl, Halogen oder C₁-C₄-alkoxy ist;
J β-Ala oder eine _{L}-Isomer- oder _{D}-Isomer-Amisäure der Struktur -N(R³)C(R⁴)(R⁵)C(=O)- ist, wobei
R³ H oder CH₃ ist;
R⁴ H oder C₁-C₃-Alkyl ist;
R⁵ H, C₁-C₈-Alkyl, C₃-C₆-Cycloalkyl, C₃-C₆-Cycloalkylmethyl, C₁-C₆-Cycloalkylethyl, Phenyl, Phenylmethyl, CH₂OH, CH₂SH, CH₂OCH₃, CH₂SCH₃, CH₂CH₂SCH₃, (CH₂)ₛNH₂, (CH₂)sNHC(=NH) (NH₂), (CH₂)ₛNHR¹⁶ ist, wobei s= 3-5;
R³ und R⁵ auch unter Bildung von -(CH₂)ₜ- (t = 2-4) oder -CH₂SC(CH₃)₂- zusammengefaßt sein können; oder
R⁴ und R⁵ unter Bildung von -(CH₂)ᵤ-, wobei u = 2-5, ebenfalls zusammengefaßt sein können;
R¹⁶ eine Aminschutzgruppe ist;
K eine _{L}-Isomer-Aminosäure der Struktur -N(R⁶)CH(R⁷)C(=O)- ist, wobei
R⁶ C₁-C₈-Alkyl ist;
R⁷ folgendes ist:
-(CH₂)ₚNHC(=NH) (NH₂), wobei p = 3-5; 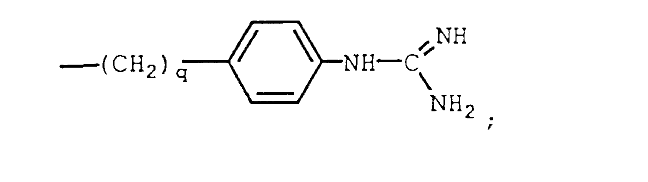 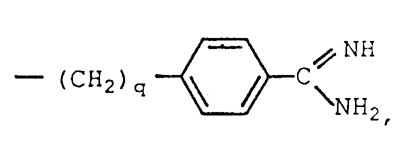
wobei q = 0, 1;
-(CH₂)ᵣX, wobei r = 4-6; 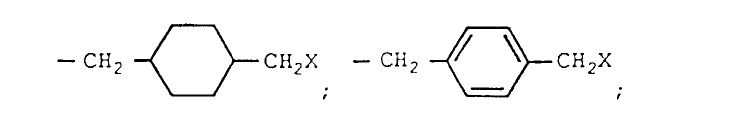 -(CH₂)ₘS(CH₂)₂X,wobei m = 1, 2;
-(C₃-C₇-Alkyl)-N-(C₁-C₆-alkyl); 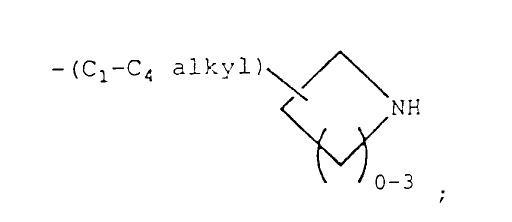 -(CH₂)ₘ-O-(C₁-C₄-Alkyl)-NH-(C₁-C₆-alkyl), wobei m = 1, 2;
-(CH₂)ₘ-S-(C₁-C₄-Alkyl)-NH-(C₁-C₆-alkyl), wobei m = 1, 2; und
X NH₂ oder -NHC(=NH) (NH₂) ist;
L -Y(CH₂)ᵥC(=O)- ist, wobei
Y NH, O oder S ist und v = 1, 2;
M eine _{L}-Isomer-Aminosäure der Struktur -NH-CH(CH₂SO₃H)C(=O)- ist;
wobei das Verfahren das Cyclisieren einer linearen Peptidvorstufe umfaßt.

11. Verfahren zur Herstellung eines Medikaments zur Behandlung von thromboembolischen Störungen, das die Verwendung einer Verbindung umfaßt, die gemäß einem der Ansprüche 1 bis 10 hergestellt wurde.

12. Verfahren zur Herstellung einer pharmazeutischen Zusammensetzung, die einen pharmazeutisch annehmbaren Träger und eine therapeutisch wirksame Menge einer Verbindung umfaßt, die gemäß einem der Ansprüche 1 bis 10 hergestellt wurde, wobei das Verfahren das Hinzufügen der Verbindung zu dem Träger umfaßt.

## Revendications (Revendications pour l'(les) Etat(s) contractant(s) suivant(s): AT, BE, CH, LI, DE, DK, FR, GB, IE, IT, LU, MC, NL, SE)

1. Composé de formule (I): 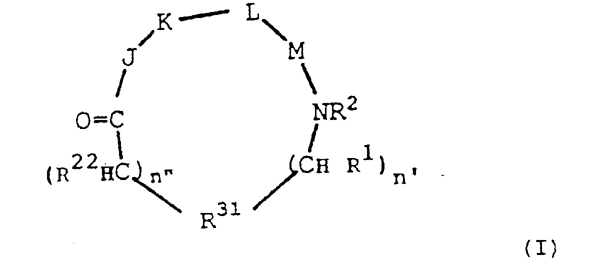 ou forme d'un sel ou d'un promédicament pharmaceutiquement acceptable de celui-ci dans lequel:
R³¹ est
(a) un anneau carbocyclique aromatique à 6 membres de formule 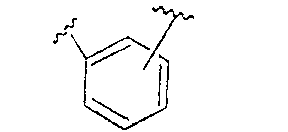 dans lequel ledit anneau carbocyclique est indépendamment substitué avec 0-2 R¹⁰, ou
(b) un anneau carboxylique bicyclique à 10 membres de formule 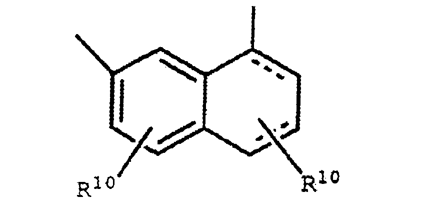 dans lequel la liaison en pointillé peut être une liaison simple ou double;
n" est 0 ou 1;
n' est 0 ou 1;
R¹ et R²² sont indépendamment choisis parmi les groupes suivants:
hydrogène,
alkyle C₁-C₈ substitué avec 0-2 R¹¹,
alcényle C₂-C₈ substitué avec 0-2 R¹¹,
alcynyle C₂-C₈ substitué avec 0-2 R¹¹,
cycloalkyle C₃-C₈ substitué avec 0-2 R¹¹,
alkyle bicyclique C₆-C₁₀ substitué avec 0-2 R¹¹,
aryle substitué avec 0-2 R¹²,
un système d'anneau hétérocyclique substitué avec 0-2 R¹², composé de 5-10 atomes, y compris 1-3 hétéroatomes de N, S ou O,
=O, F, Cl, Br, I, -CN, - CO₂R¹³, -C(=O)R¹³, -C(=O)NR¹³R¹⁴, -CHO, -CH₂OR¹³,-OC(=O)R¹³, -OC(=O)OR¹³,-OR¹³, -OC(=O)NR¹³R¹⁴, -NR¹⁴C(=O)R¹³, -NR¹⁴C(=O)OR¹³, -NR¹³C(=O)NR¹³R¹⁴, - NR¹⁴SO₂NR¹³R¹⁴, - NR¹⁴SO₂R^{13a}, -SO₃H, -SO₂R^{13a}, -SR¹³, -S(=O)R^{13a} , -SO₂NR¹³R¹⁴, -CH₂NR¹³R¹⁴, -NR¹³R¹⁴, -NHC(=NH)NHR¹³, -C(=NH)NHR¹³, NO₂;
R¹¹ est choisi parmi un ou plusieurs des groupes suivants:
=O, F, Cl, Br, I, - CF₃, -CN, CO₂R¹³, -C(=O)R¹³,-C(=O)NR¹³R¹⁴, -CH₂OR¹³, -OC(=O)R¹³, -OC(=O)OR¹³, -OR¹³,-OC(=O)NR¹³R¹⁴, -NR¹⁴C(=O)R¹³, -NR¹⁴C(=O)OR¹³, -NR¹³C(=O)NR¹³R¹⁴, - NR¹⁴SO₂NR¹³R¹⁴, - NR¹⁴SO₂R^{13a}, - SO₃H, -SO₂R^{13a}, -SR¹³, -S(=O)R^{13a},
-SO₂NR¹³R¹⁴,-CH₂NR¹³R¹⁴ , -NR¹³R¹⁴, -NHC(=NH)NHR¹³, -C(=NH)NHR¹³, =NOR¹⁴, NO₂,
alkyle C₁-C₅ , alcényle C₂-C₄, cycloalkyle C₃-C₆, cycloalkylméthyle C₃-C₆ , alkoxyalkyle C₂-C₆, alkyle C₁-C₄ (substitué avec NR¹³R¹⁴, -CF₃, NO₂, -SO₂R¹³, ou -S(=O)R^{13a})
aryle substitué avec 0-2 R¹²,
un système d'anneau hétérocyclique substitué avec 0-2 R¹², composé de 5-10 atomes, y compris 1-3 hétéroatomes d'azote, d'oxygène ou de soufre;
R¹² est choisi parmi un ou plusieurs des groupes suivants:
phényle, benzyle, phénéthyle, phénoxyle, benzyloxyle, halogène, hydroxyle, nitro , cyano, alkyle C₁-C₅, cycloalkyle C₃-C₆, cycloalkylméthyle C₃-C₆, arylalkyle C₇-C₁₀, alkoxyle C₁-C₄, -CO₂R¹³, acide hydroxamique, hydrazide, oxime, acide borique, sulfonamide, formyle, cycloalkoxyle C₃-C₆, -OC(=O)R¹³, -C(=O)R¹³, -OC(=O)OR¹³, -OR¹³ , -CH₂OR¹³, -NR¹³R¹⁴, -OC(=O)NR¹³R¹⁴, NR¹⁴C(=O)R¹³, NR¹⁴C(=O)OR¹³, -NR¹³C(=O)NR¹³R¹⁴, -NR¹⁴SO₂NR¹³R¹⁴, - NR¹⁴SO₂R^{13a}, -SO₃H, -SO₂R^{13a}, -S(=O)R^{13a}, -SR¹³, -SO₂NR¹³R¹⁴, alkoxyalkyle C₂-C₆, hydroxyalkyle C₁-C₄, méthylènedioxyle, éthylènedioxyle,
R¹³ est H, un groupe alkyle C₁-C₇, aryle, -(C₁-C₆ alkyl)aryle ou alkoxyalkyle C₃-C₆;
R^{13a} est un groupe alkyle C₁-C₇, aryle, -(C₁-C₆ alkyl)aryle ou alkoxyalkyle C₃-C₆ ;
R¹⁴ est OH, H, un groupe alkyle C₁-C₄, ou benzyle;
R² est H ou un groupe alkyle C₁-C₈;
R¹⁰ est choisi parmi un ou plusieurs des groupes suivants:
phényle, benzyle, phénéthyle, phénoxyle, benzyloxyle, halogène, hydroxyle, nitro , cyano , alkyle C₁-C₅, cycloalkyle C₃-C₆, cycloalkylméthyle C₃-C₆, arylalkyle C₇-C₁₀, alkoxyle C₁-C₄, -CO₂R¹³, -C(=O)NHOR¹³,-C(=O)NHNR¹³R¹⁴,oxime, acide borique, cycloalkoxyle C₃-C₆, OC(=O)R¹³, -C(=O)R¹³, -OC(=O)OR¹³, -OR¹³, -CH₂OR¹³, -NR¹³R¹⁴ , -OC(=O)NR¹³R¹⁴, -NR¹⁴C(=O)R¹³, - NR¹⁴C(=O)OR¹³, - NR¹³C(=O)NR¹³R¹⁴, -NR¹⁴SO₂NR¹³R¹⁴, -NR¹⁴SO₂R^{13a}, -SO₃H, -SO₂R^{13a},-S(=O)R^{13a},-SR¹³, -SO₂NR¹³R¹⁴, alkoxyalkyle C₂-C₆, hydroxyalkyle C₁-C₄, méthylènedioxyle, éthylènedioxyle, halogénoalkyle C₁-C₄, halogénoalkoxyle C₁-C₄, alkoxycarbonyle C₁-C₄, alkylcarbonyloxyle C₁-C₄, alkylcarbonyle C₁-C₄, aminoalkylcarbonyle C₁-C₄, -OCH₂CO₂H, 2-(1-morpholino)éthoxyle, alkyle C₁-C₄ (substitué avec -NR¹³R¹⁴, -CF₃, NO₂ ou S(=O)R^{13a}) ;
J est un acide aminé β-Ala ou un _{L}-isomère ou un _{D}-isomère de structure N(R³)C(R⁴)(R⁵)C(=O)- , dans lequel :
R³ est H ou CH₃ ;
R⁴ est H ou un groupe alkyle C₁-C₃;
R⁵ est H, un groupe alkyle C₁-C₈ , cycloalkyle C₃-C₆, cycloalkylméthyle C₃-C₆, cycloalkyléthyle C₁-C₆, phényle, phénylméthyle, CH₂OH, CH₂SH, CH₂OCH₃, CH₂SCH₃, CH₂CH₂SCH₃, (CH₂)_{S}NH₂, (CH₂)_{S}NHC(=NH)(NH₂), (CH₂)_{S}NHR¹⁶, où s = 3-5;
R³ et R⁵ peuvent également être pris ensemble pour former -(CH₂)ₜ-(t = 2-4) ou -CH₂SC(CH₃)₂-; ou
R⁴ et R⁵ peuvent également être pris ensemble pour former -(CH₂)ᵤ-, où u = 2-5;
R¹⁶ est un groupe protecteur de l'amine;
K est un acide aminé _{D}-isomère ou _{L}-isomère de structure -N(R⁶)CH(R⁷)C(=O)-, dans lequel:
R⁶ est un groupe alkyle C₁-C₈;
R⁷ est choisi parmi:
-(C₁-C₇ alkyl)X; 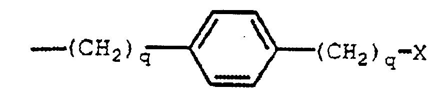 où q est indépendamment 0, 1; 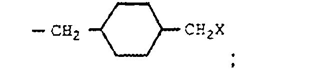 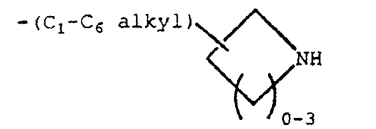 -(CH₂)ₘO-(C₁-C₄ alkyl)-X, où m = 1, 2;
-(CH₂)ₘS-(C₁-C₄ alkyl)-X, où m = 1, 2; et
X est choisi parmi: 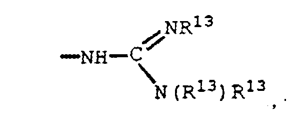 -N(R¹³)R¹³, -C(=NH)(NH₂), -SC(NH)-NH₂, dans lequel R¹³ est comme défini ci-dessus;
L est -Y(CH₂)ᵥC(=O)-, dans lequel :
Y est NH, N(C₁-C₃ alkyl), O ou S; et v = 1, 2;
M est un acide aminé _{D}-isomère ou _{L}-isomère de structure -NR¹⁷-CH(R⁸)C(=O)-, dans lequel:
R¹⁷ est H, un groupe alkyle C₁-C₃;
R⁸ est -CH₂CO₂R¹³, -CH₂SO₃R^{13a}, -CH(CH₃)CO₂R¹³, -SO₂NR¹³R¹⁴, -CH₂-acide borique ou -CH₂-tétrazole.

2. Composé de la revendication 1, dans lequel:
R³¹ est choisi parmi: 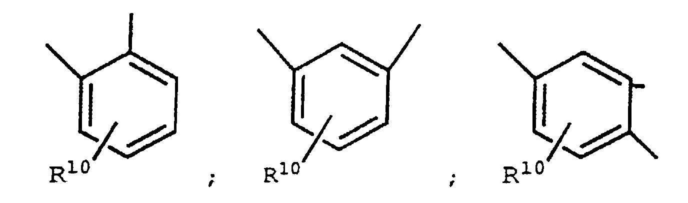 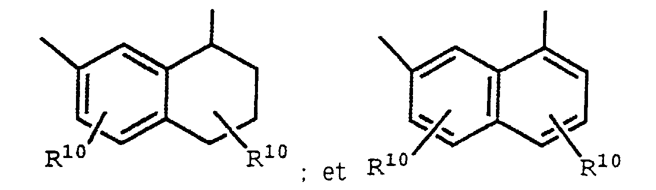 dans lequel R³¹ peut être substitué indépendamment avec 0-2 R¹⁰;
R¹ et R²² sont indépendamment choisis parmi H, un groupe alkyle C₁-C₄, phényle, benzyle, phényl-(C₂-C₄)alkyle, alkoxyle C₁-C₄;
R¹⁰ est H, un groupe alkyle C₁-C₈, phényle, halogène ou alkoxyle C₁-C₄;
K est un acide aminé _{L}-isomère de structure -N(R⁶)CH(R⁷)C(=O)-, dans lequel:
R⁶ est un groupe alkyle C₁-C₈;
R⁷ est -(CH₂)ₚNHC(=NH)(NH₂), où p = 3-5; 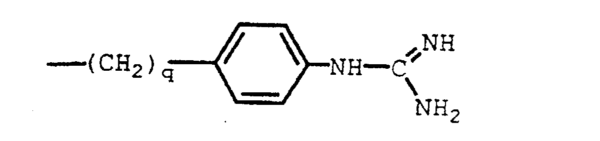 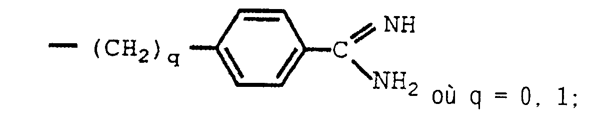 -(CH₂)ᵣX, où r = 4-6; 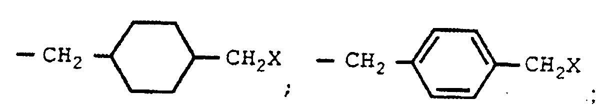 -(CH₂)ₘS(CH₂)₂X, où m = 1, 2;
-(C₃-C₇ alkyl)-N-(C₃-C₆ alkyl) 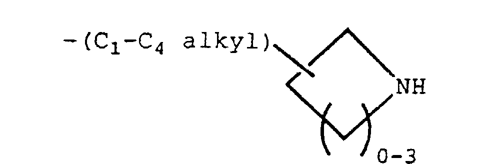 -(CH₂)ₘ-O-(C₁-C₄ alkyl)-NH-(C₁-C₆ alkyl), où m = 1, 2;
-(CH₂)ₘ-S-(C₁-C₄alkyl)-NH-(C₁-C₆ alkyl), où m = 1, 2; et
X est -NH₂ ou -NHC(=NH)(NH₂); ou
L est Y(CH₂)ᵥC(=O)- , dans lequel :
Y est NH, O, ou S: et v = 1, 2;
M est un acide aminé _{L}-isomère de structure -NH-CH(R⁸)C(=O)-, dans lequel :
R⁸ est -CH₂CO₂H ou -CH(CH₃)CO₂H.

3. Composé de la revendication 2 de formule (II): 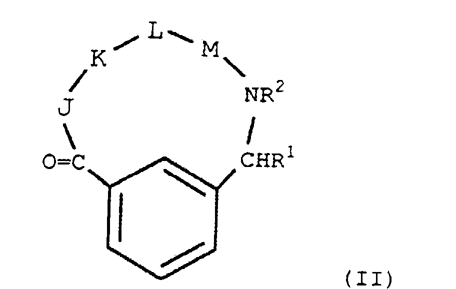 dans lequel:
R¹ est H, un groupe alkyle C₁-C₄, phényle, benzyle ou phényl-(C₂-C₄)alkyle;
R² est H ou un groupe méthyle;
J est un acide aminé β-Ala ou un _{L}-isomère ou un _{D}-isomère de structure -N(R₃)C(R₄)(R₅)C(=O)-, dans lequel:
R³ est H ou CH₃
R⁴ est H ou un groupe alkyle C₁-C₃;
R⁵ est H, un groupe alkyle C₁-C₈, cycloalkyle C₃-C₆, cycloalkylméthyle C₃-C₆, cycloalkyléthyle C₃-C₆, phényle, phénylméthyle, CH₂OH, CH₂SH, CH₂OCH₃, CH₂SCH₃, CH₂CH₂SCH₃, (CH₂)ₛNH₂ ou (CH₂)ₛNHC(=NH)(NH₂), où s = 3-5; ou
R³ et R⁵ peuvent être pris ensemble pour former -CH₂CH₂CH₂-; ou
R⁴ et R⁵ peuvent être pris ensemble pour former -(CH₂)ᵤ-, où u = 2-5;
K est un acide aminé _{L}-isomère de structure -N(R⁶)CH(R⁷)C(=O)-, dans lequel:
R⁶ est un groupe alkyle C₁-C₈;
R⁷ est -(CH₂)ₚNHC(=NH)(NH₂), où p = 3-5; 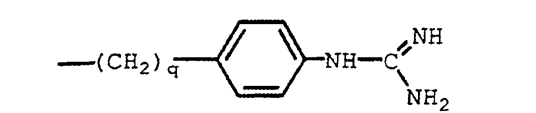 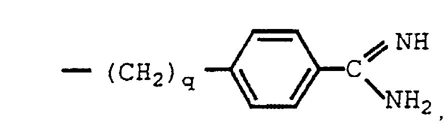 où q = 0, 1;
-(CH₂)ᵣX, où r = 4-6; 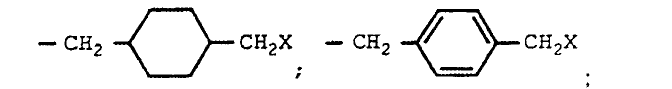 -(CH₂)ₘS(CH₂)₂X, où m = 1,2;
-(CH₂)ₘ-O-(C₁-C₄ alkyl)-NH-(C₁-C₆ alkyl), où m = 1, 2;
-(CH₂)ₘ-S-(C₁-C₄ alkyl)-NH-(C₁-C₆ alkyl), où m =1, 2; et
-(C₃-C₇ alkyl)-N-(C₁-C₆ alkyl) 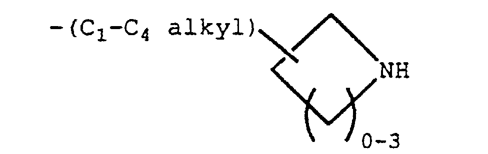
X est -NH₂ ou -NHC(=NH)(NH₂), à condition que X ne soit pas NH₂, lorsque r = 4.

4. Composé de la revendication 3 dans lequel
J est un acide aminé β-Ala ou un _{L}-isomère ou un _{D}-isomère de structure -N(R³)C(R⁴)(R⁵)C(=O)-, dans lequel:
R³ est H ou CH₃;
R⁴ est H;
R⁵ est H, un groupe alkyle C₁-C₈, cycloalkyle C₃-C₆, cycloalkylméthyle C₃-C₆, cycloalkyléthyle C₁-C₆, phényle, phénylméthyle, CH₂OH, CH₂SH, CH₂OCH₃, CH₂SCH₃, CH₂CH₂SCH₃, (CH₂)ₛNH₂ ou (CH₂)ₛNHC(=NH)(NH₂), où s = 3, 4, 5; ou
R³ et R⁵ peuvent être pris ensemble pour former -CH₂CH₂CH₂-;
K est un acide aminé L-isomère de structure -N(CH₃)CH(R⁷)C(=O), dans lequel:
R⁷ est -(CH₂)ₚNHC(=NH)(NH₂), où p = 3-4 ; 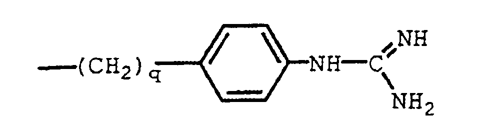 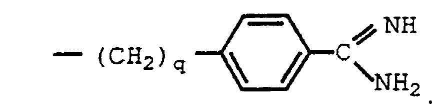 où q = 0, 1; -(CH₂)ᵣX, où r = 4-6: 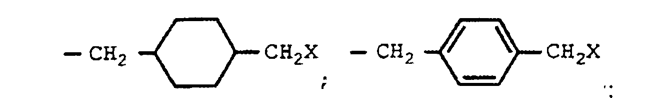 (CH₂)ₘS(CH₂)₂X, où m = 1, 2; -(C₄-C₇ alkyl)-N-(C₁-C₆ alkyl) 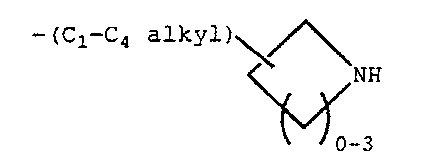 -(CH₂)ₘ-O-(C₁-C₄ alkyl)-NH-(C₁-C₆ alkyl), où m = 1,2; -(CH₂)ₘ-S-(C₁-C₄ alkyl)-NH-(C₁-C₆ alkyl), où m = 1,2; et
X est -NH₂ ou -NHC(=NH)(NH₂), à condition que X ne soit pas -NH₂, lorsque r = 4;
L est -YCH₂C(=O)-, dans lequel:
Y est NH ou O;
M est un acide aminé L-isomère de structure -NH-CH(CH₂CO₂H)C(=O)-.

5. Composé des revendications 3 et 4 dans lequel :
R¹ est H;
R² est H;
J est un acide aminé β-Ala ou un _{L}-isomère ou un _{D}-isomère de formule N(R³)CH(R⁵)C(=O)-, dans lequel :
R³ est H et R⁵ est H, CH₃, CH₂CH₃, CH(CH₃)₂, CH(CH₃)CH₂CH₃, CH₂CH₂CH₃, CH₂CH₂CH₂CH₃, CH₂CH₂SCH₃, CH₂CH(CH₃)₂, (CH₂)₄NH₂; ou
R³ est CH₃ et R⁵ est H; ou
R³ et R⁵ peuvent être pris ensemble pour former -CH₂CH₂CH₂-
K est un acide aminé L-isomère de formule -N(CH₃)CH(R⁷)C(=O)-, dans lequel :
R⁷ est -(CH₂)₃NHC(=NH)(NH₂);
L est NHCH₂C(=O)- ; et
M est un acide aminé L-isomère de formule -NHCH(CH₂COOH)C(=O).

6. Composé de la revendication 2 de formule (III) 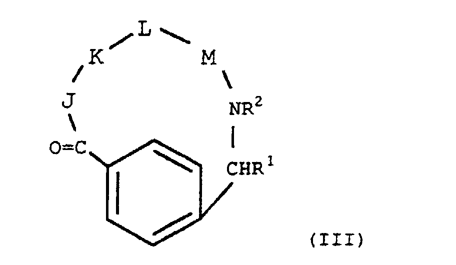 dans lequel:
R¹ est H, un groupe alkyle C₁-C₄, phényle, benzyle ou phényl-(C₂-C₄)alkyle;
R² est H ou un groupe méthyle;
J est un acide aminé β-Ala ou un L-isomère ou un o-isomère de structure -N(R³)C(R⁴)(R⁵)C(=O)-, dans lequel:
R³ est H ou CH₃ ;
R⁴ est H ou un groupe alkyle C₁-C₃;
R⁵ est H, un groupe alkyle C₁-C₈, cycloalkyle C₃-C₆, cycloalkylméthyle C₃-C₆, cycloalkyléthyle C₁-C₆, phényle, phénylméthyle, CH₂OH, CH₂SH, CH₂OCH₃, CH₂SCH₃, CH₂CH₂SCH₃, (CH₂)_{S}NH₂ ou (CH₂)_{S}NHC(=NH)(NH₂),
où s = 3-5; ou
R³ et R⁵ peuvent être pris ensemble pour former -CH₂CH₂CH₂-; ou
R⁴ et R⁵ peuvent être pris ensemble pour former -(CH₂)ᵤ-, où u = 2-5;
K est un acide aminé L-isomère de structure -N(R⁶)CH(R⁷)C(=O)-, dans lequel:
R⁶ est un groupe alkyle C₁-C₈;
R⁷ est -(CH₂)ₚNHC(=NH)(NH₂), où p = 3-4; 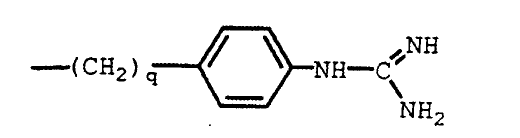 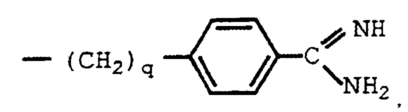 où q = 0,1;
-(CH₂)ᵣX, où r = 4-6; 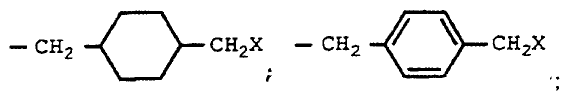 -(CH₂)ₘS(CH₂)₂X, où m = 1,2;
-(C₃-C₇ alkyl)-N-(C₁-C₆ alkyl) 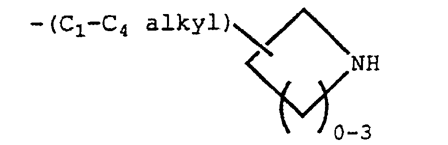 -(CH₂)ₘ-O-(C₁-C₄ alkyl)-NH-(C₁-C₆ alkyl), où m = 1,2;
-(CH₂)ₘ-S-(C₁-C₄ alkyl)-NH-(C₁-C₆ alkyl), où m = 1,2; et
X est -NH₂ ou -NHC(=NH)(NH₂), à condition que X ne soit pas NH₂, lorsque r = 4.

7. Composé de la revendication 1, qui est choisi parmi: le composé de formule (II): 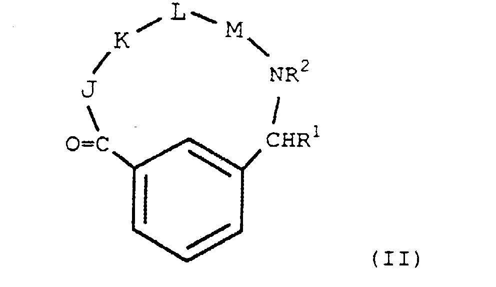 dans lequel R¹ et R² sont H; J est _{D}-Val; K est NMeArg; L est Gly; et M est Asp;
le composé de formule (II) dans lequel R¹ et R² sont H; J est l'acide _{D}-2-aminobutyrique; K est NMeArg: L est Gly; et M est Asp;
le composé de formule (II) dans lequel R¹ et R² sont H; J est _{D}-Leu; K est NMeArg; L est Gly; et M est Asp;
le composé de formule (II) dans lequel R¹ et R² sont H; J est _{D}-Ala; K est NMeArg; L est Gly; M est Asp;
le composé de formule (II) dans lequel R¹ et R² sont H; J est Gly; K est NMeArg; L est Gly; et M est Asp;
le composé de formule (II) dans lequel R¹ et R² sont H; J est _{D}-Pro; K est NMeArg; L est Gly; et M est Asp;
le composé de formule (II) dans lequel R¹ et R² sont H; J est _{D}-Lys; K est NMeArg; L est Gly; et M est Asp;
le composé de formule (II) dans lequel R¹ et R² sont H; J est β-Ala; K est NMeArg; L est Gly; et M est Asp;
le composé de formule (II) dans lequel R¹ et R² sont H; J est _{D}-Val; K est NMeArg; L est β-Ala; et M est Asp;
le composé de formule (II) dans lequel R¹ et R² sont H; J est Pro; K est NMeArg; L est Gly; et M est Asp;
le composé de formule (II) dans lequel R¹ et R² sont H; J est NMeGly; K est NMeArg; L est Gly; et M est Asp;
le composé de formule (II) dans lequel R¹ est un groupe méthyle (isomère 1); R² est H; J est _{D}-Val; K est NMeArg; L est Gly; et M est Asp;
le composé de formule (II) dans lequel R¹ est un groupe méthyle (isomère 2); R² est H; J est _{D}-Val; K est NMeArg; L est Gly; et M est Asp;
le composé de formule (II) dans lequel R¹ est un groupe phényle (isomère 1); R² est H; J est _{D}-Val; K est NMeArg; L est Gly; et M est Asp;
le composé de formule (II) dans lequel R¹ est un groupe phényle (isomère 2); R² est H; J est _{D}-Val; K est NMeArg; L est Gly; et M est Asp;
le composé de formule (II) dans lequel R¹ et R² sont H; J est D-Val; K est D-NMeArg; L est Gly; et M est Asp;
le composé de formule (II) dans lequel R¹ et R² sont H; J est D-Nle; K est NMeArg; L est Gly; et M est Asp;
le composé de formule (II) dans lequel R¹ et R² sont H; J est D-Phg; K est NMeArg; L est Gly; et M est Asp;
le composé de formule (II) dans lequel R¹ et R² sont H; J est D-Ile; K est NMeArg; L est Gly; et M est Asp;
le composé de formule (II) dans lequel R¹ et R² sont H; J est D-Phe; K est NMeArg; L est Gly; et M est Asp;
le composé de formule (II) dans lequel R¹ et R² sont H; J est NMeGly; K est NMeArg; L est Gly; et M est Asp;
le composé de formule (II) dans lequel R¹ et R² sont H; J est D-Nle; K est NMeArg; L est Gly; et M est Asp;
le composé de formule (II) dans lequel R¹ et R² sont H; J est D-Tyr; K est NMeArg; L est Gly; et M est Asp;
le composé de formule (II) dans lequel R¹ et R² sont H; J est D-Val; K est NMeAmf: L est Gly; et M est Asp;
le composé de formule (II) dans lequel R¹ et R² sont H; J est D-Val; K est NMeArg: L est Ala; et M est Asp;
le composé de formule (II) dans lequel R¹ et R² sont H; J est D-Val; K est NMeArg; L est Gly; et M est -MeAsp;
le composé de formule (II) dans lequel R¹ et R² sont H; J est D-Val: K est NMeArg; L est Gly; et M est β-MeAsp;
le composé de formule (II) dans lequel R¹ et R² sont H; J est D-Val; K est NMeArg; L est Gly; et M est NMeAsp;
le composé de formule (II) dans lequel R¹ et R² sont H; J est D-Val; K est NMeArg; L est Gly; et M est D-Asp;
le composé de formule (II) dans lequel R¹ est H; R² est CH₃; J est D-Val; K est NMeArg; L est Gly; et M est Asp;
le composé de formule (II) dans lequel R¹ et R² sont H; J est D-Abu; K est di-NMeOrn; L est Gly; et M est Asp;
le composé de formule (III): 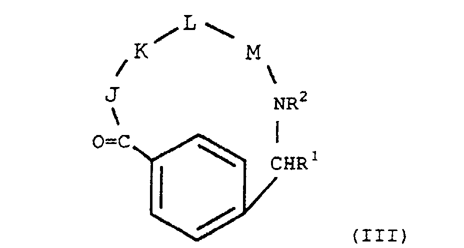 dans lequel R¹ et R² sont H; J est D-Val; K est NMeArg; L est Gly; et M est Asp;
le composé de formule (V): 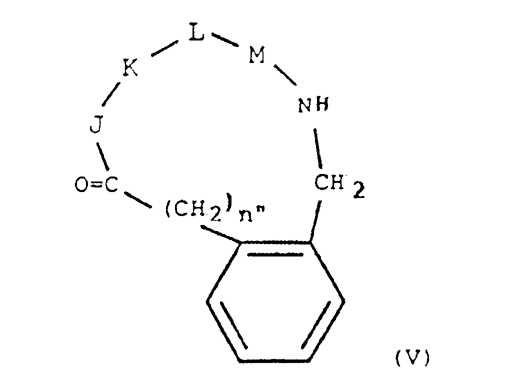 dans lequel n"=1; J est D-Val; K est NMeArg; L est Gly; et M est Asp;
le composé de formule (V) dans lequel n"=O: J est D-Val; K est NMeArg; L est Gly; et M est Asp;
le composé de formule (VI): 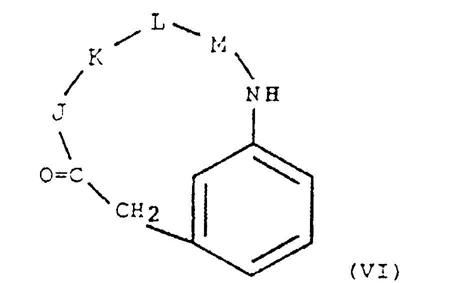 dans lequel J est D-Val; K est NMeArg; L est Gly; et M est Asp;
le composé de formule (VII): 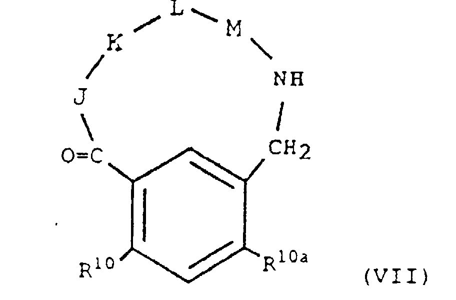 dans lequel R¹⁰ est H; R^{10a} est Cl; J est D-Val: K est NMeArg; L est Gly; et M est Asp;
le composé de formule (VII) dans lequel R¹⁰ est H; R^{10a} est I; J est D-Val; K est NMeArg; L est Gly; et M est Asp;
le composé de formule (VII) dans lequel R¹⁰ est H; R^{10a} est I; J est D-Abu; K est NMeArg; L est Gly; et M est Asp;
le composé de formule (VII) dans lequel R¹⁰ est H; R^{10a} est OMe: J est D-Val; K est NMeArg; L est Gly; et M est Asp;
le composé de formule (VII) dans lequel R¹⁰ est H; R^{10a} est Me; J est D-Val: K est NMeArg; L est Gly; et M est Asp;
le composé de formule (VII) dans lequel R^{10a} est H; R¹⁰ est Cl; J est D-Val: K est NMeArg; L est Gly; et M est Asp;
le composé de formule (VII) dans lequel R^{10a} est H; R¹⁰ est MeO; J est D-Val; K est NMeArg; L est Gly; et M est Asp:
le composé de formule (VII) dans lequel R^{10a} est H; R¹⁰ est Me; J est D-Val; K est NMeArg; L est Gly; et M est Asp;
le composé de formule (VIII): 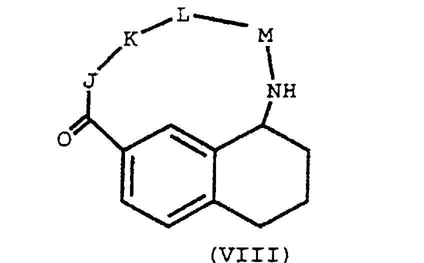 dans lequel J est D-Val; K est NMeArg; L est Gly; et M est Asp;
le composé de formule (IX) : 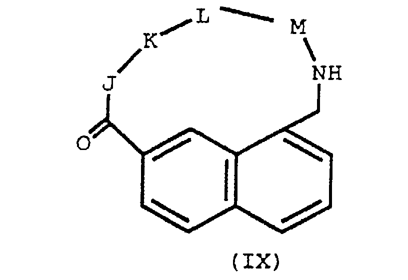 dans lequel J est D-Val; K est NMeArg; L est Gly; et M est Asp.

8. Composé de la revendication 7 qui est choisi parmi:
le composé de formule (Il) dans lequel R¹ et R² sont H; J est D-Val; K est NMeArg; L est Gly; et M est Asp;
le composé de formule (Il) dans lequel R¹ et R² sont H; J est un acide D-2-aminobutyrique; K est NMeArg; L est Gly; et M est Asp;
le composé de formule (Il) dans lequel R¹ et R² sont H; J est D-Leu; K est NMeArg; L est Gly; et M est Asp;
le composé de formule (Il) dans lequel R¹ et R² sont H; J est D-Ala; K est NMeArg; L est Gly; et M est Asp;
le composé de formule (Il) dans lequel R¹ et R² sont H; J est Gly; K est NMeArg; L est Gly; et M est Asp;
le composé de formule. (II) dans lequel R¹ et R² sont H; J est D-Pro; K est NMeArg; L est Gly; et M est Asp;
le composé de formule (Il) dans lequel R¹ et R² sont H; J est D-Lys; K est NMeArg; L est Gly; et M est Asp;
le composé de formule (il) dans lequel R¹ et R² sont H; J est β-Ala; K est NMeArg; L est Gly; et M est Asp;
le composé de formule (il) dans lequel R¹ et R² sont H; J est NMeGly; K est NMeArg; L est Gly, et M est Asp;
le composé de formule (Il) dans lequel R¹ est un groupe méthyle (isomère 1); R² est H; J est D-Val; K est NMeArg; L est Gly; et M est Asp;
le composé de formule (il) dans lequel R¹ est un groupe méthyle (isomère 2); R² est H; J est D-Val; K est NMeArg; L est Gly; et M est Asp;
le composé de formule (il) dans lequel R¹ est un groupe phényle (isomère 1); R² est H; J est D-Val; K est NMeArg; L est Gly; et M est Asp;
le composé de formule (il) dans lequel R¹ et R² sont H; J est D-Val; K est D-NMeArg; L est Gly; et M est Asp;
le composé de formule (il) dans lequel R¹ et R² sont H; J est D-Nle; K est NMeArg; L est Gly; et M est Asp;
le composé de formule (il) dans lequel R¹ et R² sont H; J est D-Phg; K est NMeArg; L est Gly; et M est Asp;
le composé de formule (II) dans lequel R¹ et R² sont H; J est D-Phe; K est NMeArg; L est Gly; et M est Asp;
le composé de formule (il) dans lequel R¹ et R² sont H; J est D-Ile; K est NMeArg; L est Gly; et M est Asp;
le composé de formule (III) dans lequel R¹ et R² sont H; J est D-Val; K est NMeArg; L est Gly; et M est Asp;
le composé de formule (V) dans lequel n" = 1; J est D-Val; K est NMeArg; L est Gly et M est Asp;
le composé de formule (V) dans lequel n" = 0; J est D-Val; K est NMeArg; L est Gly et M est Asp;
le composé de formule (VI) dans lequel J est D-Val; K est NMeArg; L est Gly; et M est Asp;
le composé de formule (VII) dans lequel R¹⁰ est H; R^{10a} est Cl; J est D-Val; K est NMeArg; L est Gly; et M est Asp;
le composé de formule (VII) dans lequel R¹⁰ est H; R^{10a} est I; J est D-Val; K est NMeArg; L est Gly; et M est Asp;
le composé de formule (VII) dans lequel R¹⁰ est H; R^{10a} est I; J est D-Abu; K est NMeArg; L est Gly; et M est Asp;
le composé de formule (VII) dans lequel R¹⁰ est H; R^{10a} est Me; J est D-Val; K est NMeArg; L est Gly; et M est Asp;
le composé de formule (VII) dans lequel R^{10a} est H; R¹⁰ est Cl; J est D-Val; K est NMeArg; L est Gly; et M est Asp;
le composé de formule (VII) dans lequel R^{10a} est H; R¹⁰ est MeO; J est D-Val; K est NMeArg; L est Gly; et M est Asp;
le composé de formule (VII) dans lequel R^{10a} est H; R¹⁰ est Me; J est D-Val; K est NMeArg; L est Gly; et M est Asp;
le composé de formule (Il) dans lequel R¹ et R² sont H; J est D-Tyr; K est NMeArg; L est Gly; et M est Asp;
le composé de formule (Il) dans lequel R¹ et R² sont H; J est D-Val; K est NMeAmf; L est Gly; et M est Asp;
le composé de formule (Il) dans lequel R¹ et R² sont H; J est D-Val; K est NMeArg; L est Gly; et M est bMeAsp;
le composé de formule (Il) dans lequel R¹ est H; R² est CH₃; J est D-Val; K est NMeArg; L est Gly; et M est Asp;
le composé de formule (III) dans lequel R¹ et R² sont H; J est D-Val; K est NMeArg; L est Gly; et M est Asp;
le composé de formule (VIII) dans lequel J est D-Val; K est NMeArg; L est Gly; et M est Asp.

9. Composé de la revendication 8 choisi parmi:
le composé de formule (Il) dans lequel R¹ et R² sont H; J est D-Val; K est NMeArg; L est Gly; et M est Asp;
le composé de formule (il) dans lequel R¹ et R² sont H; J est l'acide D-2-aminobutyrique; K est NMeArg; L est Gly; et M est Asp;
le composé de formule (Il) dans lequel R¹ et R² sont H; J est D-Leu; K est NMeArg; L est Gly; et M est Asp;
le composé de formule (Il) dans lequel R¹ et R² sont H; J est D-Ala; K est NMeArg; L est Gly; et M est Asp;
le composé de formule (Il) dans lequel R¹ et R² sont H; J est D-Pro; K est NMeArg; L est Gly; et M est Asp;
le composé de formule (Il) dans lequel R¹ et R² sont H; J est D-Lys; K est NMeArg; L est Gly; et M est Asp;
le composé de formule (Il) dans lequel R¹ et R² sont H; J est D-Nle; K est NMeArg; L est Gly; et M est Asp;
le composé de formule (Il) dans lequel R¹ et R² sont H; J est D-Phe; K est NMeArg; L est Gly; et M est Asp;
le composé de formule (Il) dans lequel R¹ et R² sont H; J est D-Ile; K est NMeArg; L est Gly; et M est Asp;
le composé de formule (V) dans lequel n" = 1; J est D-Val; K est NMeArg; L est Gly; et M est Asp;
le composé de formule (VI) dans lequel J est D-Val; K est NMeArg; L est Gly; et M est Asp;
le composé de formule (VII) dans lequel R¹⁰ est H; R^{10a} est Cl; J est D-Val; K est NMeArg; L est Gly; et M est Asp;
le composé de formule (VII) dans lequel R¹⁰ est H; R^{10a} est I; J est D-Val; K est NMeArg; L est Gly; et M est Asp;
le composé de formule (VII) dans lequel R¹⁰ est H; R^{10a} est Me; J est D-Val; K est NMeArg; L est Gly; et M est Asp;
le composé de formule (VII) dans lequel R^{10a} est H; R¹⁰ est Cl; J est D-Val; K est NMeArg; L est Gly; et M est Asp;
le composé de formule (VII) dans lequel R^{10a} est H; R¹⁰ est MeO; J est D-Val; K est NMeArg; L est Gly; et M est Asp;
le composé de formule (VII) dans lequel R^{10a} est H; R¹⁰ est Me; J est D-Val; K est NMeArg; L est Gly; et M est Asp;
le composé de formule (Il) dans lequel R¹ et R² sont H; J est D-Val; K est NMeArg; L est Gly; et M est bMeAsp.

10. Composé de formule (I): 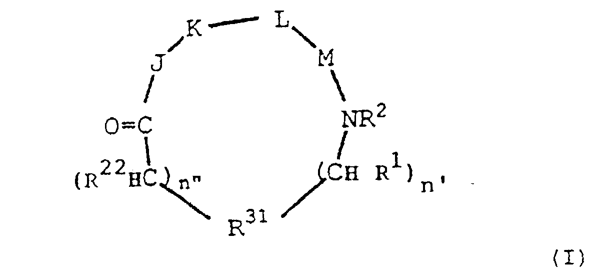 ou forme d'un sel ou d'un promédicament pharmaceutiquement acceptable de celui-ci dans lequel:
R³¹ est choisi parmi: 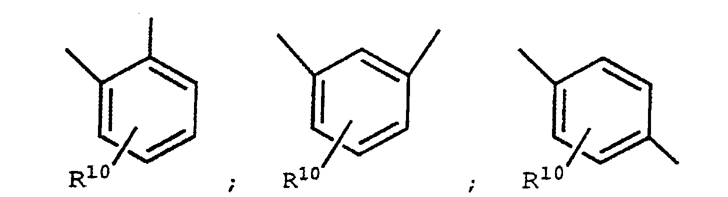 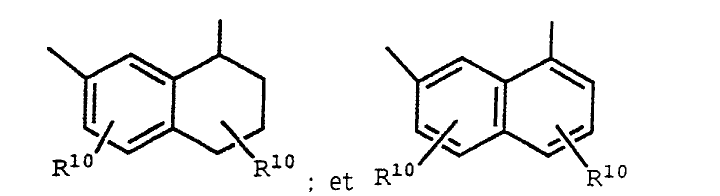 dans lequel R³¹ peut être indépendamment substitué avec 0-2 R¹⁰;
n" est 0 ou 1;
n' est 0 ou 1;
R¹ et R²² sont indépendamment choisis parmi H, un groupe alkyle C₁-C₄, phényle, benzyle, phényl-(C₂-C₄)alkyle, alkoxyle C₁-C₄;
R² est H ou un groupe alkyle C₁-C₈;
R¹⁰ est H, un groupe alkyle C₁-C₈, phényle, halogène, ou alkoxyle C₁-C₄;
J est un acide aminé β-Ala ou un L-isomère ou un D-isomère de structure -N(R³)C(R⁴)(R⁵)C(=O)-, dans lequel:
R³ est H ou CH₃
R⁴ est H ou un groupe alkyle C₁-C₃;
R⁵ est H, un groupe alkyle C₁-C₈, cycloalkyle C₃-C₆, cycloalkylméthyle C₃-C₆, cycloalkyléthyle C₁-C₆, phényle, phénylméthyle, CH₂OH, CH₂SH, CH₂OCH₃, CH₂SCH₃, CH₂CH₂SCH₃, (CH₂)ₛNH₂, (CH₂)ₛNHC(=NH)(NH₂), (CH₂)ₛNHR¹⁶, où s = 3-5;
R³ et R⁵ peuvent également être pris ensemble pour former -(CH₂)ₜ-(t = 2-4) ou -CH₂SC(CH₃)2-; ou
R⁴ et R⁵ peuvent également être pris ensemble pour former -(CH₂)ᵤ-, où u = 2-5;
R¹⁶ est un groupe protecteur de l'amine;
K est un acide aminé L-isomère de structure -N(R⁶)CH(R⁷)C(=0)-, dans lequel:
R⁶ est un groupe alkyle C₁-C₈;
R⁷ est -(CH₂)ₚNHC(=NH)(NH₂), où p = 3-5; 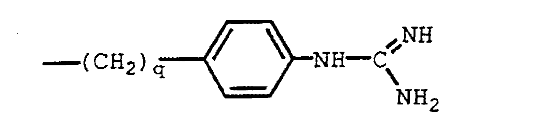 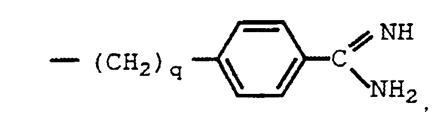 où q = 0, 1;
-(CH₂)ᵣX, où r = 4-6; 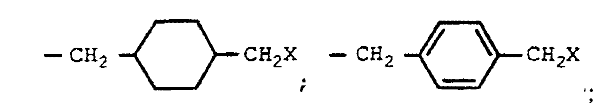 (CH₂)ₘS(CH₂)₂X, où m = 1,2;
-(C₃-C₇ alkyl)-N-(C₁-C₆ alkyl) 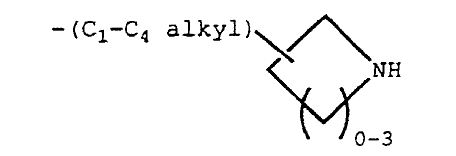 -(CH₂)ₘ-O-(C₁-C₄ alkyl)-NH-(C₁-C₆ alkyl), où m = 1, 2;
-(CH₂)ₘ-S-(C₁-C₄ alkyl)-NH-(C₁-C₆ alkyl), où m = 1, 2; et
X est -NH₂ ou -NHC(=NH)(NH₂);
L est Y(CH₂)ᵥC(=O)- , dans lequel :
Y est NH, O, ou S; et v = 1,2;
M est un acide aminé L-isomère de structure -NH-CH(CH₂S0₃H)C(=O)-.

11. Utilisation d'un composé suivant l'une quelconque des revendications 1-10 pour la préparation d'un médicament pour le traitement de désordres thromboemboliques.

12. Composition pharmaceutique comprenant un excipient pharmaceutiquement acceptable et une quantité thérapeutiquement efficace d'un composé suivant l'une quelconque des revendications 1-10.

## Revendications (Revendications pour l'(les) Etat(s) contractant(s) suivant(s): GR)

1. Composé de formule (I): 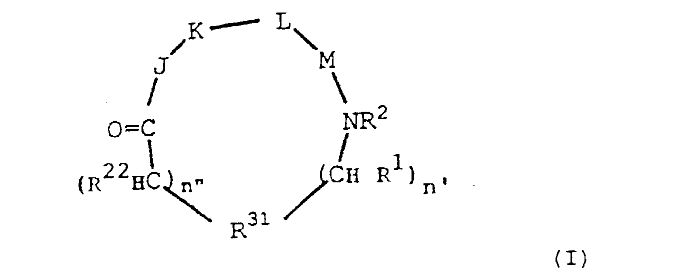 ou forme d'un sel ou d'un promédicament pharmaceutiquement acceptable de celui-ci dans lequel:
R³¹ est
(a) un anneau carbocyclique aromatique à 6 membres de formule 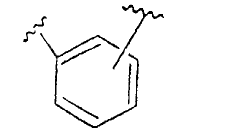 dans lequel ledit anneau carbocyclique est indépendamment substitué avec 0-2 R¹⁰, ou
(b) un anneau carboxylique bicyclique à 10 membres de formule 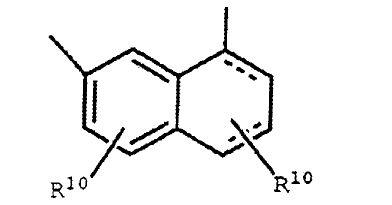 dans lequel la liaison en pointillé peut être une liaison simple ou double;
n" est 0 ou 1;
n' est 0 ou 1;
R¹ et R²² sont indépendamment choisis parmi les groupes suivants: hydrogène,
alkyle C₁-C₈ substitué avec 0-2 R¹¹,
alcényle C₂-C₈ substitué avec 0-2 R¹¹,
alcynyle C₂-C₈ substitué avec 0-2 R¹¹,
cycloalkyle C₃-C₈ substitué avec 0-2 R¹¹,
alkyle bicyclique C₆-C₁₀ substitué avec 0-2 R¹¹,
aryle substitué avec 0-2 R¹²,
un système d'anneau hétérocyclique substitué avec 0-2 R¹², composé de 5-10 atomes, y compris 1-3 hétéroatomes de N, S ou O,
=O, F, Cl, Br, I, -CN, CO₂R¹³, -C(=O)R¹³, -C(=O)NR¹³R¹⁴, -CHO, -CH₂OR¹³,-OC(=O)R¹³,-OC(=O)OR¹³, -OR¹³, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -SR¹³, -S(=O)R^{13a} , -SO₂NR¹³R¹⁴, -CH₂NR¹³R¹⁴, -NR¹³R¹⁴, -NHC(=NH)NHR¹³, -C(=NH)NHR¹³, NO₂;
R¹¹ est choisi parmi un ou plusieurs des groupes suivants:
=O, F, Cl, Br, I, -CF₃, -CN, -CO₂R¹³, -C(=O)R¹³, -C(=O)NR¹³R¹⁴, -CH₂OR¹³, -OC(=O)R¹³, -OC(=O)OR¹³, -OR¹³, -OC(=O)NR¹³R¹⁴, -NR¹⁴C(=O)R¹³, -NR¹⁴C(=O)OR¹³, NR¹³C(=O)NR¹³R¹⁴, -NR¹⁴SO₂NR¹³R¹⁴, NR¹⁴SO₂R^{13a}, -SO₃H, -SO₂R^{13a}, -SR¹³, -S(=O)R^{13a},
-SO₂NR¹³R¹⁴, -CH₂NR¹³R¹⁴, -NR¹³R¹⁴, -NHC(=NH)NHR¹³, -C(=NH)NHR¹³, =NOR¹⁴, NO₂,
alkyle C₁-C₅ , alcényle C₂-C₄, cycloalkyle C₃-C₆ , cycloalkylméthyle C₃-C₆ , alkoxyalkyle C₂-C₆, alkyle C₁-C₄ (substitué avec -NR¹³R¹⁴, -CF₃ , NO₂, -SO₂R¹³, ou -S(=O)R^{13a})
aryle substitué avec 0-2 R¹²,
un système d'anneau hétérocyclique substitué avec O-2 R¹², composé de 5-10 atomes, y compris 1-3 hétéroatomes d'azote, d'oxygène ou de soufre;
R¹² est choisi parmi un ou plusieurs des groupes suivants:
phényle, benzyle, phénéthyle, phénoxyle, benzyloxyle, halogène, hydroxyle, nitro, cyano, alkyle C₁-C₅, cycloalkyle C₃-C₆, cycloalkylméthyle C₃-C₆, arylalkyle C₇-C₁₀, alkoxyle C₁-C₄, -CO₂R¹³, acide hydroxamique, hydrazide, oxime, acide borique, sulfonamide, formyle, cycloalkoxyle C₃-C₆, -OC(=O)R¹³, -C(=O)R¹³, -OC(=O)OR¹³ , -OR¹³ , -CH₂OR¹³, -NR¹³R¹⁴, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -S(=O)R^{13a}, -SR¹³, -SO₂NR¹³R¹⁴, alkoxyalkyle C₂-C₆, hydroxyalkyle C₁-C₄, méthylènedioxyle, éthylènedioxyle,
R¹³ est H, un groupe alkyle C₁-C₇, aryle, -(C₁-C₆ alkyl)aryle ou alkoxyalkyle C₃-C₆ ;
R^{13a} est un groupe alkyle C₁-C₇, aryle, -(C₁-C₆ alkyl)aryle ou alkoxyalkyle C₃-C₆ ;
R¹⁴ est OH, H, un groupe alkyle C₁-C₄, ou benzyle;
R² est H ou un groupe alkyle C₁-C₈;
R¹⁰ est choisi parmi un ou plusieurs des groupes suivants:
phényle, benzyle, phénéthyle, phénoxyle, benzyloxyle, halogène, hydroxyle, nitro , cyano, alkyle C₁-C₅, cycloalkyle C₃-C₆, cycloalkylméthyle C₃-C₆, arylalkyle C₁-C₁₀, alkoxyle C₁-C₄, -CO₂R¹³, -C(=O)NHOR¹³,-C(=O)NHNR¹³R¹⁴,oxime, acide borique, cycloalkoxyle C₃-C₆, -OC(=O)R¹³, -C(=O)R¹³, -OC(=O)OR¹³, -OR¹³, -CH₂OR¹³, -NR¹³R¹⁴, -OC(=O)NR¹³R¹⁴, -NR¹⁴C(=O)R¹³, -NR¹⁴C(=O)OR¹³, - NR¹³C(=O)NR¹³R¹⁴, - NR¹⁴SO₂NR¹³R¹⁴, - NR¹⁴SO₂R^{13a}, -SO₃H, -SO₂R^{13a}, -S(=O)R^{13a}, -SR¹³, - SO₂NR¹³R¹⁴, alkoxyalkyle C₂-C₆, hydroxyalkyle C₁-C₄, méthylènedioxyle, éthylènedioxyle, halogénoalkyle C₁-C₄, halogénoalkoxyle C₁-C₄, alkoxycarbonyle C₁-C₄, alkylcarbonyloxyle C₁-C₄, alkylcarbonyle C₁-C₄, aminoalkylcarbonyle C₁-C₄, -OCH₂CO₂H, 2-(1-morpholino)éthoxyle, alkyle C₁-C₄ (substitué avec -NR¹³R¹⁴, -CF₃, NO₂ ou S(=O)R^{13a}) ;
J est un acide aminé β-Ala ou un L-isomère ou un D-isomère de structure -N(R³)C(R⁴)(R⁵)C(=O)-, dans lequel:
R³ est H ou CH₃;
R⁴ est H ou un groupe alkyle C₁-C₃;
R⁵ est H, un groupe alkyle C₁-C₈, cycloalkyle C₃-C₆, cycloalkylméthyle C₃-C₆, cycloalkyléthyle C₁-C₆, phényle, phénylméthyle, CH₂OH, CH₂SH, CH₂OCH₃, CH₂SCH₃, CH₂CH₂SCH₃, (CH₂)_{S}NH₂, (CH₂)_{S}NHC(=NH)(NH₂), (CH₂)_{S}NHR¹⁶, où s = 3-5;
R³ et R⁵ peuvent également être pris ensemble pour former -(CH₂)ₜ-(t = 2-4) ou -CH₂SC(CH₃)₂-; ou
R⁴ et R⁵ peuvent également être pris ensemble pour former -(CH₂)ᵤ-, où u = 2-5;
R¹⁶ est un groupe protecteur de l'amine;
K est un acide aminé D-isomère ou L-isomère de structure -N(R⁶)CH(R⁷)C(=O)-, dans lequel :
R⁶ est un groupe alkyle C₁-C₈;
R⁷ est choisi parmi:
-(C₁-C₇ alkyl)X; 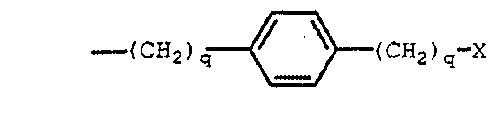 où q est indépendamment 0, 1; 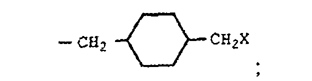 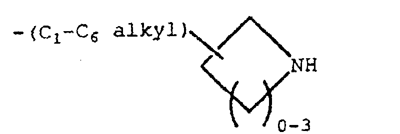 -(CH₂)ₘO-(C₁-C₄ alkyl)-X, où m = 1, 2;
-(CH₂)ₘS-(C₁-C₄ alkyl)-X, où m = 1, 2; et
X est choisi parmi : 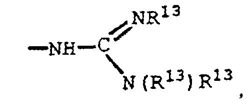 -N(R¹³)R¹³, -C(=NH)(NH₂), -SC(NH)-NH₂, dans lequel R¹³ est comme défini ci-dessus;
L est -Y(CH₂)ᵥC(=O)-, dans lequel:
Y est NH, N(C₁-C₃ alkyl), O ou S; et v = 1, 2;
M est un acide aminé D-isomère ou L-isomère de structure -NR¹⁷-CH(R⁸)C(=O)-, dans lequel :
R¹⁷ est H, un groupe alkyle C₁-C₃;
R⁸ est -CH₂CO₂R¹³, -CH₂SO₃R^{13a}, -CH(CH₃)CO₂R¹³, -SO₂NR¹³R¹⁴, -CH₂-acide borique ou -CH₂-tétrazole.

2. Composé de la revendication 1, dans lequel :
R³¹ est choisi parmi: 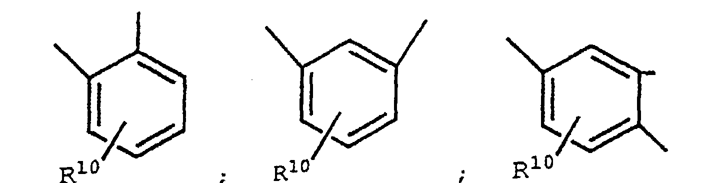 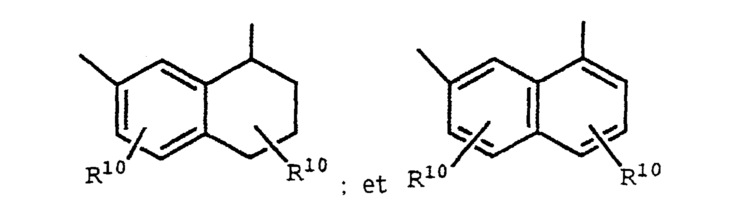 dans lequel R³¹ peut être substitué indépendamment avec 0-2 R¹⁰;
R¹ et R²² sont indépendamment choisis parmi H, un groupe alkyle C₁-C₄, phényle, benzyle, phényl-(C₂-C₄)alkyle, alkoxyle C₁-C₄;
R¹⁰ est H, un groupe alkyle C₁-C₈, phényle, halogène ou alkoxyle C₁-C₄;
K est un acide aminé L-isomère de structure -N(R⁶)CH(R⁷)C(=O)-, dans lequel:
R⁶ est un groupe alkyle C₁-C₈;
R⁷ est -(CH₂)ₚNHC(=NH)(NH₂), où p = 3-5; 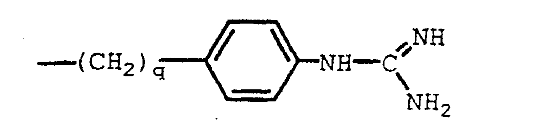 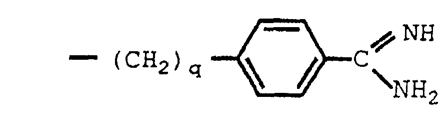 où q = 0, 1;
-(CH₂)ᵣX, où r = 4-6; 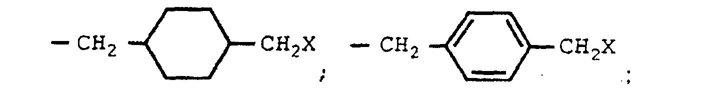 -(CH₂)ₘS(CH₂)₂X, où m = 1, 2;
-(C₃-C₇ alkyl)-N-(C₁-C₆ alkyl) 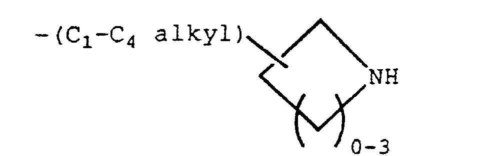 -(CH₂)ₘ-O-(C₁-C₄ alkyl)-NH-(C₁-C₆ alkyl), où m = 1, 2;
-(CH₂)ₘ-S-(C₁-C₄ alkyl)-NH-(C₁-C₆ alkyl), où m = 1, 2; et
X est -NH₂ ou -NHC(=NH)(NH₂); ou
L est Y(CH₂)ᵥC(=O)-, dans lequel :
Y est NH, O, ou S; et v = 1, 2;
M est un acide aminé L-isomère de structure -NH-CH(R⁸)C(=O)-, dans lequel :
R⁸ est -CH₂CO₂H ou -CH(CH₃)CO₂H.

3. Composé de la revendication 2 de formule (II): 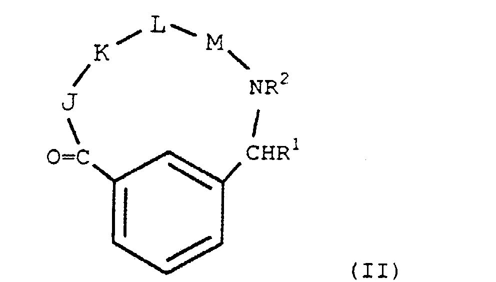 dans lequel:
R¹ est H, un groupe alkyle C₁-C₄, phényle, benzyle ou phényl-(C₂-C₄)alkyle;
R² est H ou un groupe méthyle;
J est un acide aminé β-Ala ou un _{L}-isomère ou un _{D}-isomère de structure -N(R³)C(R⁴)(R⁵)C(=O)-, dans lequel:
R³ est H ou CH₃
R⁴ est H ou un groupe alkyle C₁-C₃;
R⁵ est H, un groupe alkyle C₁-C₈, cycloalkyle C₃-C₆, cycloalkylméthyle C₃-C₆, cycloalkyléthyle C₃-C₆, phényle, phénylméthyle, CH₂OH, CH₂SH, CH₂OCH₃, CH₂SCH₃, CH₂CH₂SCH₃, (CH₂)ₛNH₂ ou (CH₂)_{S}NHC(=NH)(NH₂), où S = 3-5; ou
R³ et R⁵ peuvent être pris ensemble pour former -CH₂CH₂CH₂-; ou
R⁴ et R⁵ peuvent être pris ensemble pour former -(CH₂)ᵤ-, où u = 2-5;
K est un acide aminé _{L}-isomère de structure -N(R⁶)CH(R⁷)C(=O)-, dans lequel:
R⁶ est un groupe alkyle C₁-C₈;
R⁷ est -(CH₂)ₚNHC(=NH)(NH₂), où p = 3-5; 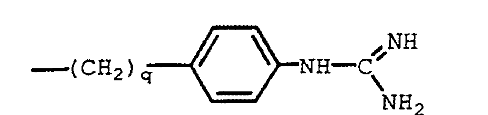 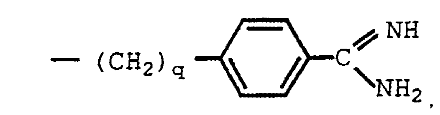 où q = 0, 1;
-(CH₂)ᵣX, où r = 4-6; 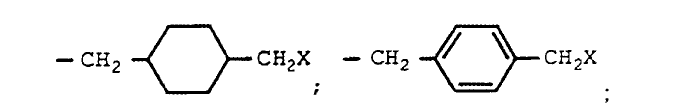 -(CH₂)ₘS(CH₂)₂X, où m = 1,2;
-(CH₂)ₘ-O-(C₁-C₄ alkyl)-NH-(C₁-C₆ alkyl), où m = 1, 2;
-(CH₂)ₘ-S-(C₁-C₄ alkyl)-NH-(C₁-C₆ alkyl), où m = 1, 2; et
-(C₃-C₇ alkyl)-N-(C₁-C₆ alkyl) 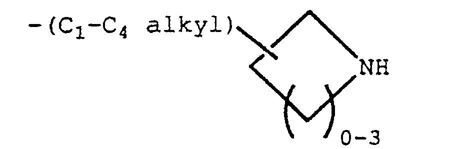
X est -NH₂ ou -NHC(=NH)(NH₂), à condition que X ne soit pas NH₂, lorsque r = 4.

4. Composé de la revendication 3 dans lequel
J est un acide aminé β-Ala ou un _{L}-isomère ou un _{D}-isomère de structure -N(R³)C(R⁴)(R⁵)C(=O) -, dans lequel:
R³ est H ou CH₃;
R⁴ est H;
R⁵ est H, un group alkyle C₁-C₈, cycloalkyle C₃-C₆, cycloalkylméthyle C₃-C₆, cycloalkyléthyle C₁-C₆, phényle, phénylméthyle, CH₂OH, CH₂SH, CH₂OCH₃, CH₂SCH₃, CH₂CH₂SCH₃,(CH₂)ₛNH₂ ou (CH₂)ₛNHC(=NH)(NH₂), où s = 3, 4, 5; ou
R³ et R⁵ peuvent être pris ensemble pour former -CH₂CH₂CH₂-;
K est un acide aminé L-isomère de structure -N(CH₃)CH(R⁷)C(=O), dans lequel:
R⁷ est -(CH₂)ₚNHC(=NH)(NH₂), où p = 3-4; 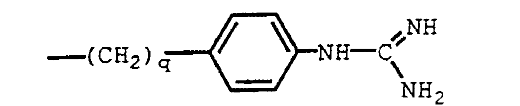 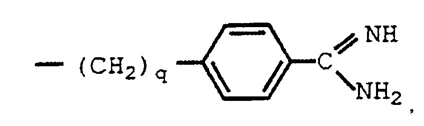 où q = 0, 1;
-(CH₂)ᵣX, où r = 4-6; 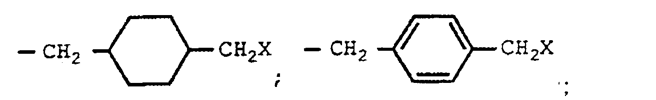 -(CH₂)ₘS(CH₂)₂X, où m = 1, 2;
-(C₄-C₇ alkyl)-N-(C₁-C₆ alkyl) 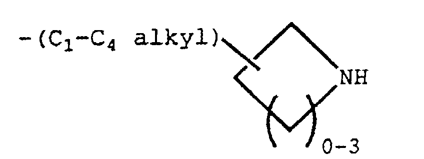 -(CH₂)ₘ-O-(C₁-C₄ alkyl)-NH-(C₁-C₆ alkyl), où m = 1,2;
-(CH₂)ₘ-S-(C₁-C₄ alkyl)-NH-(C₁-C₆ alkyl), où m = 1,2; et
X est -NH₂ ou -NHC(=NH)(NH₂), à condition que X ne soit pas -NH₂, lorsque r = 4;
L est -YCH₂C(=O)-, dans lequel:
Y est NH ou O;
M est un acide aminé L-isomère de structure -NH-CH(CH₂CO₂H)C(=O)-.

5. Composé des revendications 3 et 4 dans lequel :
R¹ est H;
R² est H;
J est un acide aminé β-Ala ou un _{L}-isomère ou un _{D}-isomère de formule -N(R³)CH(R⁵)C(=O)-, dans lequel:
R³ est H et R⁵ est H, CH₃, CH₂CH₃, CH(CH₃)₂, CH(CH₃)CH₂CH₃, CH₂CH₂CH₃, CH₂CH₂CH₂CH₃, CH₂CH₂SCH₃, CH₂CH(CH₃)₂, (CH₂)₄NH₂; ou
R³ est CH₃ et R⁵ est H; ou
R³ et R⁵ peuvent être pris ensemble pour former -CH₂CH₂CH₂-
K est un acide aminé L-isomère de formule -N(CH₃)CH(R⁷)C(=O)-, dans lequel:
R⁷ est -(CH₂)₃NHC(=NH)(NH₂);
L est NHCH₂C(=O)- ; et
M est un acide aminé L-isomère de formule -NHCH(CH₂COOH)C(=O)-

6. Composé de la revendication 2 de formule (III) 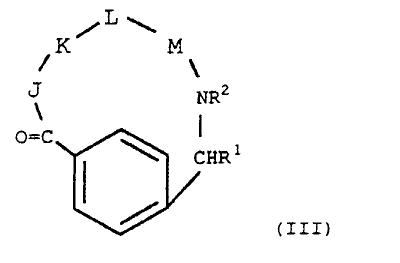 dans lequel:
R¹ est H, un groupe alkyle C₁-C₄, phényle, benzyle ou phényl-(C₂-C₄)alkyle;
R² est H ou un groupe méthyle;
J est un acide aminé β-Ala ou un _{L}-isomère ou un _{D}-isomère de structure -N(R³)C(R⁴)(R⁵)C(=O)-, dans lequel:
R³ est H ou CH₃;
R⁴ est H ou un groupe alkyle C₁-C₃;
R⁵ est H, un groupe alkyle C₁-C₈, cycloalkyle C₃-C₆, cycloalkylméthyle C₃-C₆, cycloalkyléthyle C₁-C₆, phényle, phénylméthyle, CH₂OH, CH₂SH, CH₂OCH₃, CH₂SCH₃, CH₂CH₂SCH₃, (CH₂)_{S}NH₂ ou (CH₂)_{S}NHC(=NH)(NH₂),
où s = 3-5; ou
R³ et R⁵ peuvent être pris ensemble pour former -CH₂CH₂CH₂-; ou
R⁴ et R⁵ peuvent être pris ensemble pour former -(CH₂)ᵤ-, où u = 2-5;
K est un acide aminé _{L}-isomère de structure -N(R⁶)CH(R⁷)C(=O)-, dans lequel:
R⁶ est un groupe alkyle C₁-C₈;
R⁷ est -(CH₂)ₚNHC(=NH)(NH₂), où p = 3-4; 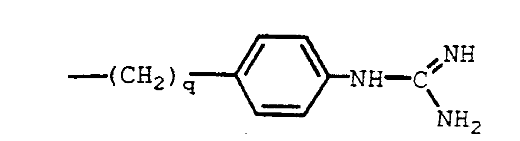 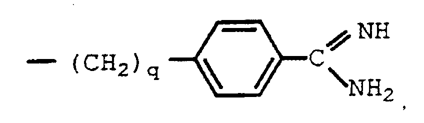 où q = 0, 1;
-(CH₂)ᵣX, où r = 4-6; 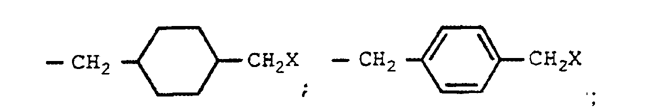 -(CH₂)ₘS(CH₂)₂X, où m = 1,2;
-(C₃-C₇ alkyl)-N-(C₁-C₆ alkyl) 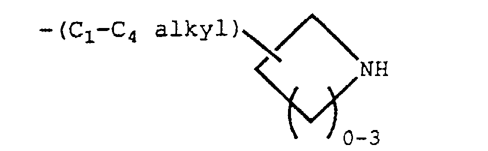 -(CH₂)ₘ-O-(C₁-C₄ alkyl)-NH-(C₁-C₆ alkyl), où m = 1,2;
-(CH₂)ₘ-S-(C₁-C₄ alkyl)-NH-(C₁-C₆ alkyl), où m = 1,2; et
X est -NH₂ ou -NHC(=NH)(NH₂), à condition que X ne soit pas NH₂, lorsque r = 4.

7. Composé de la revendication 1, qui est choisi parmi: le composé de formule (II): 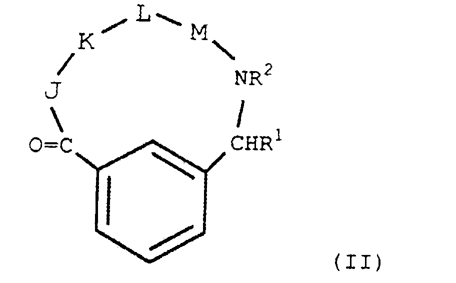 dans lequel R¹ et R² sont H; J est D-Val; K est NMeArg; L est Gly; et M est Asp;
le composé de formule (II) dans lequel R¹ et R² sont H; J est l'acide D-2-aminobutyrique; K est NMeArg; L est Gly; et M est Asp;
le composé de formule (II) dans lequel R¹ et R² sont H; J est D-Leu; K est NMeArg; L est Gly; et M est Asp;
le composé de formule (II) dans lequel R¹ et R² sont H; J est D-Ala; K est NMeArg; L est Gly; M est Asp;
le composé de formule (II) dans lequel R¹ et R² sont H; J est Gly; K est NMeArg; L est Gly; et M est Asp;
le composé de formule (II) dans lequel R¹ et R² sont H; J est D-Pro; K est NMeArg; L est Gly; et M est Asp;
le composé de formule (II) dans lequel R¹ et R² sont H; J est D-Lys; K est NMeArg; L est Gly; et M est Asp;
le composé de formule (II) dans lequel R¹ et R² sont H; J est β-Ala; K est NMeArg; L est Gly; et M est Asp;
le composé de formule (II) dans lequel R¹ et R² sont H; J est D-Val ; K est NMeArg; L est β-Ala; et M est Asp;
le composé de formule (II) dans lequel R¹ et R² sont H; J est Pro; K est NMeArg; L est Gly; et M est Asp;
le composé de formule (II) dans lequel R¹ et R² sont H; J est NMeGly; K est NMeArg; L est Gly; et M est Asp;
le composé de formule (II) dans lequel R¹ est un groupe méthyle (isomère 1); R² est H; J est D-Val; K est NMeArg; L est Gly; et M est Asp;
le composé de formule (Il) dans lequel R¹ est un groupe méthyle (isomère 2); R² est H; J est D-Val; K est NMeArg; L est Gly; et M est Asp;
le composé de formule (Il) dans lequel R¹ est un groupe phényle (isomère 1); R² est H; J est D-Val; K est NMeArg; L est Gly; et M est Asp;
le composé de formule (Il) dans lequel R¹ est un groupe phényle (isomère 2); R² est H; J est D-Val; K est NMeArg; L est Gly; et M est Asp;
le composé de formule (Il) dans lequel R¹ et R² sont H; J est D-Val; K est D-NMeArg; L est Gly; et M est Asp;
le composé de formule (Il) dans lequel R¹ et R² sont H; J est D-Nle; K est NMeArg; L est Gly; et M est Asp;
le composé de formule (Il) dans lequel R¹ et R² sont H; J est D-Phg; K est NMeArg; L est Gly; et M est Asp;
le composé de formule (Il) dans lequel R¹ et R² sont H; J est D-Ile; K est NMeArg; L est Gly; et M est Asp;
le composé de formule (Il) dans lequel R¹ et R² sont H; J est D-Phe; K est NMeArg; L est Gly; et M est Asp;
le composé de formule (Il) dans lequel R¹ et R² sont H; J est NMeGly; K est NMeArg; L est Gly; et M est Asp;
le composé de formule (Il) dans lequel R¹ et R² sont H; J est D-Nle; K est NMeArg; L est Gly; et M est Asp;
le composé de formule (Il) dans lequel R¹ et R² sont H; J est D-Tyr; K est NMeArg; L est Gly; et M est Asp;
le composé de formule (Il) dans lequel R¹ et R² sont H; J est D-Val; K est NMeAmf; L est Gly; et M est Asp;
le composé de formule (Il) dans lequel R¹ et R² sont H; J est D-Val; K est NMeArg; L est Ala; et M est Asp;
le composé de formule (Il) dans lequel R¹ et R² sont H; J est D-Val; K est NMeArg; L est Gly; et M est -MeAsp;
le composé de formule (Il) dans lequel R¹ et R² sont H; J est D-Val; K est NMeArg; L est Gly; et M est β-MeAsp;
le composé de formule (Il) dans lequel R¹ et R² sont H; J est D-Val; K est NMeArg; L est Gly; et M est NMeAsp;
le composé de formule (Il) dans lequel R¹ et R² sont H; J est D-Val; K est NMeArg; L est Gly; et M est D-Asp;
le composé de formule (Il) dans lequel R¹ est H; R² est CH₃; J est D-Val; K est NMeArg; L est Gly; et M est Asp;
le composé de formule (Il) dans lequel R¹ et R² sont H; J est D-Abu; K est di-NMeOrn; L est Gly; et M est Asp;
le composé de formule (III): 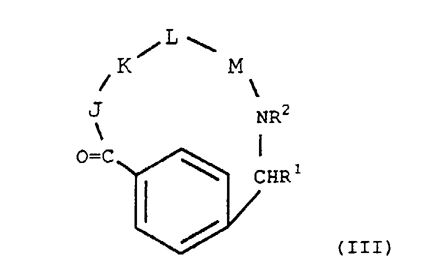 dans lequel R¹ et R² sont H; J est D-Val; K est NMeArg; L est Gly; et M est Asp;
le composé de formule (V): 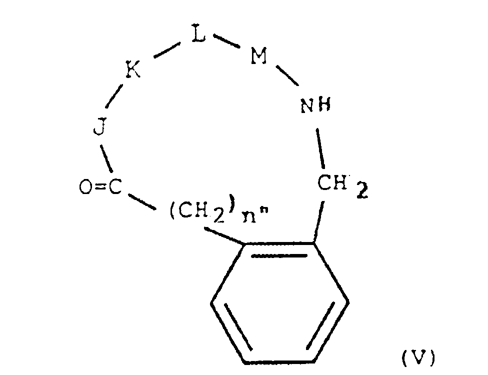 dans lequel n"=1; J est D-Val; K est NMeArg; L est Gly; et M est Asp;
le composé de formule (V) dans lequel n"=O; J est D-Val; K est NMeArg; L est Gly; et M est Asp;
le composé de formule (VI): 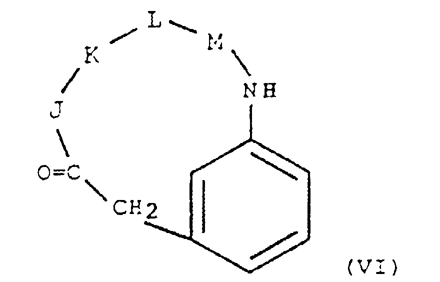 dans lequel J est D-Val; K est NMeArg; L est Gly; et M est Asp;
le composé de formule (VII): 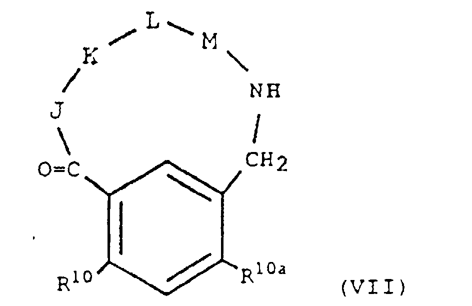 dans lequel R¹⁰ est H; R^{10a} est Cl; J est D-Val; K est NMeArg; L est Gly; et M est Asp;
le composé de formule (VII) dans lequel R¹⁰ est H; R^{10a} est I; J est D-Val; K est NMeArg; L est Gly; et M est Asp;
le composé de formule (VII) dans lequel R¹⁰ est H; R^{10a} est I; J est D-Abu; K est NMeArg; L est Gly; et M est Asp;
le composé de formule (VII) dans lequel R¹⁰ est H; R^{10a} est OMe; J est D-Val; K est NMeArg; L est Gly; et M est Asp;
le composé de formule (VII) dans lequel R¹⁰ est H; R^{10a} est Me; J est D-Val; K est NMeArg; L est Gly; et M est Asp;
le composé de formule (VII) dans lequel R^{10a} est H; R¹⁰ est Cl; J est D-Val; K est NMeArg; L est Gly; et M est Asp;
le composé de formule (VII) dans lequel R^{10a} est H; R¹⁰ est MeO; J est D-Val; K est NMeArg; L est Gly; et M est Asp;
le composé de formule (VII) dans lequel R^{10a} est H; R¹⁰ est Me; J est D-Val; K est NMeArg; L est Gly; et M est Asp; le composé de formule (VIII): 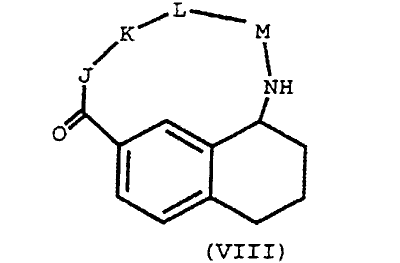 dans lequel J est D-Val; K est NMeArg; L est Gly; et M est Asp;
le composé de formule (IX): 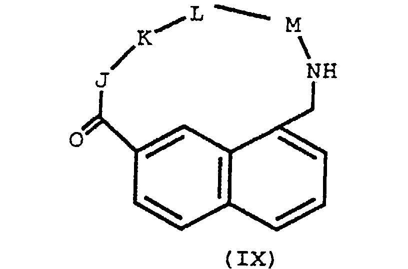 dans lequel J est D-Val; K est NMeArg; L est Gly; et M est Asp.

8. Composé de la revendication 7 qui est choisi parmi:
le composé de formule (Il) dans lequel R¹ et R² sont H; J est D-Val; K est NMeArg; L est Gly; et M est Asp;
le composé de formule (Il) dans lequel R¹ et R² sont H; J est un acide D-2-aminobutyrique; K est NMeArg; L est Gly; et M est Asp;
le composé de formule (Il) dans lequel R¹ et R² sont H; J est D-Leu; K est NMeArg; L est Gly; et M est Asp;
le composé de formule (Il) dans lequel R¹ et R² sont H; J est D-Ala; K est NMeArg; L est Gly; et M est Asp;
le composé de formule (Il) dans lequel R¹ et R² sont H; J est Gly; K est NMeArg; L est Gly; et M est Asp;
le composé de formule (II) dans lequel R¹ et R² sont H; J est D-Pro; K est NMeArg; L est Gly; et M est Asp;
le composé de formule (Il) dans lequel R¹ et R² sont H; J est D-Lys; K est NMeArg; L est Gly; et M est Asp;
le composé de formule (Il) dans lequel R¹ et R² sont H; J est β-Ala; K est NMeArg; L est Gly; et M est Asp;
le composé de formule (Il) dans lequel R¹ et R² sont H; J est NMeGly; K est NMeArg; L est Gly, et M est Asp;
le composé de formule (Il) dans lequel R¹ est un groupe méthyle (isomère 1); R² est H; J est D-Val; K est NMeArg; L est Gly; et M est Asp;
le composé de formule (Il) dans lequel R¹ est un groupe méthyle (isomère 2); R² est H; J est D-Val; K est NMeArg; L est Gly; et M est Asp;
le composé de formule (Il) dans lequel R¹ est un groupe phényle (isomère 1); R² est H; J est D-Val; K est NMeArg; L est Gly; et M est Asp;
le composé de formule (Il) dans lequel R¹ et R² sont H; J est D-Val; K est D-NMeArg; L est Gly; et M est Asp;
le composé de formule (Il) dans lequel R¹ et R² sont H; J est D-Nle; K est NMeArg; L est Gly; et M est Asp;
le composé de formule (Il) dans lequel R¹ et R² sont H; J est D-Phg; K est NMeArg; L est Gly; et M est Asp;
le composé de formule (Il) dans lequel R¹ et R² sont H; J est D-Phe; K est NMeArg; L est Gly; et M est Asp;
le composé de formule (Il) dans lequel R¹ et R² sont H; J est D-Ile; K est NMeArg; L est Gly; et M est Asp;
le composé de formule (III) dans lequel R¹ et R² sont H; J est D-Val; K est NMeArg; L est Gly; et M est Asp;
le composé de formule (V) dans lequel n" = 1; J est D-Val; K est NMeArg; L est Gly et M est Asp;
le composé de formule (V) dans lequel n" = 0; J est D-Val; K est NMeArg; L est Gly et M est Asp;
le composé de formule (VI) dans lequel J est D-Val; K est NMeArg; L est Gly; et M est Asp;
le composé de formule (VII) dans lequel R¹⁰ est H; R^{10a} est Cl; J est D-Val; K est NMeArg; L est Gly; et M est Asp;
le composé de formule (VII) dans lequel R¹⁰ est H; R^{10a} est I; J est D-Val; K est NMeArg; L est Gly; et M est Asp;
le composé de formule (VII) dans lequel R¹⁰ est H; R^{10a} est I; J est D-Abu; K est NMeArg; L est Gly; et M est Asp;
le composé de formule (VII) dans lequel R¹⁰ est H; R^{10a} est Me; J est D-Val; K est NMeArg; L est Gly; et M est Asp;
le composé de formule (VII) dans lequel R^{10a} est H; R¹⁰ est Cl; J est D-Val; K est NMeArg; L est Gly; et M est Asp;
le composé de formule (VII) dans lequel R^{10a} est H; R¹⁰ est MeO; J est D-Val; K est NMeArg; L est Gly; et M est Asp;
le composé de formule (VII) dans lequel R^{10a} est H; R¹⁰ est Me; J est D-Val; K est NMeArg; L est Gly; et M est Asp;
le composé de formule (Il) dans lequel R¹ et R² sont H; J est D-Tyr; K est NMeArg; L est Gly; et M est Asp;
le composé de formule (Il) dans lequel R¹ et R² sont H; J est D-Val; K est NMeAmf; L est Gly; et M est Asp;
le composé de formule (Il) dans lequel R¹ et R² sont H; J est D-Val; K est NMeArg; L est Gly; et M est bMeAsp;
le composé de formule (Il) dans lequel R¹ est H; R² est CH₃; J est D-Val; K est NMeArg; L est Gly; et M est Asp;
le composé de formule (III) dans lequel R¹ et R² sont H; J est D-Val; K est NMeArg; L est Gly; et M est Asp;
le composé de formule (VIII) dans lequel J est D-Val; K est NMeArg; L est Gly; et M est Asp.

9. Composé de la revendication 8 choisi parmi:
le composé de formule (Il) dans lequel R¹ et R² sont H; J est D-Val; K est NMeArg; L est Gly; et M est Asp;
le composé de formule (Il) dans lequel R¹ et R² sont H; J est l'acide D-2-aminobutyrique; K est NMeArg; L est Gly; et M est Asp;
le composé de formule (Il) dans lequel R¹ et R² sont H; J est D-Leu; K est NMeArg; L est Gly; et M est Asp;
le composé de formule (Il) dans lequel R¹ et R² sont H; J est D-Ala; K est NMeArg; L est Gly; et M est Asp;
le composé de formule (Il) dans lequel R¹ et R² sont H; J est D-Pro; K est NMeArg; L est Gly; et M est Asp;
le composé de formule (Il) dans lequel R¹ et R² sont H; J est D-Lys; K est NMeArg; L est Gly; et M est Asp;
le composé de formule (Il) dans lequel R¹ et R² sont H; J est D-Nle; K est NMeArg; L est Gly; et M est Asp;
le composé de formule (Il) dans lequel R¹ et R² sont H; J est D-Phe; K est NMeArg; L est Gly; et M est Asp;
le composé de formule (Il) dans lequel R¹ et R² sont H; J est D-Ile; K est NMeArg; L est Gly; et M est Asp;
le composé de formule (V) dans lequel n" = 1; J est D-Val; K est NMeArg; L est Gly; et M est Asp;
le composé de formule (VI) dans lequel J est D-Val; K est NMeArg; L est Gly; et M est Asp;
le composé de formule (VII) dans lequel R¹⁰ est H; R^{10a} est Cl; J est D-Val; K est NMeArg; L est Gly; et M est Asp;
le composé de formule (VII) dans lequel R¹⁰ est H; R^{10a} est I; J est D-Val; K est NMeArg; L est Gly; et M est Asp;
le composé de formule (VII) dans lequel R¹⁰ est H; R^{10a} est Me; J est D-Val; K est NMeArg; L est Gly; et M est Asp;
le composé de formule (VII) dans lequel R^{10a} est H; R¹⁰ est Cl; J est D-Val; K est NMeArg; L est Gly; et M est Asp;
le composé de formule (VII) dans lequel R^{10a} est H; R¹⁰ est MeO; J est D-Val; K est NMeArg; L est Gly; et M est Asp;
le composé de formule (VII) dans lequel R^{10a} est H; R¹⁰ est Me; J est D-Val; K est NMeArg; L est Gly; et M est Asp;
le composé de formule (Il) dans lequel R¹ et R² sont H; J est D-Val; K est NMeArg; L est Gly; et M est bMeAsp.

10. Composé de formule (I): 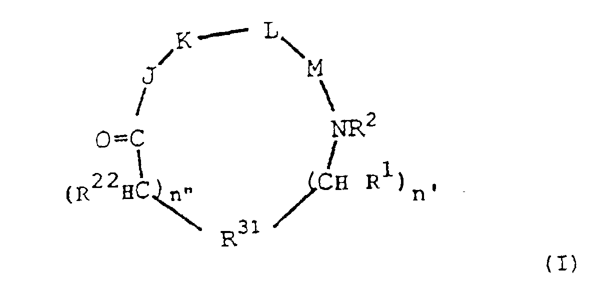 ou forme d'un sel ou d'un promédicament pharmaceutiquement acceptable de celui-ci dans lequel:
R³¹ est choisi parmi: 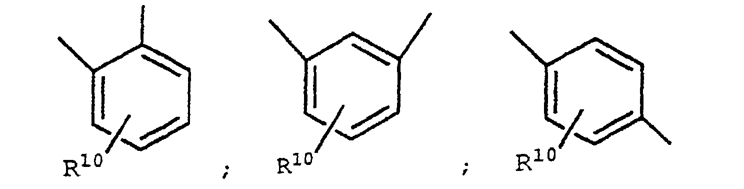 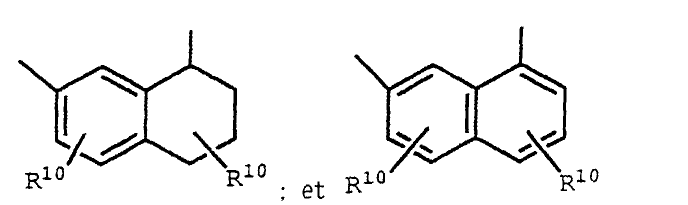 dans lequel R³¹ peut être indépendamment substitué avec 0-2 R¹⁰;
n" est 0 ou 1;
n' est 0 ou 1;
R¹ et R²² sont indépendamment choisis parmi H, un groupe alkyle C₁-C₄, phényle, benzyle, phényl-(C₂-C4)alkyle, alkoxyle C₁-C₄;
R² est H ou un groupe alkyle C₁-C₈;
R¹⁰ est H, un groupe alkyle C₁-C₈, phényle, halogène, ou alkoxyle C₁-C₄;
J est un acide aminé β-Ala ou un _{L}-isomère ou un _{D}-isomère de structure -N(R³)C(R⁴)(R⁵)C(=O)-, dans lequel:
R³ est H ou CH₃
R⁴ est H ou un groupe alkyle C₁-C₃;
R⁵ est H, un groupe alkyle C₁-C₈, cycloalkyle C₃-C₆, cycloalkylméthyle C₃-C₆, cycloalkyléthyle C₁-C₆, phényle, phénylméthyle, CH₂OH, CH₂SH, CH₂OCH₃, CH₂SCH₃, CH₂CH₂SCH₃, (CH₂)ₛNH₂, (CH₂)_{S}NHC(=NH)(NH₂), (CH₂)_{S}NHR¹⁶, où s = 3-5;
R³ et R⁵ peuvent également être pris ensemble pour former -(CH₂)ₜ-(t = 2-4) ou -CH₂SC(CH₃)2-; ou
R⁴ et R⁵ peuvent également être pris ensemble pour former -(CH₂)ᵤ-, où u = 2-5;
R¹⁶ est un groupe protecteur de l'amine;
K est un acide aminé _{L}-isomère de structure -N(R⁶)CH(R⁷)C(=O)-, dans lequel:
R⁶ est un groupe alkyle C₁-C₈;
R⁷ est -(CH₂)ₚNHC(=NH)(NH₂), où p = 3-5; 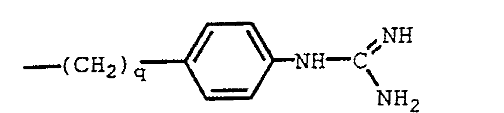 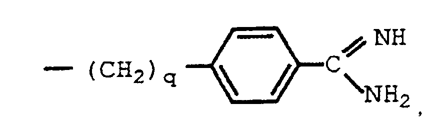 où q = 0, 1;
-(CH₂)ᵣX, où r = 4-6; 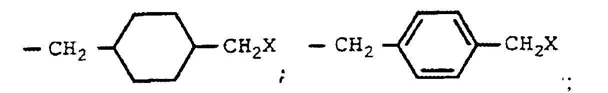 (CH₂)ₘS(CH₂)₂X, où m = 1,2;
-(C₃-C₇ alkyl)-N-(C₁-C₆ alkyl) 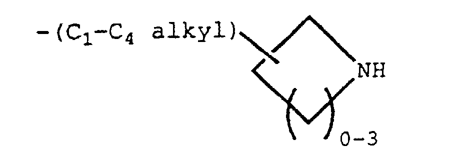 -(CH₂)ₘ-O-(C₁-C₄ alkyl)-NH-(C₁-C₆ alkyl), où m = 1, 2;
-(CH₂)ₘ-S-(C₁-C₄ alkyl)-NH-(C₁-C₆ alkyl), où m = 1, 2; et
X est -NH₂ ou -NHC(=NH)(NH₂);
L est Y(CH₂)ᵥC(=O)- , dans lequel :
Y est NH, O, ou S; et v = 1,2;
M est un acide aminé _{L}-isomère de structure -NH-CH(CH₂S0₃H)C(=O)-.

11. Utilisation d'un composé suivant l'une quelconque des revendications 1-10 pour la préparation d'un médicament pour le traitement de désordres thromboemboliques.

12. Procédé pour la préparation d'une composition pharmaceutique comprenant un excipient pharmaceutiquement acceptable et une quantité thérapeutiquement efficace d'un composé suivant l'une quelconque des revendications 1-10, lequel procédé comprend l'addition dudit composé à l'excipient.

## Revendications (Revendications pour l'(les) Etat(s) contractant(s) suivant(s): ES)

1. Procédé pour la préparation d'un composé de formule (I): 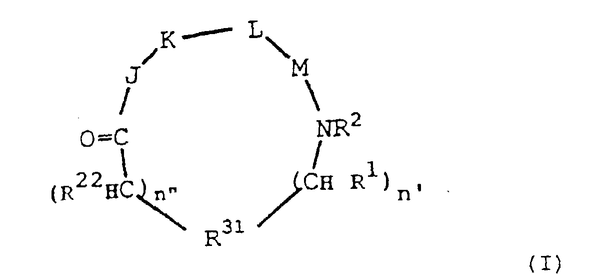 ou d'une forme d'un sel ou d'un promédicament pharmaceutiquement acceptable de celui-ci dans lequel:
R³¹ est
(a) un anneau carbocyclique aromatique à 6 membres de formule 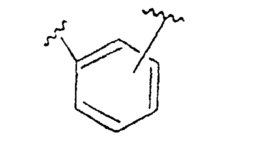 dans lequel ledit anneau carbocyclique est indépendamment substitué avec 0-2 R¹⁰, ou
(b) un anneau carboxylique bicyclique à 10 membres de formule 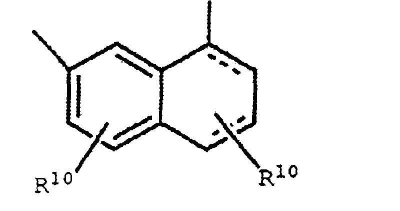 dans lequel la liaison en pointillé peut être une liaison simple ou double;
n" est 0 ou 1;
n' est 0 ou 1;
R¹ et R²² sont indépendamment choisis parmi les groupes suivants: hydrogène,
alkyle C₁-C₈ substitué avec 0-2 R¹¹,
alcényle C₂-C₈ substitué avec 0-2 R¹¹,
alcynyle C₁-C₈ substitué avec 0-2 R¹¹,
cycloalkyle C₃-C₈ substitué avec 0-2 R¹¹,
alkyle bicyclique C₆-C₁₀ substitué avec 0-2 R¹¹,
aryle substitué avec 0-2 R¹²,
un système d'anneau hétérocyclique substitué avec 0-2 R¹², composé de 5-10 atomes, y compris 1-3 hétéroatomes de N, S ou O,
=O, F, Cl , Br, I, -CN, CO₂R¹³, -C(=O)R¹³, -C(=O)NR¹³R¹⁴, -CHO, -CH₂OR¹³, -OC(=O)R¹³,-OC(=O)OR¹³, -OR¹³, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -SR¹³, -S(=O)R^{13a}, -SO₂NR¹³R¹⁴, -CH₂NR¹³R¹⁴, -NR¹³R¹⁴, -NHC(=NH)NHR¹³, -C(=NH)NHR¹³, NO₂;
R¹¹ est choisi parmi un ou plusieurs des groupes suivants:
=O, F, Cl , Br, I, -CF₃, -CN, - CO₂R¹³, -C(=O)R¹³, -C(=O)NR¹³R¹⁴, -CH₂OR¹³, -OC(=O)R¹³, -OC(=O)OR¹³, -OR¹³, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, - SO₃H, - SO₂R^{13a}, -SR¹³, -S(=O)R^{13a},
-SO₂NR¹³R¹⁴, -CH₂NR¹³R¹⁴, -NR¹³R¹⁴, -NHC(=NH)NHR¹³, -C(=NH)NHR¹³, =NOR¹⁴, NO₂,
alkyle C₁-C₅ , alcényle C₂-C₄ , cycloalkyle C₃-C₆, cycloalkylméthyle C₃-C₆, alkoxyalkyle C₂-C₆, alkyle C₁-C₄ (substitué avec -NR¹³R¹⁴, -CF₃, NO₂, -SO₂R¹³, ou -S(=O)R^{13a})
aryle substitué avec 0-2 R¹²,
un système d'anneau hétérocyclique substitué avec 0-2 R¹², composé de 5-10 atomes, y compris 1-3 hétéroatomes d'azote, d'oxygène ou de soufre;
R¹² est choisi parmi un ou plusieurs des groupes suivants:
phényle, benzyle, phénéthyle, phénoxyle, benzyloxyle, halogène, hydroxyle, nitro, cyano, alkyle C₁-C₅, cycloalkyle C₃-C₆, cycloalkylméthyle C₃-C₆, arylalkyle C₁-C₁₀, alkoxyle C₁-C₄, -CO₂R¹³, acide hydroxamique, hydrazide, oxime, acide borique, sulfonamide, formyle, cycloalkoxyle C₃-C₆, -OC(=O)R¹³, -C(=O)R¹³, -OC(=O)OR¹³, -OR¹³, -CH₂OR¹³, -NR¹³R¹⁴, -OC(=O)NR¹³R¹⁴, -NR¹⁴C(=O)R¹³, -NR¹⁴C(=O)OR¹³, -NR¹³C(=O)NR¹³R¹⁴, - NR¹⁴SO₂NR¹³R¹⁴, -NR¹⁴SO₂R^{13a}, -SO₃H, -SO₂R^{13a}, -S(=O)R^{13a}, -SR¹³, - SO₂NR¹³R¹⁴, alkoxyalkyle C₂-C₆, hydroxyalkyle C₁-C₄, méthylènedioxyle, éthylènedioxyle,
R¹³ est H, un groupe alkyle C₁-C₇, aryle, -(C₁-C₆ alkyl)aryle ou alkoxyalkyle C₃-C₆ ;
R^{13a} est un groupe alkyle C₁-C₇, aryle, -(C₁-C₆ alkyl)aryle ou alkoxyalkyle C₃-C₆ ;
R¹⁴ est OH, H, un groupe alkyle C₁-C₄, ou benzyle;
R² est H ou un groupe alkyle C₁-C₈;
R¹⁰ est choisi parmi un ou plusieurs des groupes suivants:
phényle, benzyle, phénéthyle, phénoxyle, benzyloxyle, halogène, hydroxyle, nitro, cyano, alkyle C₁-C₅, cycloalkyle C₃-C₆, cycloalkylméthyle C₃-C₆, arylalkyle C₁-C₁₀, alkoxyle C₁-C₄, -CO₂R¹³, -C(=O)NHOR¹³, -C(=O)NHNR¹³R¹⁴, oxime, acide borique, cycloalkoxyle C₃-C₆, -OC(=O)R¹³, -C(=O)R¹³, -OC(=O)OR¹³, -OR¹³, -CH₂OR¹³, -NR¹³R¹⁴, -OC(=O)NR¹³R¹⁴, -NR¹⁴C(=O)R¹³, -NR¹⁴C(=O)OR¹³, -NR¹³C(=O)NR¹³R¹⁴, -NR¹⁴SO₂NR¹³R¹⁴, -NR¹⁴SO₂R^{13a}, -SO₃H, -SO₂R^{13a}, -S(=O)R^{13a}, -SR¹³, -SO₂NR¹³R¹⁴, alkoxyalkyle C₂-C₆, hydroxyalkyle C₁-C₄, méthylènedioxyle, éthylènedioxyle, halogénoalkyle C₁-C₄, halogénoalkoxyle C₁-C₄, alkoxycarbonyle C₁-C₄, alkylcarbonyloxyle C₁-C₄, alkylcarbonyle C₁-C₄, aminoalkylcarbonyle C₁-C₄, -OCH₂CO₂H, 2-(1-morpholino)éthoxyle, alkyle C₁-C₄ (substitué avec NR¹³R¹⁴, -CF₃, NO₂ ou -S(=O)R^{13a});
J est un acide aminé β-Ala ou un _{L}-isomère ou un _{D}-isomère de structure -N(R³)C(R⁴)(R⁵)C(=O)-, dans lequel :
R³ est H ou CH₃;
R⁴ est H ou un groupe alkyle C₁-C₃;
R⁵ est H, un groupe alkyle C₁-C₈, cycloalkyle C₃-C₆, cycloalkylméthyle C₁-C₆, cycloalkyléthyle C₁-C₆, phényle, phénylméthyle, CH₂OH, CH₂SH, CH₂OCH₃, CH₂SCH₃, CH₂CH₂SCH₃, (CH₂)_{S}NH₂, (CH₂)_{S}NHC(=NH)(NH₂), (CH₂)_{S}NHR¹⁶, où s = 3-5;
R³ et R⁵ peuvent également être pris ensemble pour former -(CH₂)ₜ-(t = 2-4) ou -CH₂SC(CH₃)₂-; ou
R⁴ et R⁵ peuvent également être pris ensemble pour former -(CH₂)ᵤ-, où u = 2-5;
R¹⁶ est un groupe protecteur de l'amine;
K est un acide aminé _{D}-isomère ou _{L}-isomère de structure -N(R⁶)CH(R⁷)C(=O)-, dans lequel :
R⁶ est un groupe alkyle C₁-C₈;
R⁷ est choisi parmi:
-(C₁-C₇ alkyl)X; 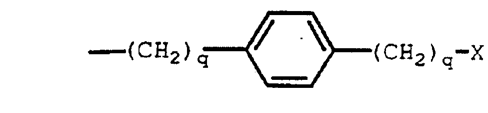 où q est indépendamment 0, 1; 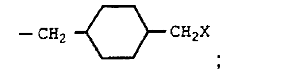 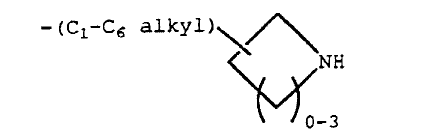 -(CH₂)ₘO-(C₁-C₄ alkyl)-X, où m = 1, 2;
-(CH₂)ₘS-(C₁-C₄ alkyl)-X, où m = 1, 2; et
X est choisi parmi : 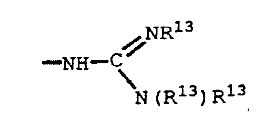 -N(R¹³)R¹³, -C(=NH)(NH₂)-SC(NH)-NH₂, dans lequel R¹³ est comme défini ci-dessus;
L est -Y(CH₂)ᵥC(=O)-, dans lequel:
Y est NH, N(C₁-C₃alkyl), O ou S; et v = 1, 2;
M est un acide aminé _{D}-isomère ou _{L}-isomère de structure -NR¹⁷-CH(R⁸)C(=O)-, dans lequel :
R¹⁷ est H, un groupe alkyle C₁-C₃;
R⁸ est -CH₂CO₂R¹³,-CH₂SO₃R^{13a}, -CH(CH₃)CO₂R¹³, -SO₂NR¹³R¹⁴, -CH₂-acide borique ou -CH₂-tétrazole;
lequel procédé comprend la cyclisation d'un peptide précurseur linéaire.

2. Procédé de la revendication 1, dans lequel:
R¹ et R²² sont indépendamment choisis parmi H, un groupe alkyle C₁-C₄, phényle, benzyle, phényl-(C₂-C₄)alkyle, alkoxyle C₁-C₄;
R³¹ est choisi parmi: 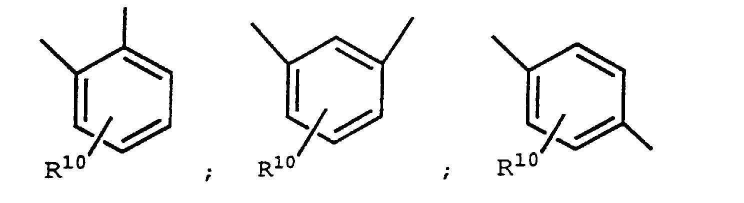 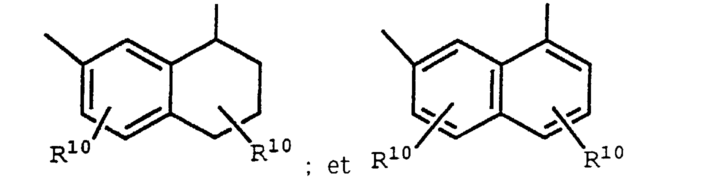 dans lequel R³¹ peut être substitué indépendamment avec 0-2 R¹⁰;
R¹⁰ est H, un groupe alkyle C₁-C₈, phényle, halogène ou alkoxyle C₁-C₄;
K est un acide aminé _{L}-isomère de structure -N(R⁶)CH(R⁷)C(=O)-, dans lequel:
R⁶ est un groupe alkyle C₁-C₈;
R⁷ est -(CH₂)ₚNHC(=NH)(NH₂), où p = 3-5; 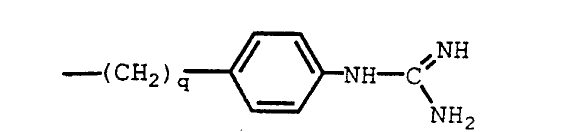 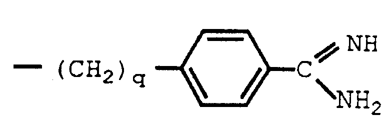 où q = 0, 1;
-(CH₂)ᵣX, où r = 4-6; 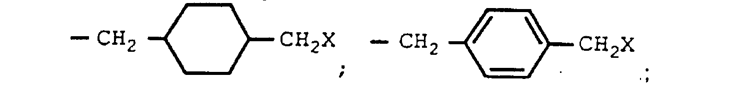 -(CH₂)ₘS(CH₂)₂X, où m = 1, 2;
-(C₃-C₇ alkyl)-N-(C₁-C₆ alkyl) 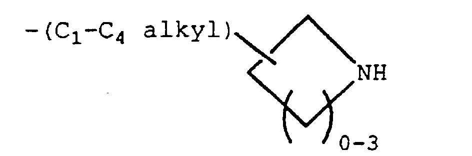 -(CH₂)ₘ-O-(C₁-C₄ alkyl)-NH-(C₁-C₆ alkyl), où m = 1, 2;
-(CH₂)ₘ-S-(C₁-C₄ alkyl)-NH-(C₁-C₆ alkyl), où m = 1, 2; et
X est -NH₂ ou -NHC(=NH)(NH₂); ou
L est Y(CH₂)ᵥC(=O)-, dans lequel :
Y est NH, O, ou S; et v = 1, 2;
M est un acide aminé _{L}-isomère de structure -NH-CH(R⁸)C(=O)-, dans lequel:
R⁸ est -CH₂CO₂H ou -CH(CH₃)CO₂H.

3. Procédé de la revendication 2 dans lequel le composé préparé a la formule (II): 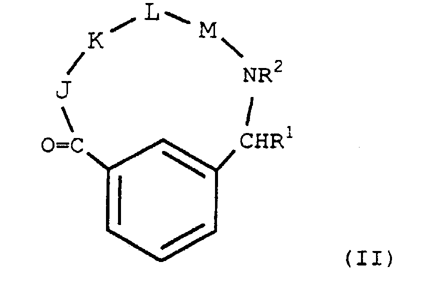 dans lequel :
R¹ est H, un groupe alkyle C₁-C₄, phényle, benzyle ou phényl-(C₂-C₄)alkyle;
R² est H ou un groupe méthyle;
J est un acide aminé β-Ala ou un _{L}-isomère ou un _{D}-isomère de structure -N(R³)C(R⁴)(R⁵)C(=O)-, dans lequel:
R³ est H ou CH₃
R⁴ est H ou un groupe alkyle C₁-C₃;
R⁵ est H, un groupe alkyle C₁-C₈, cycloalkyle C₃-C₆, cycloalkylméthyle C₃-C₆, cycloalkyléthyle C₃-C₆, phényle, phénylméthyle, CH₂OH, CH₂SH, CH₂OCH₃, CH₂SCH₃, CH₂CH₂SCH₃, (CH₂)ₛNH₂ ou (CH₂)_{S}NHC(=NH)(NH₂), où s = 3-5; ou
R³ et R⁵ peuvent être pris ensemble pour former -CH₂CH₂CH₂-; ou
R⁴ et R⁵ peuvent être pris ensemble pour former -(CH₂)ᵤ-, où u = 2-5;
K est un acide aminé _{L}-isomère de structure -N(R⁶)CH(R⁷)C(=O)-, dans lequel:
R⁶ est un groupe alkyle C₁-C₈;
R⁷ est -(CH₂)ₚNHC(=NH)(NH₂), où p = 3-5; 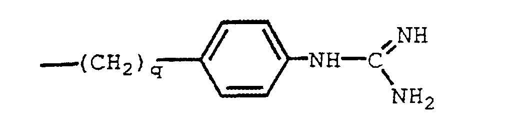 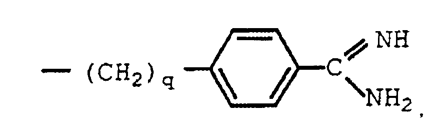 où q = 0, 1;
-(CH₂)ᵣX, où r = 4-6; 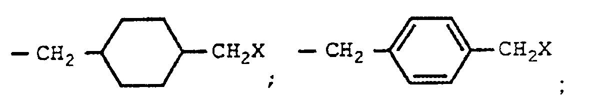 -(CH₂)ₘS(CH₂)₂X, où m = 1,2;
-(CH₂)ₘ-O-(C₁-C₄ alkyl)-NH-(C₁-C₆ alkyl), où m = 1, 2;
-(CH₂)ₘ-S-(C₁-C₄ alkyl)-NH-(C₁-C₆ alkyl), où m = 1, 2; et
-(C₃-C₇ alkyl)-N-(C₁-C₆ alkyl) 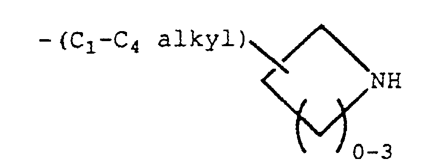
X est -NH₂ ou -NHC(=NH)(NH₂), à condition que X ne soit pas NH₂, lorsque r = 4.

4. Procédé de la revendication 3 dans lequel
J est un acide aminé β-Ala ou un _{L}-isomère ou un D-isomère de structure -N(R³)C(R⁴)(R⁵)C(=O)-, dans lequel:
R³ est H ou CH₃;
R⁴ est H;
R⁵ est H, un groupe alkyle C₁-C₈, cycloalkyle C₃-C₆, cycloalkylméthyle C₃-C₆, cycloalkyléthyle C₁-C₆, phényle, phénylméthyle, CH₂OH, CH₂SH, CH₂OCH₃, CH₂SCH₃, CH₂CH₂SCH₃, (CH₂)ₛNH₂ ou (CH₂)ₛNHC(=NH)(NH₂), où s = 3, 4, 5; ou
R³ et R⁵ peuvent être pris ensemble pour former -CH₂CH₂CH₂-;
K est un acide aminé _{L}-isomère de structure -N(CH₃)CH(R⁷)C(=O), dans lequel:
R⁷ est -(CH₂)ₚNHC(=NH)(NH₂), où p = 3-4; 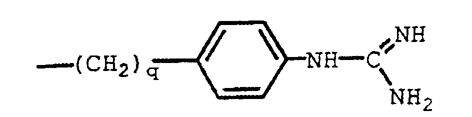 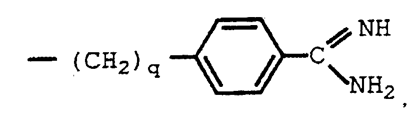 où q = 0, 1;
-(CH₂)ᵣX, où r = 4-6; 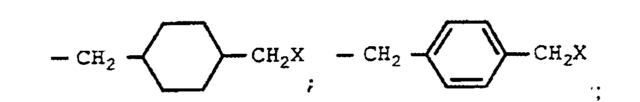 (CH₂)ₘS(CH₂)₂X, où m = 1, 2;
-(C₄-C₇ alkyl)-N-(C₁-C₆ alkyl) 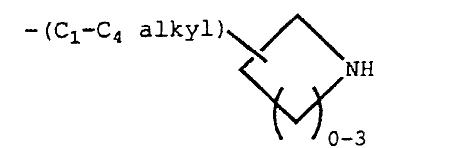 -(CH₂)ₘ-O-(C₁-C₄ alkyl)-NH-(C₁-C₆ alkyl), où m = 1,2;
-(CH₂)ₘ-S-(C₁-C₄ alkyl)-NH-(C₁-C₆ alkyl), où m = 1,2; et
X est -NH₂ ou -NHC(=NH)(NH₂), à condition que X ne soit pas -NH₂, lorsque r = 4;
L est -YCH₂C(=O)-, dans lequel:
Y est NH ou O;
M est un acide aminé L-isomère de structure -NH-CH(CH₂CO₂H)C(=O)-.

5. Procédé des revendications 3 et 4 dans lequel:
R¹ est H;
R² est H;
J est un acide aminé β-Ala ou un _{L}-isomère ou un _{D}-isomère de formule -N(R³)CH(R⁵)C(=O)-, dans lequel:
R³ est H et R⁵ est H, CH₃, CH₂CH₃, CH(CH₃)₂, CH(CH₃)CH₂CH₃, CH₂CH₂CH₃, CH₂CH₂CH₂CH₃, CH₂CH₂SCH₃, CH₂CH(CH₃)₂, (CH₂)₄NH₂; ou
R³ est CH₃ et R⁵ est H; ou
R³ et R⁵ peuvent être pris ensemble pour former -CH₂CH₂CH₂-
K est un acide aminé _{L}-isomère de formule -N(CH₃)CH(R⁷)C(=O)-, dans lequel:
R⁷ est -(CH₂)₃NHC(=NH)(NH₂);
L est -NHCH₂C(=O)-; et
M est un acide aminé _{L}-isomère de formule -NHCH(CH₂COOH)C(=O)-

6. Procédé de la revendication 2 dans lequel le composé préparé a la formule (III) 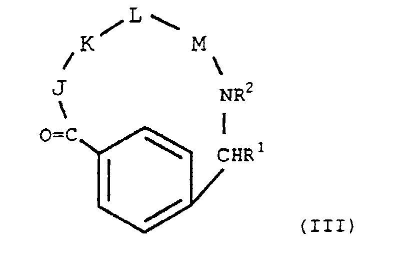 dans lequel :
R¹ est H, un groupe alkyle C₁-C₄, phényle, benzyle ou phényl-(C₂-C₄)alkyle;
R² est H ou un groupe méthyle;
J est un acide aminé β-Ala ou un _{L}-isomère ou un _{D}-isomère de structure -N(R³)C(R⁴)(R⁵)C(=O)-, dans lequel:
R³ est H ou CH₃;
R⁴ est H ou un groupe alkyle C₁-C₃;
R⁵ est H, un groupe alkyle C₁-C₈, cycloalkyle C₃-C₆, cycloalkylméthyle C₃-C₆, cycloalkyléthyle C₁-C₆, phényle, phénylméthyle, CH₂OH, CH₂SH, CH₂OCH₃, CH₂SCH₃, CH₂CH₂SCH₃, (CH₂)_{S}NH₂ ou (CH₂)_{S}NHC(=NH)(NH₂),
où s = 3-5; ou
R³ et R⁵ peuvent être pris ensemble pour former -CH₂CH₂CH₂-; ou
R⁴ et R⁵ peuvent être pris ensemble pour former -(CH₂)ᵤ-, où u = 2-5;
K est un acide aminé _{L}-isomère de structure -N(R⁶)CH(R⁷)C(=O)-, dans lequel:
R⁶ est un groupe alkyle C₁-C₈;
R⁷ est -(CH₂)ₚNHC(=NH)(NH₂), où p = 3-4; 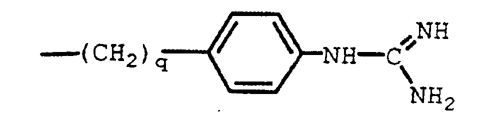 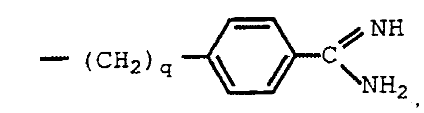 où q = 0, 1;
-(CH₂)ᵣX, où r = 4-6; 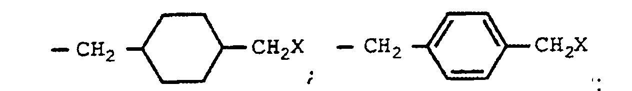 -(CH₂)ₘS(CH₂)₂X, où m = 1,2; -(C₃-C₇ alkyl)-N-(C₁-C₆ alkyl) 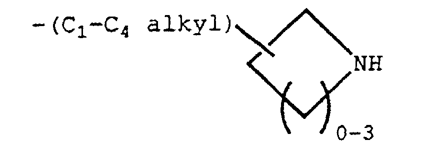 -(CH₂)ₘ-O-(C₁-C₄ alkyl)-NH-(C₁-C₆ alkyl), où m = 1,2;
-(CH₂)ₘ-S-(C₁-C₄ alkyl)-NH-(C₁-C₆ alkyl), où m = 1,2; et
X est -NH₂ ou -NHC(=NH)(NH₂), à condition que X ne soit pas NH₂, lorsque r = 4.

7. Procédé de la revendication 1, dans lequel le composé préparé est choisi parmi le composé de formule (II): 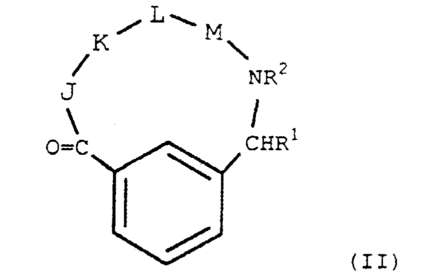 dans lequel R¹ et R² sont H; J est D-Val; K est NMeArg; L est Gly; et M est Asp;
le composé de formule (Il) dans lequel R¹ et R² sont H; J est l'acide D-2-aminobutyrique; K est NMeArg; L est Gly; et M est Asp;
le composé de formule (Il) dans lequel R¹ et R² sont H; J est D-Leu; K est NMeArg; L est Gly: et M est Asp;
le composé de formule (Il) dans lequel R¹ et R² sont H; J est D-Ala; K est NMeArg; L est Gly; M est Asp;
le composé de formule (Il) dans lequel R¹ et R² sont H; J est Gly; K est NMeArg; L est Gly: et M est Asp;
le composé de formule (Il) dans lequel R¹ et R² sont H; J est D-Pro; K est NMeArg; L est Gly; et M est Asp;
le composé de formule (Il) dans lequel R¹ et R² sont H; J est D-Lys; K est NMeArg; L est Gly; et M est Asp;
le composé de formule (Il) dans lequel R¹ et R² sont H; J est β-Ala; K est NMeArg; L est Gly; et M est Asp;
le composé de formule (Il) dans lequel R¹ et R² sont H; J est D-Val; K est NMeArg; L est β-Ala; et M est Asp;
le composé de formule (Il) dans lequel R¹ et R² sont H; J est Pro; K est NMeArg; L est Gly; et M est Asp;
le composé de formule (Il) dans lequel R¹ et R² sont H; J est NMeGly: K est NMeArg; L est Gly; et M est Asp;
le composé de formule (II) dans lequel R¹ est un groupe méthyle (isomère 1); R² est H; J est D-Val; K est NMeArg; L est Gly; et M est Asp;
le composé de formule (II) dans lequel R¹ est un groupe méthyle (isomère 2); R² est H; J est D-Val; K est NMeArg; L est Gly; et M est Asp;
le composé de formule (II) dans lequel R¹ est un groupe phényle (isomère 1); R² est H; J est D-Val; K est NMeArg; L est Gly; et M est Asp;
le composé de formule (II) dans lequel R¹ est un groupe phényle (isomère 2); R² est H; J est D-Val; K est NMeArg; L est Gly; et M est Asp;
le composé de formule (II) dans lequel R¹ et R² sont H; J est D-Val; K est D-NMeArg; L est Gly; et M est Asp;
le composé de formule (II) dans lequel R¹ et R² sont H; J est D-Nle; K est NMeArg; L est Gly; et M est Asp;
le composé de formule (II) dans lequel R¹ et R² sont H; J est D-Phg; K est NMeArg; L est Gly; et M est Asp;
le composé de formule (II) dans lequel R¹ et R² sont H; J est D-Ile; K est NMeArg; L est Gly; et M est Asp;
le composé de formule (II) dans lequel R¹ et R² sont H; J est D-Phe: K est NMeArg; L est Gly; et M est Asp;
le composé de formule (II) dans lequel R¹ et R² sont H; J est NMeGly; K est NMeArg: L est Gly: et M est Asp;
le composé de formule (II) dans lequel R¹ et R² sont H; J est D-Nle; K est NMeArg: L est Gly: et M est Asp:
le composé de formule (II) dans lequel R¹ et R² sont H; J est D-Tyr; K est NMeArg; L est Gly; et M est Asp;
le composé de formule (II) dans lequel R¹ et R² sont H; J est D-Val; K est NMeAmf: L est Gly; et M est Asp;
le composé de formule (II) dans lequel R¹ et R² sont H; J est D-Val; K est NMeArg; L est Ala; et M est Asp;
le composé de formule (II) dans lequel R¹ et R² sont H; J est D-Val; K est NMeArg; L est Gly; et M est -MeAsp;
le composé de formule (II) dans lequel R¹ et R² sont H: J est D-Val; K est NMeArg; L est Gly; et M est β-MeAsp;
le composé de formule (II) dans lequel R¹ et R² sont H; J est D-Val; K est NMeArg; L est Gly; et M est NMeAsp:
le composé de formule (II) dans lequel R¹ et R² sont H; J est D-Val; K est NMeArg; L est Gly; et M est D-Asp;
le composé de formule (II) dans lequel R¹ est H; R² est CH₃; J est D-Val; K est NMeArg; L est Gly; et M est Asp:
le composé de formule (II) dans lequel R¹ et R² sont H; J est D-Abu; K est di-NMeOrn: L est Gly; et M est Asp;
le composé de formule (III): 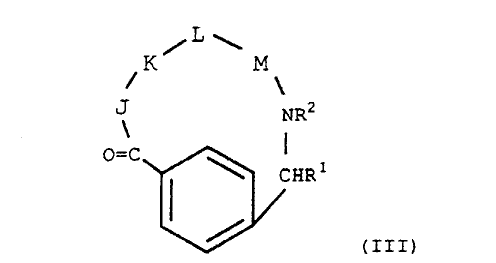 dans lequel R¹ et R² sont H; J est D-Val; K est NMeArg: L est Gly; et M est Asp;
le composé de formule (V): 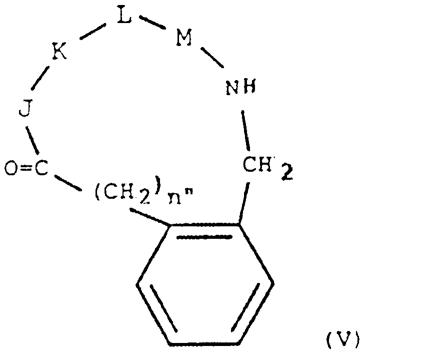 dans lequel n"=1; J est D-Val; K est NMeArg; L est Gly; et M est Asp;
le composé de formule (V) dans lequel n"=O; J est D-Val; K est NMeArg; L est Gly; et M est Asp;
le composé de formule (VI): 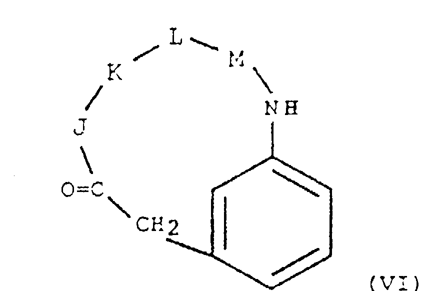 dans lequel J est D-Val; K est NMeArg; L est Gly; et M est Asp;
le composé de formule (VII): 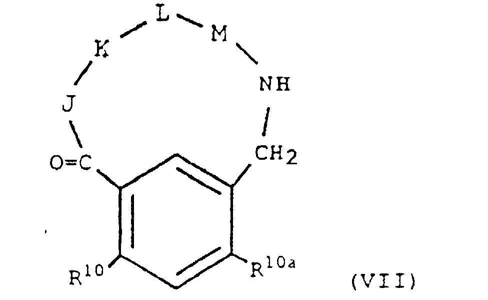 dans lequel R¹⁰ est H; R^{10a} est Cl; J est D-Val; K est NMeArg; L est Gly; et M est Asp;
le composé de formule (VII) dans lequel R¹⁰ est H; R^{10a} est I; J est D-Val; K est NMeArg; L est Gly; et M est Asp;
le composé de formule (VII) dans lequel R¹⁰ est H; R^{10a} est I; J est D-Abu; K est NMeArg; L est Gly; et M est Asp;
le composé de formule (VII) dans lequel R¹⁰ est H; R^{10a} est OMe; J est D-Val; K est NMeArg; L est Gly; et M est Asp;
le composé de formule (VII) dans lequel R¹⁰ est H; R^{10a} est Me; J est D-Val; K est NMeArg; L est Gly; et M est Asp;
le composé de formule (VII) dans lequel R^{10a} est H; R¹⁰ est Cl; J est D-Val; K est NMeArg; L est Gly; et M est Asp;
le composé de formule (VII) dans lequel R^{10a} est H; R¹⁰ est MeO; J est D-Val; K est NMeArg; L est Gly; et M est Asp;
le composé de formule (VII) dans lequel R^{10a} est H; R¹⁰ est Me; J est D-Val; K est NMeArg; L est Gly; et M est Asp;
le composé de formule (VIII): 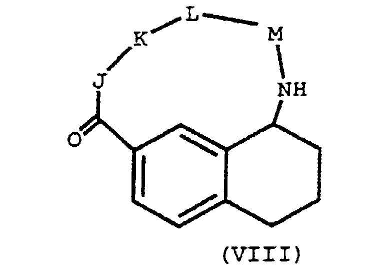 dans lequel J est D-Val; K est NMeArg; L est Gly; et M est Asp;
le composé de formule (IX): 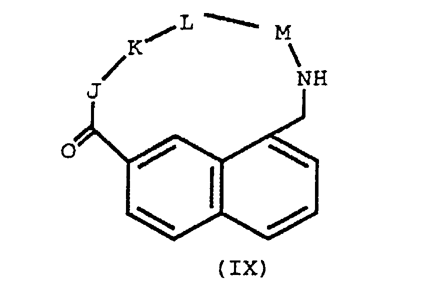 dans lequel J est D-Val; K est NMeArg; L est Gly; et M est Asp.

8. Procédé de la revendication 7 dans lequel le composé préparé est choisi parmi:
le composé de formule (Il) dans lequel R¹ et R² sont H; J est D-Val; K est NMeArg; L est Gly; et M est Asp;
le composé de formule (Il) dans lequel R¹ et R² sont H; J est un acide D-2-aminobutyrique; K est NMeArg; L est Gly: et M est Asp;
le composé de formule (Il) dans lequel R¹ et R² sont H: J est D-Leu; K est NMeArg; L est Gly; et M est Asp;
le composé de formule (Il) dans lequel R¹ et R² sont H; J est D-Ala: K est NMeArg; L est Gly; et M est Asp;
le composé de formule (Il) dans lequel R¹ et R² sont H; J est Gly; K est NMeArg; L est Gly; et M est Asp:
le composé de formule (Il) dans lequel R¹ et R² sont H; J est D-Pro; K est NMeArg; L est Gly; et M est Asp;
le composé de formule (II) dans lequel R¹ et R² sont H; J est D-Lys; K est NMeArg; L est Gly; et M est Asp;
le composé de formule (II) dans lequel R¹ et R² sont H; J est β-Ala; K est NMeArg; L est Gly; et M est Asp;
le composé de formule (II) dans lequel R¹ et R² sont H; J est NMeGly; K est NMeArg; L est Gly, et M est Asp;
le composé de formule (II) dans lequel R¹ est un groupe méthyle (isomère 1); R² est H; J est D-Val; K est NMeArg; L est Gly; et M est Asp:
le composé de formule (II) dans lequel R¹ est un groupe méthyle (isomère 2): R² est H; J est D-Val; K est NMeArg; L est Gly; et M est Asp:
le composé de formule (II) dans lequel R¹ est un groupe phényle (isomère 1); R² est H; J est D-Val; K est NMeArg; L est Gly; et M est Asp;
le composé de formule (II) dans lequel R¹ et R² sont H; J est D-Val; K est D-NMeArg; L est Gly; et M est Asp;
le composé de formule (II) dans lequel R¹ et R² sont H; J est D-Nle: K est NMeArg; L est Gly; et M est Asp;
le composé de formule (II) dans lequel R¹ et R² sont H: J est D-Phg: K est NMeArg; L est Gly: et M est Asp;
le composé de formule (II) dans lequel R¹ et R² sont H; J est D-Phe; K est NMeArg; L est Gly; et M est Asp;
le composé de formule (II) dans lequel R¹ et R² sont H; J est D-Ile; K est NMeArg; L est Gly; et M est Asp;
le composé de formule (III) dans lequel R¹ et R² sont H; J est D-Val; K est NMeArg; L est Gly; et M est Asp;
le composé de formule (V) dans lequel n" = 1; J est D-Val; K est NMeArg; L est Gly et M est Asp;
le composé de formule (V) dans lequel n" = 0; J est D-Val; K est NMeArg; L est Gly et M est Asp;
le composé de formule (VI) dans lequel J est D-Val; K est NMeArg; L est Gly; et M est Asp;
le composé de formule (VII) dans lequel R¹⁰ est H; R^{10a} est Cl; J est D-Val; K est NMeArg; L est Gly; et M est Asp;
le composé de formule (VII) dans lequel R¹⁰ est H; R^{10a} est I; J est D-Val; K est NMeArg; L est Gly; et M est Asp;
le composé de formule (VII) dans lequel R¹⁰ est H; R^{10a} est I; J est D-Abu; K est NMeArg; L est Gly; et M est Asp;
le composé de formule (VII) dans lequel R¹⁰ est H; R^{10a} est Me; J est D-Val; K est NMeArg; L est Gly; et M est Asp;
le composé de formule (VII) dans lequel R^{10a} est H; R¹⁰ est Cl; J est D-Val; K est NMeArg: L est Gly; et M est Asp;
le composé de formule (VII) dans lequel R^{10a} est H; R¹⁰ est MeO; J est D-Val; K est NMeArg; L est Gly; et M est Asp;
le composé de formule (VII) dans lequel R^{10a} est H; R¹⁰ est Me; J est D-Val; K est NMeArg; L est Gly; et M est Asp;
le composé de formule (Il) dans lequel R¹ et R² sont H; J est D-Tyr; K est NMeArg; L est Gly; et M est Asp;
le composé de formule (Il) dans lequel R¹ et R² sont H; J est D-Val; K est NMeAmf; L est Gly; et M est Asp;
le composé de formule (Il) dans lequel R¹ et R² sont H; J est D-Val; K est NMeArg; L est Gly; et M est bMeAsp;
le composé de formule (Il) dans lequel R¹ est H; R² est CH₃; J est D-Val; K est NMeArg; L est Gly; et M est Asp;
le composé de formule (III) dans lequel R¹ et R² sont H; J est D-Val; K est NMeArg; L est Gly; et M est Asp;
le composé de formule (VIII) dans lequel J est D-Val; K est NMeArg; L est Gly; et M est Asp.

9. Procédé de la revendication 8 dans lequel le composé préparé est choisi parmi :
le composé de formule (Il) dans lequel R¹ et R² sont H; J est D-Val; K est NMeArg; L est Gly; et M est Asp;
le composé de formule (Il) dans lequel R¹ et R² sont H; J est l'acide D-2-aminobutyrique; K est NMeArg; L est Gly; et M est Asp;
le composé de formule (Il) dans lequel R¹ et R² sont H; J est D-Leu; K est NMeArg; L est Gly; et M est Asp;
le composé de formule (Il) dans lequel R¹ et R² sont H; J est D-Ala; K est NMeArg; L est Gly; et M est Asp;
le composé de formule (Il) dans lequel R¹ et R² sont H; J est D-Pro; K est NMeArg; L est Gly; et M est Asp;
le composé de formule (Il) dans lequel R¹ et R² sont H; J est D-Lys; K est NMeArg; L est Gly; et M est Asp;
le composé de formule (Il) dans lequel R¹ et R² sont H; J est D-Nle; K est NMeArg; L est Gly; et M est Asp;
le composé de formule (Il) dans lequel R¹ et R² sont H; J est D-Phe; K est NMeArg; L est Gly; et M est Asp;
le composé de formule (Il) dans lequel R¹ et R² sont H; J est D-Ile; K est NMeArg; L est Gly; et M est Asp;
le composé de formule (V) dans lequel n" = 1; J est D-Val: K est NMeArg; L est Gly: et M est Asp;
le composé de formule (VI) dans lequel J est D-Val; K est NMeArg; L est Gly; et M est Asp;
le composé de formule (VII) dans lequel R¹⁰ est H; R^{10a} est Cl; J est D-Val; K est NMeArg; L est Gly; et M est Asp;
le composé de formule (VII) dans lequel R¹⁰ est H; R^{10a} est I; J est D-Val; K est NMeArg; L est Gly; et M est Asp;
le composé de formule (VII) dans lequel R¹⁰ est H; R^{10a} est Me; J est D-Val; K est NMeArg; L est Gly; et M est Asp;
le composé de formule (VII) dans lequel R^{10a} est H; R¹⁰ est Cl; J est D-Val; K est NMeArg; L est Gly; et M est Asp;
le composé de formule (VII) dans lequel R^{10a} est H; R¹⁰ est MeO; J est D-Val; K est NMeArg; L est Gly; et M est Asp;
le composé de formule (VII) dans lequel R^{10a} est H; R¹⁰ est Me; J est D-Val; K est NMeArg; L est Gly; et M est Asp:
le composé de formule (II) dans lequel R¹ et R² sont H; J est D-Val; K est NMeArg; L est Gly; et M est bMeAsp.

10. Procédé pour la préparation d'un composé de formule (I): 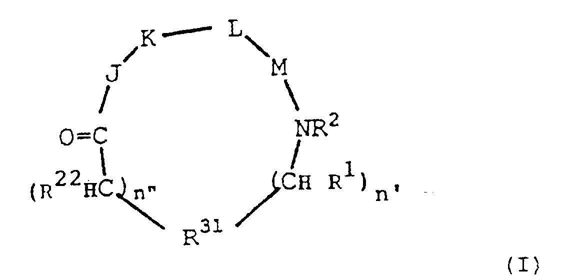 ou forme d'un sel ou d'un promédicament pharmaceutiquement acceptable de celui-ci dans lequel:
R³¹ est choisi parmi : 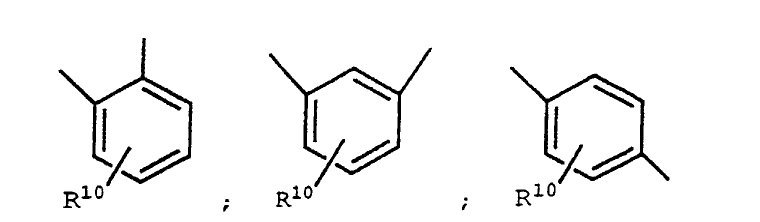 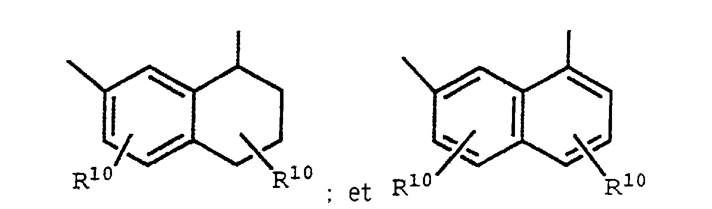 dans lequel R³¹ peut être indépendamment substitué avec 0-2 R¹⁰;
n" est 0 ou 1;
n' est 0 ou 1;
R¹ et R²² sont indépendamment choisis parmi H, un groupe alkyle C₁-C₄, phényle, benzyle, phényl-(C₂-C₄)alkyle, alkoxyle C₁-C₄;
R² est H ou un groupe alkyle C₁-C₈;
R¹⁰ est H, un groupe alkyle C₁-C₈, phényle, halogène, ou alkoxyle C₁-C₄;
J est un acide aminé β-Ala ou un _{L}-isomère ou un _{D}-isomère de structure -N(R³)C(R⁴)(R⁵)C(=O)-, dans lequel:
R³ est H ou CH₃
R⁴ est H ou un groupe alkyle C₁-C₃;
R⁵ est H, un groupe alkyle C₁-C₈, cycloalkyle C₃-C₆, cycloalkylméthyle C₃-C₆, cycloalkyléthyle C₁-C₆, phényle, phénylméthyle, CH₂OH, CH₂SH, CH₂OCH₃, CH₂SCH₃, CH₂CH₂SCH₃, (CH₂)ₛNH₂, (CH₂)ₛNHC(=NH)(NH₂), (CH₂)_{S}NHR¹⁶, où s = 3-5;
R³ et R⁵ peuvent également être pris ensemble pour former -(CH₂)ₜ-(t = 2-4) ou -CH₂SC(CH₃)2-; ou
R⁴ et R⁵ peuvent également être pris ensemble pour former -(CH₂)ᵤ-, où u = 2-5;
R¹⁶ est un groupe protecteur de l'amine;
K est un acide aminé _{L}-isomère de structure -N(R⁶)CH(R⁷)C(=O)-, dans lequel:
R⁶ est un groupe alkyle C₁-C₈;
R⁷ est -(CH₂)ₚNHC(=NH)(NH₂), où p = 3-5; 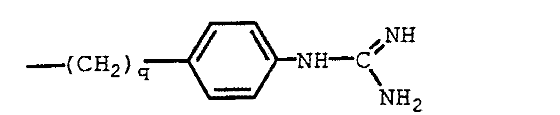 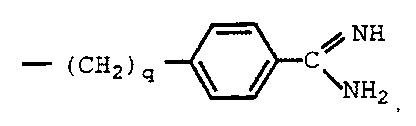 où q = 0, 1;
-(CH₂)ᵣX, où r = 4-6; 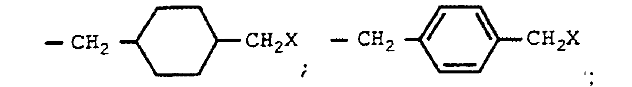 (CH₂)ₘS(CH₂)₂X, où m = 1,2;
-(C₃-C₇ alkyl)-N-(C₁-C₆ alkyl) 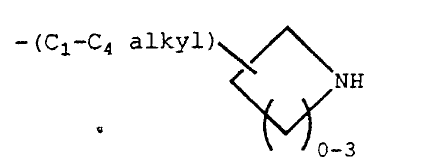 -(CH₂)ₘ-O-(C₁-C₄ alkyl)-NH-(C₁-C₆ alkyl), où m = 1, 2;
-(CH₂)ₘ-S-(C₁-C₄ alkyl)-NH-(C₁-C₆ alkyl), où m = 1, 2; et
X est -NH₂ ou -NHC(=NH)(NH₂);
L est -Y(CH₂)ᵥC(=O)-, dans lequel:
Y est NH, O, ou S; et v = 1,2;
M est un acide aminé _{L}-isomère de structure -NH-CH(CH₂SO₃H)C(=O)-;
lequel procédé comprend la cyclisation d'un peptide précurseur linéaire.

11. Procédé pour la préparation d'un médicament pour le traitement de désordres thromboemboliques comprenant l'utilisation d'un composé préparé suivant l'une quelconque des revendications 1-10.

12. Procédé pour la préparation d'une composition pharmaceutique comprenant un excipient pharmaceutiquement acceptable et une quantité thérapeutiquement efficace d'un composé préparé suivant l'une quelconque des revendications 1-10, lequel procédé comprend l'addition dudit composé à l'excipient.
